# EUROPEAN PATENT APPLICATION

(11) **EP 4 011 904 A1**
(43) Date of publication of application: **15.06.2022**
(21) Application number: 20213678.4
(22) Date of filing: 14.12.2020
(51) Int. Cl.: C07K 7/08, C07K 7/64, C07K 7/54

(54) **MASP INHIBITORY COMPOUNDS AND USES THEREOF**

(71) Applicant: Bayer Aktiengesellschaft, 51373 Leverkusen (DE); Bayer Pharma Aktiengesellschaft, 13353 Berlin (DE)
(72) Inventor: The designation of the inventor has not yet been filed
(74) Representative: BIP Patents

(57) **Abstract**

The present invention relates to novel Mannose-binding lectin (MBL)-associated serine protease (MASP) inhibitory bicyclic compounds, as well as analogues and derivatives thereof, to processes for the preparation thereof, to the use thereof alone or in combinations for treatment and/or prevention of diseases and to the use thereof for production of medicaments for treatment and/or prevention of diseases, especially for treatment and/or prevention of renal and cardiovascular disorders and of ischemia reperfusion injuries.

SEQUENCE LISTING

<110>  Bayer AG
       Bayer Pharma AG
       Bayer (China) Limited

<120>  MASP inhibitory compounds and uses thereof

<130>  BHC201057WO

<160>  12

<170>  PatentIn version 3.5

<210>  1
<211>  14
<212>  PRT
<213>  Artificial Sequence

<220>
<223>  Bicyclic SFTI-1



<220>
<221>  CHAIN
<222>  (1)..(14)
<223>  Peptide bond between G in position 1 and D in position 14 forming
       a second ring

<220>
<221>  DISULFID
<222>  (3)..(11)
<223>  disulfide bond

<400>  1

Gly Arg Cys Thr Lys Ser Ile Pro Pro Ile Cys Phe Pro Asp
1               5                   10


<210>  2
<211>  430
<212>  PRT
<213>  Artificial Sequence

<220>
<223>  MASP-1_Human_Sequence fragment_C-terminal His Tag and N-terminal
       Ig-kappa secretion signal

<400>  2

Met Glu Thr Asp Thr Leu Leu Leu Trp Val Leu Leu Leu Trp Val Pro
1               5                   10                  15


Gly Ser Thr Gly Asp Ala Gly Asn Glu Cys Pro Glu Leu Gln Pro Pro
            20                  25                  30


Val His Gly Lys Ile Glu Pro Ser Gln Ala Lys Tyr Phe Phe Lys Asp
        35                  40                  45

Gln Val Leu Val Ser Cys Asp Thr Gly Tyr Lys Val Leu Lys Asp Asn
    50                  55                  60

Val Glu Met Asp Thr Phe Gln Ile Glu Cys Leu Lys Asp Gly Thr Trp
65                  70                  75                  80

Ser Asn Lys Ile Pro Thr Cys Lys Ile Val Asp Cys Arg Ala Pro Gly
                85                  90                  95

Glu Leu Glu His Gly Leu Ile Thr Phe Ser Thr Arg Asn Asn Leu Thr
            100                 105                 110

Thr Tyr Lys Ser Glu Ile Lys Tyr Ser Cys Gln Glu Pro Tyr Tyr Lys
        115                 120                 125

Met Leu Asn Asn Asn Thr Gly Ile Tyr Thr Cys Ser Ala Gln Gly Val
    130                 135                 140

Trp Met Asn Lys Val Leu Gly Arg Ser Leu Pro Thr Cys Leu Pro Val
145                 150                 155                 160

Cys Gly Leu Pro Lys Phe Ser Arg Lys Leu Met Ala Arg Ile Phe Asn
                165                 170                 175

Gly Arg Pro Ala Gln Lys Gly Thr Thr Pro Trp Ile Ala Met Leu Ser
            180                 185                 190

His Leu Asn Gly Gln Pro Phe Cys Gly Gly Ser Leu Leu Gly Ser Ser
        195                 200                 205

Trp Ile Val Thr Ala Ala His Cys Leu His Gln Ser Leu Asp Pro Glu
    210                 215                 220

Asp Pro Thr Leu Arg Asp Ser Asp Leu Leu Ser Pro Ser Asp Phe Lys
225                 230                 235                 240

Ile Ile Leu Gly Lys His Trp Arg Leu Arg Ser Asp Glu Asn Glu Gln
                245                 250                 255

His Leu Gly Val Lys His Thr Thr Leu His Pro Gln Tyr Asp Pro Asn
            260                 265                 270

Thr Phe Glu Asn Asp Val Ala Leu Val Glu Leu Leu Glu Ser Pro Val
        275                 280                 285

Leu Asn Ala Phe Val Met Pro Ile Cys Leu Pro Glu Gly Pro Gln Gln
    290                 295                 300

Glu Gly Ala Met Val Ile Val Ser Gly Trp Gly Lys Gln Phe Leu Gln
305                 310                 315                 320

Arg Phe Pro Glu Thr Leu Met Glu Ile Glu Ile Pro Ile Val Asp His
                325                 330                 335

Ser Thr Cys Gln Lys Ala Tyr Ala Pro Leu Lys Lys Lys Val Thr Arg
            340                 345                 350

Asp Met Ile Cys Ala Gly Glu Lys Glu Gly Gly Lys Asp Ala Cys Ala
        355                 360                 365

Gly Asp Ser Gly Gly Pro Met Val Thr Leu Asn Arg Glu Arg Gly Gln
    370                 375                 380

Trp Tyr Leu Val Gly Thr Val Ser Trp Gly Asp Asp Cys Gly Lys Lys
385                 390                 395                 400

Asp Arg Tyr Gly Val Tyr Ser Tyr Ile His His Asn Lys Asp Trp Ile
                405                 410                 415

Gln Arg Val Thr Gly Val Arg Asn His His His His His His
            420                 425                 430

<210>  3
<211>  417
<212>  PRT
<213>  Artificial Sequence

<220>
<223>  MASP-2_Human_Sequence fragment_C-terminal His Tag and N-terminal
       Ig-kappa secretion signal

<400>  3

Met Glu Thr Asp Thr Leu Leu Leu Trp Val Leu Leu Leu Trp Val Pro
1               5                   10                  15

Gly Ser Thr Gly Asp Ala Gln Pro Cys Pro Tyr Pro Met Ala Pro Pro
            20                  25                  30

Asn Gly His Val Ser Pro Val Gln Ala Lys Tyr Ile Leu Lys Asp Ser
        35                  40                  45

Phe Ser Ile Phe Cys Glu Thr Gly Tyr Glu Leu Leu Gln Gly His Leu
    50                  55                  60

Pro Leu Lys Ser Phe Thr Ala Val Cys Gln Lys Asp Gly Ser Trp Asp
65                  70                  75                  80

Arg Pro Met Pro Ala Cys Ser Ile Val Asp Cys Gly Pro Pro Asp Asp
                85                  90                  95

Leu Pro Ser Gly Arg Val Glu Tyr Ile Thr Gly Pro Gly Val Thr Thr
            100                 105                 110

Tyr Lys Ala Val Ile Gln Tyr Ser Cys Glu Glu Thr Phe Tyr Thr Met
        115                 120                 125

Lys Val Asn Asp Gly Lys Tyr Val Cys Glu Ala Asp Gly Phe Trp Thr
    130                 135                 140

Ser Ser Lys Gly Glu Lys Ser Leu Pro Val Cys Glu Pro Val Cys Gly
145                 150                 155                 160

Leu Ser Ala Arg Thr Thr Gly Gly Arg Ile Tyr Gly Gly Gln Lys Ala
                165                 170                 175

Lys Pro Gly Asp Phe Pro Trp Gln Val Leu Ile Leu Gly Gly Thr Thr
            180                 185                 190

Ala Ala Gly Ala Leu Leu Tyr Asp Asn Trp Val Leu Thr Ala Ala His
        195                 200                 205

Ala Val Tyr Glu Gln Lys His Asp Ala Ser Ala Leu Asp Ile Arg Met
    210                 215                 220

Gly Thr Leu Lys Arg Leu Ser Pro His Tyr Thr Gln Ala Trp Ser Glu
225                 230                 235                 240

Ala Val Phe Ile His Glu Gly Tyr Thr His Asp Ala Gly Phe Asp Asn
                245                 250                 255

Asp Ile Ala Leu Ile Lys Leu Asn Asn Lys Val Val Ile Asn Ser Asn
            260                 265                 270

Ile Thr Pro Ile Cys Leu Pro Arg Lys Glu Ala Glu Ser Phe Met Arg
        275                 280                 285

Thr Asp Asp Ile Gly Thr Ala Ser Gly Trp Gly Leu Thr Gln Arg Gly
    290                 295                 300

Phe Leu Ala Arg Asn Leu Met Tyr Val Asp Ile Pro Ile Val Asp His
305                 310                 315                 320

Gln Lys Cys Thr Ala Ala Tyr Glu Lys Pro Pro Tyr Pro Arg Gly Ser
                325                 330                 335

Val Thr Ala Asn Met Leu Cys Ala Gly Leu Glu Ser Gly Gly Lys Asp
            340                 345                 350

Ser Cys Arg Gly Asp Ser Gly Gly Ala Leu Val Phe Leu Asp Ser Glu
        355                 360                 365

Thr Glu Arg Trp Phe Val Gly Gly Ile Val Ser Trp Gly Ser Met Asn
    370                 375                 380

Cys Gly Glu Ala Gly Gln Tyr Gly Val Tyr Thr Lys Val Ile Asn Tyr
385                 390                 395                 400

Ile Pro Trp Ile Glu Asn Ile Ile Ser Asp Phe His His His His His
                405                 410                 415

His

<210>  4
<211>  430
<212>  PRT
<213>  Artificial Sequence

<220>
<223>  MASP-1_Rat_Sequence fragment_C-terminal His Tag and N-terminal
       Ig-kappa secretion signal

<400>  4

Met Glu Thr Asp Thr Leu Leu Leu Trp Val Leu Leu Leu Trp Val Pro
1               5                   10                  15

Gly Ser Thr Gly Asp Ala Gly Asn Glu Cys Pro Lys Leu Gln Pro Pro
            20                  25                  30

Val Tyr Gly Lys Ile Glu Pro Ser Gln Ala Val Tyr Ser Phe Lys Asp
        35                  40                  45

Gln Val Leu Ile Ser Cys Asp Thr Gly Tyr Lys Val Leu Lys Asp Asn
    50                  55                  60

Glu Val Met Asp Thr Phe Gln Ile Glu Cys Leu Lys Asp Gly Ala Trp
65                  70                  75                  80

Ser Asn Lys Ile Pro Thr Cys Lys Ile Val Asp Cys Gly Val Pro Ala
                85                  90                  95

Val Leu Lys His Gly Leu Val Thr Phe Ser Thr Arg Asn Asn Leu Thr
            100                 105                 110

Thr Tyr Lys Ser Glu Ile Arg Tyr Ser Cys Gln Gln Pro Tyr Tyr Lys
        115                 120                 125

Met Leu His Asn Thr Thr Gly Val Tyr Thr Cys Ser Ala His Gly Thr
    130                 135                 140

Trp Thr Asn Glu Val Leu Lys Arg Ser Leu Pro Thr Cys Leu Pro Val
145                 150                 155                 160

Cys Gly Leu Pro Lys Phe Ser Arg Lys His Ile Ser Arg Ile Phe Asn
                165                 170                 175

Gly Arg Pro Ala Gln Lys Gly Thr Thr Pro Trp Ile Ala Met Leu Ser
            180                 185                 190

Gln Leu Asn Gly Gln Pro Phe Cys Gly Gly Ser Leu Leu Gly Ser Asn
        195                 200                 205

Trp Val Leu Thr Ala Ala His Cys Leu His His Pro Leu Asp Pro Glu
    210                 215                 220

Glu Pro Ile Leu His Asn Ser His Leu Leu Ser Pro Ser Asp Phe Lys
225                 230                 235                 240

Ile Ile Met Gly Lys His Trp Arg Arg Arg Ser Asp Glu Asp Glu Gln
                245                 250                 255

His Leu His Val Lys His Ile Met Leu His Pro Leu Tyr Asn Pro Ser
            260                 265                 270

Thr Phe Glu Asn Asp Leu Gly Leu Val Glu Leu Ser Glu Ser Pro Arg
        275                 280                 285

Leu Asn Asp Phe Val Met Pro Val Cys Leu Pro Glu His Pro Ser Thr
    290                 295                 300

Glu Gly Thr Met Val Ile Val Ser Gly Trp Gly Lys Gln Phe Leu Gln
305                 310                 315                 320

Arg Leu Pro Glu Asn Leu Met Glu Ile Glu Ile Pro Ile Val Asn Tyr
                325                 330                 335

His Thr Cys Gln Glu Ala Tyr Thr Pro Leu Gly Lys Lys Val Thr Gln
            340                 345                 350

Asp Met Ile Cys Ala Gly Glu Lys Glu Gly Gly Lys Asp Ala Cys Ala

355                 360                 365

Gly Asp Ser Gly Gly Pro Met Val Thr Lys Asp Ala Glu Arg Asp Gln
    370                 375                 380

Trp Tyr Leu Val Gly Val Val Ser Trp Gly Glu Asp Cys Gly Lys Lys
385                 390                 395                 400

Asp Arg Tyr Gly Val Tyr Ser Tyr Ile Tyr Pro Asn Lys Asp Trp Ile
                405                 410                 415

Gln Arg Val Thr Gly Val Arg Asn His His His His His His
            420                 425                 430

<210>  5
<211>  417
<212>  PRT
<213>  Artificial Sequence

<220>
<223>  MASP-2_Rat_Sequence fragment_C-terminal His Tag and N-terminal
       Ig-kappa secretion signal

<400>  5

Met Glu Thr Asp Thr Leu Leu Leu Trp Val Leu Leu Leu Trp Val Pro
1               5                   10                  15

Gly Ser Thr Gly Asp Thr Ala Gln Pro Cys Pro Asp Pro Thr Ala Pro
            20                  25                  30

Pro Asn Gly His Ile Ser Pro Val Gln Ala Thr Tyr Val Leu Lys Asp
        35                  40                  45

Ser Phe Ser Val Phe Cys Lys Thr Gly Phe Glu Leu Leu Gln Gly Ser
    50                  55                  60

Val Pro Leu Lys Ser Phe Thr Ala Val Cys Gln Lys Asp Gly Ser Trp
65                  70                  75                  80

Asp Arg Pro Ile Pro Glu Cys Ser Ile Ile Asp Cys Gly Pro Pro Asp
                85                  90                  95

Asp Leu Pro Asn Gly His Val Asp Tyr Ile Thr Gly Pro Glu Val Thr
            100                 105                 110

Thr Tyr Lys Ala Val Ile Gln Tyr Ser Cys Glu Glu Thr Phe Tyr Thr
        115                 120                 125

Met Ser Ser Asn Gly Lys Tyr Val Cys Glu Ala Asp Gly Phe Trp Thr

    130                 135                 140

Ser Ser Lys Gly Glu Lys Ser Leu Pro Val Cys Lys Pro Val Cys Gly
145                 150                 155                 160

Leu Ser Thr His Thr Ser Gly Gly Arg Ile Ile Gly Gly Gln Pro Ala
                165                 170                 175

Lys Pro Gly Asp Phe Pro Trp Gln Val Leu Leu Leu Gly Glu Thr Thr
            180                 185                 190

Ala Ala Gly Ala Leu Ile His Asp Asp Trp Val Leu Thr Ala Ala His
        195                 200                 205

Ala Val Tyr Gly Lys Thr Glu Ala Met Ser Ser Leu Asp Ile Arg Met
    210                 215                 220

Gly Ile Leu Lys Arg Leu Ser Leu Ile Tyr Thr Gln Ala Trp Pro Glu
225                 230                 235                 240

Ala Val Phe Ile His Glu Gly Tyr Thr His Gly Ala Gly Phe Asp Asn
                245                 250                 255

Asp Ile Ala Leu Ile Lys Leu Lys Asn Lys Val Thr Ile Asn Arg Asn
            260                 265                 270

Ile Met Pro Ile Cys Leu Pro Arg Lys Glu Ala Ala Ser Leu Met Lys
        275                 280                 285

Thr Asp Phe Val Gly Thr Val Ala Gly Trp Gly Leu Thr Gln Lys Gly
    290                 295                 300

Phe Leu Ala Arg Asn Leu Met Phe Val Asp Ile Pro Ile Val Asp His
305                 310                 315                 320

Gln Lys Cys Ala Thr Ala Tyr Thr Lys Gln Pro Tyr Pro Gly Ala Lys
                325                 330                 335

Val Thr Val Asn Met Leu Cys Ala Gly Leu Asp Arg Gly Gly Lys Asp
            340                 345                 350

Ser Cys Arg Gly Asp Ser Gly Gly Ala Leu Val Phe Leu Asp Asn Glu
        355                 360                 365

Thr Gln Arg Trp Phe Val Gly Gly Ile Val Ser Trp Gly Ser Ile Asn
    370                 375                 380

Cys Gly Gly Ser Glu Gln Tyr Gly Val Tyr Thr Lys Val Thr Asn Tyr
385                 390                 395                 400

Ile Pro Trp Ile Glu Asn Ile Ile Asn Asn Phe His His His His His
                405                 410                 415

His

<210>  6
<211>  11
<212>  PRT
<213>  Artificial Sequence

<220>
<223>  Fluorescence Resonance Energy Transfer substrate

<220>
<221>  Xaa
<222>  (1)..(1)
<223>  Xaa is 2-aminobenzoyl

<220>
<221>  MOD_RES
<222>  (10)..(10)
<223>  AMIDATION

<220>
<221>  Xaa
<222>  (11)..(11)
<223>  Xaa is a non peptidic capping group (2,4-dinitrophenyl; bound to
       lysine amino group on position 11)

<400>  6

Xaa Met Tyr Gly Gly Ala Arg Arg Leu Lys Xaa
1               5                   10

<210>  7
<211>  13
<212>  PRT
<213>  Artificial Sequence

<220>
<223>  Fluorescence resonance energy transfer substrate

<220>
<221>  Xaa
<222>  (1)..(1)
<223>  Xaa is 4-((4-(dimethylamino)phenyl)azo)benzoic acid

<220>
<221>  MOD_RES
<222>  (12)..(12)
<223>  AMIDATION

<220>

<221>  Xaa
<222>  (13)..(13)
<223>  Xaa is 5-[(2-Aminoethyl) amino]naphthalene-1-sulfonyl (bound to
       Glutamic acid caboxylic acid group on position 13)

<400>  7

Xaa Lys Ile Ser Pro Gln Gly Tyr Gly Arg Arg Glu Xaa
1               5                   10


<210>  8
<211>  11
<212>  PRT
<213>  Artificial Sequence

<220>
<223>  Fluorescence resonance energy transfer substrate


<220>
<221>  Xaa
<222>  (1)..(1)
<223>  Xaa is 4-((4-(dimethylamino)phenyl)azo)benzoic acid

<220>
<221>  MOD_RES
<222>  (10)..(10)
<223>  AMIDATION

<220>
<221>  Xaa
<222>  (11)..(11)
<223>  Xaa is 5-[(2-Aminoethyl) amino]naphthalene-1-sulfonyl (bound to
       Glutamic acid caboxylic acid group on position 11)

<400>  8

Xaa Met Tyr Gly Gly Ala Arg Arg Leu Glu Xaa
1               5                   10


<210>  9
<211>  11
<212>  PRT
<213>  Artificial Sequence

<220>
<223>  Fluorescence resonance energy transfer substrate


<220>
<221>  Xaa
<222>  (1)..(1)
<223>  Xaa is 2-aminobenzoyl

<220>
<221>  MOD_RES
<222>  (10)..(10)
<223>  AMIDATION

<220>
<221>  Xaa

<222>   (11)..(11)
<223>   Xaa is a non peptidic capping group (2,4-dinitrophenyl; bound to
        lysine amino group on position 11)

<400>   9

Xaa Ile Glu Gly Arg Thr Ser Glu Asp Lys Xaa
1               5                   10


<210>   10
<211>   14
<212>   PRT
<213>   Artificial Sequence

<220>
<223>   Bicyclic SFMI-1


<220>
<221>   CHAIN
<222>   (1)..(14)
<223>   Peptide bond between G in position 1 and D in position 14 forming
        a second ring

<220>
<221>   DISULFID
<222>   (3)..(11)
<223>   disulfide bond

<400>   10

Gly Ile Cys Ser Arg Ser Leu Pro Pro Ile Cys Ile Pro Asp
1               5                   10


<210>   11
<211>   14
<212>   PRT
<213>   Artificial Sequence

<220>
<223>   Bicyclic SFMI-2


<220>
<221>   CHAIN
<222>   (1)..(14)
<223>   Peptide bond between G in position 1 and D in position 14 forming
        a second ring

<220>
<221>   DISULFID
<222>   (3)..(11)
<223>   disulfide bond

<400>   11

Gly Tyr Cys Ser Arg Ser Tyr Pro Pro Val Cys Ile Pro Asp
1               5                   10


<210>   12

<211>  14
<212>  PRT
<213>  Artificial Sequence

<220>
<223>  Reference peptide

<220>
<221>  CHAIN
<222>  (1)..(14)
<223>  Peptide bond between P in position 1 and D in position 14 forming
       a second ring

<220>
<221>  DISULFID
<222>  (3)..(11)
<223>  disulfide bond

<400>  12

Pro Phe Cys Ile Pro Pro Ile Ser Lys Thr Cys Arg Gly Asp
1               5                   10

## Description

The present invention relates to novel Mannose-binding lectin (MBL)-associated serine protease (MASP) inhibitory bicyclic compounds, as well as analogues and derivatives thereof, to processes for the preparation thereof, to the use thereof alone or in combinations for treatment and/or prevention of diseases and to the use thereof for production of medicaments for treatment and/or prevention of diseases, especially for treatment and/or prevention of renal and cardiovascular disorders and of ischemia reperfusion injuries.

The complement system consists of a complex cascading network of proteins, receptors and enzymes of which many are circulating in the blood stream. The complement system is an important constituent of innate immunity and essential for the defense against invading pathogens and clearance of dead and virus infected cells. It forms a bridge between innate and adaptive immune responses. Activation of the complement system is also involved in the pathologies of sepsis and ischemia reperfusion injuries, e.g. after myocardial infarction, ischemic kidney injury or organ transplantation. Three branches of the complement system have been identified: the lectin pathway, the classical and the alternative pathway (Dunkelberger and Song, Complement and its role in innate and adaptive immune responses. Cell Res. 2010; 20(1): 34-50)*.* The lectin pathway is activated by deposition of lectins which are circulating in the blood stream and under normal conditions have a sentinel function against invading pathogens and dead cells by recognizing foreign and altered carbohydrate surface patterns, respectively, and decorating their surfaces. Mannose-binding lectin (MBL), ficolins and collectins are the major representatives of these lectins which are produced in liver, kidney and other organs (Garred et al., A journey through the lectin pathway of complement-MBL and beyond. Immunol Rev. 2016; 274(1): 74-97)*.* Their deposition is followed by further recruitment of zymogens of essentially two closely related serine proteases from the blood stream, mannose-binding lectin-associated serine protease 1 and 2 (MASP-1 and MASP-2) forming a complex in which the zymogens come into close proximity to each other. The current concept is that under in vivo conditions MASP-1 zymogen after recruitment is self-activating and then activates the MASP-2 zymogen by cleavage. Activated MASP-1 furthermore cleaves complement factor C2 into C2a and C2b. Activated MASP-2 also cleaves C2 and complement factor C4 into C4a and C4b which together with C2a forms the C4bC2a complex which serves as complement factor C3 convertase. Constitution of C3 convertase activity and consecutive C3 deposition to target cell surfaces represents the point of convergence of all three complement pathways activating the common downstream cascade that results in generation of inflammatory mediators and target cell lysis. In intact human serum activities of both MASP-enzymes are indispensable for C3 convertase formation (Héja et al, Revised mechanism of complement lectin-pathway activation revealing the role of serine protease MASP-1 as the exclusive activator of MASP-2. Proc Natl Acad Sci USA. 2012; 109(26): 10498-503)*.*

The microvascular system plays a crucial role during inflammatory and ischemic organ disorders. Barrier function, leukocyte trafficking and coagulation control are closely dependent on the integrity of the luminal endothelial cell surface in small blood vessels. The luminal endothelial surface is lined by a dense coat of glycosylation extensions from membrane integrated glycoproteins, proteoglycans, and glycolipids which in their entirety are called glycokalyx. Electron microscopic analyses of samples from animal experiments and human pathologies have shown that in particular the endothelial glycokalyx is rapidly and fundamentally being degraded upon ischemic challenge as well as under inflammatory conditions such as in sepsis. These changes lead to exposure of carbohydrate residues to the blood stream that under normal conditions are not detectable (for review see: Sieve et al., Regulation and function of endothelial glycocalyx layer in vascular diseases. Vascul Pharmacol. 2018; 100: 26-33)*.* Beside other changes of the cell surface in particular the altered carbohydrate pattern is thought to activate the lectin pathway causing deposition of the pattern recognizing lectins, subsequent C3 deposition and initiation of cell lysis. MBL and C3 deposition was shown to occur after ischemia and acute kidney injury across species including man. The lectin pathway activation was of particular relevance for reperfusion damage as targeted deletion of MBL and MASP-2 protected mice from ischemia reperfusion damages in kidney heart and intestine (Møller-Kristensen et al., Mannan-binding lectin recognizes structures on ischaemic reperfused mouse kidneys and is implicated in tissue injury. Scand J Immunol. 2005; 61(5): 426-34*;* Schwaeble et al., Targeting of mannan-binding lectin-associated serine protease-2 confers protection from myocardial and gastrointestinal ischemia/reperfusion injury. Proc Natl Acad Sci USA. 2011; 108(18): 7523-8*.* Moreover, deletion of collectin 11, another MASP activating lectin which is predominantly expressed in the kidney, made mice resistant against ischemic acute kidney injury (Farrar et al., Collectin-11 detects stress-inducedL-fucose pattern to trigger renal epithelial injury. J Clin Invest. 2016; 126(5): 1911-1925*).* Selective peptide inhibitors of MASP1 and MASP2 have been identified from phage display libraries employing natural trypsin inhibitors from sun flower or grass hoppers as a starting point. These peptides have been shown to inhibit the lectin pathway dependent C3 convertase formation in vitro (Kocsis et al., Selective inhibition of the lectin pathway of complement with phage display selected peptides against mannose-binding lectin-associated serine protease (MASP)-1 and -2: significant contribution of MASP-1 to lectin pathway activation. J Immunol. 2010; 185(7): 4169-78*;* Héja et al., Monospecific inhibitors show that both mannan-binding lectin-associated serine protease-1 (MASP-1) and are essential for lectin pathway activation and reveal structural plasticity of MASP-2. JBiol Chem. 2012; 287(24): 20290-300)*.* However, no evidence for pharmaceutical utility and in vivo efficacy of those peptide inhibitors is available, yet. Similarly, antibodies directed against MASP2 which interfere with MASP zymogen interaction have been identified and brought to clinical development for atypical hemolytic uremic syndrome and other inflammatory kidney disorders (*ClinicalTrials.gov Identifier: NCT03205995; NCT02682407; NCT03608033*)*.* However, clinical proof for utility in the prevention or treatment of acute, in particular ischemic organ damage is still missing.

WO 2004/075837 discloses anti-MASP antibodies, functionally equivalent fragments thereof and MASP binding peptides for decreasing the morbidity and mortality caused by tissue damage associated with ischemia-reperfusion injury or TAAA repair by inhibition of the complement system. Small peptides such as the sunflower MASP inhibitor-1 (SFMI-1) and sunflower MASP inhibitor-2 (SFMI-2) as well as derivatives thereof for the treatment of diseases related to the complement system, primarily the lectin pathway were first described in WO 2010/136831.

WO 2015/054298 discloses methods for preserving vision or reducing vision loss in a subject and for inhibiting or reducing photoreceptor cell death in a subject by reducing the activity of MASP-1, MASP-2 or MASP-3. WO 2004/106384, WO 2005/123128, WO 2007/117996 and WO 2014/144542 disclose anti-MASP-2 antibodies for the therapy of diseases associated with MASP-2-dependent complement activation.

It was the object of the present invention to provide novel peptides, having inhibitory effects on MASP-1 and/or MASP-2 enzymes and other beneficial properties making them suitable as efficient and safe alternatives for the prophylaxis and treatment of MASP-1 and/or MASP-2-associated disorder as defined below. It was a further object to provide novel peptides, having an improved inhibitory effect on human MASP-1 and/or MASP-2 enzyme and/or rat MASP-1 and/or MASP-2 enzyme.

The present invention generally relates to peptides acting as inhibitors of MASP-1 and/or MASP-2 enzymes and methods of making and using the same.

The invention provides bicyclic compounds, which may be isolated and/or purified, comprising, essentially consisting of, or consisting of the formula (I): 
X1—X2—X3—Ile4—Cys5—Ser6—Arg7—Ser8—X9—Pro10—X11—Ile12—X13—Ile14—X15—X16—X17
(I)
 or a derivative, analogue, pharmaceutically acceptable salt, solvate or solvate of the salt thereof, wherein
- X¹: represents a natural amino acid, which can be in D- or L-stereoconfiguration, selected from the group consisting of alanine, glycine, lysine, cysteine and glutamic acid, or a moiety selected from the group consisting of 6-aminohexanoic acid (Ahx), L-2,3-Diaminopropionic acid (Dap), L-2,4-Diaminobutyric acid (Dab), 3-azido-L-Alanine, L-2-aminobutyric acid (Abu), gamma-aminobutyric acid (gamma-Abu), 2-aminoisobutyric acid (Aib), L-Ornithine (Orn), 1,13-diamino-4,7,10-trioxatridecan-succinamic acid (TTDS), 9-Amino-4,7-dioxanonanoic acid [PEG1(10 atoms)], 12-Amino-4,7,10-trioxadodecanoic acid [PEG2(13 atoms)], 15-Amino-4,7,10,13-tetraoxapentadecanoic acid [PEG3(16 atoms)] and adipic acid, or X¹ may be absent,
- X²: represents a natural amino acid, which can be in D- or L-stereoconfiguration, selected from the group consisting of glycine and serine, or a moiety selected from the group consisting of N-methyl-glycine, L-2,3-Diaminopropionic acid (Dap), L-2,4-Diaminobutyric acid (Dab), L-2-Aminobutyric acid (Abu), gamma-aminobutyric acid (gamma-Abu), tranexamic acid (TXA), 3-(aminomethyl)benzoic acid and 4-(aminomethyl)benzoic acid, or X² may be absent,
- X³: represents a natural amino acid, which can be in D- or L-stereoconfiguration, selected from the group consisting of glycine and alanine, or X³ may be absent,
- Ile⁴: represents L-Isoleucine,
- Cys⁵: represents L-Cysteine,
- Ser⁶: represents L-Serine,
- Arg⁷: represents L-Arginine,
- Ser⁸: represents L-Serine,
- X⁹: represents L-Lysine or L-tert-Butylalanine [(tBu)A)],
- Pr0¹⁰: represents L-Proline,
- X¹¹: represents L-Proline or 2,3,3a,4,5,6,7,7a-octahydroindole-2-carboxylic acid (Oic),
- Ile¹²: represents L-Isoleucine,
- X¹³: represents L-Cysteine, L-N-Methylcysteine [(N-Me)C] or L-Penicillamine (Pen),
- Ile¹⁴: represents L-Isoleucine,
- X¹⁵: represents L-Proline or 2-aminoisobutyric acid (Aib), or X¹⁵ may be absent,
- X¹⁶: represents a natural amino acid, which can be in D- or L-stereoconfiguration, selected from the group consisting of aspartic acid and glutamic acid, or X¹⁶ may be absent,
- X¹⁷: represents a natural amino acid, which can be in D- or L-stereoconfiguration, selected from the group consisting of serine, cysteine, proline and lysine, or a moiety selected from the group consisting of L-2,3-Diaminopropionic acid (Dap), L-2,4-Diaminobutyric acid (Dab) and L-Propargylglycine, or X¹⁷ may be absent,
wherein Cys⁵ and X¹³ are linked by a disulfide bond between the sulfur atoms of the two groups forming a first ring,
wherein a second ring is formed between X¹ (in case X¹ is not absent), X² (in case X¹ is absent and X² is not absent), X³ (in case X¹ and X² are absent and X³ is not absent) or Ile⁴ (in case X¹, X² and X³ are all absent) at the N-terminus and Ile¹⁴ (in case X¹⁵, X¹⁶ and X¹⁷ are all absent), X¹⁵ (in case X¹⁶ and X¹⁷ are absent and X¹⁵ is not absent), X¹⁶ (in case X¹⁷ is absent and X¹⁶ is not absent) or X¹⁷ (in case X¹⁷ is not absent) at the C-terminus,
and wherein such second ring may be formed either via an α-peptide bond in the backbone or via one or two of the amino acid side chains, where in the case the second ring is formed not using the C-terminal carboxylic acid then the C-terminal carboxy group may be transformed in to an amide group,
wherein in the case that X¹ represents 3-azido-L-Alanine and X¹⁷ represents L-Propargylglycine the ring formation results in an 1,2,3-triazole ring, which is attached in 1-position to the alanine and in 4-position to the glycine.

The indices, e.g. 2 and 5 in X² and Cys⁵, indicate the position of the amino acid in the peptide for easy reference.

Unless otherwise defined herein, scientific and technical terms used in this application shall have the meanings that are commonly understood by those of ordinary skill in the art. Generally, nomenclature used in connection with, and techniques of, chemistry, molecular biology, cell and cancer biology, immunology, microbiology, pharmacology, and protein and nucleic acid chemistry, described herein, are those well-known and commonly used in the art.

Throughout this specification, the word "comprise" or variations thereof such as "comprises" or "comprising" will be understood to imply the inclusion of a stated integer (or components) or group of integers (or components), but not the exclusion of any other integer (or components) or group of integers (or components). The singular forms "a", "an", and "the" include the plurals unless the context clearly dictates otherwise. The term "including" and "containing" is used to mean "including but not limited to", which expressions can be used interchangeably. In particular, the expression "compound containing a peptide" means a compound which contains a defined peptide sequence and which can optionally contain further chemical groups or substituents covalently bound to the peptide, e.g. amino acids, fatty acids, chemical groups to enhance pharmacodynamic or pharmacokinetic properties of the peptide or any other chemical groups. It is also to be understood that the expression "compound containing a peptide" explicitly includes the defined peptide sequence without any further chemical groups or substituents covalently bound to that peptide.

As used herein, the following terms have the meanings ascribed to them unless specified otherwise. "Essentially consisting of" is understood as a peptide being at least 80%, at least 85%, at least 90%, at least 95%, at least 96%, at least 97%, at least 98%, or at least 99% identical to the peptide it is compared to.

The terms "protein", "polypeptide" and "peptide" are used interchangeably to refer broadly to a sequence of two or more amino acids linked together, preferable by peptide (amide) bonds. Peptide (amide) bonds are formed when the carboxyl group of one amino acid reacts with the amino group of another. It should be further understood that the terms "protein", "polypeptide" and "peptide" do not indicate a specific length of a polymer of amino acids, nor is it intended to imply or distinguish whether the polypeptide is produced using recombinant techniques, chemical or enzymatic synthesis, or is naturally occurring. It should be further understood that a peptide can contain one or more parts which are no amino acids under the definition of the present application. These parts are preferably present at the N- and C-terminal ends of the peptide.

The term "amino acid" or "any amino acid" as used herein refers to organic compounds containing amine (-NH₂) and carboxyl (-COOH) functional groups, along with a side chain and refers to any and all amino acids, including naturally occurring amino acids (e.g., α-L-amino acids), unnatural amino acids, modified amino acids, and non-natural amino acids. "Natural amino acids" include those found in nature, such as, e.g., the 23 amino acids that combine into peptide chains to form the building-blocks of a vast array of proteins. These are primarily L stereoisomers, although a few D-amino acids occur in bacterial envelopes and some antibiotics. The 20 proteinogenic, natural amino acids in the standard genetic code are listed in Table 2.

"Unnatural" or "non-natural" amino acids are non-proteinogenic amino acids (i.e., those not naturally encoded or found in the genetic code) that either occur naturally or are chemically synthesized. Over 140 natural amino acids are known and thousands of more combinations are possible. Examples of "unnatural" amino acids include β-amino acids (β³ and β²), homo-amino acids, proline and pyruvic acid derivatives, 3-substituted alanine derivatives, glycine derivatives, ring-substituted phenylalanine and tyrosine derivatives, linear core amino acids, diamino acids, D-amino acids, and N-methyl amino acids. Unnatural or non-natural amino acids also include modified amino acids. "Modified" amino acids include amino acids (e.g., natural amino acids) that have been chemically modified to include a group, groups, or chemical moiety not naturally present in the amino acid. According to the present invention preferred unnatural amino acids are listed in Table 1. Table 1 displays unnatural amino acids as D- and/or L-stereoisomers, however preferred unnatural amino acids according to the invention are both D- and L-stereoisomers of unnatural amino acids listed in Table 1.

**Table 1: Preferred unnatural amino acids**

| |
|---|
| 1,13-Diamino-4,7,10-trioxatridecan-succinamic acid (TTDS) |
| 12-Amino-4,7,10-trioxadodecanoic acid [PEG2(13 atoms)] |
| 15-Amino-4,7,10,13-tetraoxapentadecanoic acid [PEG3(16 atoms)] |
| 2,3,3a,4,5,6,7,7a-Octahydroindole-2-carboxylic acid (Oic) |
| 3-(Aminomethyl)benzoic acid |
| 3 -Azido-L-Alanine |
| 4-(Aminomethyl)benzoic acid |
| 9-Amino-4,7-dioxanonanoic acid [PEG1(10 atoms)] |
| Gamma-Aminobutyric acid (gamma-Abu) |
| L-2,3-Diaminopropionic acid (Dap) |
| L-2,4-Diaminobutyric acid (Dab) |
| L-2-Aminobutyric acid (Abu) |
| L-N-Methylcysteine ((N-Me)C) |
| L-Ornithine (Orn) |
| L-Penicillamine (Pen) |
| L-Propargylglycine |
| L-tert-Butylalanine ((tBu)A) |
| N-Methyl-Glycine ((N-Me)G) |
| 6-Aminohexanoic acid (Ahx) |
| Tranexamic acid (TXA) |
| 2-Aminoisobutyric acid (Aib) |

More preferred unnatural amino acid are selected from a list consisting of N-Methyl-Glycine ((N-Me)G), L-tert-Butylalanine ((tBu)A), 3-(Aminomethyl)benzoic acid, 4-(Aminomethyl)benzoic acid, L-2-Aminobutyric acid (Abu), 6-Aminohexanoic acid (Ahx), 2-Aminoisobutyric acid (Aib), L-2,4-Diaminobutyric acid (Dab), L-2,3-Diaminopropionic acid (Dap), Gamma-Aminobutyric acid (Gamma-Abu), L-Ornithine (Orn), 2,3,3a,4,5,6,7,7a-Octahydroindole-2-carboxylic acid (Oic), L-N-Methylcysteine ((N-Me)C), L-Penicillamine (Pen), Tranexamic acid (TXA), 1,13-Diamino-4,7,10-trioxatridecan-succinamic acid (TTDS), 12-Amino-4,7,10-trioxadodecanoic acid, 15-Amino-4,7,10,13-tetraoxapentadecanoic acid and 1,13-Diamino-4,7,10-trioxatridecan-succinamic acid (TTDS).

Most preferred unnatural amino acid are selected from a list consisting of L-tert-Butylalanine ((tBu)A), 3-(Aminomethyl)benzoic acid, 4-(Aminomethyl)benzoic acid, 6-Aminohexanoic acid (Ahx), L-2,4-Diaminobutyric acid (Dab), L-2,3-Diaminopropionic acid (Dap), L-Ornithine (Orn), 2,3,3a,4,5,6,7,7a-Octahydroindole-2-carboxylic acid (Oic), L-Penicillamine (Pen), Tranexamic acid (TXA), 1,13-Diamino-4,7,10-trioxatridecan-succinamic acid (TTDS), 12-Amino-4,7,10-trioxadodecanoic acid, 15-Amino-4,7,10,13-tetraoxapentadecanoic acid and 1,13-Diamino-4,7,10-trioxatridecan-succinamic acid (TTDS).

It should be understood that all amino acids and chemical groups of the peptides of the present invention are connected via peptide (amide) bonds. Generally, peptides are formed by linking α-amino and carboxy groups of α-amino acids, which are then linked by α-peptide bonds. According to the present invention a peptide bond can be formed by any carboxyl- and amino group being present in a respective natural or unnatural amino acid. For example, α-amino acids which contain a second amino group in addition to the α-amino group (e.g. L-lysine) or α-amino acids which, in addition to the α-carboxy group, contain a second carboxy group, (eg. L-aspartic acid and L-glutamic acid) can be connected via the additional amino- or carboxy group.

In accordance with the understanding of a person skilled in the art, the peptide sequences disclosed herein represent sequences of amino acids, which are connected via α-peptide bonds. A "+"in the sequence means that the attachment is using the amino acid side chain for attachment to form a disulfide bond [e.g. C+, (Pen)+, (N-Me)C+] of the first ring. Since the peptides of this invention contain two rings, the second ring is indicated by "**" or "++", indicating the two amino acids that are joined to form a second ring. The following two examples will illustrate, how the structure drawn in formula (I) and the linear sequence correlate.

(1) In the sequence A**GGIC+SRS-((tBu)A)-PPI-(Pen)+-IPd**, the "+"'s for C+ and (Pen)+ indicate that a disulfide bond is formed using the side chain sulfur atoms of Cys-5 and Pen-13, and the "**"'s indicate that Ala-1 and d-Asp-16 form a head-to-tail cyclization via amide bond to form a second ring (X¹⁷ is absent in this case). The structure according to formula I is the following (cf. Example 39):

Ala1–Gly2–Gly3–Ile4–Cys5–Ser6–Arg7–Ser8–(tBu)Ala9–Pro10–Pro11–Ile12–Pen13–Ile14–Pro15–asp16

Likewise, in the sequence (Ahx)**-GIC+SRS-((tBu)A)-PPI-((N-Me)C)+-IPD++-NH₂, the "+"'s for C+ and ((N-Me)C)+ indicate that a disulfide bond is being formed using the side chain sulfur atoms of Cys-5 and (N-Me)Cys-13, and the "**" and the "++" indicate that a head-to-side chain amide bond is being formed between Ahx-1 and side chain acid of Asp-16 (X² and X¹⁷ are absent in this case). The structure according to formula I is the following (cf. Example 40), where the CONH₂ group indicates that the side chain of Asp¹⁶ is used to form the bond with Ahx¹.

H2NOC
Ahx1–Gly3–Ile4–Cys5–Ser6–Arg7–Ser8–(tBu)Ala9–Pro10–Pro11–Ile12–(N-Me)C13–Ile14–Pro15–Asp16

In accordance with the understanding of a person skilled in the art, the peptide sequences disclosed herein are shown proceeding from left to right, with the left end of the sequence being the "N-terminus" ("amino terminus", "N-terminal end") of the peptide and the right end of the sequence being the "C-terminus" ("carboxy terminus", "C-terminal end") of the peptide. This terminology N-terminus (amino terminus, N-terminal end)" applies irrespective of whether the peptide actually contains an amino group at the N-terminus. This terminology C-terminus (carboxy terminus, C-terminal end) applies irrespective of whether the peptide actually contains a carboxy group at the C-terminus. The term "terminal amino group" refers to any amino group present at the N-terminus. The term "terminal carboxyl group" refers to any carboxyl group present at the C-terminus.

According to the present invention the second ring is formed between X¹ (in case X¹ is not absent), X² (in case X¹ is absent and X² is not absent), X³ (in case X¹ and X² are absent and X³ is not absent) or Ile⁴ (in case X¹, X² and X³ are all absent) at the N-terminus and Ile¹⁴ (in case X¹⁵, X¹⁶ and X¹⁷ are all absent), X¹⁵ (in case X¹⁶ and X¹⁷ are absent and X¹⁵ is not absent), X¹⁶ (in case X¹⁷ is absent and X¹⁶ is not absent) or X¹⁷ (in case X¹⁷ is not absent) at the C-terminus.

In the present invention the names of naturally occurring and non-naturally occurring aminoacyl residues used herein are preferably following the naming conventions suggested by the IUPAC Commission on the Nomenclature of Organic Chemistry and the IUPAC-IUB Commission on Biochemical Nomenclature as set out in Nomenclature of α-Amino Acids (Recommendations, 1974), Biochemistry, 14(2), (1975*).*

In the present specification naturally occurring proteinogenic amino acids are usually designated by their conventional single-letter abbreviations. Alternatively, they can also be referred to by their three-letter abbreviations (e.g. in particular in the sequence listings) or by their full name as shown in Table 2 below:

**Table 2: Standard Abbreviations for Natural Amino Acids**

| **3-Letter** | **1-Letter** | **Amino Acid** |
|---|---|---|
| Ala | A | Alanine |
| Arg | R | Arginine |
| Asn | N | Asparagine |
| Asp | D | Aspartic acid |
| Cys | C | Cysteine |
| Glu | E | Glutamic acid |
| Gln | Q | Glutamine |
| Gly | G | Glycine |
| His | H | Histidine |
| Ile | I | Isoleucine |
| Leu | L | Leucine |
| Lys | K | Lysine |
| Met | M | Methionine |
| Phe | F | Phenylalanine |
| Pro | P | Proline |
| Ser | S | Serine |
| Thr | T | Threonine |
| Trp | W | Tryptophan |
| Tyr | Y | Tyrosine |
| Val | V | Valine |

In the case of non-proteinogenic or non-naturally occurring amino acids, unless they are referred to by their full name (e.g. ornithine, etc.), frequently employed three- to six-character codes are employed for residues thereof, including those abbreviations as indicated in the abbreviation list below (Table 3).

The term "L-amino acid", as used herein, refers to the "L" isomeric form of an amino acid, and conversely the term "D-amino acid" refers to the "D" isomeric form of an amino acid. It is further a conventional manner to indicate the L-amino acid with capital letters such as Ala / A, Arg / R, etc. and the D-amino acid with small letters such as ala / a, arg / r, etc.

The three-letter code in the form as indicated in the table above, i.e. Ala, Arg, Asn etc. and as generally used in the present specification, shall generally comprise the D- and L- form as well as homo- and nor-forms, unless explicitly indicated otherwise. The prefix "nor" refers to a structural analog that can be derived from a parent compound by the removal of one carbon atom along with the accompanying hydrogen atoms. The prefix "homo" indicates the next higher member in a homologous series. A reference to a specific isomeric form will be indicated by the capital prefix L- or D- as described above (e.g. D-Arg, L-Arg etc.). A specific reference to homo- or nor-forms will accordingly be explicitly indicated by a respective prefix (e.g. homo-Arg, homo-R, nor-Arg, nor-R, homo-Cys, homo-C etc.).

Among sequences disclosed herein are sequences incorporating either an "-OH" moiety or an "-NH₂" moiety at the bond forming the second ring via the amino acid side chain. An "-OH" or an "-NH₂" moiety at such bond of the sequence indicates a hydroxy group or an amino group, corresponding to the presence of a carboxy group or an amido [-(C=O)-NH₂] group, respectively. In each sequence of the invention, a "-OH" moiety may be substituted for a C-terminal "-NH₂" moiety, and vice-versa. However, among said alternatives a C-terminal "-OH" moiety is preferred.

According to a further embodiment, the invention provides bicyclic compounds, which may be isolated and/or purified, comprising, essentially consisting of, or consisting of formula (I) or a derivative, analogue, pharmaceutically acceptable salt, solvate or solvate of the salt thereof, wherein
- X¹: represents a natural amino acid selected from the group consisting of D-alanine, L-Alanine, Glycine, D-lysine, L-Lysine, L-Cysteine and L-Glutamic acid, or a moiety selected from the group consisting of 6-aminohexanoic acid (Ahx), L-2,3-Diaminopropionic acid (Dap), L-2,4-Diaminobutyric acid (Dab), gamma-aminobutyric acid (gamma-Abu), L-Ornithine (Orn), 1,13-diamino-4,7,10-trioxatride-can-succinamic acid (TTDS), 9-Amino-4,7-dioxanonanoic acid [PEG1(10 atoms)], 15-Amino-4,7,10,13-tetraoxapentadecanoic acid [PEG3(16 atoms)] and adipic acid, or X¹ may be absent,
- X²: represents a natural amino acid selected from the group consisting of Glycine and L-Serine, or a moiety selected from the group consisting of N-methyl-glycine, L-2,3-Diaminopropionic acid (Dap), L-2,4-Diaminobutyric acid (Dab), L-2-Aminobutyric acid (Abu), tranexamic acid (TXA), and 4-(aminomethyl)benzoic acid, or X² may be absent,
- X³: represents a natural amino acidselected from the group consisting of Glycine, L-Alanine and D-alanine, or X³ may be absent,
- Ile⁴: represents L-Isoleucine,
- Cys⁵: represents L-Cysteine,
- Ser⁶: represents L-Serine,
- Arg⁷: represents L-Arginine,
- Ser⁸: represents L-Serine,
- X⁹: represents L-Lysine or L-tert-Butylalanine [(tBu)A)],
- Pr0¹⁰: represents L-Proline,
- X¹¹: represents L-proline or 2,3,3a,4,5,6,7,7a-octahydroindole-2-carboxylic acid (Oic),
- Ile¹²: represents L-Isoleucine,
- X¹³: represents L-Cysteine, L-N-Methylcysteine [(N-Me)C] or L-Penicillamine (Pen),
- Ile¹⁴: represents L-Isoleucine,
- X¹⁵: represents L-Proline, or X¹⁵ may be absent,
- X¹⁶: represents a natural amino acid selected from the group consisting of L-Aspartic acide, D-aspartic acid and L-Glutamic acid, or X¹⁶ may be absent,
- X¹⁷: represents a natural amino acid selected from the group consisting of L-Serine, L-Cysteine, L-Proline and L-Lysine, or a moiety selected from the group consisting of L-2,3-Diaminopropionic acid (Dap), or X¹⁷ may be absent,
wherein Cys⁵ and X¹³ are linked by a disulfide bond between the sulfur atoms of the two groups forming a first ring,
wherein a second ring is formed between X¹ (in case X¹ is not absent), X² (in case X¹ is absent and X² is not absent), X³ (in case X¹ and X² are absent and X³ is not absent) or Ile⁴ (in case X¹, X² and X³ are all absent) at the N-terminus and Ile¹⁴ (in case X¹⁵, X¹⁶ and X¹⁷ are all absent), X¹⁵ (in case X¹⁶ and X¹⁷ are absent and X¹⁵ is not absent), X¹⁶ (in case X¹⁷ is absent and X¹⁶ is not absent) or X¹⁷ (in case X¹⁷ is not absent) at the C-terminus,
and wherein such second ring may be formed either via an α-peptide bond in the backbone or via one or two of the amino acid side chains, where in the case the second ring is formed not using the C-terminal carboxylic acid then the C-terminal carboxy group may be transformed in to an amide group.

According to a further embodiment, the invention provides bicyclic compounds, which may be isolated and/or purified, comprising, essentially consisting of, or consisting of formula (I) or a derivative, analogue, pharmaceutically acceptable salt, solvate or solvate of the salt thereof, wherein
- X¹: represents a natural amino acid selected from the group consisting of L-Alanine, Glycine, L-Lysine and L-Glutamic acid, or a moiety selected from the group consisting of 6-aminohexanoic acid (Ahx), L-2,3-Diaminopropionic acid (Dap), L-2,4-Diaminobutyric acid (Dab), gamma-aminobutyric acid (gamma-Abu), L-Ornithine (Orn), 1,13-diamino-4,7,10-trioxatridecan-succinamic acid (TTDS), 9-Amino-4,7-dioxanonanoic acid [PEG1(10 atoms)], 15-Amino-4,7,10,13-tetraoxapentadecanoic acid [PEG3(16 atoms)] and adipic acid,
- X²: represents a natural amino acid selected from the group consisting of Glycine and L-Serine, or a moiety selected from the group consisting of N-methyl-glycine, L-2,3-Diaminopropionic acid (Dap), L-2-Aminobutyric acid (Abu), tranexamic acid (TXA), and 4-(aminomethyl)benzoic acid, or X² may be absent,
- X³: represents a natural amino acidselected from the group consisting of Glycine and L-Alanine, or X³ may be absent,
- Ile⁴: represents L-Isoleucine,
- Cys⁵: represents L-Cysteine,
- Ser⁶: represents L-Serine,
- Arg⁷: represents L-Arginine,
- Ser⁸: represents L-Serine,
- X⁹: represents L-Lysine or L-tert-Butylalanine [(tBu)A)],
- Pr0¹⁰: represents L-Proline,
- X¹¹: represents L-proline or 2,3,3a,4,5,6,7,7a-octahydroindole-2-carboxylic acid (Oic),
- Ile^{l2}: represents L-Isoleucine,
- X¹³: represents L-N-Methylcysteine [(N-Me)C] or L-Penicillamine (Pen),
- Ile¹⁴: represents L-Isoleucine,
- X¹⁵: represents L-Proline, or X¹⁵ may be absent,
- X¹⁶: represents a natural amino acid selected from the group consisting of L-Aspartic acide and L-Glutamic acid, or X¹⁶ may be absent,
- X¹⁷: represents a natural amino acid selected from the group consisting of L-Proline and L-Lysine, or a moiety selected from the group consisting of L-2,3-Diaminopropionic acid (Dap), or X¹⁷ may be absent,
wherein Cys⁵ and X¹³ are linked by a disulfide bond between the sulfur atoms of the two groups forming a first ring,
wherein a second ring is formed between X¹ at the N-terminus and Ile¹⁴ (in case X¹⁵, X¹⁶ and X¹⁷ are all absent), X¹⁵ (in case X¹⁶ and X¹⁷ are absent and X¹⁵ is not absent), X¹⁶ (in case X¹⁷ is absent and X¹⁶ is not absent) or X¹⁷ (in case X¹⁷ is not absent) at the C-terminus,
and wherein such second ring may be formed either via an α-peptide bond in the backbone or via one or two of the amino acid side chains, where in the case the second ring is formed not using the C-terminal carboxylic acid then the C-terminal carboxy group may be transformed in to an amide group.

According to a further embodiment, the invention provides bicyclic compounds, which may be isolated and/or purified, comprising, essentially consisting of, or consisting of formula (I) or a derivative, analogue, pharmaceutically acceptable salt, solvate or solvate of the salt thereof, wherein
- X¹: represents a natural amino acid selected from the group consisting of L-Alanine and Glycine, L-Lysine, or a moiety selected from the group consisting of 6-aminohexanoic acid (Ahx), L-2,3-Diaminopropionic acid (Dap), gamma-aminobutyric acid (gamma-Abu), L-Ornithine (Orn),
- X²: represents the natural amino acid Glycine, or a moiety selected from the group consisting L-2,3-Diaminopropionic acid (Dap), L-2-Aminobutyric acid (Abu), tranexamic acid (TXA), and 4-(aminomethyl)benzoic acid, or X² may be absent,
- X³: represents a natural amino acidselected from the group consisting of Glycine and L-Alanine, or X³ may be absent,
- Ile⁴: represents L-Isoleucine,
- Cys⁵: represents L-Cysteine,
- Ser⁶: represents L-Serine,
- Arg⁷: represents L-Arginine,
- Ser⁸: represents L-Serine,
- X⁹: represents L-tert-Butylalanine [(tBu)A)],
- Pr0¹⁰: represents L-Proline,
- X¹¹: represents 2,3,3a,4,5,6,7,7a-octahydroindole-2-carboxylic acid (Oic),
- Ile¹²: represents L-Isoleucine,
- X¹³: represents L-Penicillamine (Pen),
- Ile¹⁴: represents L-Isoleucine,
- X¹⁵: represents L-Proline, or X¹⁵ may be absent,
- X¹⁶: represents a natural amino acid selected from the group consisting of L-Aspartic acide and L-Glutamic acid, or X¹⁶ may be absent,
- X¹⁷: is absent,
wherein Cys⁵ and X¹³ are linked by a disulfide bond between the sulfur atoms of the two groups forming a first ring,
wherein a second ring is formed between X¹ at the N-terminus and Ile¹⁴ (in case X¹⁵ and X¹⁶ are absent), X¹⁵ (in case X¹⁶ is absent and X¹⁵ is not absent) or X¹⁶ (X¹⁶ is not absent) at the C-terminus,
and wherein such second ring may be formed either via an α-peptide bond in the backbone or via one or two of the amino acid side chains, where in the case the second ring is formed not using the C-terminal carboxylic acid then the C-terminal carboxy group may be transformed in to an amide group.

Futher embodiments of the invention are disclosed in the following.

X¹ may be present or absent.

Preferably X¹ is present.

X¹, if present, represents a natural amino acid, which can be in D- or L-stereoconfiguration, selected from the group consisting of alanine, glycine, lysine, cysteine and glutamic acid, or a moiety selected from the group consisting of 6-aminohexanoic acid (Ahx), L-2,3-Diaminopropionic acid (Dap), L-2,4-Diaminobutyric acid (Dab), 3-azido-L-Alanine, L-2-aminobutyric acid (Abu), gamma-aminobutyric acid (gamma-Abu), 2-aminoisobutyric acid (Aib), L-Ornithine (Orn), 1,13-diamino-4,7,10-trioxatridecan-succinamic acid (TTDS), 9-Amino-4,7-dioxanonanoic acid [PEG1(10 atoms)], 12-Amino-4,7,10-trioxadodecanoic acid [PEG2(13 atoms)], 15-Amino-4,7,10,13-tetraoxapentadecanoic acid [PEG3(16 atoms)] and adipic acid.

X¹, if present, preferably represents a natural amino acid selected from the group consisting of D-alanine, L-Alanine, Glycine, D-lysine, L-Lysine, L-Cysteine and L-Glutamic acid, or a moiety selected from the group consisting of 6-aminohexanoic acid (Ahx), L-2,3-Diaminopropionic acid (Dap), L-2,4-Diaminobutyric acid (Dab), gamma-aminobutyric acid (gamma-Abu), L-Ornithine (Orn), 1,13-diamino-4,7,10-trioxatridecan-succinamic acid (TTDS), 9-Amino-4,7-dioxanonanoic acid [PEG1(10 atoms)], 15-Amino-4,7,10,13-tetraoxapentadecanoic acid [PEG3(16 atoms)] and adipic acid.

X¹, if present, more preferred represents a natural amino acid selected from the group consisting of L-Alanine, Glycine, L-Lysine and L-Glutamic acid, or a moiety selected from the group consisting of 6-aminohexanoic acid (Ahx), L-2,3-Diaminopropionic acid (Dap), L-2,4-Diaminobutyric acid (Dab), gamma-aminobutyric acid (gamma-Abu), L-Ornithine (Orn), 1,13-diamino-4,7,10-trioxatridecan-succinamic acid (TTDS), 9-Amino-4,7-dioxanonanoic acid [PEG1(10 atoms)], 15-Amino-4,7,10,13-tetraoxapentadecanoic acid [PEG3(16 atoms)] and adipic acid.

In a further embodiment X¹, if present, represents a natural amino acid selected from the group consisting of L-Alanine and Glycine, L-Lysine, or a moiety selected from the group consisting of 6-aminohexanoic acid (Ahx), L-2,3-Diaminopropionic acid (Dap), gamma-aminobutyric acid (gamma-Abu), L-Ornithine (Orn).

X² may be present or absent.

X², if present, represents a natural amino acid, which can be in D- or L-stereoconfiguration, selected from the group consisting of glycine and serine, or a moiety selected from the group consisting of N-methyl-glycine, L-2,3-Diaminopropionic acid (Dap), L-2,4-Diaminobutyric acid (Dab), L-2-Aminobutyric acid (Abu), gamma-aminobutyric acid (gamma-Abu), tranexamic acid (TXA), 3-(aminomethyl)benzoic acid and 4-(aminomethyl)benzoic acid.

X², if present, preferably represents a natural amino acid selected from the group consisting of Glycine and L-Serine, or a moiety selected from the group consisting of N-methyl-glycine, L-2,3-Diaminopropionic acid (Dap), L-2,4-Diaminobutyric acid (Dab), L-2-Aminobutyric acid (Abu), tranexamic acid (TXA), and 4-(aminomethyl)benzoic acid.

X², if present, more preferred represents a natural amino acid selected from the group consisting of Glycine and L-Serine, or a moiety selected from the group consisting of N-methyl-glycine, L-2,3-Diaminopropionic acid (Dap), L-2-Aminobutyric acid (Abu), tranexamic acid (TXA), and 4-(aminomethyl)benzoic acid.

In a further embodiment X², if present, represents the natural amino acid Glycine, or a moiety selected from the group consisting L-2,3-Diaminopropionic acid (Dap), L-2-Aminobutyric acid (Abu), tranexamic acid (TXA), and 4-(aminomethyl)benzoic acid.

X³ may be present or absent.

X³, if present, represents a natural amino acid, which can be in D- or L-stereoconfiguration, selected from the group consisting of glycine and alanine.

X³, if present, preferably represents a natural amino acidselected from the group consisting of Glycine, L-Alanine and D-alanine.

X³, if present, more preferred represents a natural amino acidselected from the group consisting of Glycine and L-Alanine.

X⁹ preferably represents L-tert-Buylalanine [(tBu)A)].

X¹¹ preferably represents 2,3,3a,4,5,6,7,7a-octahydroindole-2-carboxylic acid (Oic).

X¹³ preferably represents L-N-Methylcysteine [(N-Me)C] or L-Penicillamine (Pen).

X¹³ more preferred represents L-Penicillamine (Pen).

X¹⁵ preferably represents L-Proline or X¹⁵ is absent.

X¹⁵ more preferred represents L-Proline.

X¹⁵ also more preferred is absent.

X¹⁶ may be present or absent.

X¹⁶, if present, represents a natural amino acid, which can be in D- or L-stereoconfiguration, selected from the group consisting of aspartic acid and glutamic acid.

X¹⁶, if present, preferably represents a natural amino acid selected from the group consisting of L-Aspartic acide, D-aspartic acid and L-Glutamic acid.

X¹⁶, if present, more preferred represents a natural amino acid selected from the group consisting of L-Aspartic acide and L-Glutamic acid.

X¹⁷ may be present or absent.

X¹⁷, if present, represents a natural amino acid, which can be in D- or L-stereoconfiguration, selected from the group consisting of serine, cysteine, proline and lysine, or a moiety selected from the group consisting of L-2,3-Diaminopropionic acid (Dap), L-2,4-Diaminobutyric acid (Dab) and L-Propargylglycine.

X¹⁷, if present, preferably represents a natural amino acid selected from the group consisting of L-Serine, L-Cysteine, L-Proline and L-Lysine, or a moiety selected from the group consisting of L-2,3-Diaminopropionic acid (Dap).

X¹⁷, if present, more preferred represents a natural amino acid selected from the group consisting of L-Proline and L-Lysine, or a moiety selected from the group consisting of L-2,3-Diaminopropionic acid (Dap).

In a further embodiment X¹⁷ is absent.

In another embodiment of the invention X¹ and X¹⁶ are present and X¹⁷ is absent.

In a further embodiment of the invention X¹ and X¹⁵ are present and X¹⁶ and X¹⁷ are absent.

Chemical groups, unnatural amino acids or moieties may be abbreviated herein as shown in Table 3.

**Table 3: Abbreviations/expressions and nomenclature used for chemical groups, unnatural amino acids or further moieties in the sequences.**

| **Abbreviation/Expression** | **Abbreviation/Expression Definition** |
|---|---|
| ((N-Me)C) | L-N-Methylcysteine |
| (N-Me)G | N -Methyl-Glycine |
| 3-(Aminomethyl)benzoic acid | 3-(Aminomethyl)benzoic acid |
| 4-(Aminomethyl)benzoic acid | 4-(Aminomethyl)benzoic acid |
| Abu | L-2-Aminobutyric acid |
| Adipic acid | Adipic acid |
| Ahx | 6-Aminohexanoic acid |
| Aib | 2-Aminoisobutyric acid |
| (tBu)A | L-tert-Butylalanine |
| 3-Azido-L-Alanine | 3 -Azido-L-Alanine |
| Dab | L-2,4-Diaminobutyric acid |
| Dap | L-2,3-Diaminopropionic acid |
| gamma-Abu | Gamma-Aminobutyric acid |
| L-Propargylglycine | L-Propargylglycine |
| Oic | 2,3,3a,4,5,6,7,7a-Octahydroindole-2-carboxylic acid |
| Orn | L-Ornithine |
| PEG1(10 atoms) | 9-Amino-4,7-dioxanonanoic acid |
| PEG2(13 atoms) | 12-Amino-4,7,10-trioxadodecanoic acid |
| PEG3(16 atoms) | 15-Amino-4,7,10,13 -tetraoxapentadecanoic acid |
| Pen | L-Penicillamine |
| Suberic acid | Suberic acid |
| TXA | Tranexamic acid |
| TTDS | 1,13 -Diamino-4,7,10-trioxatridecan-succinamic acid |

The term "mimetic", used in context with some amino acids in the definition of several moieties of the peptide according to formula (I) of the present invention, represents a respective amino acid mimetic, such as e.g. an arginine mimetic, an isoleucine mimetic or a proline mimetic. Generally, a "protein mimetic" indicates a molecule such as a peptide, a modified peptide or any other molecule that biologically mimics the action or activity of some other protein. In context with the use of the term "mimetic" in connection with a certain amino acid said term "mimetic" analogously indicates any other amino acid, amino acid analogue, amino acid derivative, amino acid conjugate or the like, which biologically mimics the action or activity of the respective amino acid.

Proline mimetics according to the present invention comprise in particular (1S,2S,5R)-3-Azabicyclo[3.1.0]-hexane-2-carboxylic acid, Hyp, Morpholine-3-carboxylic, Pip, (4aR,6aR,9S,11aS)-11-Oxo-2,3,4,4a,6a,-7,8,9,11,11a-decahydro-1H-pyrido[3,2-e]pyrrolo[1,2-a]azepine-9-carboxylic acid or (trans-4-Fluoro)P, (1R,2S,5S)-3-azabicyclo[3.1.0]hexane-2-carboxylic acid, Oic, Hyp, (4-CF3)P, (cis-4-Fluoro)P, 3,3-dimethyl-1,3-azasilolidine-5-carboxylic acid, (3S-OH)P, (1R,3S,5R)-2-Azabicyclo[3.1.0]hexane-3-carboxylic acid, (6S)-5-Azaspiro[2.4]heptane-6-carboxylic acid, rel-(1R,3R,5R,6R)-6-(Trifluoromethyl)-2-azabicyclo[3.1.0]-hexane-3-carboxylic acid, (2S,3aS,6aS)-Octahydrocyclopenta[b]pyrrole-2-carboxylic acid or difluoroproline, (3R,6R)-1,1-Difluoro-5-azaspiro[2.4]heptane-6-carboxylic acid (enantiomer 1), (3R,6R)-1,1-Difluoro-5-azaspiro[2.4]heptane-6-carboxylic acid (enantiomer 2) and substituted prolines.

Isoleucine mimetics according to the present invention comprise in particular (N-Methyl)-I, allo-Ile, Cba, Nva, Abu, Leu, Cpg, cyclohexyl-Gly, (S)-2-Amino-3-ethyl-pentanoic acid, 3-Chloro-Phg, allo-Ile, Chg, Cyclobutylglycine, allo-Ile, Cbg, (2S,3S)-2-((Amino)methyl)-3-methylpentanoic acid, Phg, 2-[(1S,2S)-1-(Amino)-2-methylbutyl]-1,3-oxazole-4-carboxylic acid, 2-Methyl-D-alloisoleucine, Nva, Abu or Ala.

Leucine mimetics according to the present invention comprise in particular (tBu)A, (2-Chloro)F, (2-Bromo)F, AAD, (2S)-2-Amino-4,4,4-trifluorobutanoic acid, Cnba, (4-Fluoro)L, (S)-(trifluoromethyl)-L-cysteine, (2S)-2-amino-3-(1-methylcyclopropyl)propanoic acid, Gly(tBu), 3-(Trimethylsilyl)-L-alanine, 2,5-difluoro-L-phenylalanine, 2-Amino-7-(tert-butoxy)-7-oxoheptanoic acid, 5,5,5-Trifluoro-L-leucine ((Trifluoro)L), (2-Me)F, Cba, Cpa, cyclopropylmethylalanine, trifluoromethylalanine or difluoromethylalanine, (2-Fluoro)F, (2S)-3-(2,3-difluorophenyl)-2-aminopropanoic acid, (2S)-3-(3-Cyanophenyl)-2-aminopropanoic acid, 2-Amino-5,5,5-trifluoro-4-methyl-pentanoic acid, (2S)-2-Amino-5-methyl-hexanoic acid or (2S)-3-(indol-4-yl)-2-(amino)propanoic acid.

The invention further comprises analogues and derivatives of the described peptides. The term "analogue" or "derivative" of a peptide or an amino acid sequence according to the present invention comprises in particular any amino acid sequence having a sequence identity of at least 80% or at least 85%, preferably at least 90%, more preferably at least 95%, and even more preferably of at least 99% identity to said sequence, and same or comparable properties or activity. Sequence identity can be determined by common techniques, such as visual comparison or by means of any computer tool generally used in the field. Examples comprise BLAST programs used with default parameters.

An analogue or derivative of a peptide or an amino acid sequence of the invention may result from changes derived from mutation or variation in the sequences of peptides of the invention, including the deletion or insertion of one or more amino acids or the substitution of one or more amino acids, or even to alternative splicing. Several of these modifications may be combined. Preferably, an analogue of an amino acid sequence of the invention comprises conservative substitutions relative to the sequence of amino acids.

The term "conservative substitution" as used herein denotes that one or more amino acids are replaced by another, biologically similar residue. Examples include substitution of amino acid residues with similar characteristics, e.g., small amino acids, acidic amino acids, polar amino acids, basic amino acids, hydrophobic amino acids and aromatic amino acids. See, for example, the scheme in Table 4 below, wherein conservative substitutions of amino acids are grouped by physicochemical properties. I: neutral, hydrophilic; II: acids and amides; III: basic; IV: hydrophobic; V: aromatic, bulky amino acids, VI: neutral or hydrophobic; VII: acidic; VIII: polar.

**Table 4: Amino Acids grouped according to their physicochemical properties**

| **I** | **II** | **III** | **IV** | **V** | **VI** | **VII** | **VIII** |
|---|---|---|---|---|---|---|---|
| Ala | Asn | His | Met | Phe | Ala | Glu | Met |
| Ser | Asp | Arg | Leu | Tyr | Leu | Asp | Ser |
| Thr | Glu | Lys | Ile | Trp | Ile | | Thr |
| Pro | Gln | | Val | | Pro | | Cys |
| Gly | | | Cys | | Gly | | Asn |
| | | | | | Val | | Gln |

All peptides of this invention unless otherwise noted are TFA salts. The invention comprises further pharmaceutically acceptable salts of the peptides as defined herein and salt free forms. Therein, pharmaceutically acceptable salts represent salts or zwitterionic forms of the peptides or compounds of the present invention which are water or oil-soluble or dispersible, which are suitable for treatment of diseases without undue toxicity, irritation, and allergic response; which are commensurate with a reasonable benefit/risk ratio, and which are effective for their intended use. The salts can be prepared during the final isolation and purification of the compounds or separately by reacting an amino group with a suitable acid. Representative acid addition salts include acetate, adipate, alginate, citrate, aspartate, benzoate, benzenesulfonate, bisulfate, butyrate, camphorate, camphorsulfonate, carbonate, digluconate, glycerophosphate, hemisulfate, heptanoate, hexanoate, formate, fumarate, hydrochloride, hydrobromide, hydroiodide, 2-hydroxyethansulfonate (isethionate), lactate, maleate, mesitylenesulfonate, methanesulfonate, naphthylenesulfonate, nicotinate, 2- naphthalenesulfonate, oxalate, pamoate, pectinate, persulfate, 3-phenylproprionate, picrate, pivalate, propionate, succinate, sulfate, tartrate, trichloroacetate, trifluoroacetate, phosphate, glutamate, bicarbonate, para-toluenesulfonate, and undecanoate. Preferred acid addition salts include trifluoroacetate, formate, hydrochloride, and acetate.

Also, amino groups in the compounds of the present invention can be quaternized with methyl, ethyl, propyl, and butyl chlorides, bromides, and iodides; dimethyl, diethyl, dibutyl, and diamyl sulfates; decyl, lauryl, myristyl, and steryl chlorides, bromides, and iodides; and benzyl and phenethyl bromides. Examples of acids which can be employed to form therapeutically acceptable addition salts include inorganic acids such as hydrochloric, hydrobromic, sulfuric, and phosphoric, and organic acids such as oxalic, maleic, succinic, and citric. A pharmaceutically acceptable salt may suitably be a salt chosen, e.g., among acid addition salts and basic salts. Examples of acid addition salts include chloride salts, citrate salts and acetate salts.

Examples of basic salts include salts where the cation is selected from alkali metal cations, such as sodium or potassium ions, alkaline earth metal cations, such as calcium or magnesium ions, as well as substituted ammonium ions, such as ions of the type N(R¹)(R²)(R³)(R⁴)+, where R¹, R², R³ and R⁴ independently from each other will typically designate hydrogen, optionally substituted C₁₋₆-alkyl or optionally substituted C₂₋₆-alkenyl. Examples of relevant C₁₋₆-alkyl groups include methyl, ethyl, 1-propyl and 2-propyl groups. Examples of C₂₋₆-alkenyl groups of possible relevance include ethenyl, 1-propenyl and 2-propenyl. Therein, salts where the cation is selected among sodium, potassium and calcium are preferred.

Other examples of pharmaceutically acceptable salts are described in *"*Remington's Pharmaceutical Sciences ", 17th edition, Alfonso R. Gennaro (Ed.), Mark Publishing Company, Easton, PA, USA, 1985 *(and more recent editions thereof)*, *in the "*Encyclopaedia of Pharmaceutical Technology", 3rd edition, James Swarbrick (Ed.), Informa Healthcare USA (Inc.), NY, USA, 2007*, and in* J. Pharm. Sci. 66: 2 (1977*).* Also, for a review on suitable salts, see Handbook of Pharmaceutical Salts: Properties, Selection, and Use by Stahl and Wermuth (Wiley-VCH, 2002*).* Other suitable base salts are formed from bases which form non-toxic salts. Representative examples include the aluminum, arginine, benzathine, calcium, choline, diethylamine, diolamine, glycine, lysine, magnesium, meglumine, olamine, potassium, sodium, tromethamine, and zinc salts, preferably choline. Hemisalts of acids and bases may also be formed, e.g., hemisulphate and hemicalcium salts.

The invention further comprises solvates of the peptides as defined herein. Therein the term "solvate" refers to a complex of defined stoichiometry formed between a solute (e.g., a peptide according to the invention or pharmaceutically acceptable salt thereof) and a solvent. The solvent in this connection may, for example, be water, ethanol or another pharmaceutically acceptable, typically small-molecular organic species, such as, but not limited to, acetic acid or lactic acid. When the solvent in question is water, such a solvate is normally referred to as a hydrate.

The compounds according to the invention have useful pharmacological properties and can be used for prevention and treatment of disorders in humans and animals.

In the context of the present invention, the term "treatment" or "treat" includes the inhibition, delay, arrest, amelioration, attenuation, limitation, reduction, suppression, reversal or cure of a disease, a condition, a disorder, an injury or health impairment, of the development, course or the progression of such states and/or the symptoms of such states. Here, the term "therapy" is understood to be synonymous with the term "treatment".

In the context of the present invention, the terms "prevention", "prophylaxis" or "precaution" are used synonymously and refer to the avoidance or reduction of the risk to get, to contract, to suffer from or to have a disease, a condition, a disorder, an injury or a health impairment, a development or a progression of such states and/or the symptoms of such states.

The treatment or the prevention of a disease, a condition, a disorder, an injury or a health impairment may take place partially or completely.

The compounds according to the invention are particularly suitable for the treatment and/or prevention of cardiovascular, cardiopulmonary, renal, pulmonary, fibrotic, thromboembolic, and inflammatory disorders.

Accordingly, the compounds according to the invention can be used in medicaments for the treatment and/or prevention of cardiovascular and cardiopulmonary disorders and their sequels such as, for example inflammatory heart diseases, myocarditis, endocarditis, pericarditis, rheumatic fever without and with heart involvement, acute rheumatic pericarditis, acute rheumatic endocarditis, acute rheumatic myocarditis, chronic rheumatic heart diseases with and without endocarditis, valvulitis, pericarditis, ischemic heart diseases such as unstable angina pectoris and acute myocardial infarction, atrial and ventricular arrhythmias and impaired conduction such as, for example, grade I-III atrioventricular blocks, supraventricular tachyarrhythmia, atrial fibrillation, atrial flutter, ventricular fibrillation, ventricular flutter, ventricular tachyarrhythmia, Torsade de pointes tachycardia, atrial and ventricular extrasystoles, AV-junctional extrasystoles, sick sinus syndrome, stroke due to occlusion and stenosis of cerebral arteries (Cerebral infarction following e.g. thromboembolic, atherosclerotic, infectious and inflammatory vascular lesions), for the treatment and/or prevention of stroke due to intracerebral or intracranial haemorrhage, peripheral ischemic tissue damage (e.g. atherosclerotic gangrene) due to diseases of arteries, arterioles and capillaries (e.g. thromboembolic, atherosclerotic, infectious and inflammatory vascular lesions, endarteritis deformans or obliterans, and aneurysm dissection), phlebitis and thrombophlebitis, for preventing postprocedural disorders of the circulatory system, e.g. systemic inflammatory response syndrome, vasoplegia after surgery, postcardiotomy syndrome, postprocedural hypotension and heart failure, for preventing and treating ischemia reperfusion injury and organ dysfunction for example after thrombolysis therapies, percutaneous transluminal angioplasties (PTA), percutaneous transluminal coronary angioplasties (PTCA), bypass operations and heart, lung, liver and kidney transplants, and for the prevention and treatment of delayed graft function after kidney transplantation.

The compounds according to the invention are furthermore suited for the treatment of shock such as cardiogenic shock, septic shock and anaphylactic shock by preveting MASP mediated end organ damages.

Moreover, the compounds according to the invention have antiinflammatory action and can therefore be used as antiinflammatories for treatment and/or prevention of sepsis (SIRS), multiple organ failure (MODS, MOF), inflammatory disorders of the kidney, chronic bowel inflammations (IBD, Crohn's Disease, UC), pancreatitis, peritonitis, rheumatoid disorders, inflammatory skin disorders and inflammatory eye disorders.

By virtue of their activity profile, the compounds according to the invention are particularly suitable for the treatment and/or prevention of cardiovascular, pulmonary, cerebral and renal sequels of sepsis and systemic inflammatory response syndrome.

The compounds according to the invention are particularly suitable for the treatment and/or prevention of ischemia and/or reperfusion-related damage to the heart and the kidney and other organs in the context of resuscitation and surgical interventions such as but not restricted to bypass operations, heart valve surgery, and aortic aneurysm surgery,

The compounds according to the invention can additionally also be used for preventing ischaemic and/or reperfusion-related damage to organs or tissues and also as additives for perfusion and preservation solutions of organs, organ parts, tissues or tissue parts of human or animal origin, in particular for surgical interventions or in the field of transplantation medicine.

Furthermore, the compounds according to the invention are suitable for the treatment and/or prevention of diseases of the blood and blood-forming organs and the immune system including but not limited to acquired haemolytic anaemia, haemolytic-uraemic syndrome, paroxysmal nocturnal haemoglobinuria [Marchiafava-Micheli], coagulation defects, purpura and other haemorrhagic conditions, disseminated intravascular coagulation [defibrination syndrome], essential (haemorrhagic) thrombocythaemia, purpura fulminans, thrombotic thrombocytopenic purpura, allergic purpura, allergic vasculitis, lymphopenia and Igranulocytosis, and sarcoidosis.

Furthermore, the compounds according to the invention are suitable for the treatment and/or prevention of sequels of diabetes mellitus sucha as renal complications of diabetes mellitus, diabetic nephropathy, intracapillary glomerulonephrosis, ophthalmic complications of diabetes mellitus, diabetic retinopathy, neurological complications, diabetic polyneuropathy, and circulatory complications such as microangiopathy and gangrene.

Moreover, the compounds according to the invention are suitable for the treatment and/or prevention of inflammatory diseases of the nervous system such as multiple sclerosis, meningitis and encephalitis, bacterial and viral meningitis and encephalitis, postimmunization encephalitis, inflammatory polyneuropathy, and polyneuropathy in infectious and parasitic diseases.

The compounds according to the invention are furthermore suitable for the treatment and/or prevention of diseases of the eye and its adnexa, such as acute and subacute iridocyclitis, choroidal degeneration, chorioretinal inflammation, chorioretinal inflammation in infectious and parasitic diseases, background retinopathy and retinal vascular changes, proliferative retinopathy, degeneration of macula and posterior pole, peripheral retinal degeneration, age-related macular degeneration (AMD) including dry (non-exudative) and wet (exudative, neovascular) AMD, choroidal neovascularization (CNV), choroidal neovascular membranes (CNVM), cystoid macular oedema (CME), epiretinal membranes (ERM) and macular perforations, myopia-associated choroidal neovascularization, angioid and vascular streaks, retinal detachment, diabetic retinopathy, diabetic macular oedema (DME), atrophic and hypertrophic lesions in the retinal pigment epithelium, retinal vein occlusion, choroidal retinal vein occlusion, macular oedema, macular oedema associated with retinal vein occlusion, postprocedural disorders of eye and adnexa, e.g. keratopathy following cataract surgery.

Furthermore, the compounds according to the invention are suitable for the treatment and/or prevention of diseases of the respiratory system including but not restricted to viral, bacterial, and mycotic pneumonia, radiation pneumonitis, pneumoconiosis, allergic alveolitis, airway disease due to specific organic dust, e.g. farmer lung, bronchitis, pneumonitis and pulmonary oedema due to chemicals, gases, fumes and vapours, drug-induced interstitial lung disorders, adult respiratory distress syndrome (ARDS) and acute lung injury (ALI), acute oedema of the lung, interstitial pulmonary diseases with fibrosis, rheumatoid lung disease, respiratory disorders in other diffuse connective tissue disorders, such as associated to systemic lupus erythematosus, sclerodermia and Wegener granulomatosis.

Furthermore, the compounds according to the invention are suitable for treatment and/or prevention of microvascular injury, thrombosis and consecutive thromboembolic events caused by viral infections such as, but not restricted to, Influenza viruses (e.g. caused by strains of serotypes H1N1, H5N1, H7N9), and Corona viruses (e.g. SARS-CoV, the pathogen of *severe acute respiratory syndrome (SARS),* MERS-CoV, the pathogen of *Middle East respiratory syndrome (MERS),* and *SARS-CoV-2* the pathogen of *COVID-19 pandemic*).

Furthermore, the compounds according to the invention are suitable for the treatment and/or prevention of diseases of the digestive system including but not restricted to noninfective enteritis and colitis such as Crohn disease and ulcerative colitis, pancreatitis (including acute alcohol- and drug induced pancreatitis), cholecystitis, inflammatory liver diseases, hepatorenal syndrome, postprocedural disorders of the liver, e.g. after liver surgery.

By virtue of their activity profile, the compounds according to the invention are particularly suitable for the treatment and/or prevention of diseases of the genitourinary system including but not restricted to acute renal failure, acute kidney injury (AKI), surgery associated AKI, sepsis associated AKI, contrast media and chemotherapy induced AKI, ischaemia and infarction of the kidney, complications such as hypersensitivity in the context of hemodialysis and hemodiafiltration, cystitis, irradiation cystitis, inflammatory diseases of the prostate, and endometriosis.

The compounds according to the invention are furthermore suitable for the treatment and/or prevention of sequels of burns and injuries including but not restricted to early complications of trauma, traumatic anuria, crush syndrome, renal failure following crushing, traumatic ischaemia of muscle, traumatic brain injury, organ damage after exposure to electric current, radiation and extreme ambient air temperature and pressure, after exposure to smoke, fire and flames, after contact with venomous animals and plants.

By virtue of their activity profile, the compounds according to the invention are furthermore suitable for the treatment of inflammatory skin diseases for example dermal lupus erythematosus, bullous disorders and acantholytic skin diseases such as pemphigus subtypes, papulosquamous disorders such as psoriasis, dermatitis and eczema, urticaria and erythema.

According to a further embodiment, the invention provides a bicyclic compound which may be isolated and/or purified, comprising, essentially consisting of, or consisting of formula (I) or a derivative, analogue or pharmaceutically acceptable salts or solvates thereof for the use in the prophylaxis and treatment of diseases.

According to a further embodiment, the invention provides a bicyclic compound which may be isolated and/or purified, comprising, essentially consisting of, or consisting of formula (I) or a derivative, analogue or pharmaceutically acceptable salts or solvates thereof for the use in the prophylaxis and treatment of MASP-associated disorders.

According to a further embodiment, the invention provides a bicyclic compound which may be isolated and/or purified, comprising, essentially consisting of, or consisting of, formula (I) or a derivative, analogue or pharmaceutically acceptable salt, solvate or solvate of the salt, which acts as a MASP-1 and/or MASP-2 inhibitor and/or which inhibits C3 deposition, for the use in the prophylaxis and treatment of MASP-associated disorders.

According to a further embodiment, the invention provides a bicyclic compound which may be isolated and/or purified, comprising, essentially consisting of, or consisting of, formula (I) or a derivative, analogue or pharmaceutically acceptable salts or solvates thereof for the use in the prophylaxis and treatment of cardiovascular and cardiopulmonary disorders, shock, inflammatory disorders, cardiovascular, pulmonary, cerebral and renal sequels of sepsis, ischemia and/or reperfusion-related damage, acute kidney injury, transplant protection and delayed graft function, diseases of the blood and blood-forming organs and the immune system, sequels of diabetes mellitus, inflammatory diseases of the nervous system, diseases of the eye, diseases of the skin, diseases of the respiratory, digestive or genitourinary system and sequels of burns and injuries.

According to a further embodiment, the invention provides a bicyclic compound which may be isolated and/or purified, comprising, essentially consisting of, or consisting of, formula (I) or a derivative, analogue or pharmaceutically acceptable salts or solvates thereof for the use in the prophylaxis and treatment of diseases of the genitourinary system including but not restricted to acute renal failure, acute kidney injury (AKI), surgery associated AKI, sepsis associated AKI, contrast media and chemotherapy induced AKI, ischaemia and infarction of the kidney, complications such as hypersensitivity in the context of hemodialysis and hemodiafiltration, cystitis, irradiation cystitis, inflammatory diseases of the prostate, and endometriosis.

The invention further relates to a method of treating or ameliorating MASP-associated disorders, as defined above, in a subject or patient by administering at least one peptide, derivative or analogue as defined herein or a pharmaceutically acceptable salt or solvate thereof, a complex or a pharmaceutical composition as defined above, to said subject or patient in need thereof.

As used herein, the terms "patient", "subject" or "individual" may be used interchangeably and refer to either a human or a non-human animal. These terms include mammals such as humans, primates, livestock animals (e.g., bovines, porcines), companion animals (e.g., canines, felines) and rodents (e.g., mice and rats). The term "mammal" refers to any mammalian species such as a human, mouse, rat, dog, cat, hamster, guinea pig, rabbit, livestock, and the like.

According to the invention the at least one peptide, derivative or analogue as defined herein or the pharmaceutically acceptable salt or solvate thereof, or the complex as defined above is administered to a patient or subject in a therapeutically effective amount, wherein a "therapeutically effective amount" of a compound of the present invention is meant to describe a sufficient amount of a compound of the present invention to treat an MASP-associated disorder as defined herein. In particular the therapeutically effective amount will achieve a desired benefit/risk ratio applicable to any medical treatment.

A bicyclic compound or derivative or analogue thereof as defined herein or the pharmaceutically acceptable salt or solvate thereof or the complex or the pharmaceutical composition (as defined below), are hereinafter commonly also referred to as "MASP inhibitory peptide of the present invention".

In some embodiments, a MASP inhibitory peptide of the present invention binds to MASP-1 and/or MASP-2, e.g. human MASP-1 and/or MASP-2. In certain embodiments, a MASP inhibitory peptide of the present invention specifically binds to human MASP-1 and/or MASP-2. As used herein, "specifically binds" refers to a specific binding agent's preferential interaction with a given ligand over other agents in a sample. For example, a specific binding agent that specifically binds a given ligand binds the given ligand, under suitable conditions, in an amount or a degree that is observable over that of any nonspecific interaction with other components in the sample. Suitable conditions are those that allow interaction between a given specific binding agent and a given ligand. These conditions include pH, temperature, concentration, solvent, time of incubation, and the like, and may differ among given specific binding agent and ligand pairs, but may be readily determined by those skilled in the art. In some embodiments, a MASP inhibitory peptide of the present invention binds MASP-1 and/or MASP-2 with greater specificity than a MASP inhibitory peptide reference compound (e.g. any one of the MASP inhibitory peptide reference compounds provided herein).

The invention thus further relates to a complex comprising at least one bicyclic compound, derivative or analogue as defined herein bound to MASP-1 or MASP-2.

In some embodiments, a MASP inhibitory peptide of the present invention exhibits specific binding to MASP-1 and/or MASP-2, especially human MASP-1 and/or MASP-2, that is at least about 10%, 20%, 30%, 40%, 50%, 60%, 70%, 80%, 90%, 100%, 200%, 300%, 400%, 500%, 700%, 1000%, or 10,000% higher than a selected MASP inhibitory peptide reference compound.

In some embodiments, a MASP inhibitory peptide of the present invention exhibits specific binding to MASP-1 and/or MASP-2, especially human MASP-1 and/or MASP-2, that is at least about 1, 2, 3, 4, 5 fold, or at least about 10, 20, 50, or 100 fold higher than a selected MASP inhibitory peptide reference compound.

In some embodiments, a MASP inhibitory peptide of the present invention exhibits a binding affinity to MASP-1 and/or MASP-2 that is at least about 10%, 20%, 30%, 40%, 50%, 60%, 70%, 80%, 90%, 100%, 200%, 300%, 400%, 500%, 700%, 1000%, or 10,000% higher than a selected MASP inhibitory peptide reference compound.

In some embodiments, a MASP inhibitory peptide of the present invention exhibits a binding affinity to MASP-1 and/or MASP-2 that is at least about 1, 2, 3, 4, 5 fold, or at least about 10, 20, 50, 100 or 1000 fold higher than a selected MASP inhibitory peptide reference compound.

In some embodiments, a MASP inhibitory peptide of the present invention exhibits an inhibition of MASP-1 and/or MASP-2 (e.g., rat or human MASP-1 and/or MASP-2) activity. In some embodiments, the activity is an *in vitro* or an *in vivo* activity, e.g. an *in vitro* or *in vivo* activity described herein. In some embodiments, a MASP inhibitory peptide of the present invention inhibits at least about 10%, 20%, 30%, 40%, 50%, 60%, 70%, 80%, 90%, 100%, 200%, 300%, 400%, 500%, 700%, 1000%, or 10,000% of the MASP-1 and/or MASP-2 activity inhibited by a selected MASP inhibitory peptide reference compound.

In certain embodiments, the MASP inhibitory peptide of the present invention exhibits 1.5, 2, 3, 4, 5, 6, 7, 8, 9, 10, 11, 12, 13, 14, 15, 16, 17, 18, 19, 20, 30, 40, 50, 60, 70, 80, 90, 100, 120, 140, 160, 180, or 200-fold greater MASP-1 and/or MASP-2 inhibition than a selected MASP inhibitory peptide reference compound. In further particular embodiments, the MASP-1 and/or MASP-2 inhibitory activity of the MASP inhibitory peptides according to the present invention is determined by measurement of their IC₅₀ for MASP-1 and/or MASP-2 (e.g., rat human MASP-1 and/or MASP-2). Determination of the IC₅₀ for MASP-1 and/or MASP-2 can be done with the biochemical assays shown herein. It is particularly preferred that a MASP inhibitory peptide of the present invention exhibits an IC₅₀ for MASP-1 and/or MASP-2 of < 1,000 nM, preferably ≤ 500 nM, more preferably ≤ 300 nM, more preferably ≤ 250 nM, more preferably ≤ 200 nM, more preferably ≤ 150 nM, more preferably ≤ 100 nM, more preferably ≤ 75 nM, more preferably ≤ 50 nM, more preferably ≤ 45 nM, more preferably ≤ 40nM, more preferably ≤ 35nM, more preferably ≤ 30 nM.

In some embodiments, a MASP inhibitory peptide of the present invention has a lower IC₅₀ (i.e. higher binding affinity) for MASP-1 and/or MASP-2, (e.g., rat or human MASP-1 and/or MASP-2) compared to a selected MASP inhibitory peptide reference compound. In some embodiments, a MASP inhibitory peptide according to the present invention has an IC₅₀ in a MASP-1 and/or MASP-2 competitive binding assay which is at least about 5%, 10%, 20%, 30%, 40%, 50%, 60%, 70%, 80%, 90%, 100%, 200%, 300%, 400%, 500%, 700%, 1000% or 10,000% lower than that of a selected MASP inhibitory peptide reference compound.

In some embodiments, a MASP inhibitory peptide of the present invention exhibits at least about 5%, 10%, 20%, 30%, 40%, 50%, 60%, 70%, 80%, 90%, 95%, 97%, 98%, 99%, or greater than 99%, 100%, 200% 300%, 400%, 500%, 700%, 1000% or 10,000% greater *in vitro* inhibition of human MASP-1 and/or MASP-2 activity as that of a selected MASP inhibitory peptide reference compound.

In some embodiments, a MASP inhibitory peptide of the present invention exhibits at least about 5%, 10%, 20%, 30%, 40%, 50%, 60%, 70%, 80%, 90%, 95%, 97%, 98%, 99%, or greater than 99%, 100%, 200% 300%, 400%, 500%, 700%, 1000% or 10,000% greater *in vivo* inhibition of human MASP-1 and/or MASP-2 activity as that of a selected MASP inhibitory peptide reference compound.

As used herein, in certain embodiments, a MASP inhibitory peptide having a "MASP-1 and/or MASP-2 inhibitory activity" means that the compound has the ability to inhibit C3 deposition *in vitro* or in subjects (e.g. mice or humans), when administered thereto (e.g. by the parenteral route, e.g. by injection, or by the pulmonary, nasal, sublingual, lingual, buccal, dermal, transdermal, conjunctival, optic route or as implant or stent orally administered), in a dose-dependent and time-dependent manner.

In some embodiments, a MASP inhibitory peptide of the present invention exhibits an inhibition of C3 deposition (e.g., human C3 deposition. In some embodiments, the inhibition of C3 deposition is mdetermined by an *in vitro* or an *in vivo* inhibition. In some embodiments, a MASP inhibitory peptide of the present invention inhibits at least about 10%, 20%, 30%, 40%, 50%, 60%, 70%, 80%, 90%, 100%, 200%, 300%, 400%, 500%, 700%, 1000%, or 10,000% of the C3 deposition inhibited by a selected MASP inhibitory peptide reference compound.

In certain embodiments, the MASP inhibitory peptide of the present invention exhibits 1.5, 2, 3, 4, 5, 6, 7, 8, 9, 10, 11, 12, 13, 14, 15, 16, 17, 18, 19, 20, 30, 40, 50, 60, 70, 80, 90, 100, 120, 140, 160, 180, or 200-fold greater inhibition of C3 deposition than a selected MASP inhibitory peptide reference compound.

In further particular embodiments, the MASP-1 and/or MASP-2 inhibitory activity of the MASP inhibitory peptides according to the present invention is determined by measurement of their IC₅₀ for inhibition of C3 deposition *in vitro* or in subjects (e.g. mice or humans). Determination of the IC₅₀ for C3-deposition can be done with the C3 Human Deposition assay shown herein. It is particularly preferred that a MASP inhibitory peptide of the present invention exhibits an IC₅₀ for C3 deposition of < 1,000 nM, preferably ≤ 500 nM, more preferably ≤ 300 nM, more preferably ≤ 250 nM, more preferably ≤ 200 nM, more preferably ≤ 150 nM, more preferably ≤ 100 nM, more preferably ≤ 75 nM, more preferably ≤ 50 nM, more preferably ≤ 45 nM, more preferably ≤ 40nM, more preferably ≤ 35nM, more preferably ≤ 30 nM.

In some embodiments, a MASP inhibitory peptide of the present invention exhibits at least about 5%, 10%, 20%, 30%, 40%, 50%, 60%, 70%, 80%, 90%, 95%, 97%, 98%, 99%, or greater than 99%, 100%, 200% 300%, 400%, 500%, 700%, 1000% or 10,000% greater *in vitro* inhibition of C3-deposition as that of a selected MASP inhibitory peptide reference compound.

In some embodiments, a MASP inhibitory peptide of the present invention exhibits at least about 5%, 10%, 20%, 30%, 40%, 50%, 60%, 70%, 80%, 90%, 95%, 97%, 98%, 99%, or greater than 99%, 100%, 200% 300%, 400%, 500%, 700%, 1000% or 10,000% greater *in vivo* inhibition of C3-deposition as that of a selected MASP inhibitory peptide reference compound.

It is particularly preferred that a peptide according to the present invention acts as a MASP inhibitory peptide with its activity being determined in accordance with at least one of the specific assays and/or the *in vivo* studies according to the examples of the present invention.

Due to their aforesaid MASP-1 and/or MASP-2 inhibitory activity and inhibitory activity of C3-deposition, a compound containing the peptide or the peptide of the present invention (including analogues, derivatives, and pharmaceutically acceptable salts or solvates thereof as well as the above mentioned complex) are suitable for the use in in the prophylaxis and treatment of MASP-1 and/or MASP-2-associated disorders.

According to a further embodiment, the invention provides a bicyclic compound which may be isolated and/or purified, comprising, essentially consisting of, or consisting of formula (I) or a derivative, analogue, pharmaceutically acceptable salt, solvate or solvate of the salt, which acts as a MASP-1 and/or MASP-2 inhibitor and/or which inhibits C3 deposition.

The compounds according to the invention can be used alone or in combination with other active compounds if necessary. The present invention further relates to medicaments containing at least one of the compounds according to the invention and one or more further active compounds, in particular for the treatment and/or prophylaxis of the aforementioned diseases. As suitable combination active compounds, we may mention for example and preferably:
- compounds that inhibit the degradation of cyclic guanosine monophosphate (cGMP) and/or cyclic adenosine monophosphate (cAMP), for example inhibitors of phosphodiesterases (PDE) 1, 2, 3, 4 and/or 5, in particular PDE 4 inhibitors such as roflumilast or revamilast and PDE 5 inhibitors such as sildenafil, vardenafil, tadalafil, udenafil, dasantafil, avanafil, mirodenafil or lodenafil;
- NO-independent but haem-dependent stimulators of guanylate cyclase, in particular riociguat, nelociguat, vericiguat and the compounds described in WO 00/06568, WO 00/06569, WO 02/42301, WO 03/095451, WO 2011/147809, WO 2012/004258, WO 2012/028647 and WO 2012/059549;
- prostacyclin analogs and IP receptor agonists, for example and preferably iloprost, beraprost, treprostinil, epoprostenol, NS-304, selexipag, or ralinepag;
- endothelin receptor antagonists, for example and preferably bosentan, darusentan, ambrisentan, macicentan or sitaxsentan;
- vasopressin receptor antagonists, for example tolvaptan, conivaptan, relcovaptan
- human neutrophile elastase (HNE) inhibitors, for example and preferably sivelestat or DX-890 (Reltran);
- compounds which inhibit the signal transduction cascade, in particular from the group of the tyrosine kinase inhibitors, for example and preferably dasatinib, nilotinib, bosutinib, regorafenib, sorafenib, sunitinib, cediranib, axitinib, telatinib, imatinib, brivanib, pazopanib, vatalanib, gefitinib, erlotinib, lapatinib, canertinib, lestaurtinib, pelitinib, semaxanib, masitinib, or tandutinib;
- signal transductuion modulators from the group of ASK1 kinase inhibitors, for example selonsertib;
- Rho kinase inhibitors, for example and preferably fasudil, Y-27632, SLx-2119, BF-66851, BF-66852, BF-66853, KI-23095 or BA-1049;
- active ingredients which reduce vascular wall permeability (oedema formation), by way of example and with preference inhibitors of the ALK1-Smad1/5 signalling pathway, inhibitors of the VEGF and/or PDGF signalling pathways, cyclooxygenase inhibitors, inhibitors of the kallikrein-kinin system or inhibitors of the sphingosine-1-phosphate signalling pathways; and/or
- corticosteroids, for example cortisone, cortisol, prednisolone, methylprednisolone, triamcinolone or dexamethasone;
- active ingredients which reduce damage to organs under oxidative stress, by way of example and with preference inhibitors of the complement system, especially antagonists of the complement C5a receptor, anti C5 antibodies or agonists of the 5-HT1A receptor;
- modulators, stimulators and enhancers of the transcription factor Nrf2, for example CXA-10, Oltipraz, dimethyl fumarate or Bardoxolone;
- adrenomedullin and adrenomedullin derivatives, for example pegylated adrenomedullin, and adrenomedullin stabilizing agents, for example adrecizumab;
- compounds which inhibit hypoxia inducible factor prolyl hydroxylase (HIF-PH inhibitors), for example molidustat, vadadustat, roxadustat, daprodustat or desidustat;
- compounds which inhibit induction of cell death and apoptosis pathway, for example QPI-1002;
- C-Met agonists and hepatocyte growth factor mimetics, for example refanalin;
- alkaline phosphatase and recombinant alkaline phosphatase;
- compounds which inhibit inflammatory response and T cell proliferation, for example CD28 antagonistic compounds such as Reltecimod;
- compounds which modulate the activation of Th17 T cells, for example modulators of the RORc/ROR-gamma transcription factor;
- compounds antagonizing the Th17 T cell response for example anti IL-17 and anti IL-23 antibodies, for example Ixekizumab, Secukinumab, Brodalumab, Ustekinumab, Guselkumab or PTG-200;
- antithrombotic agents, for example and preferably from the group of platelet aggregation inhibitors, anticoagulants or profibrinolytic substances;
- In a preferred embodiment of the invention, the compounds according to the invention are administered in combination with a platelet aggregation inhibitor, for example and preferably aspirin, clopidogrel, ticlopidine or dipyridamole.
- In a preferred embodiment of the invention, the compounds according to the invention are administered in combination with a thrombin inhibitor, for example and preferably ximelagatran, melagatran, dabigatran, bivalirudin or Clexane.
- In a preferred embodiment of the invention, the compounds according to the invention are administered in combination with a GPIIb/IIIa antagonist, for example and preferably tirofiban or abciximab.
- In a preferred embodiment of the invention, the compounds according to the invention are administered in combination with a factor Xa inhibitor, for example and preferably rivaroxaban, apixaban, fidexaban, razaxaban, fondaparinux, idraparinux, DU-176b, PMD-3112, YM-150, KFA-1982, EMD-503982, MCM-17, MLN-1021, DX 9065a, DPC 906, JTV 803, SSR-126512 or SSR-128428.
- In a preferred embodiment of the invention, the compounds according to the invention are administered in combination with heparin or a low molecular weight (LMW) heparin derivative.
- In a preferred embodiment of the invention, the compounds according to the invention are administered in combination with direct inhibitors of coagulation factor XI, inhibitors of coagulation factor XI expression, and anti-coagulation factor XI antibodies such as Xisomab 3G3;
- In a preferred embodiment of the invention, the compounds according to the invention are administered in combination with a mineralocorticoid-receptor antagonist, for example and preferably spironolactone, eplerenone or finerenone.
- In a preferred embodiment of the invention, the compounds according to the invention are administered in combination with a diuretic, for example and preferably furosemide, bumetanide, Torsemide, bendroflumethiazide, chlorthiazide, hydrochlorthiazide, hydroflumethiazide, methyclothiazide, polythiazide, trichlormethiazide, chlorthalidone, indapamide, metolazone, quinethazone, acetazolamide, dichlorphenamide, methazolamide, glycerol, isosorbide, mannitol, amiloride or triamterene.
- In a preferred embodiment of the invention, the compounds according to the invention are administered in combination with a PPAR-gamma agonist, for example and preferably pioglitazone or rosiglitazone.
- In a preferred embodiment of the invention, the compounds according to the invention are administered in combination with a PPAR-delta agonist, for example and preferably GW 501516 or BAY 68-5042.

According to a further embodiment, the invention provides a pharmaceutical composition comprising at least one bicyclic compound which may be isolated and/or purified, comprising, essentially consisting of, or consisting of formula (I) or a derivative, analogue, pharmaceutically acceptable salt, solvate or solvate of the salt, in combination with one or more further active ingredients selected from the group consisting of inhibitors of phosphodiesterases, stimulators or activators of guanylate cyclase, IP receptor agonists, mineralocorticoid-receptor antagonist, diuretic, PPAR-gamma agonist, PPAR-delta agonist, corticosteroids, active ingredients which reduce damage to organs under oxidative stress, compounds which inhibit induction of cell death and apoptosis pathway, compounds which inhibit inflammatory response and T cell proliferation, antithrombotic agents, platelet aggregation inhibitor, thrombin inhibitor, GPIIb/IIIa antagonist, factor Xa inhibitor, heparin or a low molecular weight (LMW) heparin derivative and inhibitors of coagulation factor XI.

The invention further relates to a kit-of-parts combination comprising at least one peptide, derivative or analogue as defined herein or a pharmaceutically acceptable salt or solvate thereof, a complex or a pharmaceutical composition as defined above, and at least one selected from a reagent, medical device, instruction letter or any combination thereof.

The invention further relates to a medical device comprising at least one peptide, derivative or analogue as defined herein or a pharmaceutically acceptable salt or solvate thereof, a complex or a pharmaceutical composition as defined above, for delivery of the peptide, derivative, analogue or complex thereof or of the pharmaceutical composition to a subject.

The pharmaceutical composition, kit-of-parts combination or medical device as defined above is in particular for the use in the prophylaxis or treatment of the disorders or diseases as defined as defined herein.

It is possible for the MASP inhibitory peptide of the present invention to have systemic and/or local activity. For this purpose, they can be administered in a suitable manner, such as, for example, via the oral, parenteral, pulmonary, nasal, sublingual, lingual, buccal, rectal, vaginal, dermal, transdermal, conjunctival, otic route or as an implant or stent.

For these administration routes, it is possible for the compounds according to the invention to be administered in suitable administration forms.

For oral administration, it is possible to formulate the compounds according to the invention to dosage forms known in the art that deliver the compounds of the invention rapidly and/or in a modified manner, such as, for example, tablets (uncoated or coated tablets, for example with enteric or controlled release coatings that dissolve with a delay or are insoluble), orally-disintegrating tablets, films/wafers, films/lyophylisates, capsules (for example hard or soft gelatine capsules), sugar-coated tablets, granules, pellets, powders, emulsions, suspensions, aerosols or solutions. It is possible to incorporate the compounds according to the invention in crystalline and/or amorphised and/or dissolved form into said dosage forms.

Parenteral administration can be effected with avoidance of an absorption step (for example intravenous, intraarterial, intracardial, intraspinal or intralumbal) or with inclusion of absorption (for example intramuscular, subcutaneous, intracutaneous, percutaneous or intraperitoneal, intraocular). Administration forms which are suitable for parenteral administration are, inter alia, preparations for injection and infusion in the form of solutions, suspensions, emulsions, lyophylisates or sterile powders.

Examples which are suitable for other administration routes are pharmaceutical forms for inhalation [inter alia powder inhalers, nebulizers], nasal drops, nasal solutions, nasal sprays; tablets/films/wafers/capsules for lingual, sublingual or buccal administration; suppositories; eye drops, eye ointments, eye baths, ocular inserts, ear drops, ear sprays, ear powders, ear-rinses, ear tampons; vaginal capsules, topical application, aqueous suspensions (lotions, mixturae agitandae), lipophilic suspensions, emulsions, ointments, creams, transdermal therapeutic systems (such as, for example, patches), milk, pastes, foams, dusting powders, implants or stents.

According to a further embodiment, the invention provides a pharmaceutical composition comprising at least one bicyclic compound which may be isolated and/or purified, comprising, essentially consisting of, or consisting of formula (I) or a derivative, analogue, pharmaceutically acceptable salt, solvate or solvate of the salt, in combination with one or more inert, nontoxic, pharmaceutically suitable excipients.

The compounds according to the invention can be incorporated into the stated administration forms. This can be effected in a manner known per se by mixing with pharmaceutically suitable excipients. Pharmaceutically suitable excipients include, inter alia,
- fillers and carriers (for example cellulose, microcrystalline cellulose (such as, for example, Avicel^{®}), lactose, mannitol, starch, calcium phosphate (such as, for example, Di-Cafos^{®})),
- ointment bases (for example petroleum jelly, paraffins, triglycerides, waxes, wool wax, wool wax alcohols, lanolin, hydrophilic ointment, polyethylene glycols),
- bases for suppositories (for example polyethylene glycols, cacao butter, hard fat),
- solvents (for example water, ethanol, isopropanol, glycerol, propylene glycol, medium chain-length triglycerides fatty oils, liquid polyethylene glycols, paraffins),
- surfactants, emulsifiers, dispersants or wetters (for example sodium dodecyl sulfate), lecithin, phospholipids, fatty alcohols (such as, for example, Lanette^{®}), sorbitan fatty acid esters (such as, for example, Span^{®}), polyoxyethylene sorbitan fatty acid esters (such as, for example, Tween^{®}), polyoxyethylene fatty acid glycerides (such as, for example, Cremophor^{®}), polyoxethylene fatty acid esters, polyoxyethylene fatty alcohol ethers, glycerol fatty acid esters, poloxamers (such as, for example, Pluronic^{®}),
- buffers, acids and bases (for example phosphates, carbonates, citric acid, acetic acid, hydrochloric acid, sodium hydroxide solution, ammonium carbonate, trometamol, triethanolamine),
- isotonicity agents (for example glucose, sodium chloride),
- adsorbents (for example highly-disperse silicas),
- viscosity-increasing agents, gel formers, thickeners and/or binders (for example polyvinylpyrrolidone, methylcellulose, hydroxypropylmethylcellulose, hydroxypropylcellulose, carboxymethylcellulose-sodium, starch, carbomers, polyacrylic acids (such as, for example, Carbopol^{®}); alginates, gelatine),
- disintegrants (for example modified starch, carboxymethylcellulose-sodium, sodium starch glycolate (such as, for example, Explotab^{®}), cross- linked polyvinylpyrrolidone, croscarmellose-sodium (such as, for example, AcDiSol^{®})),
- flow regulators, lubricants, glidants and mould release agents (for example magnesium stearate, stearic acid, talc, highly-disperse silicas (such as, for example, Aerosil^{®})),
- coating materials (for example sugar, shellac) and film formers for films or diffusion membranes which dissolve rapidly or in a modified manner (for example polyvinylpyrrolidones (such as, for example, Kollidon^{®}), polyvinyl alcohol, hydroxypropylmethylcellulose, hydroxypropylcellulose, ethylcellulose, hydroxypropylmethylcellulose phthalate, cellulose acetate, cellulose acetate phthalate, polyacrylates, polymethacrylates such as, for example, Eudragit^{®})),
- capsule materials (for example gelatine, hydroxypropylmethylcellulose),
- synthetic polymers (for example polylactides, polyglycolides, polyacrylates, polymethacrylates (such as, for example, Eudragit^{®}), polyvinylpyrrolidones (such as, for example, Kollidon^{®}), polyvinyl alcohols, polyvinyl acetates, polyethylene oxides, polyethylene glycols and their copolymers and blockcopolymers),
- plasticizers (for example polyethylene glycols, propylene glycol, glycerol, triacetine, triacetyl citrate, dibutyl phthalate),
- penetration enhancers,
- stabilisers (for example antioxidants such as, for example, ascorbic acid, ascorbyl palmitate, sodium ascorbate, butylhydroxyanisole, butylhydroxytoluene, propyl gallate),
- preservatives (for example parabens, sorbic acid, thiomersal, benzalkonium chloride, chlorhexidine acetate, sodium benzoate),
- colourants (for example inorganic pigments such as, for example, iron oxides, titanium dioxide),
- flavourings, sweeteners, flavour- and/or odour-masking agents.

The present invention furthermore relates to a pharmaceutical composition comprising at least one peptide, derivative or analogue as defined herein or a pharmaceutically acceptable salt or solvate thereof or a complex as defined above.

In particular, the present invention relates to a pharmaceutical composition comprising at least one peptide, derivative or analogue as defined herein or a pharmaceutically acceptable salt or solvate thereof or a complex as defined above, conventionally together with one or more pharmaceutically suitable excipient(s), and to their use according to the present invention.

A pharmaceutical composition according to the present invention may comprise at least one additional active ingredient, such as preferably an additional active ingredient which is active in the prophylaxis or treatment of the disorders or diseases as defined herein.

The at least one peptide, derivative or analogue as defined herein or the pharmaceutically acceptable salt or solvate thereof or the complex or the pharmaceutical compositions as defined above may be administered enterally or parenterally, including intravenous, intramuscular, intraperitoneal, intrasternal, subcutaneous, intradermal and intraarticular injection and infusion, orally, intravaginally, intraperitoneally, intrarectally, topically or buccally. Suitable formulations for the respective administration routes are well known to a skilled person and include, without being limited thereto: pills, tablets, enteric-coated tablets, film tablets, layer tablets, sustained-release or extended-release formulations for oral administration, plasters, topical extended-release formulations, dragees, pessaries, gels, ointments, syrup, granules, suppositories, emulsions, dispersions, microcapsules, microformulations, nanoformulations, liposomal formulations, capsules, enteric-coated capsules, powders, inhalation powders, microcrystalline formulations, inhalation sprays, powders, drops, nose drops, nasal sprays, aerosols, ampoules, solutions, juices, suspensions, infusion solutions or injection solutions, etc.

According to a further embodiment, the invention provides a pharmaceutical composition comprising at least one bicyclic compound which may be isolated and/or purified, comprising, essentially consisting of, or consisting of formula (I) or a derivative, analogue, pharmaceutically acceptable salt, solvate or solvate of the salt, in combination with one or more inert, nontoxic, pharmaceutically suitable excipients for the use in the prophylaxis and treatment of cardiovascular and cardiopulmonary disorders, shock, inflammatory disorders, cardiovascular, pulmonary, cerebral and renal sequels of sepsis, ischemia and/or reperfusion-related damage, acute kidney injury, transplant protection and delayed graft function, diseases of the blood and blood-forming organs and the immune system, sequels of diabetes mellitus, inflammatory diseases of the nervous system, diseases of the eye, diseases of the skin, diseases of the respiratory, digestive or genitourinary system and sequels of burns and injuries.

According to a further embodiment, the invention provides a pharmaceutical composition comprising at least one bicyclic compound which may be isolated and/or purified, comprising, essentially consisting of, or consisting of formula (I) or a derivative, analogue, pharmaceutically acceptable salt, solvate or solvate of the salt, in combination with one or more further active ingredients selected from the group consisting of inhibitors of phosphodiesterases, stimulators or activators of guanylate cyclase, IP receptor agonists, mineralocorticoid-receptor antagonist, diuretic, PPAR-gamma agonist, PPAR-delta agonist, corticosteroids, active ingredients which reduce damage to organs under oxidative stress, compounds which inhibit induction of cell death and apoptosis pathway, compounds which inhibit inflammatory response and T cell proliferation, antithrombotic agents, platelet aggregation inhibitor, thrombin inhibitor, GPIIb/IIIa antagonist, factor Xa inhibitor, heparin or a low molecular weight (LMW) heparin derivative and inhibitors of coagulation factor XI for the use in the prophylaxis and treatment of cardiovascular and cardiopulmonary disorders, shock, inflammatory disorders, cardiovascular, pulmonary, cerebral and renal sequels of sepsis, ischemia and/or reperfusion-related damage, acute kidney injury, transplant protection and delayed graft function, diseases of the blood and blood-forming organs and the immune system, sequels of diabetes mellitus, inflammatory diseases of the nervous system, diseases of the eye, diseases of the skin, diseases of the respiratory, digestive or genitourinary system and sequels of burns and injuries.

According to a further embodiment, the invention provides a method for treatment and/or prevention of of cardiovascular and cardiopulmonary disorders, shock, inflammatory disorders, cardiovascular, pulmonary, cerebral and renal sequels of sepsis, ischemia and/or reperfusion-related damage, acute kidney injury, transplant protection and delayed graft function, diseases of the blood and blood-forming organs and the immune system, sequels of diabetes mellitus, inflammatory diseases of the nervous system, diseases of the eye, diseases of the skin, diseases of the respiratory, digestive or genitourinary system and sequels of burns and injuries in humans and animals by administration of an effective amount of a pharmaceutical composition comprising at least one bicyclic compound which may be isolated and/or purified, comprising, essentially consisting of, or consisting of formula (I) or a derivative, analogue, pharmaceutically acceptable salt, solvate or solvate of the salt, or of a pharmaceutical composition comprising at least one bicyclic compound which may be isolated and/or purified, comprising, essentially consisting of, or consisting of formula (I) or a derivative, analogue, pharmaceutically acceptable salt, solvate or solvate of the salt, in combination with one or more inert, nontoxic, pharmaceutically suitable excipients and/or one or more further active ingredients.

The suitable dosage of the MASP inhibitory peptide of the present invention can be decided by the attending physician within the scope of sound medical judgment. The specific therapeutically effective dose level for any particular subject will depend upon a variety of factors including: a) the disorder being treated and the severity of the disorder; b) activity of the specific compound employed; c) the specific composition employed, the age, body weight, general health, sex and diet of the patient; d) the time of administration, route of administration, and rate of excretion of the specific hepcidin analogue employed; e) the duration of the treatment; f) drugs used in combination or coincidental with the MASP inhibitory peptide employed, and like factors well known in the medical arts.

In particular embodiments, the total daily dose of the MASP inhibitory peptide of the invention to be administered to a subject or patient in single or divided doses may be in amounts, for example, from 0.0001 to 300 mg/kg body weight daily or 1 to 300 mg/kg body weight daily, or from about 0.0001 to about 100 mg/kg body weight per day, such as from about 0.0005 to about 50 mg/kg body weight per day, such as from about 0.001 to about 10 mg/kg body weight per day, e.g. from about 0.01 to about 1 mg/kg body weight per day, administered in one or more doses, such as from one to three doses. Generally, the MASP inhibitory peptide of the invention may be administered continuously (e.g. by intravenous administration or another continuous drug administration method), or may be administered to a subject at intervals, typically at regular time intervals, depending on the desired dosage and the pharmaceutical composition selected by the skilled practitioner for the particular subject. Regular administration dosing intervals include, e.g., once daily, twice daily, once every two, three, four, five or six days, once or twice weekly, once or twice monthly, and the like.

The invention further comprises the use of the MASP inhibitory peptide as described herein for the manufacture of a medicament, in particular for the manufacture of a medicament for the prophylaxis or treatment of a disorder or disease as defined herein.

The invention further comprises a process for manufacturing the peptids of the present invention, derivative or analogue or the pharmaceutically acceptable salt or solvate thereof or a complex, each as described herein. The process for manufacturing comprises the steps as shown in the examples of the present invention.

Generally, the MASP inhibitory peptide of the present invention may be manufactured synthetically, or semi-recombinantly.

According to a further embodiment, the invention provides a process for preparing a bicyclic compound which may be isolated and/or purified, comprising, essentially consisting of, or consisting of formula (I) or a derivative, analogue or pharmaceutically acceptable salts or solvates thereof by using solid phase peptide synthesis.

According to a further embodiment, the invention provides a process for preparing a bicyclic compound which may be isolated and/or purified, comprising, essentially consisting of, or consisting of formula (I) or a derivative, analogue, pharmaceutically acceptable salt, solvate or solvate of the salt, containing the steps
1. Use of a 2-chlorotrityl-type resin with a loading of 0.2 - 1.2 mmol/g, with or without the first amino acid preloaded,
2. Loading the c-terminal amino acid of the sequence onto the resin (if needed),
3. Removal of fmoc protection with a 15-30% piperidine solution in DMF or NMP,
4. Coupling of the next amino acid in the sequence with coupling reagents such as HBTU, HATU or DIC/Oxyma using stoichiometries between 3-8 equivalents,
5. Repeating steps 3 and 4 until the sequence is completed,
6. Cleavage of the peptide from the solid support using a cleavage cocktail that involves 1-5% TFA or HFIP,
7. Optional removal of an orthogonal protecting group (e.g. allyl or alloc) with reagents such as Pd(PPh₃)₄ and PhSiH,
8. Optional removal of an orthogonal protecting group (e.g. Dde, Dmab, or ivDde) with a 1-5% solution of hydrazine hydrate,
9. Cyclization of the peptide via amide bond formation using coupling reagents such as HBTU, HATU, PyBop, PyAop or DIC/Oxyma using stoichiometries between 3-8 equivalents, or formation a disulfide bond between two cysteines when they are present at the amine and C-terminus,
10. Removal of protecting groups from the peptide using a cleavage cocktail that involves TFA and a thiol scavenger,
11. Cyclization of two cysteines in the sequence under oxidative conditions (air or 12),
12. Purification of the cleaved peptide using reversed-phase HPLC.

According to a further embodiment, the invention provides a process for preparing a bicyclic compound which may be isolated and/or purified, comprising, essentially consisting of, or consisting of formula (I) or a derivative, analogue, pharmaceutically acceptable salt, solvate or solvate of the salt, containing the steps
1. Use of a 2-chlorotrityl-type resin with a loading of 0.2 - 1.2 mmol/g, with or without the first amino acid preloaded,
2. Loading the c-terminal amino acid of the sequence onto the resin (if needed),
3. Removal of fmoc protection with a 15-30% piperidine solution in DMF or NMP,
4. Coupling of the next amino acid in the sequence with coupling reagents such as HBTU, HATU or DIC/Oxyma using stoichiometries between 3-8 equivalents,
5. Repeating steps 3 and 4 until the sequence is completed,
6. Cleavage of the peptide from the solid support using a cleavage cocktail that involves 1-5% TFA or HFIP,
7. Optional removal of an orthogonal protecting group (e.g. allyl or alloc) with reagents such as Pd(PPh₃)₄ and PhSiH,
8. Optional removal of an orthogonal protecting group (e.g. Dde, Dmab, or ivDde) with a 1-5% solution of hydrazine hydrate,
9. Cyclization of the peptide via amide bond formation using coupling reagents such as HBTU, HATU, PyBop, PyAop or DIC/Oxyma using stoichiometries between 3-8 equivalents, or formation a disulfide bond between two cysteines when they are present at the amine and C-terminus,
10. Removal of protecting groups from the peptide using a cleavage cocktail that involves TFA and a thiol scavenger,
11. Cyclization of two cysteines in the sequence under oxidative conditions (air or 12),
12. Purification of the cleaved peptide using reversed-phase HPLC,
13. Conversion to the HCl salt.

According to a further embodiment, the invention provides a process for preparing a bicyclic compound which may be isolated and/or purified, comprising, essentially consisting of, or consisting of formula (I) or a derivative, analogue, pharmaceutically acceptable salt, solvate or solvate of the salt, containing the steps
1. Use of a Rink amide-type resin such as MBHA Rink amide resin with a loading of 0.2 - 1.4 mmol/g, with or without the first amino acid preloaded,
2. Removal of fmoc protection with a 15-30% piperidine solution in DMF or NMP,
3. Coupling of the next amino acid in the sequence with coupling reagents such as HBTU, HATU or DIC/Oxyma using stoichiometries between 3-8 equivalents,
4. Repeating steps 3 and 4 until the sequence is completed,
5. Cleavage of the peptide from the solid support using a cleavage cocktail that involves 1-5% TFA or HFIP,
6. Optional removal of an orthogonal protecting group (e.g. allyl or alloc) with reagents such as Pd(PPh₃)₄ and PhSiH,
7. Optional removal of an orthogonal protecting group (e.g. Dde, Dmab, or ivDde) with a 1-5% solution of hydrazine hydrate,
8. Cyclization of the peptide via amide bond formation using coupling reagents such as HBTU, HATU, PyBop, PyAop or DIC/Oxyma using stoichiometries between 3-8 equivalents, or formation a disulfide bond between two cysteines when they are present at the amine and C-terminus,
9. Removal of protecting groups from the peptide using a cleavage cocktail that involves TFA and a thiol scavenger,
10. Cyclization of two cysteines in the sequence under oxidative conditions (air or 12),
11. Purification of the cleaved peptide using reversed-phase HPLC.

According to a further embodiment, the invention provides a process for preparing a bicyclic compound which may be isolated and/or purified, comprising, essentially consisting of, or consisting of formula (I) or a derivative, analogue, pharmaceutically acceptable salt, solvate or solvate of the salt, containing the steps
1. Use of a Rink amide-type resin such as MBHA Rink amide resin with a loading of 0.2 - 1.4 mmol/g, with or without the first amino acid preloaded,
2. Removal of fmoc protection with a 15-30% piperidine solution in DMF or NMP,
3. Coupling of the next amino acid in the sequence with coupling reagents such as HBTU, HATU or DIC/Oxyma using stoichiometries between 3-8 equivalents,
4. Repeating steps 3 and 4 until the sequence is completed,
5. Cleavage of the peptide from the solid support using a cleavage cocktail that involves 1-5% TFA or HFIP,
6. Optional removal of an orthogonal protecting group (e.g. allyl or alloc) with reagents such as Pd(PPh₃)₄ and PhSiH,
7. Optional removal of an orthogonal protecting group (e.g. Dde, Dmab, or ivDde) with a 1-5% solution of hydrazine hydrate,
8. Cyclization of the peptide via amide bond formation using coupling reagents such as HBTU, HATU, PyBop, PyAop or DIC/Oxyma using stoichiometries between 3-8 equivalents, or formation a disulfide bond between two cysteines when they are present at the amine and C-terminus,
9. Removal of protecting groups from the peptide using a cleavage cocktail that involves TFA and a thiol scavenger,
10. Cyclization of two cysteines in the sequence under oxidative conditions (air or 12),
11. Purification of the cleaved peptide using reversed-phase HPLC,
12. Conversion to the HCl salt.

The at least one peptide, derivative or analogue as defined herein or the pharmaceutically acceptable salt or solvate thereof or the complex as defined herein may also be used as a biochemical agent in a biochemical assay, such as e.g. in a diagnostic assay to measure responsiveness to MASP inhibitors or in any biochemical assay being based on MASP inhibitor binding.

The present invention also includes polynucleotides comprising a sequence encoding a MASP inhibitory peptide according to the present invention, as well as a vector comprising a polynucleotide comprising a sequence encoding a MASP inhibitory peptide according to the present invention.

The invention is further illustrated by the following examples, which relate to certain specific embodiments of the present invention. The examples were carried out using well known standard techniques within the routine to those of skill in the art, unless indicated otherwise. The following examples are for illustrative purposes only and do not purport to be wholly definitive as to conditions or scope of the invention. As such, they should not be construed in any way as limiting the scope of the present invention.

### Examples

### List of Abbreviations used in Experimental Section:

- Å: Angstroms
- ACN: Acetonitrile
- aq.: Aqueous, aqueous solution
- bar: Unit of pressure
- BPR: Back-pressure regulator
- conc: Concentrated
- d: Doublet (NMR)
- dd: Doublet of doublet (NMR)
- DCM: Dichloromethane
- DEA: Diethylamine
- DIPEA: N,N,-diisopropylethylamine (Hünig's base)
- DMAP: 4-Dimethylaminopyridine
- DMF: N,N-dimethylformamide
- DMSO: Dimethylsulfoxide
- dt: Doublet of triplet (NMR)
- EA: Ethyl acetate
- ee: Enantiomeric excess
- ent: Enantiomeric
- eq: Equivalent(s)
- equiv: Equivalent(s) (ion chromatography)
- ESI: Electrospray-ionisation (mass spectroscopy)
- Fmoc-OSu: 1-{[(9H-Fluoren-9-ylmethoxy)carbonyl]oxy}pyrrolidine-2,5-dione
- GC-MS: Gas chromatography coupled with mass spectrometry
- h: Hour(s)
- HATU: O-(7-Azabenzotriazol-1-yl)-N,N,N',N'-tetramethyluronium-hexafluorophosphate
- HFIP: 1,1,1,3,3,3-Hexafluoro-2-propanol
- HPLC: High pressure liquid chromatography
- IC: Ion chromatography
- ID: Internal diameter
- IRA: part of a trade name for Amberlite IRA ion exchange resin
- L: Liter
- LC-MS: Liquid chromatography coupled to mass spectroscopy
- LiHMDS: Lithium bis(trimethylsilyl)amide
- lit.: Literature
- m: Multiplet (NMR)
- MALDI: Matrix Assisted Laser Desorption/Ionization (mass spectrometry)
- Me: Methyl
- mL: milliliter
- min: Minute(s)
- mm: millimeter
- µL: microliter
- µm: micrometer (micron)
- M: Molar
- MPLC: Medium pressure liquid chromatography
- MS: Mass spectroscopy
- MTBE: tert-Butyl methyl ether
- MTP: Microtiter plate
- m/z: mass-to-charge ratioi (mass spectrometry)
- nm: nanometer
- NMM: N-Methyl morpholine
- NMP: N-Methyl-2-pyrrolidone
- NMR: Nuclear magnetic resonance spectroscopy
- PBS: Phosphate buffered saline
- PE: Petroleum ether
- PEG: Polyethylene glycol
- pos: Positive
- ppm: parts per million
- Pr: Propyl
- Psi: Pounds per square inch (pressure)
- q / quart: Quartet (NMR)
- qd: Quartet of doublet (NMR)
- quint: Quintet (NMR)
- rac: racemic
- Rf: Retention factor (TLC)
- RP: reversed-phase (for liquid chromatography)
- Rt: Retention time (chromatography)
- s: Singlet (NMR)
- s: Seconds (time)
- SPPS: solid phase peptide synthesis
- t: Triplet (NMR)
- TBTU: O (Benzotriazol-1-yl)-N,N,N',N'-tetramethyluronium tetrafluoroborate
- tBu: tert-Butyl
- TEA: triethylamine
- THF: Tetrahydrofuran
- TLC: Thin layer chromatography
- UPLC: Ultra-performance liquid chromatography
- UV: Ultraviolet

Further abbreviations can be found in Table 2 and 3.

### Analytical LC-MS Methods

### Method 1

Equipment type MS: ThermoFisherScientific LTQ-Orbitrap-XL; Equipment type HPLC: Agilent 1200SL; Column: Agilent, POROSHELL 120, 3 x 150 mm, SB - C18 2.7 µm; eluent A: 1 L water + 0.1% trifluoroacetic acid; eluent B: 1 L acetonitrile + 0.1% trifluoroacetic acid; gradient: 0.0 min 2% B → 1.5 min 2% B → 15.5 min 98% B → 18.0 min 98% B; oven: 40°C; flow rate: 0.75 mL/min; UV-detection: 210 nm.

### Method 2

Equipment type MS: ThermoFisherScientific LTQ-Orbitrap-XL; Equipment type HPLC: Agilent 1200SL; Column: Agilent, POROSHELL 120; 3 x 150 mm, SB - C18 2.7 µm; eluent A: 1 L water + 0.1% trifluoroacetic acid; eluent B: 1 L acetonitrile + 0.1% trifluoroacetic acid; gradient: 0.0 min 5% B → 0.3 min 5% B → 7.0 min 98% B → 10 min 98% B; oven: 40°C; flow rate: 0.75 mL/min; UV-detection: 210 nm.

### Method 3

Equipment type MS: Waters TOF instrument; Equipment type UPLC: Waters Acquity I-CLASS; Column: YMC, TRIART C18, 75 x 1 mm, 3.0 µm x 12nm ; eluent A: 1 L water + 0.01% formic acid; eluent B: 1 L acetonitrile + 0.01% formic acid; gradient: 0.0 min 1% B → 2.0 min 1% B → 8.0 min 95% B → 10.0 min 95% B; oven: 50°C; flow rate: 0.63 mL/min; UV-detection: 210 nm.

### Method 4

Equipment type MS: Waters TOF instrument; Equipment type UPLC: Waters Acquity I-CLASS; Column: YMC, TRIART C18, 75 x 1 mm, 3.0 µm 12nm ; eluent A: 1 L water + 0.01% formic acid; eluent B: 1 L acetonitrile + 0.01% formic acid; gradient: 0.0 min 1% B → 1.0 min 1% B → 15.0 min 50% B → 18.0 min 95% B; oven: 50°C; flow rate: 0.63 mL/min; UV-detection: 210 nm.

### Method 5

Equipment type MS: Waters TOF instrument; Equipment type UPLC: Waters Acquity I-CLASS; Column: Waters, HSST3, 2.1 x 50 mm, C18 1.8 µm; eluent A: 1 L water + 0.01% formic acid; eluent B: 1 L acetonitrile + 0.01% formic acid; gradient: 0.0 min 2% B → 0.5 min 2% B → 7.5 min 95% B → 10.0 min 95% B; oven: 50°C; flow rate: 1.00 mL/min; UV-detection: 210 nm.

### Method 6

Equipment type MS: Waters Synapt G2S; Equipment type UPLC: Waters Acquity I-CLASS; Column: Waters, BEH300, 2.1 x 150 mm, C18 1.7 µm; eluent A: 1 L water + 0.01% formic acid; eluent B: 1 L acetonitrile + 0.01% formic acid; gradient: 0.0 min 2% B → 1.5 min 2% B → 8.5 min 95% B → 10.0 min 95% B; oven: 50°C; flow rate: 0.50 mL/min; UV-detection: 220 nm.

### Method 7

Instrument type MS: Agilent 6410 Triple Quad; Instrument type HPLC: Agilent 1200; Column: Gemini-NX C18 5µm 110Å 150 x 4.6 mm; eluent A: 0.1%TFA in H₂O; eluent B: 0.1%TFA in ACN; gradient: 0.0 min 20% B → 20 min 50% B → 20.1 min 90% B → 23 min 90% B; oven temperature: 50°C; flow rate: 1.0 mL/min; UV-detection: 220 nm.

### Method 8

Instrument type MS: Agilent 6410 Triple Quad; Instrument type HPLC: Agilent 1200; Column: Discovery BIO Wide Pore C18 5µm 300Å x 4.6 mm; eluent A: 0.1%TFA in H₂O; eluent B: 0.1%TFA in ACN; gradient: 0.0 min 10% B → 20 min 80% B → 20.1 min 90% B → 23 min 90% B; oven temperature: 50°C; flow rate: 1.0 mL/min; UV-detection: 220 nm.

### Method 9

Instrument: Waters ACQUITY SQD UPLC System; column: Waters Acquity UPLC HSS T3 1.8 µm 50 x 1 mm; eluent A: 1 L water + 0.25 mL 99% formic acid, eluent B: 1 L acetonitrile + 0.25 mL 99% formic acid; gradient: 0.0 min 90% A → 1.2 min 5% A → 2.0 min 5% A; oven: 50 °C; flow: 0.40 mL/min; UV-detection: 210 nm.

### Method 10

Instrument MS: Thermo Scientific FT-MS; Instrument UHPLC: Thermo Scientific UltiMate 3000; Column: Waters, HSST3, 2.1 x 75 mm, C18 1.8 µm: eluent A: 1 L watMethod 10er + 0.01% formic acid; eluent B: 1L acetonitrile + 0.01% formic acid; gradient: 0.0 min 10% B → 2.5 min 95% B → 3.5 min 95% B; oven: 50°C; flow rate: 0.90 mL/min; UV-detection: 210 nm/ Optimum Integration Path 210-300 nm.

### Method 11

MS Instrument: Agilent MS Quad 6150; HPLC: Agilent 1290; Column: Waters Acquity UPLC HSS T3 1.8 µm 50 x 2.1 mm; eluent A: 1 L water + 0.25 mL formic acid, eluent B: 1 L acetonitrile + 0.25 mL formic acid; gradient: 0.0 min 90% A → 0.3 min 90% A → 1.7 min 5% A → 3.0 min 5% A oven: 50°C; flow rate: 1.20 mL/min; UV-detection: 205 - 305 nm.

### Method 12

Instrument: Waters ACQUITY SQD UPLC System; column: Waters Acquity UPLC HSST3 1.8 µm 50 x 1 mm; eluent A: 1 L water + 0.25 mL 99% formic acid, eluent B: 1 L acetonitrile + 0.25 mL 99% formic acid; gradient: 0.0 min 95% A → 6.0 min 5% A → 7.5 min 5% A oven: 50°C; flow rate: 0.35 mL/min; UV-detection: 210 nm.

### Method 13

Instrument: Waters Single Quad MS System; Instrument Waters UPLC Acquity; column: Waters BEH C18 1.7 µm 50 x 2.1 mm; eluent A: 1 L water + 1.0 mL (25% ammonia)/L, eluent B: 1 L acetonitrile; gradient: 0.0 min 92% A → 0.1 min 92% A → 1.8 min 5% A → 3.5 min 5% A; oven: 50°C; flow rate: 0.45 mL/min; UV-detection: 210 nm.

### Method 14

System MS: Waters TOF instrument; System UPLC: Waters Acquity I-CLASS; Column: Waters Acquity UPLC Peptide BEH C18 300Å, 1.7 µm 150 x 2.1 mm; Eluent A: 11 Water + 0.100 ml 99% Formic acid, Eluent B: 11 Acetonitrile + 0.100 ml 99% Formic acid; Gradient: 0.0 min 90% A →0.25 min 90% A → 8.0 min 45% A → 10.0 min 2% → 12.0 min 2% A Oven: 50°C; Flow: 0.475 ml/min; UV-Detection: 210 nm.

### Method 15

MS instrument type: Agilent G6110A; HPLC instrument type: Agilent 1200 Series LC; UV DAD; column: Chromolith Flash RP-18e 25 x 2.0mm; mobile phase A: 0.0375% TFA in water (v/v), mobile phase B: 0.01875% TFA in acetonitrile (V/V): gradient: 0.01 min 5% B → 0.80 min 95% B → 1.20 min 95% B → 1.21 min 5% B → 1.5 min 5% B; flow rate: 1.50 mL/min; oven temperature: 50 °C; UV detection: 220 nm & 254 nm.

### MALDI Method

Exact mass measurements were performed on selected peptides using a Matrix Assisted Laser Desorption/Ionization (MALDI) mass spectrometry method on a Bruker autoflex maX LRF MALDI MS Time-of-Flight (ToF-MS) system. Samples were prepared on a Bruker MALDI target plate using α-cyano-4-hydroxycinnamic acid (CAS 28166-41-8) as the matrix. A solution of the sample peptide 0.1 to 1.0 mg in 1.0 mL acetonitrile-water (50/50 or 30/70) and a stock solution of the matrix (10 mg/mL) in 50% acetonitrile in water containing 0.05% trifluoroacetic acid are prepared. 1.0 uL of each solution is placed onto the MALDI target plate and allowed to dry. The sample is then ready for analysis. Recommended sample preparations for MALDI target plates can be found in the documention provided by Bruker.

### Analytical Ion Chromatography Method

### Method: IC - Quantitative

Quantitative Measurement of Cations and Anions using external standards; Instrument: Thermo Scientific ICS 5000+; Capillary IC Columns: IonPac AS11-HC und IonPac CS16; eluent: gradient eluent [H]+ [OH]-; Detector: Conductivity detection; routine anions possible: acetate, bromide, citrate, chloride, fluoride, formate, lactate, mesylate, phosphate, sulfate, tartrate, trifluoroacetate; routine cations possible: ammonium, barium, calcium, potassium, lithium, sodium, magnesium, choline.

### Analytical Gas Chromatography Mass Spec Method

### GC-MS Method

Instrument: Thermo Scientific DSQII, Thermo Scientific Trace GC Ultra; column: Restek RTX-35MS, 15 m x 200 µm x 0.33 µm; constant flow with Helium: 1.20 mL/min; oven: 60°C; Inlet: 220°C; gradient: 60°C, 30°C/min → 300°C (3.33 min hold).

### NMR

The 1H-NMR data of selected compounds are listed in the form of 1H-NMR peaklists. Therein, for each signal peak the δ value in ppm is given, followed by the signal intensity, reported in round brackets. The δ value-signal intensity pairs from different peaks are separated by commas. Therefore, a peaklist is described by the general form: δ1 (intensity1), δ2 (intensity2), ... , δi (intensityi), ... , δn (intensityn).

The intensity of a sharp signal correlates with the height (in cm) of the signal in a printed NMR spectrum. When compared with other signals, this data can be correlated to the real ratios of the signal intensities. In the case of broad signals, more than one peak, or the center of the signal along with their relative intensity, compared to the most intense signal displayed in the spectrum, are shown. A 1H-NMR peaklist is similar to a classical 1H-NMR readout, and thus usually contains all the peaks listed in a classical NMR interpretation. Moreover, similar to classical 1H-NMR printouts, peaklists can show solvent signals, signals derived from stereoisomers of the particular target compound, peaks of impurities, 13C satellite peaks, and/or spinning sidebands. The peaks of stereoisomers, and/or peaks of impurities are typically displayed with a lower intensity compared to the peaks of the target compound (e.g., with a purity of >90%). Such stereoisomers and/or impurities may be typical for the particular manufacturing process, and therefore their peaks may help to identify a reproduction of the manufacturing process on the basis of "by-product fingerprints". An expert who calculates the peaks of the target compound by known methods (MestReC, ACD simulation, or by use of empirically evaluated expectation values), can isolate the peaks of the target compound as required, optionally using additional intensity filters. Such an operation would be similar to peak-picking in classical 1H-NMR interpretation. A detailed description of the reporting of NMR data in the form of peaklists can be found in the publication "Citation of NMR Peaklist Data within Patent Applications" (cf. http://www.researchdisclosure.com/searching-disclosures, Research Disclosure Database Number 605005, 2014, 01 Aug 2014). In the peak picking routine, as described in the Research Disclosure Database Number 605005, the parameter "MinimumHeight" can be adjusted between 1% and 4%. However, depending on the chemical structure and/or depending on the concentration of the measured compound it may be reasonable to set the parameter "MinimumHeight" <1%.

### Preparation Examples

### General Methods for the Synthesis

Solid Phase Peptide Synthesis (SPPS) was carried out either using an automatic peptide synthesizer or performed manually by hand. Peptide synthesis was typically carried out in scale ranges from 0.1 to 1.0 mmol. When peptide synthesis was carried out by hand, the general procedures Methods C, D and E described below were used. Automated peptide synthesis was performed on a Symphony X peptide synthesizer (Protein Technologies).

Fmoc-protected amino acids were purchased from Novabiochem, Bachem, Iris Biotech, Sigma-Aldrich, Alfa, Enamine, Amatek, Anichem, ACBR, Combiblocks, ArZa Bioscience, Ark Pharm, Acroteinchem, Apollo Scientific, Biofine, Broadpharm, VWR, or Gyros Protein Technologies (Fluorenylmethoxycarbonyl = Fmoc), GL Biochem (Shanghai) Ltd, Chengdu aminotp Pharmaceutical Technology Ltd, Suzhou Highfine Biotech Co., Ltd, or sourced through other Chinese vendors. Some special fmoc-protected amino acids were synthesized internally, and these synthetic methods are described herein. Some of the fmoc amino acids synthesized internally are also commercially available. In some cases where the Fmoc-protected amino acid was not commercially available but the Boc-protected unnatural amino acid was commercially available, the fmoc-protected amino acid was prepared from the Boc-protected amino acid by deprotection and reprotection using methods commonly employed in the art. CAS Numbers for commercially available, unnatural amino acids used in the synthesis of peptides of this invention have in most cases been included in Table 5. In cases where a racemic amino acid (Fmoc or Boc) was purchased, one skilled in the art should recognize that the enantiomers can be separated using chiral chromatography, and this was in fact sometimes done to optain the enantiomerically pure amino acid prior to peptide synthesis.

The following unnatural amino acids have been used in preparing peptides of the invention. The Fmoc- or Boc- protected amino acids were either obtained through commercial sources (CAS Number is available) or, synthesized internally by methods described herein. Table 5 shows the CAS number of the chemical groups / amino acids which were used for the peptide synthesis (right column) and the corresponding chemical group / amino acid present in the peptides (left column).

**Table 5: Availability of unnatural amino acids and chemical groups of this Invention**

| **Abbreviation/Expression Definition** | CAS **Number for Fmoc-/Boc-protected amino acid, chemical group or building block used for peptide synthesis** |
|---|---|
| L-N-Methylcysteine | 944797-51-7 |
| N-Methyl-Glycine | 77128-70-2 |
| 3-(Aminomethyl)benzoic acid | 117445-22-4 |
| 4-(Aminomethyl)benzoic acid | 33233-67-9 |
| L-2-Aminobutyric acid | 135112-27-5 |
| Adipic acid | 124-04-9 |
| 6-Aminohexanoic acid | 88574-06-5 |
| L-tert-Butylalanine | 139551-74-9 |
| 3-Azido-L-Alanine | 684270-46-0 |
| L-2,4-Diaminobutyric acid | 125238-99-5; 607366-21-2 (ivDde); 851392-68-2 (MTT) |
| L-2,3-Diaminopropionic acid | 162558-25-0; 607366-20-1 (ivDde); 654670-89-0 (MTT) |
| Gamma-Aminobutyric acid | 116821-47-7; 57294-38-9 (Boc) |
| L-Propargylglycine | 1435854-95-7 |
| 2,3,3a,4,5,6,7,7a-Octahydroindole-2-carboxylic acid | 130309-37-4 |
| L-Ornithine | 109425-55-0; 269062-80-8 (DDE); 1198321-33-3 (ivDDE); 147290-11-7 (Alloc) |
| 9-Amino-4,7-dioxanonanoic acid | 872679-70-4 |
| 12-Amino-4,7,10-trioxadodecanoic acid | 867062-95-1 |
| 15-Amino-4,7,10,13-tetraoxapentadecanoic acid | 557756-85-1 |
| L-Penicillamine | 201531-88-6 |
| Suberic acid | 505-48-6 |
| Tranexamic acid | 167690-53-1; 27687-14-5 (Boc) |
| 1,13-Diamino-4,7,10-trioxatridecan-succinamic acid | 172089-14-4 |
| 2-Aminoisobutyric acid | 94744-50-0 |

Solid-phase resins were purchased from Novabiochem, Bachem, Iris Biotech, Pcas Biomatrix, GL Biochem (Shanghai) Ltd, CEM, or Protein Technologies. The resin loading was 0.3 - 1.0 mmol/g. Peptides were synthesized on 2-Chlorotrityl resin, on Wang resin, or on Rink amide-type resins depending on the desired C-terminus. In some cases, a 2-chlorotrityl resin or Wang-type resin containing the first amino acid already attached (e.g. Fmoc-Asp(Ot-Bu)-2-chlorotrityl resin) was used. Cleavage of the fluorenylmethoxycarbonyl (Fmoc) protecting group was achieved using 20% piperidine in dimethylformamide at room temperature. Each Fmoc cleavage step was carried out twice. Amino acids were coupled on an automated synthesizer (Symphony X) using 8 equivalents of the Fmoc-amino acid, with 8 equivalents of DIC (Diispropylcar-bodiimide) (0.5 M in DMF) and 8 equivalents of Oxyma (Ethyl cyanohydroxyiminoacetate) (0.5M in DMF). Amino acid couplings were conducted at room temperature and under a nitrogen atmosphere, when the Symphony X was used. When expensive or self-prepared fmoc-amino acids were used, the coupling was performed manually using 3 equivalents of the Fmoc-amino acid, with 3 equivalents of DIC (0.5 M in DMF) and 3 equivalents of Oxyma (0.5M in DMF). Each amino acid coupling step was carried out twice (double coupling). Alternatively peptides were prepared by SPPS manually using 3 equivalents of the Fmoc-amino acid, 2.85 equivalents of HBTU ((2-(1H-benzotriazol-1-yl)-1,1,3,3-tetramethyluronium hexafluorophosphate, **H**exafluorophosphate **B**enzotriazole **T**etramethyl **U**ronium) (0.5 M in DMF) and 6 equivalents of DIPEA (0.5M in DMF). The coupling reaction was monitored using the ninhydrin test.

Pepides were removed from 2-chlorotrityl resin using a 1%TFA solution or HFIP. The cleaved peptide was then futher modified by amide bond formation and/or disulfide bond formation.

Peptides were completely deprotected using trifluoroacetic acid (TFA)/thioanisole (TA)/1,2-ethanedithiol (EDT) (90:7:3) or with 92.5%TFA/2.5%EDT/2.5%TIS (triisopropylsilane)/2.5%H₂O. For peptides containing methionine, the peptides were treated with a solution of 1.6% EDT and 1.2% trimethylsilylbromide in TFA for 2 h at room temperature to reduce oxidized methionine.

### Peptide Cyclization:

Head-to-tail cyclization of the peptide via amide bond formation was accomplished in solution using coupling reagents such as HBTU, HATU, PyBop, PyAop or DIC/Oxyma using stoichiometries between 3-8 equivalents after cleavage from the 2-chlorotrityl resin. When a Rink amide-type resin like MBHA Rink Amide Resin, a side chain-to-tail, a head to side chain, or a side chain-to-side chain cyclization was normally performed on the resin using coupling reagents such as HBTU, HATU, PyBop, PyAop or DIC/Oxyma using stoichiometries between 3-8 equivalents, after which full cleavage from the resin was performed. After amide bond formation in solution, a cleavage cocktail such as trifluoroacetic acid (TFA)/thioanisole (TA)/1,2-ethanedithiol (EDT) (90:7:3) or with 92.5%TFA/2.5%EDT/2.5%TIS (triisopropylsilane)/2.5%H₂O was used to remove the remaining protecting groups prior to disulfide bond formation.

### Disulfide Cyclization:

Disulfide bridges were formed by shaking peptides in 0.1 M ammonium bicarbonate buffer (pH 7.83) at a concentration of 0.5 mg/mL overnight. The solution was then lyophilized. Alternatively, disulfide bridges were formed formed by shaking peptides in mixture of acetonitrile/water (often 3:7) adjusted to pH 9.0 with solid ammonium bicarbonate buffer at a concentration of 1-3 mg/mL overnight. Alternatively, disulfide bridges were prepared by oxidation with iodine (I₂) (0.1 M in MeOH) at a concentration of 1 - 1.3 mg/mL in acetonitrile/water (1:1) at 20°C for 2min, followed by treatment with sodium thiosulfate (0.1 M in water) followed by lyophilization.

### Optional Acetylation:

N-terminal acetylation was performed using 10 equivalents acetic anhydride (or another anhydride reagent, e.g. adipic) in DMF (2 mL) and 2.5 equivalents DIPEA by shaking the suspension at RT for 1 h on an orbital shaker. The solvent was removed, and the resin was washed with DMF (5x) and DCM (5x). The procedure was then repeated again. Alternatively, N-terminal acetylation was performed using 10 mL of a capping solution consisting of acetic anhydride/N-methyl morpholine (NMM)/DMF (10:5:85) by shaking the suspention at RT for 30 min on an orbital shaker.

### Peptide cleavage:

A cleavage cocktail containing TFA/EDTThioanisol (90:3:7) was prepared. The cleavage cocktail (2 mL) was added to the peptide containing resin and the suspension was shaken on an orbital shaker for 2.5 hours. Cold ether (-20°C) was added to precipitate the peptide. The resulting solution was centrifuged under nitrogen (Sigma 2-16KL), and the resulting solid obtained after decantation was washed with cold ether 3 more times, by centrifugation and decantation. The resulting solid was purified by preparative HPLC.

Alternatively, a cleavage cocktail containing TFA/EDT/TIS/H₂O (92.5:2.5:2.5:2.5) was prepared. The cleavage cocktail (6 mL (0.3 mmol scale)) was added to the peptide containing resin and the suspension was shaken on an orbital shaker for 2.5 hours. Cold tert-butyl methyl ether (-20°C) was added to precipitate the peptide. The resulting solution was centrifuged at 3000 rpm for 3 min, and the resulting solid obtained after decantation was washed with cold tert-butyl methyl ether 3 more times (20 mL x 3), by centrifugation and decantation. The resulting crude peptide was dried over vacuum for 2 hours and then purified by preparative HPLC. Those skilled in the art should recognize that alternatie cleavage cocktails sometimes might need to be modified to improve yield or minimize side products.

### Preparative HPLC:

An Agilent 1260 Prep reversed-phase HPLC or a Knauer AZURA Prep reversed-phase HPLC was used for purification. The column is chosen based on the results of a column screen. The peptide is dissolved in 10 - 30% ACN/water (typically the starting point of the gradient). Water and acetonitrile both contain 0.1% TFA. Flow rate 20mL/min, 10-30% ACN/water to 85-90% ACN/water was typically used. Fractions were analysed by HPLC (Agilent 1260 Infinity) using using a Chromolith Speedrod column, 5-95% ACN/water gradient over 8 min) and by one or more of the following LC-MS methods: Method 1, Method 2, Method 3, Method 4, Method 5, Method 6.

Alternatively, a Gilson GX-281 Prep reversed-phase HPLC was used for purification. The column was chosen based on the results of a column screen. The peptide is dissolved in 10 - 30% ACN/water (typically the starting point of the gradient). The water contained 0.075% TFA. Normally a Luna column (25 x 200 mm, C18 10 um, 110 Å) or a Gemini column (30 x 150 mm, C18 5 um,110 Å) was used. Conditions for prep HPLC: flow rate 20mL/min, 10-30% ACN/water to 85-90% ACN/water, wavelength 214/254 nm, oven temperature 30°C. Fractions were analysed by HPLC (Agilent 1260 Infinity) using Method 7. The peptides were thereafter analyzed by one or more of the following methods: Method 1, Method 2, Method 3, Method 4, Method 5, Method 6.

Disulfide mimetics, wherein the -S-S- disulfide bond is replaced by a -CH₂-S-, -S-CH₂-, -CH₂-CH₂-, -S-(CH₂)₂-, -(CH₂)₂-S- or a -CH₂-S-CH₂- can be prepared according to procedure described in the following references in combination with methods described herein: (1) Hong-Kui Cui, Ye Guo, Yao He, Feng-Liang Wang, Hao-Nan Chang, Yu-Jia Wang, Fang-Ming Wu, Chang-Lin Tian, Lei Liu Angew. Chem. Int. Ed. 2013, 52, 9558-9562; (2) Ye Guo, De-Meng Sun, Feng-Liang Wang, Yao He, Lei Liu, Chang-Lin Tian Angew. Chem. Int. Ed. 2015, 54, 14276-14281; (3) Yang Xu, Tao Wang, Chao-Jian Guan, Yi-Ming Li, Lei Liu, Jing Shi, Donald Bierer Tetrahedron Letters 2017, 58, 1677-1680; (4) Tao Wang, Yi-Fu Kong, Yang Xu, Jian Fan, Hua-Jian Xu, Donald Bierer, Jun Wang, Jing Shi, Yi-Ming Li Tetrahedron Letters 2017, 58, 3970-3973; (5) Tao Wang, Jian Fan, Xiao-Xu Chen, Rui Zhao, Yang Xu, Donald Bierer, Lei Liu, Yi-Ming Li, Jing Shi, Ge-Min Fang Org. Lett. 2018, 20, 6074-6078; (6) Jan-Patrick Fischer, Ria Schonauer, Sylvia Els-Heindl, Donald Bierer, Johannes Koebberling, Bernd Riedl, Annette G. Beck-Sickinger J Pep Sci. 2019; e3147; (7) Dong-Liang Huang, Jing-Si Bai, Meng Wu, Xia Wang, Bernd Riedl, Elisabeth Pook, Carsten Alt, Marion Erny, Yi-Ming Li, Donald Bierer, Jing Shi, Ge-Min Fang Chem. Commun., 2019, 55, 2821-2824; (8) ShuaiShuai Sun, Junyou Chen, Rui Zhao, Donald Bierer, Jun Wang, Ge-Min Fang, Yi-Ming Li Tetrahedron Letters 2019, 60, 1197-1201; (9) C. M. B. K. Kourra and N. Cramer Chem. Sci., 2016, 7, 7007-7012; (10) Qian Qu, Shuai Gao, Fangming Wu, Meng-Ge Zhang, Ying Li, Long-Hua Zhang, Donald Bierer, Chang-Lin Tian, Ji-Shen Zheng, Lei Liu Angew. Chem. Int. Ed. 2020, 59, 6037-6045; (11) Rui Zhao, Pan Shi, Junyou Chen, Shuaishuai Sun, Jingnan Chen, Jibin Cui, Fangming Wu, Gemin Fang, Changlin Tian, Jing Shi, Donald Bierer, Lei Liu, Yi-Ming Li Chem. Sci., 2020, 11, 7927-7932; 10.1039/d0sc02374d; (12) Junyou Chen, Shuaishuai Sun, Rui Zhao, Chen-Peng Xi, Wenjie Qiu, Ning Wang, Ya Wang, Donald Bierer, Jing Shi, Yi-Ming Li ChemistrySelect 2020, 5, 1359-1363; 10.1002/slct.201904042; (13) Yun-Kun Qi, Qian Qu, Donald Bierer, Lei Liu Chem Asian J. 2020, 15, 2793-2802; 10.1002/asia.202000609.

All peptides of this invention unless otherwise noted are TFA Salts.

### General Method for the Automated SPPS of Masp Peptides (Method A)

The synthesis of (Ahx)**-GIC+SRSLPPIC+IPD** (Example 13) is representative.

The peptide was synthesized using standard Fmoc chemistry. 2-Chlorotrityl resins or 2-chlorotrityl resins with the first amino acid preloaded were typically used.

Automated SPPS was performed on a Symphony X peptide synthesizer (Protein Technologies). Fmoc-Asp(O-tBu)-chlorotrityl resin was typically used (loading 0.3 - 0.8 mmol/gram) on a 0.1 mmol scale for peptides containing Asp at the C-terminus. For Example 13, the loading used was 0.389 mmol/gram. The resin was placed into the reaction vessel and placed onto the instrument. The following soluitons were prepared and used during the synthesis:
1) Fmoc Amino Acids: 0.2 M (8 eq)
2) Activator 1: 0.5 M DIC in DMF (7.5 eq - 8 eq)
3) Activator 2: 0.5 M Oxyma in DMF (7.5 eq - 8 eq)
4) Fmoc Deprotection: 30% piperidine in DMF

Double couplings were typically performed for each amino acid. For expensive unnatural Fmoc or Boc amino acids, in-house synthesized Fmoc amino acids, or N-methylated amino acid, the sequence was interupted and this amino acid was coupled manually (double coupling, but typically with less reagent (3-5 equiv). After this coupling was completed, the synthesis was typically continued on the synthesizer. If an N-methyl amino acid was added to the sequence, typically the next amino acid was coupled manually as well. All steps were performed at room temperature under nitrogen. Fmoc-Pen and Fmoc-Oic were typically coupled using automated SPPS. Fmoc(N-Me)Gly was typically coupled manually. Ahx was best and most often coupled manually.

The resin was swelled and washed with DMF (3 × 3 mL, 10 min). If the resin contained Fmoc, then the Fmoc was removed with 30% piperidine solution (2×3 mL, 10 min). The resin was washed with DMF (6×3 mL, 30 sec).

### Fmoc cleavage:

The Fmoc protecting group was removed by adding 30% piperidine solution (2 x 3 mL, 10 min). The resin was washed with DMF (6 x 3 mL, 30 sec).

### Coupling:

Fmoc-Pro (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (1 x 3 mL, 30 sec). The coupling step was repeated. Fmoc-Pro (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (6 x 3 mL, 30 sec).

### Fmoc cleavage:

The Fmoc protecting group was removed by adding 30% piperidine solution (2 x 3 mL, 10 min). The resin was washed with DMF (6 x 3 mL, 30 sec).

### Coupling:

Fmoc-Ile (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (1 x 3 mL, 30 sec). The coupling step was repeated. Fmoc-Ile (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (6 x 3 mL, 30 sec).

### Fmoc cleavage:

The Fmoc protecting group was removed by adding 30% piperidine solution (2 x 3 mL, 10 min). The resin was washed with DMF (6 x 3 mL, 30 sec).

### Coupling:

Fmoc-Cys(Trt) (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (1 x 3 mL, 30 sec). The coupling step was repeated. Fmoc-Cys(Trt) (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (6 x 3 mL, 30 sec).

### Fmoc cleavage:

The Fmoc protecting group was removed by adding 30% piperidine solution (2 x 3 mL, 10 min). The resin was washed with DMF (6 x 3 mL, 30 sec).

### Coupling:

Fmoc-Ile (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (1 x 3 mL, 30 sec). The coupling step was repeated. Fmoc-Ile (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (6 x 3 mL, 30 sec).

### Fmoc cleavage:

The Fmoc protecting group was removed by adding 30% piperidine solution (2 x 3 mL, 10 min). The resin was washed with DMF (6 x 3 mL, 30 sec).

### Coupling:

Fmoc-Pro (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (1 x 3 mL, 30 sec). The coupling step was repeated. Fmoc-Pro (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (6 x 3 mL, 30 sec).

### Fmoc cleavage:

The Fmoc protecting group was removed by adding 30% piperidine solution (2 x 3 mL, 10 min). The resin was washed with DMF (6 x 3 mL, 30 sec).

### Coupling:

Fmoc-Pro (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (1 x 3 mL, 30 sec). The coupling step was repeated. Fmoc-Pro (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (6 x 3 mL, 30 sec).

### Fmoc cleavage:

The Fmoc protecting group was removed by adding 30% piperidine solution (2 x 3 mL, 10 min). The resin was washed with DMF (6 x 3 mL, 30 sec).

### Coupling:

Fmoc-Leu (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (1 x 3 mL, 30 sec). The coupling step was repeated. Fmoc-Leu (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (6 x 3 mL, 30 sec).

### Fmoc cleavage:

The Fmoc protecting group was removed by adding 30% piperidine solution (2 x 3 mL, 10 min). The resin was washed with DMF (6 x 3 mL, 30 sec).

### Coupling:

Fmoc-Ser(t-Bu) (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (1 x 3 mL, 30 sec). The coupling step was repeated. Fmoc-Ser(t-Bu) (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (6 x 3 mL, 30 sec).

### Fmoc cleavage:

The Fmoc protecting group was removed by adding 30% piperidine solution (2 x 3 mL, 10 min). The resin was washed with DMF (6 x 3 mL, 30 sec).

### Coupling:

Fmoc-Arg(Pbf) (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (1 x 3 mL, 30 sec). The coupling step was repeated. Fmoc-Arg(Pbf) (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (6 x 3 mL, 30 sec).

### Fmoc cleavage:

The Fmoc protecting group was removed by adding 30% piperidine solution (2 x 3 mL, 10 min). The resin was washed with DMF (6 x 3 mL, 30 sec).

### Coupling:

Fmoc-Ser(t-Bu) (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (1 x 3 mL, 30 sec). The coupling step was repeated. Fmoc-Ser(t-Bu) (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (6 x 3 mL, 30 sec).

### Fmoc cleavage:

The Fmoc protecting group was removed by adding 30% piperidine solution (2 x 3 mL, 10 min). The resin was washed with DMF (6 x 3 mL, 30 sec).

### Coupling:

Fmoc-Cys(Trt) (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (1 x 3 mL, 30 sec). The coupling step was repeated. Fmoc-Cys(Trt) (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (6 x 3 mL, 30 sec).

### Fmoc cleavage:

The Fmoc protecting group was removed by adding 30% piperidine solution (2 x 3 mL, 10 min). The resin was washed with DMF (6 x 3 mL, 30 sec).

### Coupling:

Fmoc-Ile (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (1 x 3 mL, 30 sec). The coupling step was repeated. Fmoc-Ile (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (6 x 3 mL, 30 sec).

### Fmoc cleavage:

The Fmoc protecting group was removed by adding 30% piperidine solution (2 x 3 mL, 10 min). The resin was washed with DMF (6 x 3 mL, 30 sec).

### Coupling:

Fmoc-Gly (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (1 x 3 mL, 30 sec). The coupling step was repeated. Fmoc-Gly (4.0 mL) was added. Activator 1 solution (DIC, 1.6 mL) and Activator 2 solution (Oxyma, 1.6 mL) were added and the coupling was allowed to proceed with nitrogen bubbling for 2 hours. The solution was drained and washed with DMF (6 x 3 mL, 30 sec).

### Fmoc cleavage:

The Fmoc protecting group was removed by adding 30% piperidine solution (2 x 3 mL, 10 min). The resin was washed with DMF (6 x 3 mL, 30 sec).

### Manual Coupling during automated SPPS:

Fmoc-Ahx (0.2 M in DMF, 3 equiv) was added to the resin. Activator 1 solution (DIC, 0.6 mL) and Activator 2 solution (Oxyma, 0.6 mL) were added and the coupling was allowed to proceed with shaking (Thermomixer, rt) for 2 hours. The solution was filtered and washed with DMF (1 x 3 mL, 30 sec). The coupling step was repeated. Fmoc-Ahx (0.2 M in DMF, 3 equiv) was added. Activator 1 solution (DIC, 0.6 mL) and Activator 2 solution (Oxyma, 0.6 mL) were added and the coupling was allowed to proceed with shaking (Thermomixer, rt) for 2 hours. The solution was filtered and washed with DMF (6 x 3 mL, 30 sec).

### Fmoc cleavage:

The Fmoc protecting group was removed by adding 30% piperidine solution (2 x 3 mL, 10 min). The resin was washed with DMF (6 x 3 mL, 30 sec).

If additional amino acids were present in the sequence, they were coupled using using the steps above.

### Test Cleavage:

When manual couplings were performed, a test cleavage was typically performed to monitor the reactions. The test cleavage cocktail was TFA/EDT/Thioanisol (90:3:7); 1,5 h shaking on a Thermomixer at room temperature and 750 rpm. Analysis was performed by LC-MS using one of the methods above.

### Cleavage of the peptide from the 2-chlorotrityl resin:

The resin containing the peptide was placed into a syringe and 3.0 mL of cleavage buffer HFIP/DCM (1:4) was added to the resin. The mixture was shaken at room temperature for 2.5 hours. The solution was collected by filtration and the resin washed with DCM (2 x 20 mL). The combined HFIP/DCM solution was concentrated using a rotary evaporator and washed with DCM and then concentrated (2 x 20 mL).

### Peptide Cyclization (Amide head-to-tail formation):

The crude peptide (510 mg) was dissolved into DMF (enough to dissolve it) and then divided and placed into two round-bottomed flasks. DIC (5 equiv), Oxyma (5 equiv) and DCM (1000 mL) were added to achieve a final concentration of 1 mg peptide/2 mL solution volume. The reaction mixture was shaken on an orbital shaker for 2 hours at rt. Additional DIC (5 equiv) and Oxyma (5 equiv) were added and the reaction mixture was further shaken overnight at rt. The reaction mixture was then evaporated to dryness using a rotary evaporator.

### Full Cleavage:

The crude peptide was placed into a 5 mL syringe and a mixture of cleavage buffer TFA/EDT/Thioanisol (90:3:7) (1 mL) was added, and then the solution was shaken for 2 hours at rt. The peptide was precipitated by adding cold diethyl ether (-20°C). The solution was centrifuged (3000 rpm) in a Falcon tube (60 mL) under a nitrogen atmosphere. The ether was decanted, and the solid residue was washed repeatedly with cold diethyl ether (5 x 10 mL). The solid residue was then dried.

### Disulfide Cyclization:

The crude peptide (173 mg) was dissolved in 0.1 M ammonium bicarbonate buffer (pH 7.8-8.2) (this example pH = 7.81) at a concentration of 1 mg/2 mL (350 mL). The solution was allowed to shake on an orbital shaker overnight in a round-bottomed flask open to the air. The solution was then lyophilized to obtain a white powder.

### Column Screening for HPLC Purification:

The peptide was dissolved in 5% CH₃CN and 95% water. Column screening was performed on each peptide to determine which preparative HPLC method to use for purification. The following analytical columns were screened.

### Two methods are available for column screening:

1) 5-60% ACN_8 Min_1 mL/min_25°C
2) 30-85% ACN_8 Min_1 mL/min_25°C

### Available columns (50mm x ID 4,6 mm) (available also as as Prep columns):

1) Aeris C18 (Phenomenex)
2) X-Bridge C18 (Waters)
3) Kinetex C18 (Coreshell Material) (Phenomenex)
4) YMC Triart C18 (elution with 100% water possible)
5) Kinetix Biphenyl (Phenomenex)
6) X-Select C18 (positive charge)
7) Jupiter Proteo C18 (Phenomenex)
8) Luna C18 (Phenomenex)

### For peptides of this invention, one of the following 5 preparative columns was used:

1) Column: Phenomenex, Aeris Peptide 5µ XB-C18, AXIA Packed, 21,2x250mm + Cartridge 5µ
2) Column: Phenomenex, Kinetex C18 5µ 21,5x250mm + Cartridge 5µ
3) Column: Phenomenex, Kinetex 5µ Biphenyl 100A, AXIA Packed, 21,2x250mm + Cartridge 5µ
4) Column: YMC Actus Triart Prep. C18 12nm, 8-10µm 250x20mm + Cartridge 3µm (10x4mm)
5) Column: Waters, Xbridge Prep.C18 5µ OBD 19x250mm + Cartridge 10µ

### Once the column was chosen, one of the following methods was used:

1) method: Gradient 5-60% ACN in water (0,10% TFA)
2) method: Gradient 30-85% ACN in water (0,10% TFA)
3) focused gradient based on results of the column screening.

### Flow rate 20 mL/min

The combined fractions were analyzed by HPLC (5-95 gradient in 8 min, Chromolith SpeedROD & Poroshell 120SB C18 5-95 in 18 min and using one of the LC-MS methods described above.

For Example 13, the crude peptide was dissolved in 30% CH3CN/water and purified on a Waters XBridge Prep C18 5µ, OBD 19x250mm + Cartridge 5µ, Flow rate: 20 mL/min, Method: 5-60% ACN in water (each containing 0.10% TFA) over 40 min. The combined fractions were lyophilized to provide 8.24 mg of example 13 (95% pure) and an additional 16.0 mg of 83-87% purity.

**Table 6: Note of materials used and conditions:**

| # | **Materials** | **Coupling reagents (2x for double coupling)** | **Coupling time** |
|---|---|---|---|
| 1 | Fmoc-Asp(Ot-Bu)-CT Resin loading 0.389 mmol/g (0.1 mmol scale) | --- | |
| 2 | Fmoc-Pro-OH (8.0 eq) | DIC (8.0 eq) Oxyma (8.0 eq) | 120 min |
| 3 | Fmoc-Ile-OH (8.0 eq) | DIC (8.0 eq) Oxyma (8.0 eq) | 120 min |
| 4 | Fmoc-Cys(Trt)-OH (8.0 eq) | DIC (8.0 eq) Oxyma (8.0 eq) | 120 min |
| 5 | Fmoc-Ile-OH (8.0 eq) | DIC (8.0 eq) Oxyma (8.0 eq) | 120 min |
| 6 | Fmoc-Oic-OH (8.0 eq) | DIC (8.0 eq) Oxyma (8.0 eq) | 120 min |
| 7 | Fmoc-Pro-OH (8.0 eq) | DIC (8.0 eq) Oxyma (8.0 eq) | 120 min |
| 8 | Fmoc-Leu-OH (8.0 eq) | DIC (8.0 eq) Oxyma (8.0 eq) | 120 min |
| 9 | Fmoc-Ser(tBu)-OH (8.0 eq) | DIC (8.0 eq) Oxyma (8.0 eq) | 120 min |
| 10 | Fmoc-Arg(Pbf)-OH (8.0 eq) | DIC (8.0 eq) Oxyma (8.0 eq) | 120 min |
| 11 | Fmoc-Ser(t-Bu)-OH (8.0 eq) | DIC (8.0 eq) Oxyma (8.0 eq) | 120 min |
| 12 | Fmoc-Cys(Trt)-OH (8.0 eq) | DIC (8.0 eq) Oxyma (8.0 eq) | 120 min |
| 13 | Fmoc-Ile-OH (8.0 eq) | DIC (8.0 eq) Oxyma (8.0 eq) | 120 min |
| 14 | Fmoc-6-Ahx-OH (3.0 eq) | DIC (3.0 eq) Oxyma (3.0 eq) | 120 min |
| 15 | Head-to-tail cyclization step | DIC (5.0 eq) Oxyma (5.0 eq) | 120 min + overnight |

When the first amino acid was not available as a preloaded resin, it was added manually and chlorotrityl resin was used for the synthesis.

### Loading of first amino acid on 2-chlorotrityl resin:

2-Chlortritylresin (500 mg, 0.775 mmol) was allowed to swell with 10 mL of DCM in a 50 mL Falcon tube for 15 min. The first amino acid (e.g. Fmoc-Ile-OH) (0.775 mmol) was dissolved in DCM with DIEA (6 equiv, 0.81 mL) added, and the solution was added to the resin. The solution was purged with argon and shaken overnight at room temperature. The mixture was filtered and washed with DMF (3 x 5 mL) and DCM (3 x 5 mL). Methanol was added (5 mL), the mixture was shaken for 30 minutes, and then filtered. The resin was washed with DMF (3 x 5 mL) and DCM (3 x 5 mL). Methanol was again added (5 mL), the mixture was shaken for 30 minutes, and then filtered. The resin was washed with DMF (3 x 5 mL) and DCM (3 x 5 mL). The loading was determined to be 0.42 mmol/g based on the determination of loading procedure described below.

The resin was then used for automated SPPS on the Symphony X synthesizer from Protein Technologies on 0.1 mmol scale.

### Determination of Resin Loading:

To determine the loading of a resin the FMOC protecting group is cleaved from a defined amount of resin and afterwards the concentration of the resulting fluorenyl compound in the supernatant cleavage solution is measured via photometry at 301nm. This correlates directly with the amount of amino acid loaded on the resin.
1) 1-3 mg of resin are weighed into a 2mL Eppendorf tube or similar (note the exact amount)
2) 1000 µL of a solution of 20% piperidine in DMF are added.
3) The mixture is agitated for 30 minutes to cleave the FMOC group.
4) 100 µL of the supernatant solution are then transferred into a quartz glass cuvette and diluted with 900µL of 20% piperidine / DMF
5) A blank sample of 1000µl piperidine / DMF is prepared in a second cuvette.
6) After determining the blank value from the reference sample, the extinction of the test sample at λ = 301nm is then measured in a UV-Vis photometer (Thermo Scientific Evolution 201).
7) For greater accuracy, multiple test samples (typically two) can be fashioned; the arithmetic mean of the measured extinctions is then used for calculation.

### Calculation of resin loading:

The resin loading L₃₀₁ in mmol/g is calculated by the following formula: ${L}_{301} = \frac{E \left(301nm\right) ⋅ V}{ε \left(301nm\right) ⋅ D ⋅ m} ⋅ VF ⋅ 1000$
E = extinction
ε = extinction coefficient at 301nm wavelength (7800 L/mol**cm)
m = amount of resin used (g)
V = volume of sample (L)
D = layer thickness of the cuvette (cm)
VF = dilution factor ( = 10)

### General Method for the Automated SPPS of Masp Peptides (Method B)

The synthesis of G**GIC+SRSLPPIC+IPD** (Example 15) is representative.

The synthesis is the same as method A except that a solution of 20% piperidine in DMF was used for the Fmoc cleavage steps.

Automated SPPS was performed on a Symphony X peptide synthesizer (Protein Technologies). Fmoc-Asp(O-tBu)-chlorotrityl resin was typically used (loading 0.3 - 0.8 mmol/gram) on a 0.1 mmol scale for peptides containing Asp at the C-terminus. For Example 15, the loading used was 0.80 mmol/gram. The resin was placed into the reaction vessel and placed onto the instrument. The following soluitons were prepared and used during the synthesis:
1) Fmoc Amino Acids: 0.2 M (8 eq)
2) Activator 1: 0.5 M DIC in DMF (7.5 eq - 8 eq)
3) Activator 2: 0.5 M Oxyma in DMF (7.5 eq - 8 eq)
4) Fmoc Deprotection: 20% piperidine in DMF

For Example 15, the crude peptide was dissolved in CH₃CN/water and purified on a Waters XBridge Prep C18 5µ, OBD 19x250mm + Cartridge 5µ, Flow rate: 20 mL/min, Method: 5-60% ACN in water (each containing 0.10% TFA) over 40 min. The combined fractions were lyophilized to provide 4.43 mg of example 15 (>99% pure).

### General Method for the Manual SPPS of Masp Peptides (Method C)

The synthesis of K++GIC+SRSLPPIC+IPD** (Example 22) is representative.

The linear synthesis of the peptide was carried out according to Method B. To prepare for the side-chain to tail amide cyclization, an orthogonally protected amino acid (e.g. in this case Boc-Lys(Fmoc) needs to be used. The cleavage step from the resin with HFIP/DCM is the same as exemplified in Method B.

### Peptide Cyclization (Side chain-to-tail amide formation):

The crude peptide (476 mg) was dissolved into DMF (enough to dissolve it) and then divided and placed into two round-bottomed flasks. DIC (5 equiv), Oxyma (5 equiv) and DCM (900 mL) were added to achieve a final concentration of 1 mg peptide/2 mL solution volume. Three round-bottemed flasks were used. The reaction mixtures were shaken shaken on an orbital shaker for 2 hours at rt. Additional DIC (5 equiv) and Oxyma (5 equiv) were added and the reaction mixture was further shaken overnight at rt. The reaction mixture was then evaporated to dryness using a rotary evaporator.

### Full Cleavage:

The crude peptide was placed into a 10 mL syringe and a mixture of cleavage buffer TFA/EDT/Thioanisol (90:3:7) (2 mL) was added, and then the solution was shaken for 2 hours at rt. The peptide was precipitated by adding cold diethyl ether (-20°C). The solution was centrifuged (3000 rpm) in a Falcon tube (60 mL) under a nitrogen atmosphere. The ether was decanted, and the solid residue was washed repeatedly with cold diethyl ether (5 x 10 mL). The solid residue was then dried.

### Disulfide Cyclization:

The crude peptide (280 mg) was dived into three portions and placed into 3 round-bottomed flasks (100 mg, 90 mg, 90 mg). The peptide was dissolved in 0.1 M ammonium bicarbonate buffer (pH 7.8-8.2) (this example pH = 7.77 - 7.83) at a concentration of 1 mg/2 mL (200 mL, 180 mL, 180 mL, respectively). The solutions were allowed to shake on an orbital shaker overnight in a round-bottomed flask open to the air. The combined solution was then lyophilized to obtain a white powder.

### Column Screening for HPLC Purification:

Column screening according to Method A led to the choice of YMC Triart Prep C18 5µ, 20x250mm + Cartridge 5µ as the best preparaie column for this peptide. The peptide was dissolved in 5% CH₃CN in water and purified using the YMC Triart column, Flow rate: 20 mL/min, Method: 5-60% ACN in water (each containing 0.10% TFA) over 40 min. The combined fractions were lyophilized to provide 50.36 mg mg of example 22 (> 99% pure).

**Table 7: Note of materials used and conditions:**

| # | **Materials** | **Coupling reagents (2x for double coupling)** | **Coupling time** |
|---|---|---|---|
| 1 | Fmoc-Asp(Ot-Bu)-CT Resin loading 0.380 mmol/g (0.1 mmol scale) | --- | |
| 2 | Fmoc-Pro-OH (8.0 eq) | DIC (8.0 eq) Oxyma (8.0 eq) | 120 min |
| 3 | Fmoc-Ile-OH (8.0 eq) | DIC (8.0 eq) Oxyma (8.0 eq) | 120 min |
| 4 | Fmoc-Cys(Trt)-OH (8.0 eq) | DIC (8.0 eq) Oxyma (8.0 eq) | 120 min |
| 5 | Fmoc-Ile-OH (8.0 eq) | DIC (8.0 eq) Oxyma (8.0 eq) | 120 min |
| 6 | Fmoc-Pro-OH (8.0 eq) | DIC (8.0 eq) Oxyma (8.0 eq) | 120 min |
| 7 | Fmoc-Pro-OH (8.0 eq) | DIC (8.0 eq) Oxyma (8.0 eq) | 120 min |
| 8 | Fmoc-Leu-OH (8.0 eq) | DIC (8.0 eq) Oxyma (8.0 eq) | 120 min |
| 9 | Fmoc-Ser(tBu)-OH (8.0 eq) | DIC (8.0 eq) Oxyma (8.0 eq) | 120 min |
| 10 | Fmoc-Arg(Pbf)-OH (8.0 eq) | DIC (8.0 eq) Oxyma (8.0 eq) | 120 min |
| 11 | Fmoc-Ser(t-Bu)-OH (8.0 eq) | DIC (8.0 eq) Oxyma (8.0 eq) | 120 min |
| 12 | Fmoc-Cys(Trt)-OH (8.0 eq) | DIC (8.0 eq) Oxyma (8.0 eq) | 120 min |
| 13 | Fmoc-Ile-OH (8.0 eq) | DIC (8.0 eq) Oxyma (8.0 eq) | 120 min |
| 14 | Fmoc-Gly-OH (8.0 eq) | DIC (8.0 eq) Oxyma (8.0 eq) | 120 min |
| 15 | Boc-Lys(Fmoc)-OH (8.0 eq) | DIC (8.0 eq) Oxyma (8.0 eq) | 120 min + overnight |
| 16 | Side chain-to-tail cyclization step | DIC (5.0 eq) Oxyma (5.0 eq) | 120 min + overnight |

### General Method for the Manual SPPS of Masp Peptides (Method D)

The synthesis of (Ahx)**-GIC+SRS-((tBu)A)-PPI-((N-Me)C)+-IPD** (Example 30) is representative.

### Peptide Synthesis:

The peptide was synthesized using standard Fmoc chemistry.
1) Resin preparation: To the 2-chlorotrityl resin (0.267 g, 0.30 mmol, loading = 1.12 mmol/g) was added the amino amino acid (Fmoc-Asp(Ot-Bu)-OH) (1 equiv), DIEA (4 equiv), and DCM (15 mL), and the mixture was agitated with N₂ bubbling for 0.5 h at 20°C. The column reaction solution was removed, and the column was washed with DCM. Then a solution of 1:1 DCM/MeOH was added and nitrogen bubbling continued for 30 min (to cap the resin). The resin was aspirated to remove the DCM/MeOH, then washed with DCM, and DMF. Then 20% piperidine in DMF (10 mL) was added and the mixture was agitated with N₂ for 20 min at 20°C. The solution was removed, and the resin was washed with DMF (10 mL x 5) and vacuum filtered to get the resin.
2) Coupling: The next amino acid in the sequence, Fmoc-Pro-OH (0.9 mmol, 0.303 g, 3.0 eq) HBTU (0.33 g, 0.85 mmol, 2.85 eq) and DIEA (1.80 mmol, 0.31 mL, 6.00 eq) in DMF (30 mL) was added to the resin and agitated with N₂ for 30 min at 20°C. The resin was then washed with DMF (30 mL x 3).
3) Deprotection: 20% piperidine in DMF (10.0 mL) was added to the resin and the mixture was agitated with N₂ for 20 min at 20 °C.
4) Repeat Step 2 to 3 for all other amino acids.

**Table 8: Note of materials used and conditions:**

| # | **Materials** | **Coupling reagents** | **Coupling time** |
|---|---|---|---|
| 1 | Fmoc-Asp(Ot-Bu)-OH (1.0 eq) | HBTU(2.85 eq) DIEA(6.0 eq) | 30 min |
| 2 | Fmoc-Pro-OH (3.0 eq) | HBTU(2.85 eq) DIEA(6.0 eq) | 30 min |
| 3 | Fmoc-Ile-OH (3.0 eq) | HBTU(2.85 eq) DIEA(6.0 eq) | 30 min |
| 4 | Fmoc -Cys(N-Me)-OH (3.0 eq) | HBTU(2.85 eq) DIEA(6.0 eq) | 30 min |
| 5 | Fmoc-Ile-OH (3.0 eq) | HBTU(2.85 eq) DIEA(6.0 eq) | 30 min |
| 6 | Fmoc-Pro-OH (3.0 eq) | HBTU(2.85 eq) DIEA(6.0 eq) | 30 min |
| 7 | Fmoc-Pro-OH (3.0 eq) | HBTU(2.85 eq) DIEA(6.0 eq) | 30 min |
| 8 | Fmoc-Ala(t-Bu)-OH (3.0 eq) | HBTU(2.85 eq) DIEA(6.0 eq) | 30 min |
| 9 | Fmoc-Ser(tBu)-OH (3.0 eq) | HBTU(2.85 eq) DIEA(6.0 eq) | 30 min |
| 10 | Fmoc-Arg(Pbf)-OH (3.0 eq) | HBTU(2.85 eq) DIEA(6.0 eq) | 30 min |
| 11 | Fmoc-Ser(tBu)-OH (3.0 eq) | HBTU(2.85 eq) DIEA(6.0 eq) | 30 min |
| 12 | Fmoc-Cys(Trt)-OH (3.0 eq) | HBTU(2.85 eq) DIEA(6.0 eq) | 30 min |
| 13 | Fmoc-Ile-OH (3.0 eq) | HBTU(2.85 eq) DIEA(6.0 eq) | 30 min |
| 14 | Fmoc-Gly-OH (3.0 eq) | HBTU(2.85 eq) DIEA(6.0 eq) | 30 min |
| 15 | Fmoc-Ahx-OH (3.0 eq) | HBTU(2.85 eq) DIEA(6.0 eq) | 30 min |

20% piperidine in DMF was used for Fmoc deprotection for 30 min. The coupling reaction was monitored by ninhydrin (all amino acids except Pro) and chloranil test (Pro), and the resin was washed with DMF (5.0 mL) for 5 times.

### Peptide Cleavage:

After the peptide elongation was finished, the resin was washed with MeOH (10 mL x 3) and dried under vacuum to get the peptide resin. Then 10.0 mL of cleavage buffer 1% TFA/DCM was added to the vessel containing the resin and the mixture allowed to swell for 10 min. The mixture was filtered and the filtrate was collected. The process was repeated and the combined filtrate was used for the next step.

### Amide Cyclization and Protecting Group Removal:

The peptide was diluted with DCM to adjust the peptide concentration to ImM. DIEA was added to adjust the pH to about 8. Then TBTU (289 mg, 3.0 eq) and HOBT (122 mg, 3.0 eq) were added to the solution and the reaction mixture was allowed to react for about 3 h. Then the solution was washed with IN HCl (1 x 150 mL) and the organic layer was collected and concentrated under vacuum. The resulting residue was treated with a cocktail of 90%TFA/5%TIPS/2.5%H₂O/2.5%EDT (10 mL) and swelled for about 2 h. The crude peptide was was precipitated with cold tert-butyl methyl ether (50 mL) and centrifuged (3 min at 3000 rpm) to get the solid crude peptide. The crude peptide precipitate was washed with tert-butyl methyl ether for three more times (20.0 mL x 3), and then the crude peptide was dried under vacuum.

### Disulfide bond formation:

The crude peptide was dissolved in H₂O/ACN (1:1) to adjust the concentration to 1 mM. Then 1 M NH4HCO3 was added to the above solution to adjust the pH to about 8-9. The solution was allowed to react for about 8 h at room temperature. The reaction was monitored by LC-MS. After the reaction was complete, the reaction was quenched by adding acetic acid to adjust the pH to about 6. The reaction mixture was then lyophilized, and the resulting solid was resulting solid was purified by reversed-phase HPLC.

### Purification:

The crude peptide was purified by preparative HPLC (conditions: A: 0.075% TFA in water B: CH₃CN) and lyophilized to obtain 74.4 mg (97.2% pure by Method 7; 94.4% pure by Method 8) of the desired peptide (Example 30) as a white solid and TFA salt. Purification conditions: Peptide was dissolved in TFA/H₂O (7:3); flow rate 20 mL/min; gradient 12-42% over 60 min; Run time= 42 min; purification over a Luna 25 x 200 mm, C18 10 um, 110 Å column.

### General Method for the Manual SPPS of Masp Peptides (Method E)

The synthesis of A**GAIC+SRS-((tBu)A)-PPI-((N-Me)C)+-IPD** (Example 31) is representative.

### Peptide Synthesis:

The peptide was synthesized using standard Fmoc chemistry.
1) Resin preparation: To the 2-chlorotrityl resin (0.267 g, 0.30 mmol, loading = 1.12 mmol/g) was added the amino amino acid (Fmoc-Asp(Ot-Bu)-OH) (1 equiv), DIEA (4 equiv), and DCM (15 mL), and the mixture was agitated with N₂ bubbling for 0.5 h at 20°C. The column reaction solution was removed, and the column was washed with DCM. Then a solution of 1:1 DCM/MeOH was added and nitrogen bubbling continued for 30 min (to cap the resin). The resin was aspirated to remove the DCM/MeOH, and then washed sequentially with DCM and DMF. Then 20% piperidine in DMF (10 mL) was added and the mixture was agitated with N₂ for 20 min at 20°C. The reaction solvents were removed by vacuum filtration and the resin was washed with DMF (10 mL x 5), and then filtered to get the resin.
2) Coupling: The next amino acid in the sequence, Fmoc-Pro-OH (0.9 mmol, 0.303 g, 3.0 eq) HBTU (0.33 g, 0.88 mmol, 2.95 eq) and DIEA (1.80 mmol, 0.31 mL, 6.00 eq) in DMF (30 mL) was added to the resin and agitated with N₂ for 30 min at 20°C. The resin was then washed with DMF (30 mL x 3).
3) Deprotection: 20% piperidine in DMF (10.0 mL) was added to the resin and the mixture was agitated with N₂ for 20 min at 20 °C.
4) Repeat Step 2 to 3 for all other amino acids.

**Table 9: Note of materials used and conditions:**

| # | **Materials** | **Coupling reagents** | **Coupling time** |
|---|---|---|---|
| 1 | Fmoc-Asp(Ot-Bu)-OH (1.0 eq) | HBTU(2.95 eq) DIEA(6.0 eq) | 30 min |
| 2 | Fmoc-Pro-OH (3.0 eq) | HBTU(2.95 eq) DIEA(6.0 eq) | 30 min |
| 3 | Fmoc-Ile-OH (3.0 eq) | HBTU(2.95 eq) DIEA(6.0 eq) | 30 min |
| 4 | Fmoc -Cys(N-Me)-OH (3.0 eq) | HBTU(2.95 eq) DIEA(6.0 eq) | 30 min |
| 5 | Fmoc-Ile-OH (3.0 eq) | HBTU(2.95 eq) DIEA(6.0 eq) | 30 min |
| 6 | Fmoc-Pro-OH (3.0 eq) | HBTU(2.95 eq) DIEA(6.0 eq) | 30 min |
| 7 | Fmoc-Pro-OH (3.0 eq) | HBTU(2.95 eq) DIEA(6.0 eq) | 30 min |
| 8 | Fmoc-Ala(t-Bu)-OH (3.0 eq) | HBTU(2.95 eq) DIEA(6.0 eq) | 30 min |
| 9 | Fmoc-Ser(tBu)-OH (3.0 eq) | HBTU(2.95 eq) DIEA(6.0 eq) | 30 min |
| 10 | Fmoc-Arg(Pbf)-OH (3.0 eq) | HBTU(2.95 eq) DIEA(6.0 eq) | 30 min |
| 11 | Fmoc-Ser(tBu)-OH (3.0 eq) | HBTU(2.95 eq) DIEA(6.0 eq) | 30 min |
| 12 | Fmoc-Cys(Trt)-OH (3.0 eq) | HBTU(2.95 eq) DIEA(6.0 eq) | 30 min |
| 13 | Fmoc-Ile-OH (3.0 eq) | HBTU(2.95 eq) DIEA(6.0 eq) | 30 min |
| 14 | Fmoc-Gly-OH (3.0 eq) | HBTU(2.95 eq) DIEA(6.0 eq) | 30 min |
| 15 | Fmoc-Ahx-OH (3.0 eq) | HBTU(2.95 eq) DIEA(6.0 eq) | 30 min |

20% piperidine in DMF was used for Fmoc deprotection for 30 min. The coupling reaction was monitored by ninhydrin (all amino acids except Pro) and chloranil test (Pro), and the resin was washed with DMF (5.0 mL) for 5 times.

### Peptide Cleavage:

After the peptide elongation was finished, the resin was washed with MeOH (10 mL x 3) and dried under vacuum to get the peptide resin. Then 10.0 mL of cleavage buffer 1% TFA/DCM was added to the vessel containing the resin and the mixture allowed to swell for 10 min. The mixture was filtered, and the filtrate was collected. The process was repeated, and the combined filtrate was used for the next step.

### Amide Cyclization and Protecting Group Removal:

The peptide was diluted with DCM to adjust the peptide concentration to ImM. DIEA was added to adjust the pH to about 8. Then TBTU (289 mg, 3.0 eq) and HOBT (122 mg, 3.0 eq) were added to the solution and the reaction mixture was allowed to react for about 3 h. Then the solution was washed with IN HCl (1 x 150 mL) and the organic layer was collected and concentrated under vacuum. The resulting residue was treated with a cocktail of 90%TFA/5%TIPS/2.5%H₂O/2.5%EDT (10 mL) and swelled for about 2 h. The crude peptide was was precipitated with cold tert-butyl methyl ether (50 mL) and centrifuged (3 min at 3000 rpm) to get the solid crude peptide. The crude peptide precipitate was washed with tert-butyl methyl ether for three more times (20.0 mL x 3), and then the crude peptide was dried under vacuum.

### Disulfide bond formation:

The crude peptide was dissolved in H₂O/ACN (1:1) to adjust the concentration to 1 mM. Then 1 M NH4HCO3 was added to the above solution to adjust the pH to about 8-9. The solution was allowed to react for about 8 h at room temperature. The reaction was monitored by LC-MS. After the reaction was complete, the reaction was quenched by adding acetic acid to adjust the pH to about 6. The reaction mixture was then lyophilized, and the resulting solid was resulting solid was purified by reversed-phase HPLC.

### Purification:

The crude peptide was purified by preparative HPLC (conditions: A: 0.075% TFA in water B: CH₃CN) and lyophilized to obtain 36.2 mg (95.0% pure by Method 7; 92.80% pure by Method 8) of the desired peptide (Example 30) as a white solid and TFA salt. Purification conditions: Peptide was dissolved in TFA/H₂O (7:3); flow rate 20 mL/min; gradient 12-42% over 60 min; Run time= 42 min; purification over a Luna 25 x 200 mm, C18 10 um, 110 Å column.

### General Method for the Manual SPPS of Masp Peptides (Method F)

The synthesis of (PEG1(10 atoms))**-AIC+SRS-((tBu)A)-PPI-(Pen)+-IPD** (Example 34) is representative.

### Peptide Synthesis:

The peptide was synthesized using standard Fmoc chemistry.
1) Resin preparation: 2-Chlorotrityl resin is allowed to swell in DCM in a column for 30 min, and then the DCM was pushed from the column with nitrogen. To the 2-chlorotrityl resin (0.267 g, 0.30 mmol, loading = 1.12 mmol/g) was added the amino amino acid (Fmoc-Asp(Ot-Bu)-OH) (1 equiv), DIEA (4 equiv), and DCM (10 mL), and the mixture was agitated with N₂ bubbling for 0.5 h at 20°C. The reaction solution was removed, and the resin was washed with DCM. Then a solution of 1:1 DCM/MeOH was added and nitrogen bubbling continued for 30 min (to cap the resin).
2) The solvents were removed from the column and the resin was washed sequentially with DCM (10 mL) and DMF (10 mL x 3).
3) A 20% piperidine solution in DMF (10 mL) was added and the mixture was agitated with N₂ for 20 min at rt.
4) The solvent from the column was removed and the resin was washed with DMF (10 mL x 5) and filtered to get the resin.
5) Preparing (or activating) the amino acid: the next amino acid Fmoc-Pro-OH (0.9 mmol, 3 equiv) and HBTU (2.95 eq) were weighed out and then dissolved in DMF. DIEA (6 equiv) was added to the above solution. The activated solution was then added to the column containing the resin and reacted for about 2 h.
6) The solvents from the column were removed and the resin was washed with DMF (10 mL x 3).
7) Repeat steps 3-6 for all other amino acids in the sequence

**Table 10: Note of materials used and conditions:**

| # | **Materials** | **Coupling reagents** | **Coupling time** |
|---|---|---|---|
| 1 | Fmoc-Asp(Ot-Bu)-OH (1.0 eq) | HBTU(2.95 eq) DIEA(6.0 eq) | 30 min |
| 2 | Fmoc-Pro-OH (3.0 eq) | HBTU(2.95 eq) DIEA(6.0 eq) | 30 min |
| 3 | Fmoc-Ile-OH (3.0 eq) | HBTU(2.95 eq) DIEA(6.0 eq) | 30 min |
| 4 | Fmoc-Pen(Trt)-OH (3.0 eq) | HBTU(2.95 eq) DIEA(6.0 eq) | 30 min |
| 5 | Fmoc-Ile-OH (3.0 eq) | HBTU(2.95 eq) DIEA(6.0 eq) | 30 min |
| 6 | Fmoc-Pro-OH (3.0 eq) | HBTU(2.95 eq) DIEA(6.0 eq) | 30 min |
| 7 | Fmoc-Pro-OH (3.0 eq) | HBTU(2.95 eq) DIEA(6.0 eq) | 30 min |
| 8 | Fmoc-Ala(t-Bu)-OH (3.0 eq) | HBTU(2.95 eq) DIEA(6.0 eq) | 30 min |
| 9 | Fmoc-Ser(tBu)-OH (3.0 eq) | HBTU(2.95 eq) DIEA(6.0 eq) | 30 min |
| 10 | Fmoc-Arg(Pbf)-OH (3.0 eq) | HBTU(2.95 eq) DIEA(6.0 eq) | 30 min |
| 11 | Fmoc-Ser(tBu)-OH (3.0 eq) | HBTU(2.95 eq) DIEA(6.0 eq) | 30 min |
| 12 | Fmoc-Cys(Trt)-OH (3.0 eq) | HBTU(2.95 eq) DIEA(6.0 eq) | 30 min |
| 13 | Fmoc-Ile-OH (3.0 eq) | HBTU(2.95 eq) DIEA(6.0 eq) | 30 min |
| 14 | Fmoc-Ala-OH (3.0 eq) | HBTU(2.95 eq) DIEA(6.0 eq) | 30 min |
| 15 | Fmoc-PEG1(10 atoms)-OH (3.0 eq) | HBTU(2.95 eq) DIEA(6.0 eq) | 30 min |

20% piperidine in DMF was used for Fmoc deprotection for 30 min. The coupling reaction was monitored by ninhydrin (all amino acids except Pro) and chloranil test (Pro), and the resin was washed with DMF (5.0 mL) for 5 times.

### Peptide Cleavage:

After the peptide elongation was finished, the resin was washed with MeOH (10 mL x 3) and dried under vacuum to get the peptide resin. Then 10.0 mL of cleavage buffer 1% TFA/DCM was added to the vessel containing the resin and the mixture allowed to swell for 10 min. The mixture was filtered, and the filtrate was collected. The process was repeated, and the combined filtrate was used for the next step.

### Amide Cyclization and Protecting Group Removal:

The peptide was diluted with DCM to adjust the peptide concentration to ImM. DIEA was added to adjust the pH to about 8. Then TBTU (289 mg, 3.0 eq) and HOBT (122 mg, 3.0 eq) were added to the solution and the reaction mixture was allowed to react for about 3 h. Then the solution was washed with IN HCl (1 x 150 mL) and the organic layer was collected and concentrated under vacuum. The resulting residue was treated with a cocktail of 90%TFA/5%TIPS/2.5%H₂O/2.5%EDT (10 mL) and swelled for about 2 h. The crude peptide was was precipitated with cold tert-butyl methyl ether (50 mL) and centrifuged (3 min at 3000 rpm) to get the solid crude peptide. The crude peptide precipitate was washed with tert-butyl methyl ether for three more times (20.0 mL x 3), and then the crude peptide was dried under vacuum.

### Disulfide bond formation:

The crude peptide was dissolved in H₂O/ACN (1:1) to adjust the concentration to 1 mM. Then 1 M NH₄HCO₃ was added to the above solution to adjust the pH to about 8-9. The solution was allowed to react for about 8 h at room temperature. The reaction was monitored by LC-MS. After the reaction was complete, the reaction was quenched by adding acetic acid to adjust the pH to about 6. The reaction mixture was then lyophilized, and the resulting solid was resulting solid was purified by reversed-phase HPLC.

### Purification:

The crude peptide was purified by preparative HPLC (conditions: A: 0.075% TFA in water B: CH₃CN) and lyophilized to obtain 133.1 mg (96.80% pure by Method 7; 94.90% pure by Method 8) of the desired peptide (Example 34) as a white solid and TFA salt. Purification conditions: Peptide was dissolved in TFA/H₂O (7:3); flow rate 20 mL/min; gradient 12-42% over 60 min; Run time= 42 min; purification over a Luna 25 x 200 mm, C18 10 um, 110 Å column.

### General Method for the Manual SPPS of Masp Peptides (Method G)

The synthesis of A**GAIC+SRSLP-(Oic)-I-(Pen)+-IPD++-NH₂ (Example 41) is representative.

### Peptide Synthesis:

The peptide was synthesized using standard Fmoc chemistry.
1) Resin preparation: MBHA Rink Amide Resin (666.67 mg, 0.30 mmol, loading 0.45 mmol/g) is allowed to swell in DMF in a column for 30 min, and then the DMF was pushed from the column with nitrogen.
2) A 20% piperidine solution in DMF (10 mL) was added and the mixture was agitated with N₂ for 20 min at rt to cleave the Fmoc group.
3) After the Fmoc group was removed, the solvent from the column was removed and the resin was washed with DMF (10 mL x 3) and then removed.
4) Preparing (or activating) the amino acid: the first amino acid Fmoc-Asp(OAlly)-OH (0.9 mmol, 3 equiv) and HBTU (2.95 eq) were weighed out and then dissolved in DMF. DIEA (6 equiv) was added to the above solution. The activated solution was then added to the column containing the resin and reacted for about 2 h.
5) The solvents from the column were removed and the resin was washed with DMF (10 mL x 3).
6) Repeat steps 2-5 for all other amino acids in the sequence.

**Table 11: Note of materials used and conditions:**

| # | **Materials** | **Coupling reagents** | **Coupling time** |
|---|---|---|---|
| 1 | Fmoc-Asp(OAllyl)-OH (3.0 eq) | HBTU(2.95 eq) DIEA(6.0 eq) | 30 min |
| 2 | Fmoc-Pro-OH (3.0 eq) | HBTU(2.95 eq) DIEA(6.0 eq) | 30 min |
| 3 | Fmoc-Ile-OH (3.0 eq) | HBTU(2.95 eq) DIEA(6.0 eq) | 30 min |
| 4 | Fmoc-Pen(Trt)-OH (3.0 eq) | HBTU(2.95 eq) DIEA(6.0 eq) | 30 min |
| 5 | Fmoc-Ile-OH (3.0 eq) | HBTU(2.95 eq) DIEA(6.0 eq) | 30 min |
| 6 | Fmoc-Oic-OH (3.0 eq) | HBTU(2.95 eq) DIEA(6.0 eq) | 30 min |
| 7 | Fmoc-Pro-OH (3.0 eq) | HBTU(2.95 eq) DIEA(6.0 eq) | 30 min |
| 8 | Fmoc-Leu-OH (3.0 eq) | HBTU(2.95 eq) DIEA(6.0 eq) | 30 min |
| 9 | Fmoc-Ser(tBu)-OH (3.0 eq) | HBTU(2.95 eq) DIEA(6.0 eq) | 30 min |
| 10 | Fmoc-Arg(Pbf)-OH (3.0 eq) | HBTU(2.95 eq) DIEA(6.0 eq) | 30 min |
| 11 | Fmoc-Ser(tBu)-OH (3.0 eq) | HBTU(2.95 eq) DIEA(6.0 eq) | 30 min |
| 12 | Fmoc-Cys(Trt)-OH (3.0 eq) | HBTU(2.95 eq) DIEA(6.0 eq) | 30 min |
| 13 | Fmoc-Ile-OH (3.0 eq) | HBTU(2.95 eq) DIEA(6.0 eq) | 30 min |
| 14 | Fmoc-Ala-OH (3.0 eq) | HBTU(2.95 eq) DIEA(6.0 eq) | 30 min |
| 15 | Fmoc-Gly-OH (3.0 eq) | HBTU(2.95 eq) DIEA(6.0 eq) | 30 min |
| 16 | Fmoc-Ala-OH (3.0 eq) | HBTU(2.95 eq) DIEA(6.0 eq) | 30 min |

20% piperidine in DMF was used for Fmoc deprotection for 30 min. The coupling reaction was monitored by ninhydrin (all amino acids except Pro) and chloranil test (Pro), and the resin was washed with DMF (5.0 mL) for 5 times.

### Allyl Group Cleavage:

A solution of Pd(PPh₃)₄ (69.3 mg, 0.2 equiv) and PhSiH (324.6 mg,10 eq) in DCM (20 mL) was added to the column and the mixture was bubbled with nitrogen for 2 h. The solution was drained and washed with DCM three times. The process was repeated two more times.

The resin was washed with DCM (10 mL) 3 times, then washed with a solution of 0.5% sodium diethyldithiocarbamate trihydrate and 0.5% DIEA (1:1) in DMF (10 mL 3 times.

The resin was then washed with MeOH 3 times and then dried.

### Amide Cyclization (on resin):

A solution of PyBOP (468 mg, 3.0 eq) and DIEA (6.0 eq) in DMF was added to the resin and the reaction mixture was agitated with nitrogen bubbling. The reaction progress was monitored using LC-MS. When the reaction was completed, the DMF solution was drained and the resin was washed with DMF (10 mL x 3 mL), DCM (10 mL x 3), and then dried.

### Peptide Cleavage:

The resin was treated with a cocktail of 90%TFA/5%TIPS/2.5%H₂O/2.5%EDT (10 mL) and swelled for about 2 h. The solution was collected and the crude peptide was was precipitated with cold tert-butyl methyl ether (50 mL) and centrifuged (3 min at 3000 rpm) to get the solid crude peptide. The crude peptide precipitate was washed with tert-butyl methyl ether for three more times (20.0 mL x 3), and then the crude peptide was dried under vacuum.

### Disulfide bond formation:

The crude peptide was dissolved in H₂O/ACN (1:1) (300 mL) to adjust the concentration to 1 mM. Then 1 M NH4HCO3 was added to the above solution to adjust the pH to about 8-9. The solution was allowed to react for about 10 h at room temperature. The reaction was monitored by LC-MS. After the reaction was complete, the reaction was quenched by adding acetic acid to adjust the pH to about 6-7. The reaction mixture was then lyophilized, and the resulting solid was resulting solid was purified by reversed-phase HPLC.

### Purification:

The crude peptide was purified by preparative HPLC (conditions: A: 0.075% TFA in water B: CH₃CN) and lyophilized to obtain 10.3 mg (95.20% pure by Method 7; 95.30% pure by Method 8) of the desired peptide (Example 41) as a white solid and TFA salt. Purification conditions: Peptide was dissolved in TFA/H₂O (7:3); flow rate 20 mL/min; gradient 12-42% over 60 min; Run time= 42 min; purification over a Luna 25 x 200 mm, C18 10 um, 110 Å column.

### General Method for the Manual SPPS of Masp Peptides (Method H)

The synthesis of (Dap)**-IC+SRS-((tBu)A)-PPI-(Pen)+-IP** (Example 55) is representative.

### Peptide Synthesis:

The peptide was synthesized using standard Fmoc chemistry.
1) Resin preparation: 2-Chlorotrityl resin (0.667 g, 0.30 mmol, loading = 0.45 mmol/g) was allowed to swell for 30 min in DCM, and then the DCM was removed. To the 2-chlorotrityl resin was added the amino amino acid (Fmoc-Pro-OH) (3 equiv), DIEA (4 equiv), and DCM (15 mL), and the mixture was agitated with N₂ bubbling for 0.5 h at 20°C. The reaction solution was removed, and the resin was washed with DCM. Then a solution of 1:1 DCM/MeOH was added and nitrogen bubbling continued for 30 min (to cap the resin).
2) The solvents were removed from the column and the resin was washed sequentially with DCM (10 mL) and DMF (10 mL x 3).
3) A 20% piperidine solution in DMF (10 mL) was added and the mixture was agitated with N₂ for 20 min at rt.
4) The solvent from the column was removed and the resin was washed with DMF (10 mL x 5) and filtered to get the resin.
5) Preparing (or activating) the amino acid: the next amino acid Fmoc-Ile-OH (0.9 mmol, 3 equiv) and HBTU (2.95 eq) were weighed out and then dissolved in DMF. DIEA (6 equiv) was added to the above solution. The activated solution was then added to the column containing the resin and reacted for about 2 h.
6) The solvents from the column were removed and the resin was washed with DMF (10 mL x 3).
7) Repeat steps 3-6 for all other amino acids in the sequence.

**Table 12: Note of materials used and conditions:**

| # | **Materials** | **Coupling reagents** | **Coupling time** |
|---|---|---|---|
| 1 | Fmoc-Pro-OH (3.0 eq) | HBTU(2.95 eq) DIEA(6.0 eq) | 30 min |
| 2 | Fmoc-Ile-OH (3.0 eq) | HBTU(2.95 eq) DIEA(6.0 eq) | 30 min |
| 3 | Fmoc-Pen(Trt)-OH (3.0 eq) | HBTU(2.95 eq) DIEA(6.0 eq) | 30 min |
| 4 | Fmoc-Ile-OH (3.0 eq) | HBTU(2.95 eq) DIEA(6.0 eq) | 30 min |
| 5 | Fmoc-Pro-OH (3.0 eq) | HBTU(2.95 eq) DIEA(6.0 eq) | 30 min |
| 6 | Fmoc-Pro-OH (3.0 eq) | HBTU(2.95 eq) DIEA(6.0 eq) | 30 min |
| 7 | Fmoc-Ala(t-Bu)-OH (3.0 eq) | HBTU(2.95 eq) DIEA(6.0 eq) | 30 min |
| 8 | Fmoc-Ser(tBu)-OH (3.0 eq) | HBTU(2.95 eq) DIEA(6.0 eq) | 30 min |
| 9 | Fmoc-Arg(Pbf)-OH (3.0 eq) | HBTU(2.95 eq) DIEA(6.0 eq) | 30 min |
| 10 | Fmoc-Ser(tBu)-OH (3.0 eq) | HBTU(2.95 eq) DIEA(6.0 eq) | 30 min |
| 11 | Fmoc-Cys(Trt)-OH (3.0 eq) | HBTU(2.95 eq) DIEA(6.0 eq) | 30 min |
| 12 | Fmoc-Ile-OH (3.0 eq) | HBTU(2.95 eq) DIEA(6.0 eq) | 30 min |
| 13 | Fmoc-Dap(Boc)-OH (3.0 eq) | HBTU(2.95 eq) DIEA(6.0 eq) | 30 min |

20% piperidine in DMF was used for Fmoc deprotection for 30 min. The coupling reaction was monitored by ninhydrin (all amino acids except Pro) and chloranil test (Pro), and the resin was washed with DMF (5.0 mL) for 5 times.

### Peptide Cleavage:

After the peptide elongation was finished, the resin was washed with MeOH (10 mL x 3) and dried under vacuum to get the peptide resin. Then 10.0 mL of cleavage buffer 1% TFA/DCM was added to the vessel containing the resin and the mixture allowed to swell for 10 min. The mixture was filtered, and the filtrate was collected. The process was repeated, and the combined filtrate was used for the next step.

### Amide Cyclization and Protecting Group Removal:

The peptide was diluted with DCM to adjust the peptide concentration to ImM. DIEA was added to adjust the pH to about 8. Then TBTU (289 mg, 3.0 eq) and HOBT (122 mg, 3.0 eq) were added to the solution and the reaction mixture was allowed to react for about 3 h. Then the solution was washed with IN HCl (1 x 150 mL) and the organic layer was collected and concentrated under vacuum. The resulting residue was treated with a cocktail of 90%TFA/5%TIPS/2.5%H₂O/2.5%EDT (10 mL) and swelled for about 2 h. The crude peptide was was precipitated with cold tert-butyl methyl ether (50 mL) and centrifuged (3 min at 3000 rpm) to get the solid crude peptide. The crude peptide precipitate was washed with tert-butyl methyl ether for three more times (20.0 mL x 3), and then the crude peptide was dried under vacuum.

### Disulfide bond formation:

The crude peptide was dissolved in H₂O/ACN (1:1) to adjust the concentration to 1 mM. To the above solution was slowly added 0.5 MI₂/MeOH solution until the solution was turned to yellow. The progress of the reaction was monitored by LC-MS. After the reaction was completed, the reaction mixture was quenched by adding 1 M Na₂S₂O₃ until the solution turned to colorless. The reaction mixture was then lyophilized, and the resulting solid was resulting solid was purified by reversed-phase HPLC.

### Purification:

The crude peptide was purified by preparative HPLC (conditions: A: 0.075% TFA in water B: CH₃CN) and lyophilized to obtain 36.2 mg (95.0% pure by Method 7; 92.80% pure by Method 8) of the desired peptide (Example 30) as a white solid and TFA salt. Purification conditions: Peptide was dissolved in TFA/H₂O (7:3); flow rate 20 mL/min; gradient 12-42% over 60 min; Run time= 42 min; purification over a Luna 25 x 200 mm, C18 10 um, 110 Å column.

### General Method for the Manual SPPS of Masp Peptides (Method I)

Synthesis of (Adipic acid)**-IC+SRS-((tBu)A)-PPI-(Pen)+-IPD-(Dap)++-NH₂ (Example 60) is representative.

### Peptide Synthesis:

The peptide was synthesized using standard Fmoc chemistry.
1) Resin preparation: MBHA Rink Amide Resin (666.67 mg, 0.30 mmol, loading 0.45 mmol/g) is allowed to swell in DMF in a column for 30 min, and then the DMF was pushed from the column with nitrogen.
2) A 20% piperidine solution in DMF (10 mL) was added and the mixture was agitated with N₂ for 20 min at rt to cleave the Fmoc group.
3) After the Fmoc group was removed, the solvent from the column was removed and the resin was washed with DMF (10 mL x 3) and then removed.
4) Preparing (or activating) the amino acid: the first amino acid Fmoc-Dap(Dde)-OH (0.9 mmol, 3 equiv) and HBTU (2.95 eq) were weighed out and then dissolved in DMF. DIEA (6 equiv) was added to the above solution. The activated solution was then added to the column containing the resin and reacted for about 2 h.
5) The solvents from the column were removed and the resin was washed with DMF (10 mL x 3).
6) Repeat steps 2-5 for all other amino acids in the sequence.

**Table 13: Note of materials used and conditions:**

| # | **Materials** | **Coupling reagents** | **Coupling time** |
|---|---|---|---|
| 1 | Fmoc-Dap(Dde)-OH | HBTU(2.95 eq) DIEA(6.0 eq) | 30 min |
| 2 | Fmoc-Asp(Ot-Bu)-OH | HBTU(2.95 eq) DIEA(6.0 eq) | 30 min |
| 3 | Fmoc-Pro-OH (3.0 eq) | HBTU(2.95 eq) DIEA(6.0 eq) | 30 min |
| 4 | Fmoc-Ile-OH (3.0 eq) | HBTU(2.95 eq) DIEA(6.0 eq) | 30 min |
| 5 | Fmoc-Pen(Trt)-OH (3.0 eq) | HBTU(2.95 eq) DIEA(6.0 eq) | 30 min |
| 6 | Fmoc-Ile-OH (3.0 eq) | HBTU(2.95 eq) DIEA(6.0 eq) | 30 min |
| 7 | Fmoc-Pro-OH (3.0 eq) | HBTU(2.95 eq) DIEA(6.0 eq) | 30 min |
| 8 | Fmoc-Pro-OH (3.0 eq) | HBTU(2.95 eq) DIEA(6.0 eq) | 30 min |
| 9 | Fmoc-Ala(t-Bu)-OH (3.0 eq) | HBTU(2.95 eq) DIEA(6.0 eq) | 30 min |
| 10 | Fmoc-Ser(tBu)-OH (3.0 eq) | HBTU(2.95 eq) DIEA(6.0 eq) | 30 min |
| 11 | Fmoc-Arg(Pbf)-OH (3.0 eq) | HBTU(2.95 eq) DIEA(6.0 eq) | 30 min |
| 12 | Fmoc-Ser(tBu)-OH (3.0 eq) | HBTU(2.95 eq) DIEA(6.0 eq) | 30 min |
| 13 | Fmoc-Cys(Trt)-OH (3.0 eq) | HBTU(2.95 eq) DIEA(6.0 eq) | 30 min |
| 14 | Fmoc-Ile-OH (3.0 eq) | HBTU(2.95 eq) DIEA(6.0 eq) | 30 min |
| 15 | Adipic acid anhydride (3.0 eq) | | 30 min |

20% piperidine in DMF was used for Fmoc deprotection for 30 min. The coupling reaction was monitored by ninhydrin (all amino acids except Pro) and chloranil test (Pro), and the resin was washed with DMF (5.0 mL) for 5 times.

### Acetylation:

A solution cocktail of adipic acid anhydride (246 mg, 3 equiv) in NMM/DMF (15:85) was prepared and added to the resin and allowed to react for 30 min with nitrogen bubbling. The solvents were removed and the resin was washed with DMF (3x).

### Dde Group Cleavage:

A solution of 3% hydrazine hydrate in DMF was prepared and added to the resin. After 20 min, the solution was removed. The process was repeated one more time. The column was then washed with DMF (3x).

### Amide Cyclization (on resin):

A solution of PyBOP (468 mg, 3.0 eq) and DIEA (6.0 eq) in DMF was added to the resin and the reaction mixture was agitated with nitrogen bubbling. The reaction progress was monitored using LC-MS. When the reaction was completed, the DMF solution was drained and the resin was washed with MeOH (10 mL x 3 mL), DCM (10 mL x 3), and then dried.

### Peptide Cleavage:

The resin was treated with a cocktail of 90%TFA/5%TIPS/2.5%H₂O/2.5%EDT (10 mL) and swelled for about 2 h. The solution was collected, and the crude peptide was was precipitated with cold tert-butyl methyl ether (50 mL) and centrifuged (3 min at 3000 rpm) to get the solid crude peptide. The crude peptide precipitate was washed with tert-butyl methyl ether for three more times (20.0 mL x 3), and then the crude peptide was dried under vacuum.

### Disulfide bond formation:

The crude peptide was dissolved in H₂O/ACN (1:1) (300 mL) to adjust the concentration to 1 mM. Then 1 M NH₄HCO₃ was added to the above solution to adjust the pH to about 8-9. The solution was allowed to react for about 10 h at room temperature. The reaction was monitored by LC-MS. After the reaction was complete, the reaction was quenched by adding acetic acid to adjust the pH to about 6-7. The reaction mixture was then lyophilized, and the resulting solid was resulting solid was purified by reversed-phase HPLC.

### Purification:

The crude peptide was purified by preparative HPLC (conditions: A: 0.075% TFA in water B: CH₃CN) and lyophilized to obtain 12.8 mg (98.10% pure by Method 7; 92.80% pure by Method 8) of the desired peptide (Example 60) as a white solid and TFA salt. Purification conditions: Peptide was dissolved in TFA/H₂O (7:3); flow rate 20 mL/min; gradient 12-42% over 60 min; Run time= 42 min; purification over a Luna 25 x 200 mm, C18 10 um, 110 Å column.

### General Method for the Manual SPPS of Masp Peptides (Method J)

Synthesis of G**-(TXA)-GIC+SRS-((tBu)A)-PPI-((N-Me)C)+-IPD++-NH₂ (Example 69) is representative.

### Peptide Synthesis:

The peptide was synthesized using standard Fmoc chemistry.
1) Resin preparation: MBHA Rink Amide Resin (666.67 mg, 0.30 mmol, loading 0.45 mmol/g) is allowed to swell in DMF in a column for 30 min, and then the DMF was pushed from the column with nitrogen.
2) A 20% piperidine solution in DMF (10 mL) was added and the mixture was agitated with N₂ for 20 min at rt to cleave the Fmoc group.
3) After the Fmoc group was removed, the solvent from the column was removed and the resin was washed with DMF (10 mL x 3) and then removed.
4) Preparing (or activating) the amino acid: the first amino acid Fmoc-Asp(OAlly)-OH (0.9 mmol, 3 equiv) and HBTU (2.95 eq) were weighed out and then dissolved in DMF. DIEA (6 equiv) was added to the above solution. The activated solution was then added to the column containing the resin and reacted for about 2 h.
5) The solvents from the column were removed and the resin was washed with DMF (10 mL x 3).
6) Repeat steps 2-5 for all other amino acids in the sequence.

**Table 14: Note of materials used and conditions:**

| # | **Materials** | **Coupling reagents** | **Coupling time** |
|---|---|---|---|
| 1 | Fmoc-Asp(OAllyl)-OH (3.0 eq) | HBTU(2.95 eq) DIEA(6.0 eq) | 30 min |
| 2 | Fmoc-Pro-OH (3.0 eq) | HBTU(2.95 eq) DIEA(6.0 eq) | 30 min |
| 3 | Fmoc-Ile-OH (3.0 eq) | HBTU(2.95 eq) DIEA(6.0 eq) | 30 min |
| 4 | Fmoc-(N-Me)Cys(Trt)-OH (3.0 eq) | HBTU(2.95 eq) DIEA(6.0 eq) | 30 min |
| 5 | Fmoc-Ile-OH (3.0 eq) | HBTU(2.95 eq) DIEA(6.0 eq) | 30 min |
| 6 | Fmoc-Pro-OH (3.0 eq) | HBTU(2.95 eq) DIEA(6.0 eq) | 30 min |
| 7 | Fmoc-Pro-OH (3.0 eq) | HBTU(2.95 eq) DIEA(6.0 eq) | 30 min |
| 8 | Fmoc-Ala(t-Bu)-OH (3.0 eq) | HBTU(2.95 eq) DIEA(6.0 eq) | 30 min |
| 9 | Fmoc-Ser(tBu)-OH (3.0 eq) | HBTU(2.95 eq) DIEA(6.0 eq) | 30 min |
| 10 | Fmoc-Arg(Pbf)-OH (3.0 eq) | HBTU(2.95 eq) DIEA(6.0 eq) | 30 min |
| 11 | Fmoc-Ser(tBu)-OH (3.0 eq) | HBTU(2.95 eq) DIEA(6.0 eq) | 30 min |
| 12 | Fmoc-Cys(Trt)-OH (3.0 eq) | HBTU(2.95 eq) DIEA(6.0 eq) | 30 min |
| 13 | Fmoc-Ile-OH (3.0 eq) | HBTU(2.95 eq) DIEA(6.0 eq) | 30 min |
| 14 | Fmoc-Ala-OH (3.0 eq) | HBTU(2.95 eq) DIEA(6.0 eq) | 30 min |
| 15 | Fmoc-Gly-OH (3.0 eq) | HBTU(2.95 eq) DIEA(6.0 eq) | 30 min |
| 16 | Fmoc-TXA-OH (3.0 eq) | HBTU(2.95 eq) DIEA(6.0 eq) | 30 min |
| 17 | Fmoc-Gly-OH (3.0 eq) | HBTU(2.95 eq) DIEA(6.0 eq) | 30 min |

20% piperidine in DMF was used for Fmoc deprotection for 30 min. The coupling reaction was monitored by ninhydrin (all amino acids except Pro) and chloranil test (Pro), and the resin was washed with DMF (5.0 mL) for 5 times.

### Allyl Group Cleavage:

A solution of Pd(PPh₃)₄ (69.3 mg, 0.2 equiv) and PhSiH (324.6 mg,10 eq) in DCM (20 mL) was added to the column and the mixture was bubbled with nitrogen for 2 h. The solution was drained and washed with DCM three times. The process was repeated two more times.

The resin was washed with DCM (10 mL) 3 times, then washed with a solution of 0.5% sodium diethyldithiocarbamate trihydrate in DMF (10 mL) and with 0.5% DIEA in DMF (10 mL). The resin was washed alternating twice more each with 0.5% sodium diethyldithiocarbamate trihydrate in DMF (10 mL) and with 0.5% DIEA in DMF (10 mL).

The resin was then washed with MeOH 3 times, and then it was dried.

### Amide Cyclization (on resin):

A solution of PyBOP (468 mg, 3.0 eq) and DIEA (6.0 eq) in DMF was added to the resin and the reaction mixture was agitated with nitrogen bubbling. The reaction progress was monitored using LC-MS. When the reaction was completed, the DMF solution was drained and the resin was washed with DMF (10 mL x 3 mL), DCM (10 mL x 3), and then dried.

### Peptide Cleavage:

The resin was treated with a cocktail of 90%TFA/5%TIPS/2.5%H₂O/2.5%EDT (10 mL) and swelled for about 2 h. The solution was collected,2580 and the crude peptide was was precipitated with cold tert-butyl methyl ether (50 mL) and centrifuged (3 min at 3000 rpm) to get the solid crude peptide. The crude peptide precipitate was washed with tert-butyl methyl ether for three more times (20.0 mL x 3), and then the crude peptide was dried under vacuum.

### Disulfide bond formation:

The crude peptide was dissolved in H₂O/ACN (1:1) (300 mL) to adjust the concentration to 1 mM. Then 1 M NH₄HCO₃ was added to the above solution to adjust the pH to about 8-9. The solution was allowed to react for about 10 h at room temperature. The reaction was monitored by LC-MS. After the reaction was complete, the reaction was quenched by adding acetic acid to adjust the pH to about 6-7. The reaction mixture was then lyophilized, and the resulting solid was resulting solid was purified by reversed-phase HPLC.

### Purification:

The crude peptide was purified by preparative HPLC (conditions: A: 0.075% TFA in water B: CH₃CN) and lyophilized to obtain 10.3 mg (95.20% pure by Method 7; 95.30% pure by Method 8) of the desired peptide (Example 41) as a white solid and TFA salt. Purification conditions: Peptide was dissolved in TFA/H₂O (7:3); flow rate 20 mL/min; gradient 12-42% over 60 min; Run time= 42 min; purification over a Luna 25 x 200 mm, C18 10 um, 110 Å column.

### General Method for the Manual SPPS of Masp Peptides (Method K)

The synthesis of C++AIC+SRS-((tBu)A)-PPI-(Pen)+-IPDC++-NH₂ (Example 50) is representative.

### Peptide Synthesis:

The peptide was synthesized using standard Fmoc chemistry.
1) Resin preparation: MBHA Rink Amide Resin (666.67 mg, 0.30 mmol, loading 0.45 mmol/g) is allowed to swell in DMF in a column for 30 min, and then the DMF was pushed from the column with nitrogen.
2) A 20% piperidine solution in DMF (10 mL) was added and the mixture was agitated with N₂ for 20 min at rt to cleave the Fmoc group.
3) After the Fmoc group was removed, the solvent from the column was removed and the resin was washed with DMF (10 mL x 3) and then removed.
4) Preparing (or activating) the amino acid: the first amino acid Fmoc-Cys(Acm)-OH (0.9 mmol, 3 equiv) and HBTU (2.95 eq) were weighed out and then dissolved in DMF. DIEA (6 equiv) was added to the above solution. The activated solution was then added to the column containing the resin and reacted for about 2 h.
5) The solvents from the column were removed and the resin was washed with DMF (10 mL x 3).
6) Repeat steps 2-5 for all other amino acids in the sequence.

**Table 15: Note of materials used and conditions:**

| # | **Materials** | **Coupling reagents** | **Coupling time** |
|---|---|---|---|
| 1 | Fmoc-Cys(Acm)-OH | HBTU(2.95 eq) DIEA(6.0 eq) | 30 min |
| 2 | Fmoc-Asp(Ot-Bu)-OH (3.0 eq) | HBTU(2.95 eq) DIEA(6.0 eq) | 30 min |
| 3 | Fmoc-Pro-OH (3.0 eq) | HBTU(2.95 eq) DIEA(6.0 eq) | 30 min |
| 4 | Fmoc-Ile-OH (3.0 eq) | HBTU(2.95 eq) DIEA(6.0 eq) | 30 min |
| 5 | Fmoc-Pen(Trt)-OH (3.0 eq) | HBTU(2.95 eq) DIEA(6.0 eq) | 30 min |
| 6 | Fmoc-Ile-OH (3.0 eq) | HBTU(2.95 eq) DIEA(6.0 eq) | 30 min |
| 7 | Fmoc-Pro-OH (3.0 eq) | HBTU(2.95 eq) DIEA(6.0 eq) | 30 min |
| 8 | Fmoc-Pro-OH (3.0 eq) | HBTU(2.95 eq) DIEA(6.0 eq) | 30 min |
| 9 | Fmoc-Ala(t-Bu)-OH (3.0 eq) | HBTU(2.95 eq) DIEA(6.0 eq) | 30 min |
| 10 | Fmoc-Ser(tBu)-OH (3.0 eq) | HBTU(2.95 eq) DIEA(6.0 eq) | 30 min |
| 11 | Fmoc-Arg(Pbf)-OH (3.0 eq) | HBTU(2.95 eq) DIEA(6.0 eq) | 30 min |
| 12 | Fmoc-Ser(tBu)-OH (3.0 eq) | HBTU(2.95 eq) DIEA(6.0 eq) | 30 min |
| 13 | Fmoc-Cys(Trt)-OH (3.0 eq) | HBTU(2.95 eq) DIEA(6.0 eq) | 30 min |
| 14 | Fmoc-Ile-OH (3.0 eq) | HBTU(2.95 eq) DIEA(6.0 eq) | 30 min |
| 15 | Fmoc-Ala-OH (3.0 eq) | HBTU(2.95 eq) DIEA(6.0 eq) | 30 min |
| 16 | Fmoc-Gly-OH (3.0 eq) | HBTU(2.95 eq) DIEA(6.0 eq) | 30 min |
| 17 | Fmoc-Ala-OH (3.0 eq) | HBTU(2.95 eq) DIEA(6.0 eq) | 30 min |
| 18 | Fmoc-Cys(Acm)-OH | HBTU(2.95 eq) DIEA(6.0 eq) | 30 min |

20% piperidine in DMF was used for Fmoc deprotection for 30 min. The coupling reaction was monitored by ninhydrin (all amino acids except Pro) and chloranil test (Pro), and the resin was washed with DMF (5.0 mL) for 5 times.

### Peptide Cleavage:

The resin was treated with a cocktail of 90%TFA/5%TIPS/2.5%H₂O/2.5%EDT (10 mL) and swelled for about 2 h. The solution was collected, and the crude peptide was was precipitated with cold tert-butyl methyl ether (50 mL) and centrifuged (3 min at 3000 rpm) to get the solid crude peptide. The crude peptide precipitate was washed with tert-butyl methyl ether for three more times (20.0 mL x 3), and then the crude peptide was dried under vacuum.

### 1^{st} Disulfide bond formation:

The crude peptide was dissolved in H₂O/ACN (1:1) (300 mL) to adjust the concentration to 1 mM. Then 1 M NH₄HCO₃ was added to the above solution to adjust the pH to about 8-9. The solution was allowed to react for about 8 h at room temperature. The reaction was monitored by LC-MS. After the reaction was complete, the reaction was quenched by adding acetic acid to adjust the pH to about 6-7. The reaction mixture was then lyophilized.

### Purification:

The crude peptide was purified by preparative HPLC (conditions: A: 0.075% TFA in water B: CH₃CN) and lyophilized to obtain 10.3 mg (95.20% pure by Method 7; 95.30% pure by Method 8) of the desired peptide (Example 41) as a white solid and TFA salt. Purification conditions: Peptide was dissolved in TFA/H₂O (7:3); flow rate 20 mL/min; gradient 12-42% over 60 min; Run time= 42 min; purification over a Luna 25 x 200 mm, C18 10 um, 110 Å column.

### 2^{nd} Disulfide bond formation:

To the purified peptide in water and CH₃CN (10 mg/ml, H₂O:CH₃CN=0.7:0.3) was added a solution of Iodine (I₂) in AcOH (0.1 M) dropwise at 20°C. The reaction progress was monitored by LC-MS. When LC-MS indicated that the reaction was competed, the reaction mixture was then lyophilized, and the resulting solid was resulting solid was purified by reversed-phase HPLC.

### Purification:

The crude peptide was purified by preparative HPLC (conditions: A: 0.075% TFA in water B: CH₃CN) and lyophilized to obtain 10.2 mg (95.0% pure by Method 7; 96.8% pure by Method 8) of the desired peptide (Example 50) as a white solid and TFA salt. Purification conditions: Peptide was dissolved in TFA/H₂O (7:3); flow rate 20 mL/min; gradient 12-42% over 60 min; Run time= 42 min; purification over a Luna 25 x 200 mm, C18 10 um, 110 Å column.

### General Method for the Manual SPPS of Masp Peptides (Method L)

The synthesis of (3-Azido-L-Alanine)++-GAIC+SRS-((tBu)A)-PPIC+IP-(L-Propargylglycine)++-NH₂ (1,2,3-triazole-1,4-diyl) (Example 81) is representative.

### Peptide Synthesis:

The peptide was synthesized using standard Fmoc chemistry.
1) Resin preparation: MBHA Rink Amide Resin (666.67 mg, 0.30 mmol, loading 0.45 mmol/g) is allowed to swell in DMF in a column for 30 min, and then the DMF was pushed from the column with nitrogen.
2) A 20% piperidine solution in DMF (10 mL) was added and the mixture was agitated with N₂ for 20 min at rt to cleave the Fmoc group.
3) After the Fmoc group was removed, the solvent from the column was removed and the resin was washed with DMF (10 mL x 3) and then removed.
4) Preparing (or activating) the amino acid: the first amino acid Fmoc-Asp(OAlly)-OH (0.9 mmol, 3 equiv) and HBTU (2.95 eq) were weighed out and then dissolved in DMF. DIEA (6 equiv) was added to the above solution. The activated solution was then added to the column containing the resin and reacted for about 2 h.
5) The solvents from the column were removed and the resin was washed with DMF (10 mL x 3).
6) Repeat steps 2-5 for all other amino acids in the sequence.

**Table 16: Note of materials used and conditions:**

| # | **Materials** | **Coupling reagents** | **Coupling time** |
|---|---|---|---|
| 1 | Fmoc-(Propargyl-Gly)-OH (3.0 eq) | HBTU(2.95 eq) DIEA(6.0 eq) | 30 min |
| 2 | Fmoc-Pro-OH (3.0 eq) | HBTU(2.95 eq) DIEA(6.0 eq) | 30 min |
| 3 | Fmoc-Ile-OH (3.0 eq) | HBTU(2.95 eq) DIEA(6.0 eq) | 30 min |
| 4 | Fmoc-Cys(Acm)-OH (3.0 eq) | HBTU(2.95 eq) DIEA(6.0 eq) | 30 min |
| 5 | Fmoc-Ile-OH (3.0 eq) | HBTU(2.95 eq) DIEA(6.0 eq) | 30 min |
| 6 | Fmoc-Pro-OH (3.0 eq) | HBTU(2.95 eq) DIEA(6.0 eq) | 30 min |
| 7 | Fmoc-Pro-OH (3.0 eq) | HBTU(2.95 eq) DIEA(6.0 eq) | 30 min |
| 8 | Fmoc-Ala(t-Bu)-OH (3.0 eq) | HBTU(2.95 eq) DIEA(6.0 eq) | 30 min |
| 9 | Fmoc-Ser(tBu)-OH (3.0 eq) | HBTU(2.95 eq) DIEA(6.0 eq) | 30 min |
| 10 | Fmoc-Arg(Pbf)-OH (3.0 eq) | HBTU(2.95 eq) DIEA(6.0 eq) | 30 min |
| 11 | Fmoc-Ser(tBu)-OH (3.0 eq) | HBTU(2.95 eq) DIEA(6.0 eq) | 30 min |
| 12 | Fmoc-Cys(Acm)-OH (3.0 eq) | HBTU(2.95 eq) DIEA(6.0 eq) | 30 min |
| 13 | Fmoc-Ile-OH (3.0 eq) | HBTU(2.95 eq) DIEA(6.0 eq) | 30 min |
| 14 | Fmoc-Ala-OH (3.0 eq) | HBTU(2.95 eq) DIEA(6.0 eq) | 30 min |
| 15 | Fmoc-Gly-OH (3.0 eq) | HBTU(2.95 eq) DIEA(6.0 eq) | 30 min |
| 16 | Fmoc-3-Azido-Alanine-OH (3.0 eq) | HBTU(2.95 eq) DIEA(6.0 eq) | 30 min |

20% piperidine in DMF was used for Fmoc deprotection for 30 min. The coupling reaction was monitored by ninhydrin (all amino acids except Pro) and chloranil test (Pro), and the resin was washed with DMF (5.0 mL) for 5 times.

### Peptide Cleavage:

The resin was treated with a cocktail of 90%TFA/5%TIPS/2.5%H₂O/2.5%EDT (10 mL) and swelled for about 2 h. The solution was collected, and the crude peptide was was precipitated with cold tert-butyl methyl ether (50 mL) and centrifuged (3 min at 3000 rpm) to get the solid crude peptide. The crude peptide precipitate was washed with tert-butyl methyl ether for three more times (20.0 mL x 3), and then the crude peptide was dried under vacuum.

### Click Reaction:

The crude peptide was dissolved in water-t-BuOH (2:1) and treated with CuSO₄ x 5H₂O (10 equiv) and ascorbic acid (10 equiv). The reaction mixture was strirred overnight, concentrated, and then lyophilized.

### Disulfide bond formation:

To the crude peptide in water and CH₃CN (10 mg/ml, H₂O:CH₃CN=0.7:0.3) was added a solution of Iodine (I₂) in AcOH (0.1 M) dropwise at 20°C. The reaction progress was monitored by LC-MS. When LC-MS indicated that the reaction was competed, the reaction mixture was then lyophilized, and the resulting solid was resulting solid was purified by reversed-phase HPLC.

### Purification:

The crude peptide was purified by preparative HPLC (conditions: A: 0.075% TFA in water B: CH₃CN) and lyophilized to obtain 5.0 mg (95.10% pure by Method 7; 95.80% pure by Method 8) of the desired peptide (Example 41) as a white solid and TFA salt. Purification conditions: Peptide was dissolved in TFA/H₂O (7:3); flow rate 20 mL/min; gradient 12-42% over 60 min; Run time= 42 min; purification over a Luna 25 x 200 mm, C18 10 um, 110 Å column.

### General Method for the Manual SPPS of Masp Peptides (Method M)

The synthesis of sequence (Ahx)**-AIC+SRSLP-(Oic)-IC+IP** is representative (Example 25).

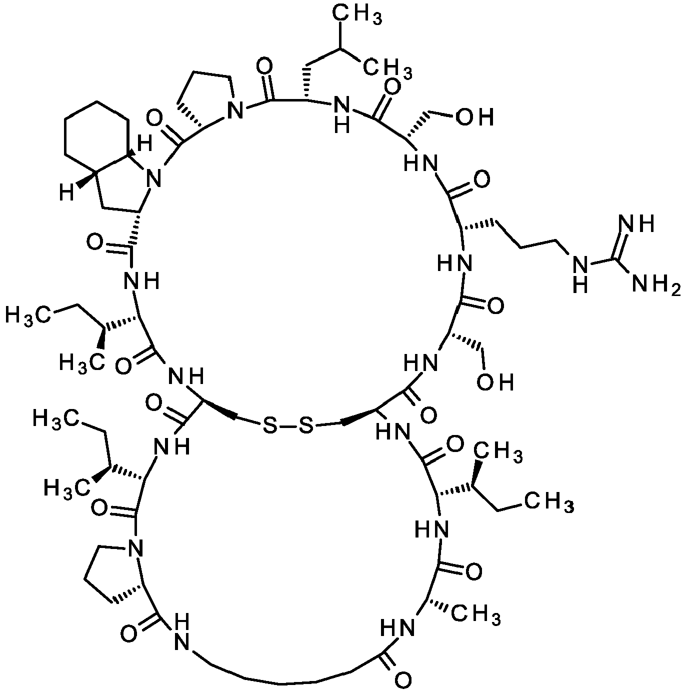

### Peptide Synthesis:

The peptide was synthesized using standard Fmoc chemistry.

The dark ball in the chemical structures below indicates the solid polymer support used for solid-phase peptide synthesis (SPPS), e.g. 2-chlorotrityl resin, Rink amide resin, etc.

At various points in the synthesis, a small amount of resin was taken and the sample treated with DCM/HFIP (4:1) to prepare an LC-MS sample for reaction monitoring.

### Example 1A

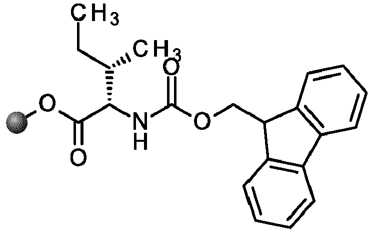

The reaction was carried out under an argon atmosphere. To a solution of N-[(9H-fluoren-9-ylmethoxy)carbonyl]-L-isoleucine (9.401 g, 26.6 mmol) in absolute dichloromethane (53 mL, 0.5 molar solution) was added N,N-diisopropylethylamine (18.533 mL, 106.4 mmol). The 2-chlorotrityl chloride resin (10g, 13.3mmol) was then added, and the mixture was shaken overnight at room temperature under argon atmosphere. The resin was aspirated and washed three times with DMF. The resin was shaken together with a solution of 1:1 DCM/MeOH for 30 min (to cap the resin). The resin was aspirated to remove the DCM/MeOH and washed with MeOH and DCM. After the final DCM wash, the resin was first dried using a rotary evaporator and then further dried under high vacuum, providing 15.16 g of resin. The loading was determined to be 0.45 mmol/g using the method described in Method B.

### Example 2A

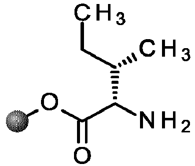

To the resin from Example 1A (15.16 g, 6.822 mmol) was added a solution of DMF/piperidine (4:1, 200 mL) and the mixture was shaken for 15 min at rt. The solution was removed by aspiration and the resin was washed three times thoroughly with DMF. The Fmoc deprotection procedure was repeated once more under the same conditions. The DMF/piperidine solution was removed by aspiration and the resin was washed three times with DMF (200 mL). Then resin was then washed with MeOH (200 mL) and DCM (200 mL). The washing with MeOH and DCM was repeated two more times. After the final DCM wash, the resin was dried using a rotary evaporator, providing 11.4 g of resin.

### Example 3A

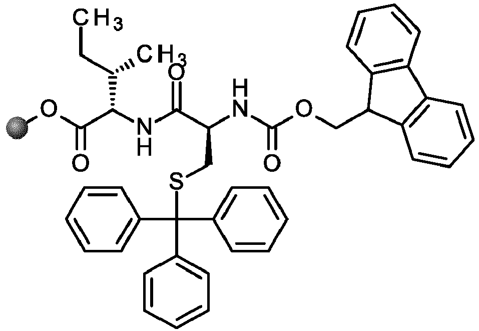

To the resin from Example 2A (11.4 g, 5.13 mmol) was added DMF (100 mL) and the resin was allowed to swell for 5 minutes. A solution of N-[(9H-fluoren-9-ylmethoxy)carbonyl]-S-tritylcysteine (6.01 g, 10.26 mmol) in DMF (50 mL) was added, followed by the addition of N,N'-diisopropylcarbodiimide (1.549 mL, 10.004 mmol) and ethyl-(hydroxyimino)cyanoacetate (1.422 g, 10.004 mmol). The mixture was shaken for 2 h at room temperature. The reaction mixure was aspirated and the resin was washed three times thoroughly with DMF. The coupling process was repeated using the same conditions. The resin was aspirated to remove the solution and the resin was washed three times with DMF (150 mL). The resin was further washed with MeOH (150 mL) DCM (150 mL). The washing with MeOH and DCM was repeated two more times. After the final DCM wash, the resin was dried using a rotary evaporator, the further dried under high vacuum, providing 18.9 g of resin.

### Example 4A

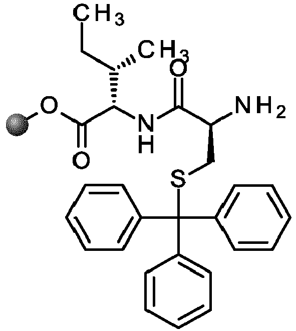

To the resin from Example 3A (18.9 g, 8.505 mmol) was added a solution of DMF/piperidine (4:1, 200 mL) and the mixture was shaken for 15 min at rt. The solution was removed by aspiration and the resin was washed three times thoroughly with DMF. The Fmoc deprotection procedure was repeated once more under the same conditions. The DMF/piperidine solution was removed by aspiration and the resin was washed three times with DMF (200 mL). Then resin was then washed with MeOH (200 mL) and DCM (200 mL). The washing with MeOH and DCM was repeated two more times. After the final DCM wash, the resin was dried using a rotary evaporator, providing 16.27 g of resin.

### Example 5A

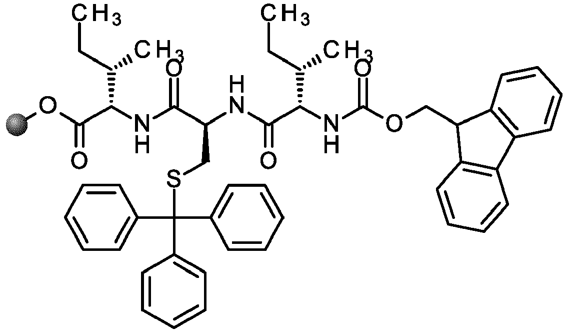

To the resin from Example 4A (16.27 g, 7.32 mmol) was added DMF (150 mL) and the resin was allowed to swell for 5 minutes. A solution of N-[(9H-fluoren-9-ylmethoxy)carbonyl]-L-isoleucine (5.17 g, 14.64 mmol) in DMF (50 mL) was added, followed by the addition of N,N'-diisopropylcarbodiimide (2.211 mL, 14.277 mmol) and ethyl-(hydroxyimino)cyanoacetate (2.029g, 14.277mmol). The mixture was shaken for 2 h at room temperature. The reaction mixure was aspirated and the resin was washed three times thoroughly with DMF. The coupling process was repeated using the same conditions. The resin was aspirated to remove the solution and the resin was washed three times with DMF (200 mL). The resin was further washed with MeOH (200 mL) DCM (200 mL). The washing with MeOH and DCM was repeated two more times. After the final DCM wash, the resin was dried using a rotary evaporator, the further dried under high vacuum, providing 19.96 g of resin. The loading test was repeated using the method described in General Method 2 and determined to be 0.388 mmol/g.

### Example 6A

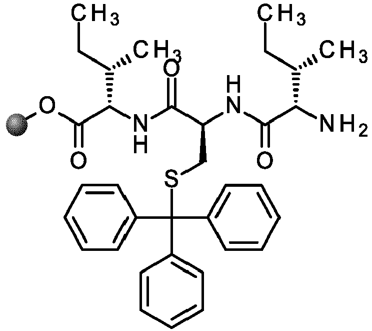

To the resin from Example 5A (19.96 g, 8.982 mmol) was added a solution of DMF/piperidine (4:1, 200 mL) and the mixture was shaken for 15 min at rt. The solution was removed by aspiration and the resin was washed three times thoroughly with DMF. The Fmoc deprotection procedure was repeated once more under the same conditions. The DMF/piperidine solution was removed by aspiration and the resin was washed three times with DMF (200 mL). Then resin was then washed with MeOH (200 mL) and DCM (200 mL). The washing with MeOH and DCM was repeated two more times. After the final DCM wash, the resin was dried using a rotary evaporator, providing 17.75 g of resin.

### Example 7A

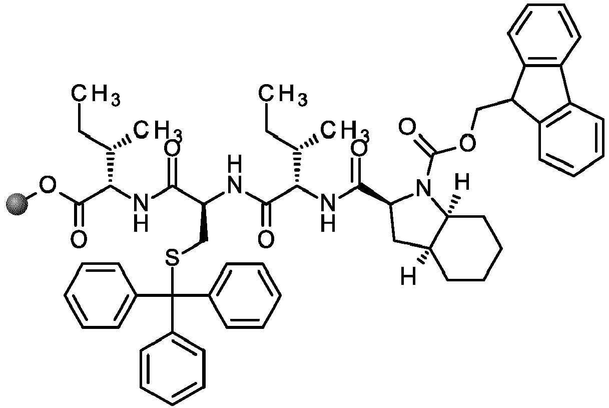

To the resin from Example 6A (17.75 g, 7.988 mmol) was added DMF (150 mL) and the resin was allowed to swell for 5 minutes. A solution of (2S,3aS,7aS)-1-[(9H-fluoren-9-ylmethoxy)carbonyl]-octahydro-1H-indole-2-carboxylic acid (6.254 g, 15.975 mmol) in DMF (50 mL) was added, followed by the addition of N,N'-diisopropylcarbodiimide (2.412 mL, 15.576 mmol) and ethyl-(hydroxyimino)cyanoacetate 2.213 g, 15.576 mmol). The mixture was shaken for 2 h at room temperature. The reaction mixure was aspirated and the resin was washed three times thoroughly with DMF. The coupling process was repeated using the same conditions. The resin was aspirated to remove the solution and the resin was washed three times with DMF (200 mL). The resin was further washed with MeOH (200 mL) DCM (200 mL). The washing with MeOH and DCM was repeated two more times. After the final DCM wash, the resin was dried using a rotary evaporator, the further dried under high vacuum, providing 21.22 g of resin.

### Example 8A

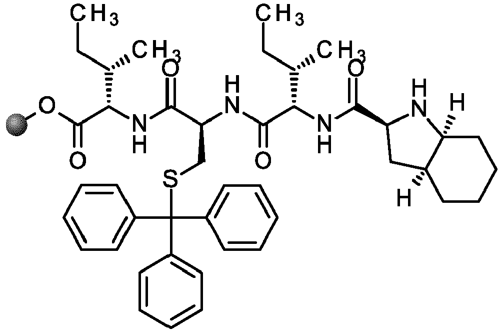

To the resin from Example 7A (21.5g, 9.675mmol) was added a solution of DMF/piperidine (4:1, 200 mL) and the mixture was shaken for 30 min at rt. The solution was removed by aspiration and the resin was washed three times thoroughly with DMF. The Fmoc deprotection procedure was repeated once more under the same conditions. The DMF/piperidine solution was removed by aspiration and the resin was washed three times with DMF (200 mL). Then resin was then washed with MeOH (200 mL) and DCM (200 mL). The washing with MeOH and DCM was repeated two more times. After the final DCM wash, the resin was dried using a rotary evaporator, providing 18.07 g of resin.

### Example 9A

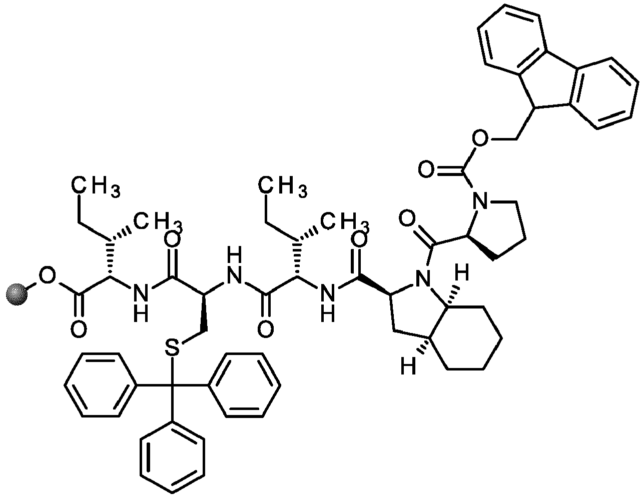

To the resin from Example 8A (18.07 g, 8.132 mmol) was added DMF (150 mL) and the resin was allowed to swell for 5 minutes. A solution of 1-[(9H-fluoren-9-ylmethoxy)carbonyl]-L-proline (10.974 g, 32.526 mmol) in DMF (50 mL) was added, followed by the addition of N,N'-diisopropylcarbodiimide (4.911 mL, 31.713 mmol) and ethyl-(hydroxyimino)cyanoacetate (4.507 g, 31.713 mmol). The mixture was shaken for 2 h at room temperature. The reaction mixure was aspirated and the resin was washed three times thoroughly with DMF. The coupling process was repeated using the same conditions. The resin was aspirated to remove the solution and the resin was washed three times with DMF (200 mL). The resin was further washed with MeOH (200 mL) DCM (200 mL). The washing with MeOH and DCM was repeated two more times. After the final DCM wash, the resin was dried using a rotary evaporator, the further dried under high vacuum, providing 20.36 g of resin.

### Example 10A

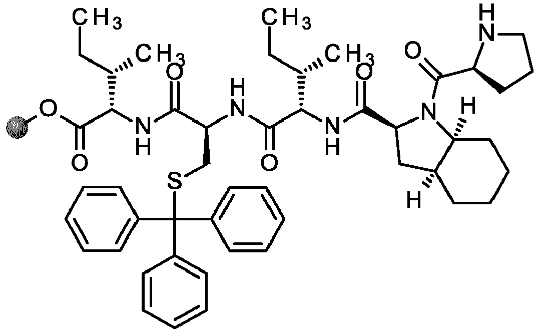

To the resin from Example 9A (20.36 g, 9.162 mmol) was added a solution of DMF/piperidine (4:1, 150 mL) and the mixture was shaken for 30 min at rt. The solution was removed by aspiration and the resin was washed three times thoroughly with DMF. The Fmoc deprotection procedure was repeated once more under the same conditions. The DMF/piperidine solution was removed by aspiration and the resin was washed three times with DMF (200 mL). Then resin was then washed with MeOH (200 mL) and DCM (200 mL). The washing with MeOH and DCM was repeated two more times. After the final DCM wash, the resin was dried using a rotary evaporator, providing 18.18 g of resin.

### Example 11A

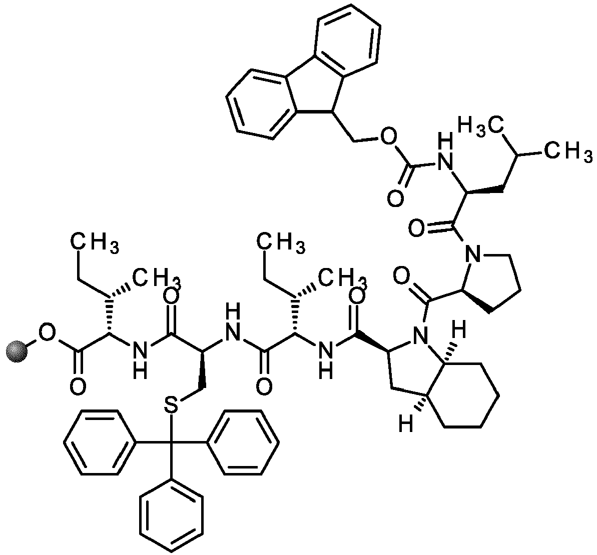

To the resin from Example 10A (18.18 g, 8.181 mmol) was added DMF (150 mL) and the resin was allowed to swell for 5 minutes. A solution of N-[(9H-fluoren-9-ylmethoxy)carbonyl]-L-leucine (11.565 g, 32.724 mmol) n DMF (50 mL) was added, followed by the addition of N,N'-diisopropylcarbodiimide (4.94 mL, 31.906 mmol) and ethyl-(hydroxyimino)cyanoacetate (4.534 g, 31.906 mmol). The mixture was shaken for 2 h at room temperature. The reaction mixure was aspirated and the resin was washed three times thoroughly with DMF. The coupling process was repeated using the same conditions. The resin was aspirated to remove the solution and the resin was washed three times with DMF (200 mL). The resin was further washed with MeOH (200 mL) DCM (200 mL). The washing with MeOH and DCM was repeated two more times. After the final DCM wash, the resin was dried using a rotary evaporator, the further dried under high vacuum, providing 20.93 g of resin.

### Example 12A

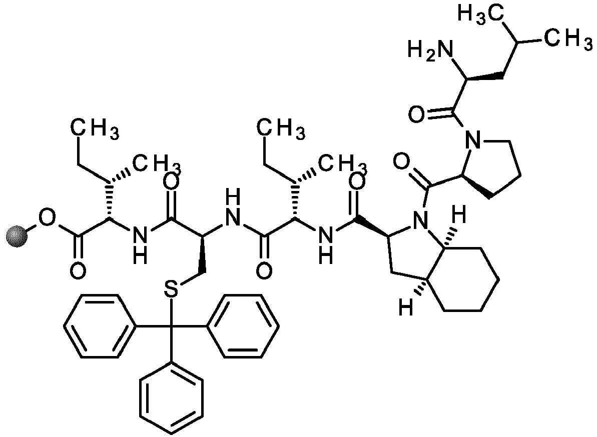

To the resin from Example 11A (20.93 g, 9.419 mmol) was added a solution of DMF/piperidine (4:1, 150 mL) and the mixture was shaken for 30 min at rt. The solution was removed by aspiration and the resin was washed three times thoroughly with DMF. The Fmoc deprotection procedure was repeated once more under the same conditions. The DMF/piperidine solution was removed by aspiration and the resin was washed three times with DMF (200 mL). Then resin was then washed with MeOH (200 mL) and DCM (200 mL). The washing with MeOH and DCM was repeated two more times. After the final DCM wash, the resin was dried using a rotary evaporator, providing 18.02 g of resin.

### Example 13A

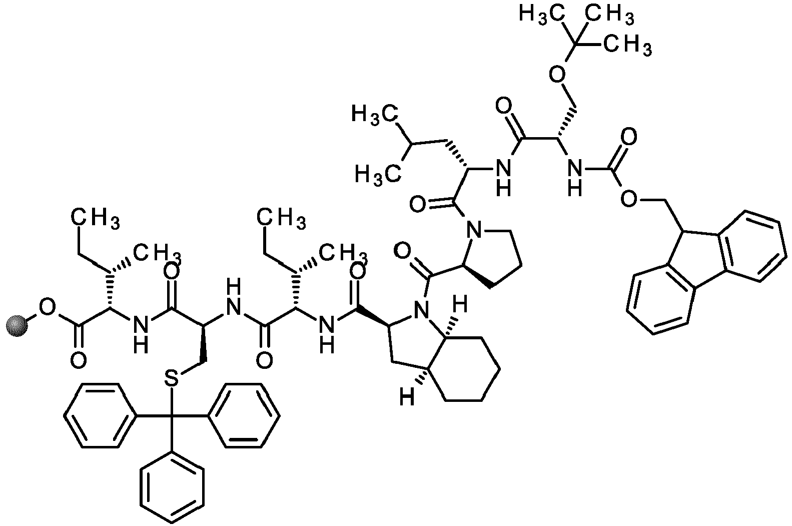

To the resin from Example 12A (18.02 g, 8.109 mmol) was added DMF (150 mL) and the resin was allowed to swell for 5 minutes. A solution of O-tert-butyl-N-[(9H-fluoren-9-ylmethoxy)carbonyl]-L-serine (12.438 g, 32.436 mmol) in DMF (50 mL) was added, followed by the addition of N,N'-diisopropylcarbodiimide (4.897 mL, 31.625 mmol) and ethyl-(hydroxyimino)cyanoacetate (4.494 g, 31.625 mmol). The mixture was shaken for 2 h at room temperature. The reaction mixure was aspirated and the resin was washed three times thoroughly with DMF. The coupling process was repeated using the same conditions. The resin was aspirated to remove the solution and the resin was washed three times with DMF (200 mL). The resin was further washed with MeOH (200 mL) DCM (200 mL). The washing with MeOH and DCM was repeated two more times. After the final DCM wash, the resin was dried using a rotary evaporator, the further dried under high vacuum, providing 21.43 g of resin.

### Example 14A

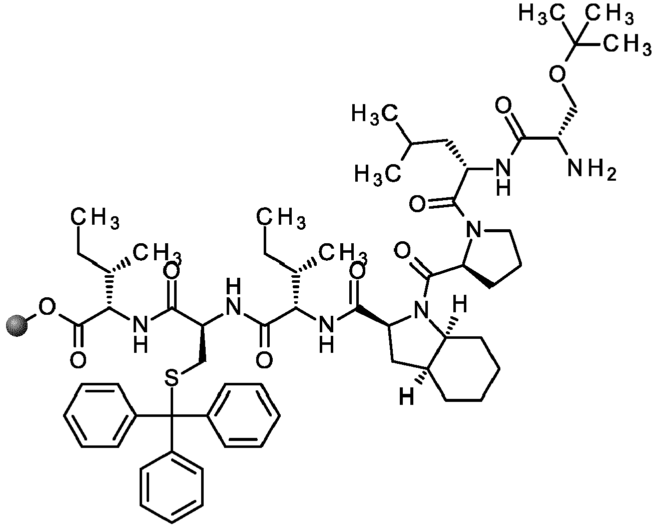

To the resin from Example 13A (21.43 g, 9.644 mmol) was added a solution of DMF/piperidine (4:1, 200 mL) and the mixture was shaken for 30 min at rt. The solution was removed by aspiration and the resin was washed three times thoroughly with DMF. The Fmoc deprotection procedure was repeated once more under the same conditions. The DMF/piperidine solution was removed by aspiration and the resin was washed three times with DMF (200 mL). Then resin was then washed with MeOH (200 mL) and DCM (200 mL). The washing with MeOH and DCM was repeated two more times. After the final DCM wash, the resin was dried using a rotary evaporator, providing 18.91 g of resin.

### Example 15A

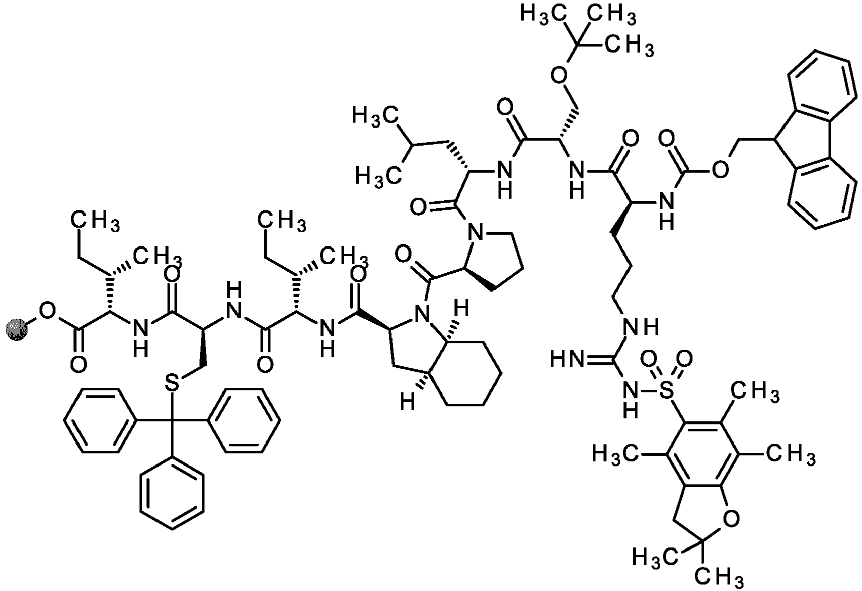

To the resin from Example 14A (18.91 g, 8.510 mmol) was added DMF (150 mL) and the resin was allowed to swell for 5 minutes. A solution of N²-[(9H-fluoren-9-ylmethoxy)carbonyl]-N⁵-{N-[(2,2,4,6,7-pentamethyl-2,3-dihydro-1-benzofuran-5-yl)sulfonyl]carbamimidoyl}-L-ornithine (22.083 g, 34.038 mmol) in DMF (50 mL) was added, followed by the addition of N,N'-diisopropylcarbodiimide (5.139 mL, 33.187 mmol) and ethyl-(hydroxyimino)cyanoacetate (4.716 g, 33.187 mmol). The mixture was shaken for 2 h at room temperature. The reaction mixure was aspirated and the resin was washed three times thoroughly with DMF. The coupling process was repeated using the same conditions. The resin was aspirated to remove the solution and the resin was washed three times with DMF (200 mL). The resin was further washed with MeOH (200 mL) DCM (200 mL). The washing with MeOH and DCM was repeated two more times. After the final DCM wash, the resin was dried using a rotary evaporator, the further dried under high vacuum, providing 23.77 g of resin.

### Example 16A

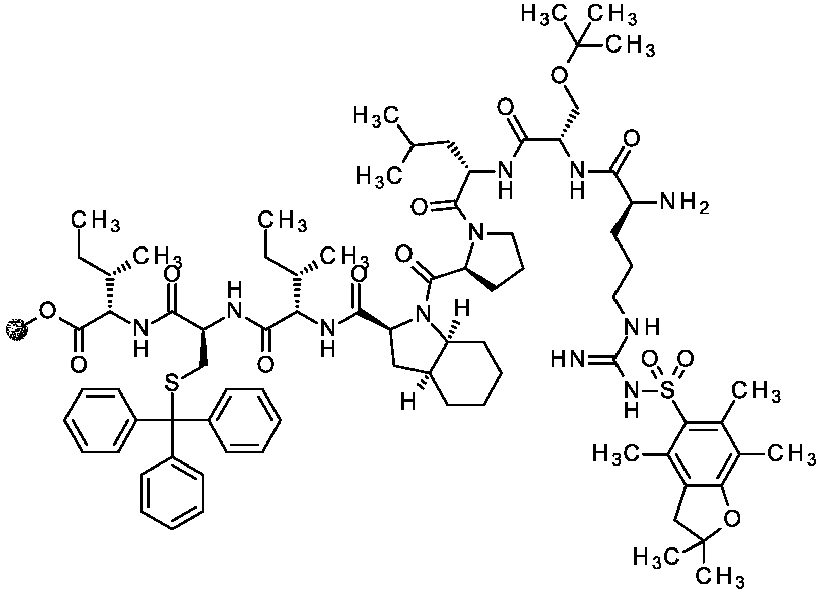

To the resin from Example 15A (23.77 g, 10.697 mmol) was added a solution of DMF/piperidine (4:1, 200 mL) and the mixture was shaken for 30 min at rt. The solution was removed by aspiration and the resin was washed three times thoroughly with DMF. The Fmoc deprotection procedure was repeated once more under the same conditions. The DMF/piperidine solution was removed by aspiration and the resin was washed three times with DMF (200 mL). Then resin was then washed with MeOH (200 mL) and DCM (200 mL). The washing with MeOH and DCM was repeated two more times. After the final DCM wash, the resin was dried using a rotary evaporator, providing 22.1 g of resin.

### Example 17A

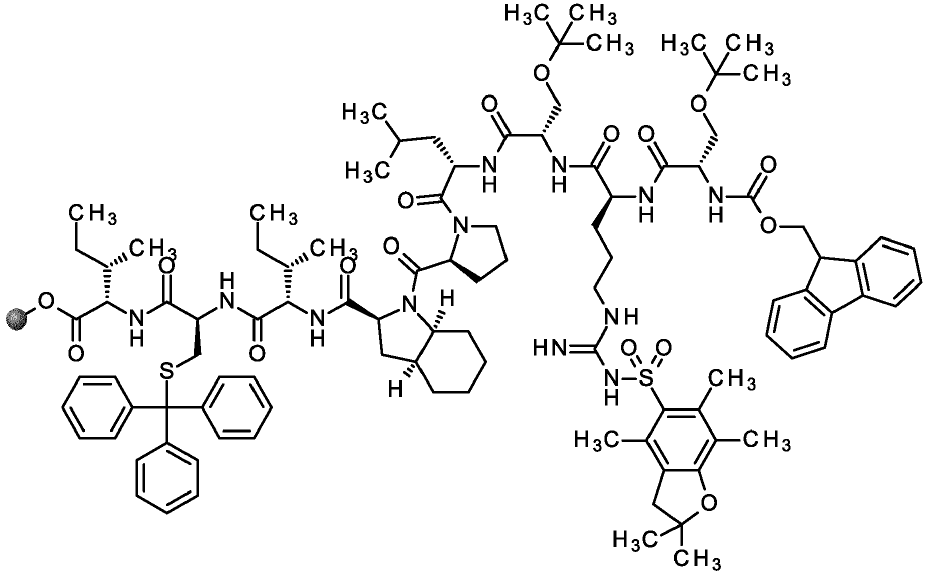

To the resin from Example 16A (22.1 g, 9.945 mmol) was added DMF (150 mL) and the resin was allowed to swell for 5 minutes. A solution of O-tert-butyl-N-[(9H-fluoren-9-ylmethoxy)carbonyl]-L-serine (15.254g, 39.78mmol) in DMF (50 mL) was added, followed by the addition of N,N'-diisopropylcarbodiimide (6.006 mL, 38.786 mmol) and ethyl-(hydroxyimino)cyanoacetate (5.512 g, 38.786 mmol). The mixture was shaken for 2 h at room temperature. The reaction mixure was aspirated and the resin was washed three times thoroughly with DMF. The coupling process was repeated using the same conditions. The resin was aspirated to remove the solution and the resin was washed three times with DMF (200 mL). The resin was further washed with MeOH (200 mL) DCM (200 mL). The washing with MeOH and DCM was repeated two more times. After the final DCM wash, the resin was dried using a rotary evaporator, the further dried under high vacuum, providing 24.65 g of resin.

### Example 18A

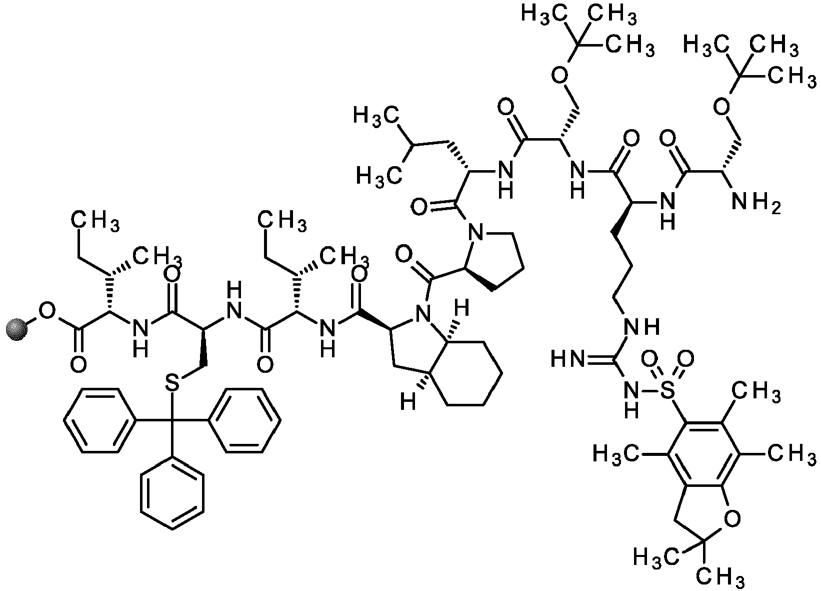

To the resin from Example 17A (24.65 g, 11.093 mmol) was added a solution of DMF/piperidine (4:1, 200 mL) and the mixture was shaken for 30 min at rt. The solution was removed by aspiration and the resin was washed three times thoroughly with DMF. The Fmoc deprotection procedure was repeated once more under the same conditions. The DMF/piperidine solution was removed by aspiration and the resin was washed three times with DMF (200 mL). Then resin was then washed with MeOH (200 mL) and DCM (200 mL). The washing with MeOH and DCM was repeated two more times. After the final DCM wash, the resin was dried using a rotary evaporator, providing 22.20 g of resin.

### Example 19A

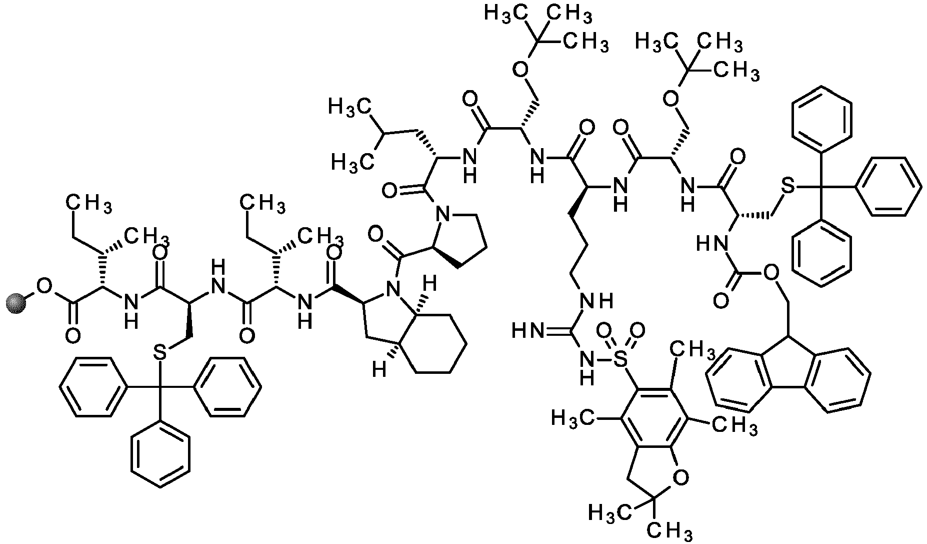

To the resin from Example 18A (22.2 g, 9.99 mmol) was added DMF (150 mL) and the resin was allowed to swell for 5 minutes. A solution of N-[(9H-fluoren-9-ylmethoxy)carbonyl]-S-tritylcysteine (23.406 g, 39.96 mmol) in DMF (50 mL) was added, followed by the addition of N,N'-diisopropylcarbodiimide (6.033 mL, 38.961 mmol) and ethyl-(hydroxyimino)cyanoacetate (5.537 g, 38.961 mmol). The mixture was shaken for 2 h at room temperature. The reaction mixure was aspirated and the resin was washed three times thoroughly with DMF. The coupling process was repeated using the same conditions. The resin was aspirated to remove the solution and the resin was washed three times with DMF (200 mL). The resin was further washed with MeOH (200 mL) DCM (200 mL). The washing with MeOH and DCM was repeated two more times. After the final DCM wash, the resin was dried using a rotary evaporator, the further dried under high vacuum, providing 24.53 g of resin.

### Example 20A

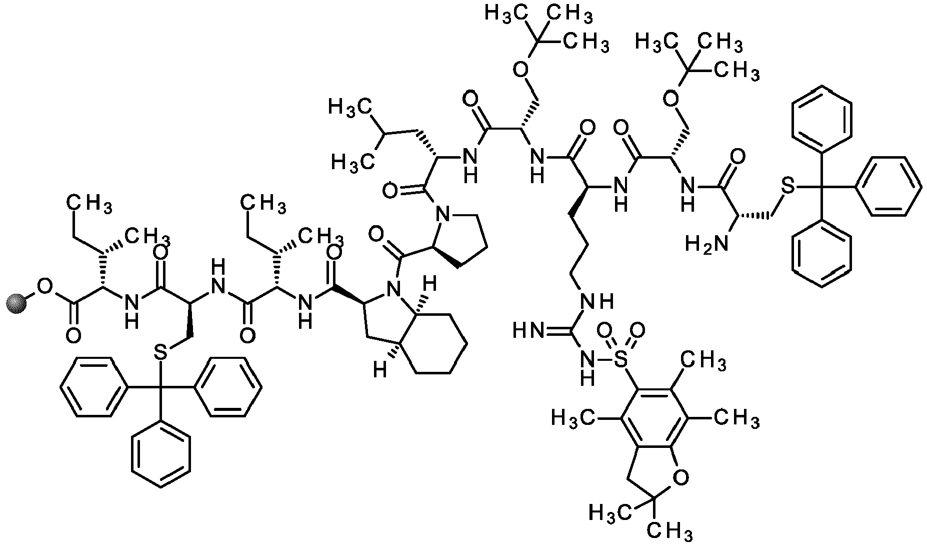

To the resin from Example 19A (24.53 g, 11.039 mmol) was added a solution of DMF/piperidine (4:1, 200 mL) and the mixture was shaken for 30 min at rt. The solution was removed by aspiration and the resin was washed three times thoroughly with DMF. The Fmoc deprotection procedure was repeated once more under the same conditions. The DMF/piperidine solution was removed by aspiration and the resin was washed three times with DMF (200 mL). Then resin was then washed with MeOH (200 mL) and DCM (200 mL). The washing with MeOH and DCM was repeated two more times. After the final DCM wash, the resin was dried using a rotary evaporator, providing 22.25 g of resin.

### Example 21A

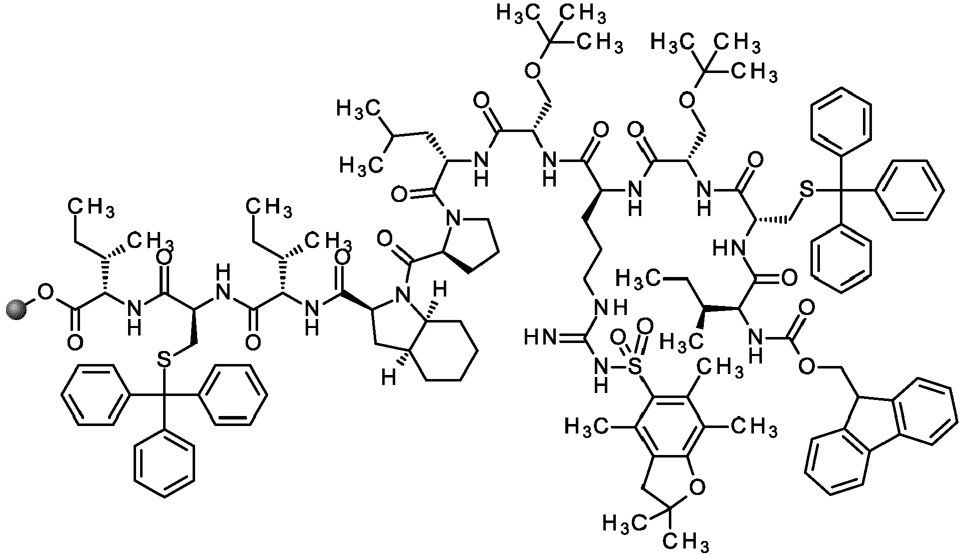

To the resin from Example 20A (22.25 g, 10.013 mmol) was added DMF (150 mL) and the resin was allowed to swell for 5 minutes. A solution of N-[(9H-fluoren-9-ylmethoxy)carbonyl]-L-isoleucine (14.155 g, 40.0 5mmol) in DMF (50 mL) was added, followed by the addition of N,N'-diisopropylcarbodiimide (6.047 mL, 39.049 mmol) and ethyl-(hydroxyimino)cyanoacetate (5.549 g, 39.049 mmol). The mixture was shaken for 2 h at room temperature. The reaction mixure was aspirated and the resin was washed three times thoroughly with DMF. The coupling process was repeated using the same conditions. The resin was aspirated to remove the solution and the resin was washed three times with DMF (200 mL). The resin was further washed with MeOH (200 mL) DCM (200 mL). The washing with MeOH and DCM was repeated two more times. After the final DCM wash, the resin was dried using a rotary evaporator, the further dried under high vacuum, providing 24.25 g of resin. The resin was used for further conversions in smaller portions. A calculated load of 0.25 mmol/g used for this purpose.

### Example 22A

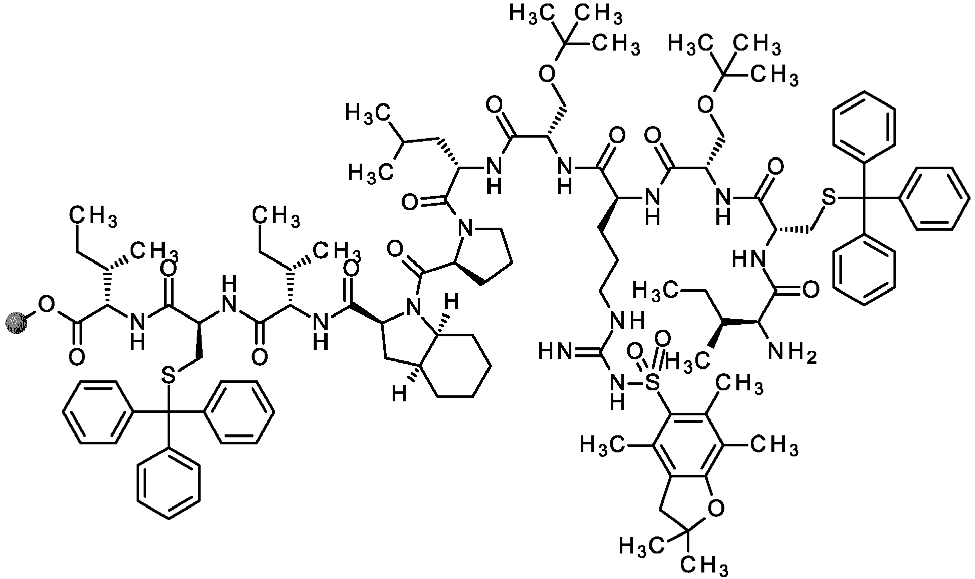

To the resin from Example 21A (4.0 g, 1.0 mmol) (in two syringes, 2 grams each) was added a solution of DMF/piperidine (4:1, 15 mL) to each syringe and the mixtures were shaken for 30 min at rt. The solution was removed by aspiration and the resin was washed three times thoroughly with DMF. The Fmoc deprotection procedure was repeated once more under the same conditions. The DMF/piperidine solution was removed by aspiration and the resin was washed three times with DMF. Then resin was then washed with MeOH and DCM. The washing with MeOH and DCM was repeated two more times. After the final DCM wash, the resin was dried using a rotary evaporator, providing the resin that was used for subsequent steps.

### Example 23A

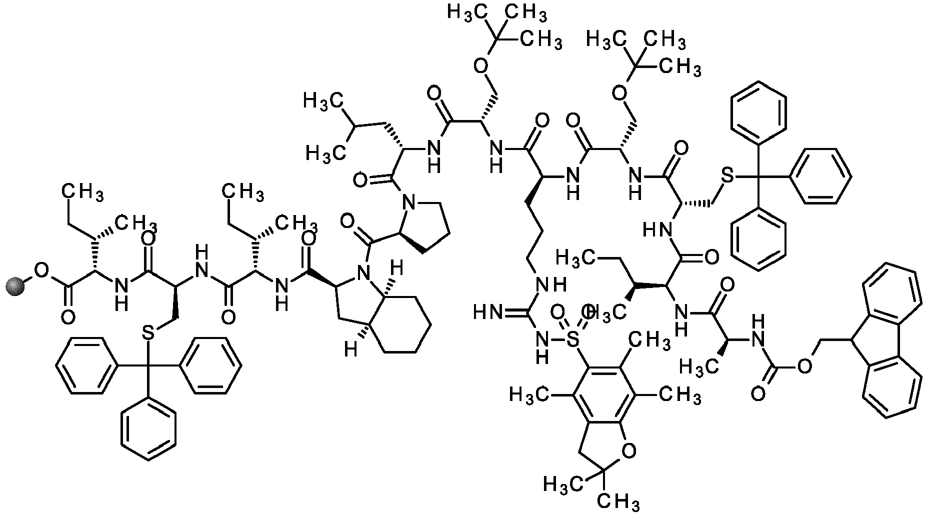

To the resin from Example 22A (2.0 g, 0.5 mmol) was added DMF (10 mL) and the resin was allowed to swell for 5 minutes. A solution of N-[(9H-fluoren-9-ylmethoxy)carbonyl]-L-alanine (0.623 g, 2.0 mmol) in DMF (4 mL) was added, followed by the addition of N,N'-diisopropylcarbodiimide (0.246 g, 1.95 mmol) and ethyl-(hydroxyimino)cyanoacetate (0.277 g, 1.95 mmol). The mixture was shaken for 2 h at room temperature. The reaction mixure was aspirated and the resin was washed three times thoroughly with DMF. The coupling process was repeated using the same conditions. The resin was aspirated to remove the solution and the resin was washed three times with DMF. The resin was further washed with MeOH and DCM. The washing with MeOH and DCM was repeated two more times. After the final DCM wash, the resin was dried using a rotary evaporator, the further dried under high vacuum, providing resin that was used for further steps.

### Example 24A

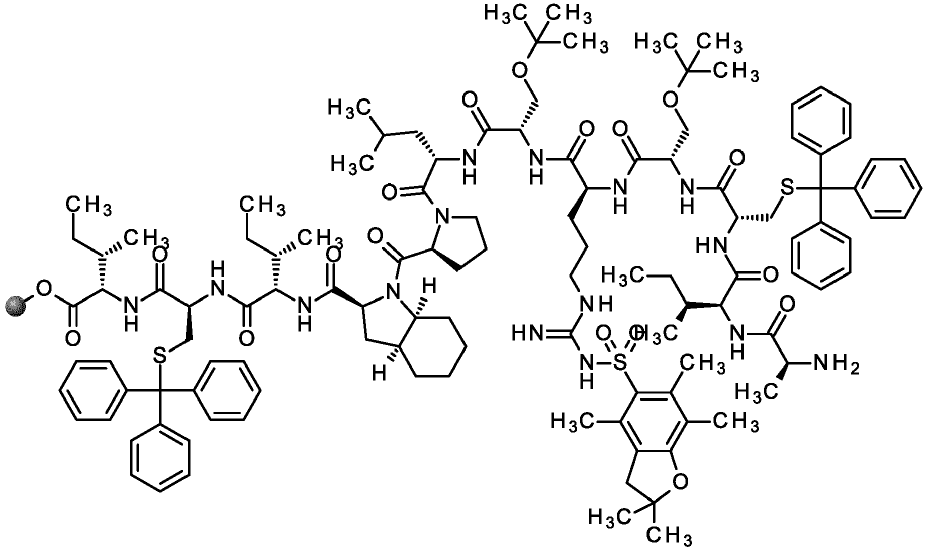

To the resin from Example 23A (3.0 g, 0.75 mmol) (in three syringes, 1 gram each) was added a solution of DMF/piperidine (4:1, 7.5 mL) to each syringe and the mixtures were shaken for 30 min at rt. The solution was removed by aspiration and the resin was washed three times thoroughly with DMF. The Fmoc deprotection procedure was repeated once more under the same conditions. The DMF/piperidine solution was removed by aspiration and the resin was washed three times with DMF. Then resin was then washed with MeOH and DCM. The washing with MeOH and DCM was repeated two more times. After the final DCM wash, the resin was dried under high vacuum, providing the resin that was used for subsequent steps.

### Example 25A

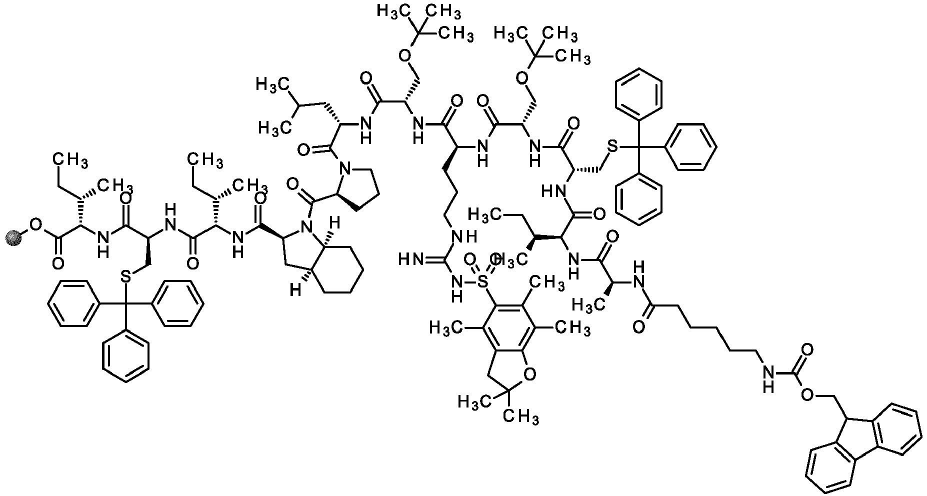

To the resin from Example 24A (2.0 g, 0.5 mmol) in two syringes (1.0 gram each) was added DMF (5 mL) and the resin was allowed to swell for 5 minutes. A solution of 6-{[(9H-fluoren-9-ylmethoxy)carbonyl]amino}hexanoic acid (354 mg, 1.0 mmol) in DMF (1 mL) was added, followed by the addition of a solution of N,N'-diisopropylcarbodiimide (151 µL, 0.975 mmol) and ethyl-(hydroxyimino)cyanoacetate (139 mg, 0.975 mmol) in DMF (1 mL). The mixtures were shaken overnight at room temperature. The reaction mixure was aspirated and the resin was washed three times thoroughly with DMF. The coupling process was repeated using the same conditions. The resin was aspirated to remove the solution and the resin was washed three times with DMF. The resin was further washed with MeOH and DCM. The washing with MeOH and DCM was repeated two more times. After the final DCM wash, the resin was dried using a rotary evaporator, the further dried under high vacuum, providing resin that was used for further steps.

### Example 26A

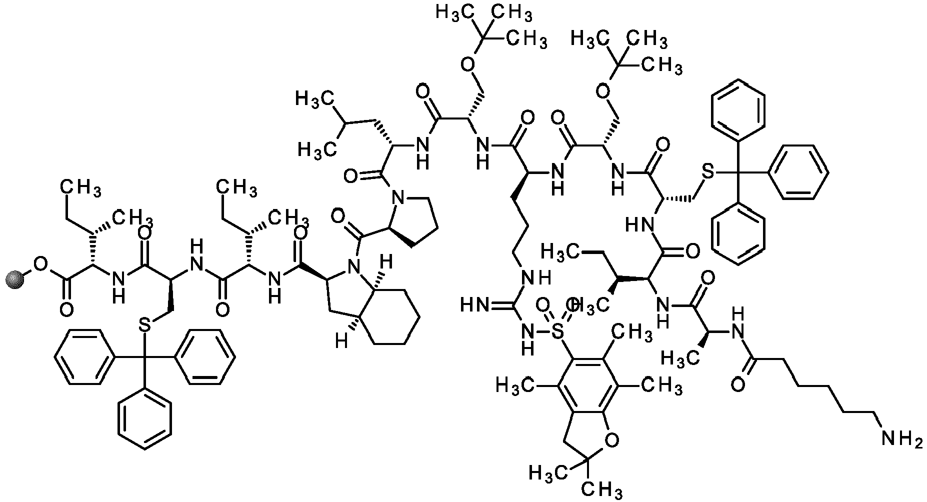

To the resin from Example 25A (2.0 g, 0.5 mmol) (in two syringes, 1 gram each) was added a solution of DMF/piperidine (4:1, 7.5 mL) to each syringe and the mixtures were shaken for 30 min at rt. The solution was removed by aspiration and the resin was washed three times thoroughly with DMF. The Fmoc deprotection procedure was repeated once more under the same conditions. The DMF/piperidine solution was removed by aspiration and the resin was washed three times with DMF. Then resin was then washed with MeOH and DCM. The washing with MeOH and DCM was repeated two more times. After the final DCM wash, the resin was dried under high vacuum, providing the resin that was used for subsequent steps.

### Example 27A

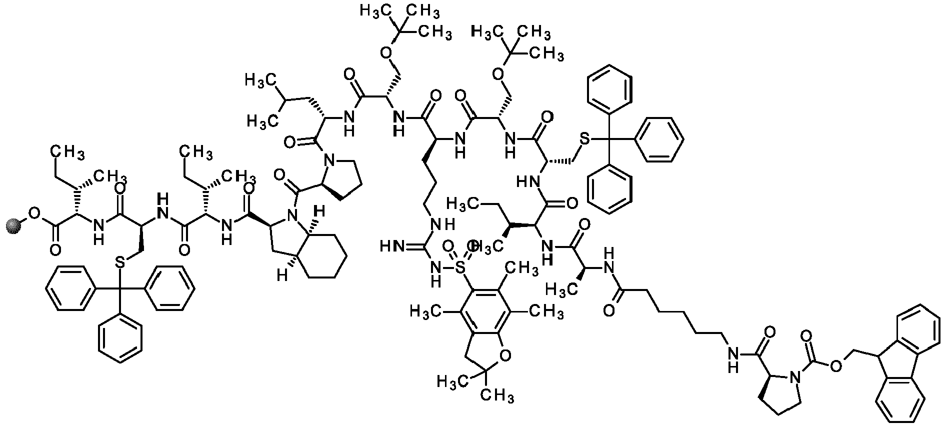

To the resin from Example 26A (1.0 g, 0.25 mmol) was added DMF (5 mL) and the resin was allowed to swell for 5 minutes. A solution of 1-[(9H-fluoren-9-ylmethoxy)carbonyl]-L-proline (0.337 g, 1.0 mmol) in DMF (1 mL) was added, followed by the addition of a solution of N,N'-diisopropylcarbodiimide (151 µL, 0.975 mmol) and ethyl-(hydroxyimino)cyanoacetate (139 mg, 0.975 mmol) in DMF (1 mL). The mixtures were shaken overnight at room temperature. The reaction mixure was aspirated and the resin was washed three times thoroughly with DMF. The coupling process was repeated using the same conditions. The resin was aspirated to remove the solution and the resin was washed three times with DMF. The resin was further washed with MeOH and DCM. The washing with MeOH and DCM was repeated two more times. After the final DCM wash, the resin was dried using a rotary evaporator, the further dried under high vacuum, providing resin that was used for further steps.

### Example 28A

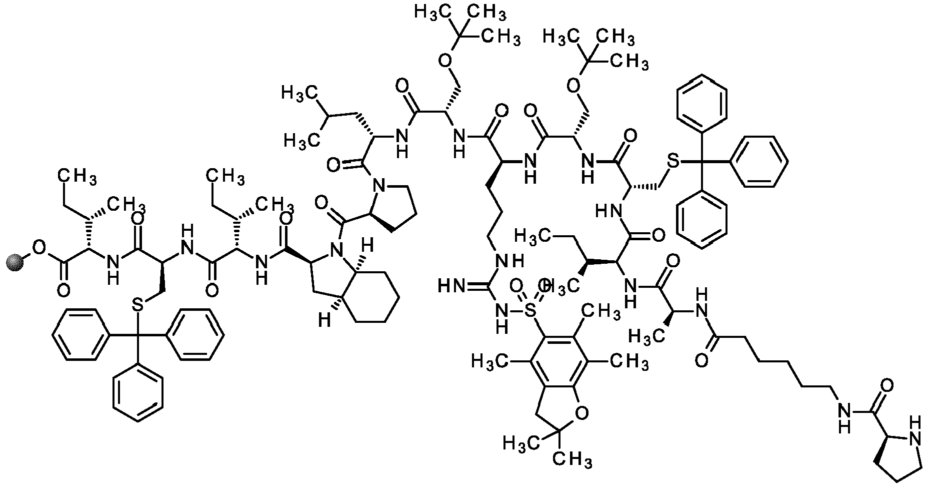

To the resin from Example 27A (1.0 g, 0.25 mmol) was added a solution of DMF/piperidine (4:1, 7.5 mL) and the mixture was shaken for 30 min at rt. The solution was removed by aspiration and the resin was washed three times thoroughly with DMF. The Fmoc deprotection procedure was repeated once more under the same conditions. The DMF/piperidine solution was removed by aspiration and the resin was washed three times with DMF. Then resin was then washed with MeOH and DCM. The washing with MeOH and DCM was repeated two more times. After the final DCM wash, the resin was dried under high vacuum, providing the resin that was used for the subsequent step.

### Example 29A

(2S,3S)-2-{[(2R)-2-{[(2S,3S)-2-({[(2S,3aS,7aS)-1-{[(2S)-1-{(2S,5S,8S,11S,14R,17S,20S)-17-[(2S)-Butan-2-yl]-5,11-bis(tert-butoxymethyl)-2-isobutyl-20-methyl-4,7,10,13,16,19,22,29-octaoxo-8-(3-{N'-[(2,2,4,6,7-pentamethyl-2,3-dihydro-1-benzofuran-5-yl)sulibnyl]carbamimidamido}propyl)-29-[(2S)-pyrrolidin-2-yl]-14-[(tritylsulfanyl)methyl]-3,6,9,12,15,18,21,28-octaazanonacosan-1-oyl}pyrrolidin-2-yl]carbonyl}octahydro-10H-indol-2-yl]carbonyl}amino)-3-methylpentanoyl]amino}-3-(tritylsulfanyl)propanoyl]amino}-3-methylpentanoic acid

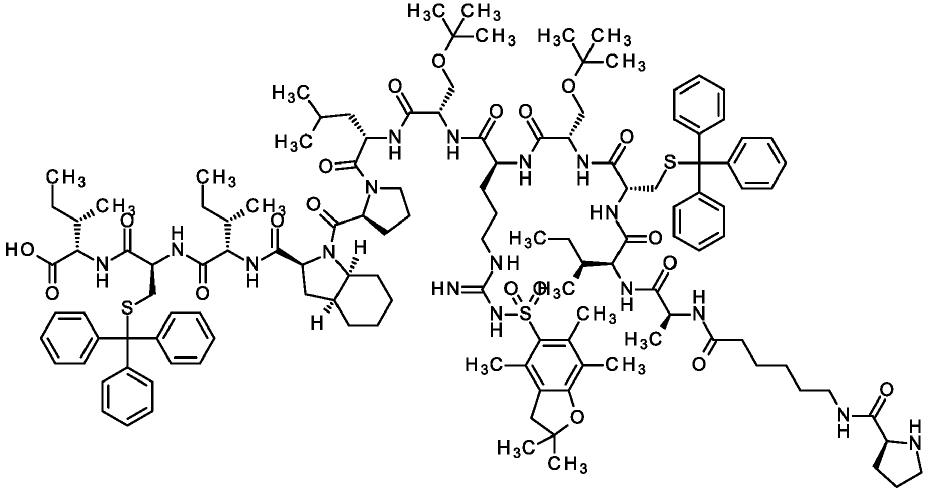

The resin from Example 28A (1.0 g, 0.250 mmol) was mixed with a solution of a DCM/HFIP 4:1 (7 mL) and the mixture was shaken at room temperature for 20 min. Then the released solution was filtered and collected in a flask, and the resin was washed thoroughly with DCM. The combined solution was evaporated and dried under high vacuum, providing 573.8 mg of a yellowish-hard foam.

### Example 30A

N-[N-(3-{(3aS,13S,16S,19R,22S,25S,28S,31S,36aS,38aS,42aS,43aS,46S,49R,52S)-52-[(2S)-Butan-2-yl]-22,28-bis(tert-butoxymethyl)-16, 46-di-sec-butyl-31-isobutyl-13-methyl-4,11,14,17,20,23,26,29,32, 37,44,47, 50,53-tetradecaoxo-19,49-bis[(tritylsulfanyl)methyl]tetrapentacontahydro-1H,34H-dipyrrolo[2',1':18,19;2", 1":3,4][1,4,7,10,13,16,19,22,25,28,31,34,37,40]tetradecaazacyclohexatetracontino[16,15-a]indol-25-yl}propyl)carbamimidoyl]-2,2,4,6,7-pentamethyl-2,3-dihydro-1-benzofuran-5-sulfonamide

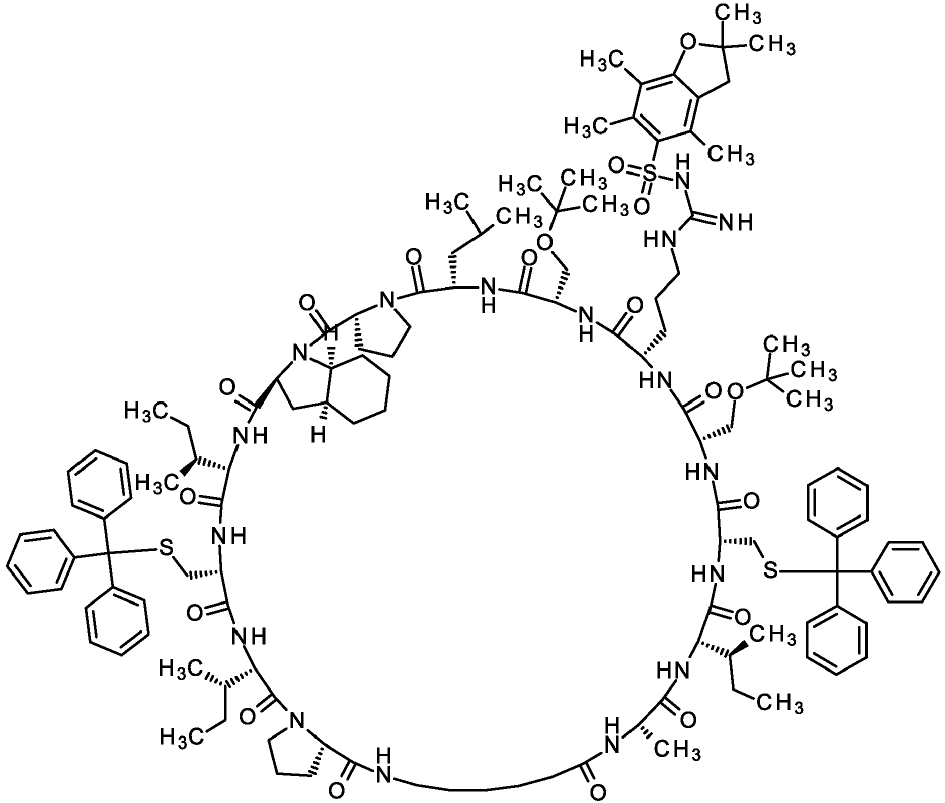

The crude residue from Example 29A (573.8 mg, 0.240 mmol) was dissolved in DMF (20 mL). N,N'-diisopropylcarbodiimide (0.149 mL, 0.962 mmol) and ethyl-(hydroxyimino)cyanoacetate (136.69 mg, 0.962 mmol) were added and the reaction mixture was immediately diluted with DCM (1150 mL) to achieve a concentration of 1 mg peptide per 2mL solution. The reaction mixture was stirred overnight at room temperature. The reaction mixure was concentrated and dried under high vacuum, providing 760 mg of an amorphous residue. The crude product was used directly for the next step.

### Example 31A

1-{3-[(3aS,13S,16S,19R,22S,25S,28S,31S,36aS,38aS,42aS,43aS,46S,49R,52S)-52-[(2S)-Butan-2-yl]-16,46-di-sec-butyl-22,28-bis(hydroxymethyl)-31-isobutyl-13-methyl-4, 11,14,17,20,23,26,29,32,37,44,47,50,53-tetradecaoxo-19,49-bis(sulfanylmethyl)tetrapentacontahydro-1H,34H-dipyrrolo[2',1':18,19;2",1":3,4][1,4,7,10, 13,16,19,22,25,28,31,34,37,40]tetradecaazacyclohexatetracontino[16,15-a]indol-25-yl]propyl}guanidine

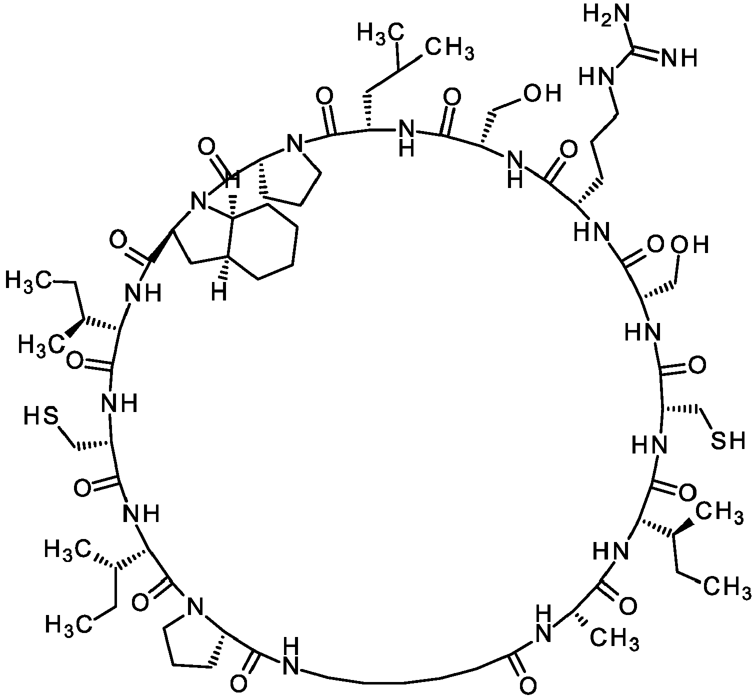

The crude product from Example 30 A (760 mg, 0.321 mmol) was mixed with 5 mL of a mixture of TFA/EDTTThioanisole 90:3:7 and stirred for 2.5 h at room temperature. The solution was diluted with DCM and evaporated. The residue was treated again with DCM and then dried using the rotary evaporator. The residue stirred with diethyl ether, vacuumed, washed twice with diethyl ether and dried under high vacuum, providing 523.4 mg of a beige solid that was used directly for the next step.

### Example 25

1-{3-[(3aS,13S,16S,19R,22S,25S,28S,31S,36aS,38aS,42aS,43aS,46S,49R,52S)-16,46,52-Tri-sec-butyl-22,28-bis(hydroxymethyl)-31-isobutyl-13-methyl-4,11,14,17,20,23,26,29,32,37,44,47,50,53-tetradeca-oxotetrapentacontahydro-1H,34H-19,49-(methanodithiomethano)dipyrrolo[2',1':18,19;2",1":3,4][1,4,7,10,13,16,19,22,25,28, 31,34,37,40]tetradecaazacyclohexatetracontino[16,15-a]indol-25-yl]propyl}guanidine

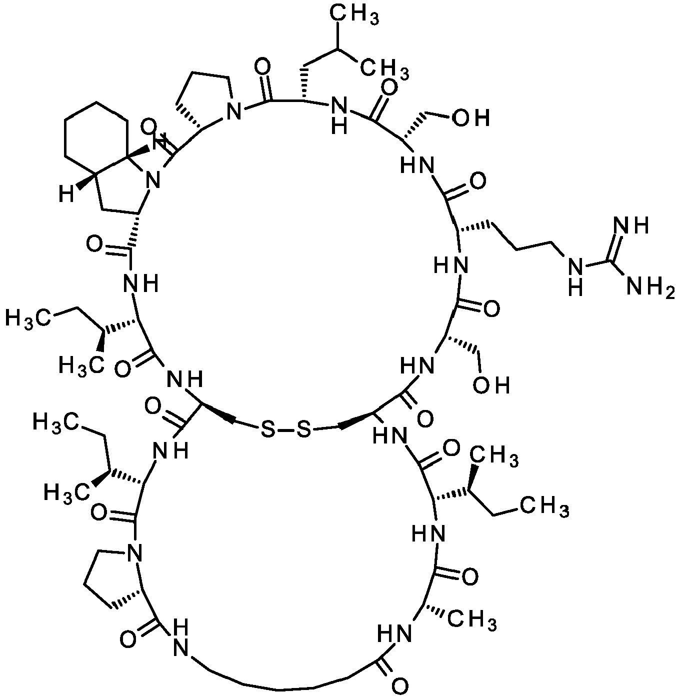

The crude peptide from Example 31A (523 mg, 0.344 mmol) was mixed with 1050 mL of 0.1 molar ammonium bicarbonate solution (pH= 7.83). While stirring, air passed through the solution for 5 min. The reaction mixture was stirred overnight in an open flask (suspension). The reaction mixture was lyophilized to afford 5.64 g of a white lyophilizate. A portion (1000 mg) was desalinated using a Pur-A-Lyzer Mega 1000 (Dialysis Kit- Article no.: PURG10010-1Kt; Sigma-Aldrich).

1000 mg of the crude peptide was suspended in a 5% acetonitrile-H₂O mixture (18 mL). The suspension was filled into the Pur-A-Lyzer Mega 1000 Dialysis Kit. The dialysis kit was placed (floating) into a beaker containing 1.6 L of water while slowly stirring. After 1.5 hours, the water was reülaced with fresh water and the kit was left stirring for another 1.5 hours. The suspension was removed from the kit and then lyophilized to offord 133 mg of crude peptide. The crude peptide was dissolved in 5% ACN/water and purified by preparative HPLC (Column: Phenomenex, Kinetex 5µ Biphenyl 100A, AXIA Packed, 21,2 x 250mm + Cartridge 5µ; Flow: 20 mL/min, method: Gradient 30-85% ACN in Water (0,10 % TFA). The product-containing fractions were combined (analyzed by analytical HLPC (5-95 in 8 min, Chromolith Speedrod & YMC) to afford 1.10 mg (>99 pure) of the title compound.

### General Method N Conversion of Peptide TFA salts to HCl salts

The synthesis of I**C+SRS-((tBu)A)-PPI-(Pen)+-IP** (HCl Salt) (example 88) is representative.

The synthesis of I**C+SRS-((tBu)A)-PPI-(Pen)+-IP** as the TFA salt was carried out using Method A. This peptide has a starting TFA content of 11.4% TFA (1.49 eq).

### Procedure of Automatic Ion Exchange Station (Method N1):

Peristaltic pump of the company Hirschmann (Rotarus volume 50), Tubes: Tygon 2001 (ID 0,64mm)
Settings:
Washing with H₂O: run-time 1200s; 80 min⁻¹; 1 cycle (is 35 mL volume)
Sample circulation with peptide: run-time 1200s; 80 min⁻¹; 1 cycle (is 70 mL volume)
Wash with H₂O (or %ACN in H₂O: run-time 1200s; 80 min⁻¹; 1 cycle (is 35 mL volume)

Amberlite IRA 410 (HCl form) was used. 700 mg of the resin was placed into 2 filter cartridges and washed with deionized water (10 times).

The peptide (56 mg) dissolved in 3 mL of a 5% ACN/H₂O solution was loaded onto the column and cycled through the column 10 times. The column was washed with water, and the solution collected into a Falcon tube and lyophilized
45.5 mg of the desired peptide was obtained as the HCl salt: LC-MS (> 99%); Ion Chromatography analysis: 2.7 wt% Cl- (1.01 eq C1-), < 1 wt % TFA.

### The ion exchange process can also be performed using the following protocol (Method N2):

The synthesis of (Ahx)**-GIC+SRSLPPIC+IPD** (HCl Salt) (example 94) is representative.

The synthesis of (Ahx)**-GIC+SRSLPPIC+IPD** (Example 13) as the TFA salt was carried out using Method B (LC-MS (95.0%). This peptide has a starting TFA content of 15.9% TFA (2.6 eq).

### Procedure of Manual Ion Exchange (Method N2):

Amberlite IRA 410 resin (HCl form) (1-2 g) was placed into a 10 mL frit-syringe (100 mg peptide needs 1g IRA 410 resin)
1) The resin is washed with water (10 times x 3 mL)
2) The resin is washed with 5% ACN in water (1 times x 3 mL)
3) The peptide was dissolved in 5% ACN in water
4) The peptide was added to the syringe and the solution was cycled through the column 10 -20 times. The eluent is collected into a Falcon Tube.
5) The resin is washed with 5%ACN in water (10 times x 3 mL); this solution is added to the solution in the Falcon-tube
6) The combined solution is lyophilized.

Using this procedure and 1500 g of IRA 410 resin, 106 mg of the desired peptide was obtained as the HCl salt: LC-MS (> 95.4%); Ion Chromatography analysis: 3.1 wt% Cl- (1.4 eq C1-), < 0.5 wt % TFA.

### The conversion to an HCl salt can also be performed using the following protocol (Method N3):

When the purification using the above Methods A-M is carried out using an HPLC modifier of 0.075% HCl in H2O instead of using TFA or formic acid as the modifier, an HCl salt is directly obtained (e.g. Example 79: (Ahx)**-aIC+SRSLP-(Oic)-I-(Pen)+-IPE++-NH₂ (HCl Salt), Ion Chromatography analysis: 4.9 wt% Cl-(2.4 eq Cl-), <1 wt % TFA.

### The conversion to an HCl salt can also be performed using the following protocol (Method N4):

From a salt-free form prepared in General Method P, the peptide can be dissolved in ACN/water, and a stoichiometric amount HCl (aq), based on the number of basic equivalents in the peptide, can be added. The solution is then lyophilized to provide the salt.

### General Method O: Conversion of Peptide TFA salts to other salts

### Other salt forms:

The chloride counter ion can be exchanged with other counterions in a similar manner by passing a solution of the desired salt form (e.g sodium acetate) repeatedly through the column, then washing the column repeatedly with water. The peptide is then loaded onto the resin and the above ion exchange procedures described in General Method N are followed. Peptide acetate, tartrate, citrate, and lactate salts of MASP peptides have been prepared.

Salt-free forms of MASP peptides prepared according to Method P can be used to prepare other salt-forms by dissolving the peptide in ACN-water and adding a stoichiometric amount of the counterion acid (e.g. acetic acid), and then the solution is then lyophilized to provide the salt.

### General Method P: Preparation of a Salt-Free Form

A salt-free form of a MASP peptide can be prepared by further purifying a TFA salt or an HCl salt of a peptide of this invention by reversed-phase preparative HPLC using an acetonitrile water gradient at 70°C with no acid modifier. The desired fractions are combined and lyophilized, to obtain a peptide nearly free of counterion; LC-MS (> 99% pure); Ion Chromatography (<1% TFA)

**Table 17: Reference Peptides**

| **Ref. No** | **Identifier** | **Sequence** | **Method of Preparation** |
|---|---|---|---|
| 2 | Bicyclic SFTI-1 | G**RC+TKSIPPIC+FPD** | Method D |
| 7 | Bicyclic SFMI-1 | G**IC+SRSLPPIC+IPD** | Method B |
| 8 | Bicyclic SFMI-2 | G**YC+SRSYPPVC+IPD** | Method A |
| 12 | | P* *FC+IPPISKTC+RGD** | Method B |

**Table 18: Peptides prepared according, to the invention**

| **Ex. No** | **Sequence** | **MoP†** |
|---|---|---|
| 13 | (Ahx)**-GIC+SRSLPPIC+IPD** | A, B |
| 14 | (PEG3(16 atoms))**-GIC+SRSLPPIC+IPD** | A |
| 15 | G**GIC+SRSLPPIC+IPD** | B |
| 16 | (Ahx)**-GIC+SRSLPPIC+IPd** | B |
| 17 | A**GGIC+SRSLPPIC+IPd** | B |
| 18 | A**GGIC+SRSLPPIC+IPD** | B |
| 19 | a**GGIC+SRSLPPIC+IPD** | B |
| 20 | (Dap)++-GGIC+SRSLPPIC+IPD** | C |
| 21 | G**SGIC+SRSLPPIC+IPDS** | B |
| 22 | K++GIC+SRSLPPIC+IPD** | C |
| 23 | (Dap)**-(Dap)-GIC+SRSLPPIC+IPD** | B |
| 24 | (PEG1(10 atoms))**-GIC+SRSLPPIC+IPD** | B |
| 25 | (Ahx)**-AIC+SRSLP-(Oic)-IC+IP** | M |
| 26 | A**GAIC+SRS-((tBu)A)-PPI-(Pen)+-IPD** | D |
| 27 | (Ahx)**-AIC+SRS-((tBu)A)-PPI-(Pen)+-IPD** | E |
| 28 | (Ahx)**-GIC+SRS-((tBu)A)-PPI-(Pen)+-IPD** | E |
| 29 | A**GGIC+SRS-((tBu)A)-PPI-(Pen)+-IPD** | E |
| 30 | (Ahx)**-GIC+SRS-((tBu)A)-PPI-((N-Me)C)+-IPD** | D |
| 31 | A**GAIC+SRS-((tBu)A)-PPI-((N-Me)C)+-IPD** | E |
| 32 | (Ahx)**-AIC+SRS-((tBu)A)-PPI-((N-Me)C)+-IPD** | E |
| 33 | (Ahx)**-(Abu)-IC+SRS-((tBu)A)-PPI-((N-Me)C)+-IPD** | E |
| 34 | (PEG1(10 atoms))**-AIC+SRS-((tBu)A)-PPI-(Pen)+-IPD** | F |
| 35 | (Gamma-Abu)**-IC+SRS-((tBu)A)-PPI-(Pen)+-IPD** | F |
| 36 | (Gamma-Abu)**-IC+SRS-((tBu)A)-PPI-((N-Me)C)+-IPD** | F |
| 37 | G**AIC+SRSLPPIC+IPD++-NH₂ | G |
| 38 | (Ahx)**-(Abu)-IC+SRS-((tBu)A)-PPI-(Pen)+-IPD** | E |
| 39 | A**GGIC+SRS-((tBu)A)-PPI-(Pen)+-IPd** | E |
| 40 | (Ahx)**-GIC+SRS-((tBu)A)-PPI-((N-Me)C)+-IPD++-NH₂ | G |
| 41 | A**GAIC+SRSLP-(Oic)-I-(Pen)+-IPD++-NH₂ | G |
| 42 | A**GGIC+SRSLP-(Oic)-I-(Pen)+-IPD++-NH₂ | G |
| 43 | (Ahx)**AIC+SRSLP-(Oic)-I-(Pen)+-IPD++-NH₂ | G |
| 44 | (Orn)++-AIC+SRS-((tBu)A)-PPI-((N-Me)C)+-IPD++-NH₂ | G |
| 45 | (Ahx)**-AIC+SRSLPPIC+IPD** | E |
| 46 | (Ahx)**-AIC+SRS-((tBu)A)-PPIC+IPD** | E |
| 47 | A**GGIC+SRS-((tBu)A)-PPI-((N-Me)C)+-IPd** | E |
| 48 | (Ahx)**-GIC+SRS-((tBu)A)-PPI-(Pen)+-IPD++-NH₂ | G |
| 49 | (Ahx)**-GIC+SRSLP-(Oic)-I-(Pen)+-IPD++-NH₂ | G |
| 50 | C++AIC+SRS-((tBu)A)-PPI-(Pen)+-IPDC++-NH₂ | K |
| 51 | (Ahx)**-IC+SRS-((tBu)A)-PPI-(Pen)+-IP** | E |
| 52 | (Ahx)**-IC+SRS-((tBu)A)-PPI-(Pen)+-I** | E |
| 53 | (Ahx)**-AIC+SRS-((tBu)A)-PPI-(Pen)+-IP** | E |
| 54 | (Ahx)**-((N-Me)G)-IC+SRS-((tBu)A)-PPI-(Pen)+-IP** | E |
| 55 | (Dap)**-IC+SRS-((tBu)A)-PPI-(Pen)+-IP** | H |
| 56 | (Dab)**-IC+SRS-((tBu)A)-PPI-(Pen)+-IP** | H |
| 57 | (Dap)**-(Dap)-IC+SRS-((tBu)A)-PPI-(Pen)+-I** | H |
| 58 | I* *C+SRS-((tBu)A)-PPI-(Pen)+-IPP** | H |
| 59 | (Ahx)**-(TXA)-IC+SRS-((tBu)A)-PPI-(Pen)+-IP** | E |
| 60 | (Adipic acid)**-IC+SRS-((tBu)A)-PPI-(Pen)+-IPD-(Dap)++-NH₂ | I |
| 61 | (Orn)++-AIC+SRS-((tBu)A)-PPI-(Pen)+-IPD++-NH₂ | G |
| 62 | G**-(TXA)-GIC+SRS-((tBu)A)-PPI-(Pen)+-IPD++-NH₂ | G |
| 63 | (TTDS)**-AIC+SRS-((tBu)A)-PPI-(Pen)+-IPD** | E |
| 64 | (Ahx)**-((N-Me)G)-IC+SRS-((tBu)A)-PPI-(Pen)+-I** | E |
| 65 | (Orn)**-GIC+SRS-((tBu)A)-PPI-(Pen)+-I** | E |
| 66 | (Orn)++-IC+SRS-((tBu)A)-PPI-(Pen)+-I** | E |
| 67 | K++-IC+SRS-((tBu)A)-PPI-(Pen)+-I** | E |
| 68 | (Ahx)**-(4-(Aminomethyl)benzoic acid)-IC+SRS-((tBu)A)-PPI-(Pen)+-IP** | E |
| 69 | G**-(TXA)-GIC+SRS-((tBu)A)-PPI-((N-Me)C)+-IPD++-NH₂ | J |
| 71 | (Orn)++-IC+SRS-((tBu)A)-PPI-(Pen)+-IP** | H |
| 72 | (Ahx)**-AIC+SRS-((tBu)A)-PPI-(Pen)+-I** | B, E |
| 73 | (Dap)**-(Dab)-IC+SRS-((tBu)A)-PPI-(Pen)+-I** | H |
| 74 | (Ahx)**-AIC+SRSLP-(Oic)-I-(Pen)+-IPE++-NH₂ | G |
| 75 | (Ahx)**-IC+SRSLP-(Oic)-I-(Pen)+-IPE++-NH₂ | G |
| 76 | (Ahx)**-((N-Me)G)-GIC+SRSLP-(Oic)-I-(Pen)+-IPE++-NH₂ | G |
| 77 | (Ahx)**-((N-Me)G)-AIC+SRSLP-(Oic)-I-(Pen)+-IPE++-NH₂ | G |
| 78 | k++-IC+SRS-((tBu)A)-PPI-(Pen)+-I** (HCl Salt) | F, N |
| 79 | (Ahx)**-aIC+SRSLP-(Oic)-I-(Pen)+-IPE++-NH₂ (HCl Salt) | G, N |
| 80 | k++-IC+SRS-((tBu)A)-PPI-(Pen)+-I** | F |
| 81 | (3-Azido-L-Alanine)++-GAIC+SRS-((tBu)A)-PPIC+IP-(L-Propargylglycine)++-NH₂ (1,2,3-triazole-1,4-diyl) | L |
| 82 | (Ahx)**-GAIC+SRSLP-(Oic)-I-(Pen)+-IPE++-NH₂ | G |
| 83 | (Ahx)**-GIC+SRSLP-(Oic)-I-(Pen)+-IPE++-NH₂ | G |
| 84 | (Ahx)**-GaIC+SRSLP-(Oic)-I-(Pen)+-IPE++-NH₂ | G |
| 85 | (Dap)++-IC+SRSLP-(Oic)-I-(Pen)+-IP** | C |
| 86 | (Dap)++-(Dap)-IC+SRSLP-(Oic)-I-(Pen)+-IP** | C |
| 87 | (Dap)**-IC+SRSLP-(Oic)-I-(Pen)+-IP** | B |
| 88 | I**C+SRS-((tBu)A)-PPI-(Pen)+-IP** (HCl Salt) | A, N |
| 89 | (Dap)**-(Dap)-IC+SRSLP-(Oic)-I-(Pen)+-IP** | B |
| 90 | E++GIC+SRSLP-(Oic)-I-(Pen)+-IPK++-NH₂ | G |
| 91 | E++GIC+SRSLP-(Oic)-I-(Pen)+-IPDK++-NH₂ | G |
| 92 | A**GGIC+SRSLP-(Oic)-I-(Pen)+-IPD** | E |
| 93 | E++GIC+SRSLP-(Oic)-I-(Pen)+-IPD-(Dap)++-NH₂ | I, J |
| 94 | (Ahx)**-GIC+SRSLPPIC+IPD**(HCl Salt) | A, N |
| 95 | (Ahx)**-IC+SRSLP-(Oic)-IC+I** | M |
| 96 | (Orn)**-GIC+SRS-((tBu)A)-PPI-(Pen)+-IP** | F |
| 97 | (Dap)**-(Dap)-IC+SRS-((tBu)A)-PPI-(Pen)+-IP** | F |
| 98 | A**GGIC+SRSLP-(Oic)-I-(Pen)+-IPd** | E |

| | | |
|---|---|---|
| † MoP = Method of Preparation | | |

**Table 19: Analytical data for the reference peptides**

| **Ref. No** | **Retention time (min)** | **LC-MS Method** | **Purity (%)** | **Exact Mass Calcd (g/mol)** | **Exact Mass Found (g/mol)** | **Ionization** | **LC-MS Purity (%) Method 7** | **LC-MS Purity (%) Method 8** |
|---|---|---|---|---|---|---|---|---|
| 2 | 6,66 | Method 4 | 100 | 1512,7217 | 1512,7438 | [M+2H]²⁺ | 96,50 | 92,50 |
| 7 | 4,56 | Method 2 | 100 | 1449,7108 | 1449,7254 | [M+2H]²⁺ | | |
| 8 | 4,29 | Method 2 | 100 | 1535,6537 | 1535,6540 | [M+2H]²⁺ | | |
| 12 | 9,63 | Method 4 | 98,68 | 1512,7217 | 1512,742 | [M+2H]²⁺ | | |

**Table 20: Analytical data for the peptides prepared according to the invention**

| **Ex. No** | **Retention time (min)** | **LC-MS Method** | **Purity (%)** | **Exact Mass Calcd (g/mol)** | **Exact Mass Found (g/mol)** | **Ionization** | **LC-MS Purity (%) Method 7** | **LC-MS Purity (%) Method 8** |
|---|---|---|---|---|---|---|---|---|
| 13 | 4,68 | Method 2 | 92,05 | 1562,7949 | 1562,7992 | [M+2H]²⁺ | | |
| 14 | 4,65 | Method 2 | 99,53 | 1696,8528 | 1696,8648 | [M+2H]²⁺ | | |
| 15 | 4,51 | Method 2 | 100 | 1506,7323 | 1506,7386 | [M+2H]²⁺ | | |
| 16 | 4,62 | Method 2 | 100 | 1562,7949 | 1562,8072 | [M+2H]²⁺ | | |
| 17 | 4,60 | Method 2 | 100 | 1577,7694 | 1577,7788 | [M+2H]²⁺ | | |
| 18 | 4,54 | Method 2 | 100 | 1577,7694 | 1577,7784 | [M+2H]²⁺ | | |
| 19 | 4,52 | Method 2 | 100 | 1577,7694 | 1577,7780 | [M+2H]²⁺ | | |
| 20 | 4,42 | Method 2 | 100 | 1592,7803 | 1592,7918 | [M+2H]²⁺ | | |
| 21 | 4,49 | Method 2 | 100 | 1680,7964 | 1680,8048 | [M+2H]²⁺ | | |
| 22 | 4,48 | Method 2 | 100 | 1577,8058 | 1577,8132 | [M+2H]²⁺ | | |
| 23 | 4,30 | Method 2 | 100 | 1621,8069 | 1621,8152 | [M+2H]²⁺ | | |
| 24 | 9,42 | Method 1 | 92,94 | 1608,8004 | 1608,8106 | [M+2H]²⁺ | | |
| 25 | 10,90 | Method 1 | 100 | 1515,8306 | 1515,8434 | [M+2H]²⁺ | | |
| 26 | 9,75 | Method 1 | 94,72 | 1633,8320 | 1633,8442 | [M+2H]²⁺ | 97,2 | 96,5 |
| 27 | 10,15 | Method 4 | 97,33 | 1618,8575 | 1618,8748 | [M+2H]²⁺ | 98,2 | 96,4 |
| 28 | 10,28 | Method 1 | 88,20 | 1604,8419 | 1604,8510 | [M+2H]²⁺ | 96,90 | 95,60 |
| 29 | 9,52 | Method 1 | 98,95 | 1619,8164 | 1619,8280 | [M+2H]²⁺ | 96,80 | 96,40 |
| 30 | 10,32 | Method 1 | 86,22 | 1590,8262 | 1590,8344 | [M+2H]²⁺ | 97,20 | 94,40 |
| 31 | 10,07 | Method 1 | 100 | 1619,8164 | 1619,8214 | [M+2H]²⁺ | 95,00 | 92,80 |
| 32 | 10,35 | Method 4 | 98,07 | 1604,8419 | 1604,8578 | [M+2H]²⁺ | 95,50 | 91,40 |
| 33 | 11,12 | Method 1 | 95,04 | 1618,8575 | 1618,8622 | [M+2H]²⁺ | 96,30 | 84,20 |
| 34 | 10,18 | Method 1 | 96,33 | 1664,8630 | 1664,8806 | [M+2H]²⁺ | 96,80 | 94,90 |
| 35 | 9,80 | Method 1 | 100 | 1519,7891 | 1519,805 | [M+2H]²⁺ | 99,0 | 97,90 |
| 36 | 10,02 | Method 1 | 100 | 1505,7734 | 1505,7900 | [M+2H]²⁺ | 97,10 | 96,90 |
| 37 | 9,22 | Method 1 | 100 | 1519,7639 | 1519,7802 | [M+2H]²⁺ | 96,02 | 93,57 |
| 38 | 10,85 | Method 1 | 60,23 | 1632,8732 | 1632,8916 | [M+2H]²⁺ | 95,20 | 97,60 |
| 39 | 9,66 | Method 1 | 100 | 1619,8164 | 1619,8314 | [M+2H]²⁺ | 95,80 | 96,10 |
| 40 | 9,29 | Method 4 | 100 | 1589,8422 | 1589,8616 | [M+2H]²⁺ | 95,20 | 92,40 |
| 41 | 10,09 | Method 1 | 100 | 1672,8793 | 1672,8966 | [M+2H]²⁺ | 95,20 | 95,30 |
| 42 | 8,94 | Method 4 | 100 | 1658,8637 | 1658,8768 | [M+2H]²⁺ | 92,60 | 91,70 |
| 43 | 10,43 | Method 1 | 93,88 | 1657,9048 | 1657,9208 | [M+2H]²⁺ | 95,00 | 94,20 |
| 44 | 9,53 | Method 1 | 74,12 | 1604,8531 | 1604,8698 | [M+2H]²⁺ | 92,80 | 95,40 |
| 45 | 9,05 | Method 4 | 95,95 | 1576,8106 | 1576,8252 | [M+2H]²⁺ | 98,20 | 95,50 |
| 46 | 9,56 | Method 4 | 92,72 | 1590,8262 | 1590,8444 | [M+2H]²⁺ | 97,60 | 95,80 |
| 47 | 9,37 | Method 4 | 100 | 1605,8007 | 1605,8172 | [M+2H]²⁺ | 96,00 | 95,50 |
| 48 | 8,66 | Method 4 | 100 | 1603,8578 | 1603,8752 | [M+2H]²⁺ | 92,10 | 90,30 |
| 49 | 9,67 | Method 4 | 100 | 1643,8891 | 1643,9060 | [M+2H]²⁺ | 96,00 | 96,60 |
| 50 | 7,82 | Method 4 | 100 | 1726,8027 | 1726,8166 | [M+2H]²⁺ | 95,00 | 96,80 |
| 51 | 9,83 | Method 4 | 98,41 | 1432,7934 | 1432,8126 | [M+2H]²⁺ | 94,80 | 95,50 |
| 52 | 10,14 | Method 4 | 100 | 1335,7407 | 1335,7584 | [M+2H]²⁺ | 97,60 | 97,00 |
| 53 | 10,04 | Method 4 | 100 | 1503,8306 | 1503,8482 | [M+2H]²⁺ | 98,20 | 98,90 |
| 54 | 9,81 | Method 4 | 100 | 1503,8306 | 1503,8482 | [M+2H]²⁺ | 96,80 | 97,40 |
| 55 | 7,37 | Method 4 | 100 | 1405,7574 | 1405,7768 | [M+2H]²⁺ | 97,10 | 95,60 |
| 56 | 7,36 | Method 4 | 100 | 1419,7730 | 1419,7938 | [M+2H]²⁺ | 97,00 | 96,50 |
| 57 | 5,06 | Method 4 | 100 | 1394,7526 | 1394,7741 | [M+3H]³⁺ | 93,10 | 94,60 |
| 58 | 9,87 | Method 4 | 100 | 1416,7621 | 1416,7774 | [M+2H]²⁺ | 96,5 | 99,4 |
| 59 | 10,20 | Method 4 | 100 | 1571,8932 | 1571,9084 | [M+2H]²⁺ | 96,9 | 95,7 |
| 60 | 8,89 | Method 4 | 95,70 | 1647,8477 | 1647,8648 | [M+2H]²⁺ | 98,1 | 92,8 |
| 61 | 7,36 | Method 4 | 100 | 1618,8687 | 1618,8858 | [M+2H]²⁺ | 78,0 | 74,7 |
| 62 | 5,74 | Method 5 | 100 | 1686,8950 | 1687,9146 | [M+2H]²⁺ | 94,90 | 89,10 |
| 63 | 9,87 | Method 4 | 100 | 1807,9576 | 1807,9742 | [M+2H]²⁺ | 98,70 | 99,0 |
| 64 | 9,91 | Method 4 | 100 | 1406,7778 | 1406,7934 | [M+2H]²⁺ | 99,70 | 99,70 |
| 65 | 6,10 | Method 4 | 100 | 1393,7574 | 1393,7756 | [M+2H]²⁺ | 92,60 | 96,20 |
| 66 | 7,28 | Method 4 | 100 | 1336,7359 | 1336,7526 | [M+2H]²⁺ | 98,4 | 93,5 |
| 67 | 7,08 | Method 4 | 97,43 | 1350,7516 | 1350,7672 | [M+2H]²⁺ | 95,9 | 96,2 |
| 68 | 10,34 | Method 4 | 100 | 1565,8462 | 1565,8532 | [M+2H]²⁺ | 99,3 | 99,8 |
| 69 | 8,77 | Method 4 | 100 | 1672,8793 | 1672,8952 | [M+2H]²⁺ | 98,8 | 98,70 |
| 71 | 7,43 | Method 4 | 96,28 | 1433,7887 | 1433,8060 | [M+2H]²⁺ | 97,80 | 96,30 |
| 72 | 9,64 | Method 4 | 100 | 1406,7778 | 1406,80 | [M+2H]²⁺ | | |
| 73 | 4,85 | Method 4 | 97,34 | 1408,7683 | 1408,7934 | [M+3H]³⁺ | 87,9 | 88,80 |
| 74 | 9,90 | Method 4 | 94,15 | 1671,9204 | 1671,9346 | [M+2H]²⁺ | 82,50 | 77,50 |
| 75 | 9,71 | Method 4 | 100 | 1600,8833 | 1600,90 | [M+2H]²⁺ | 97,90 | 93,40 |
| 76 | 9,42 | Method 4 | 98,91 | 1728,9419 | 1728,9546 | [M+2H]²⁺ | 95,40 | 93,80 |
| 77 | 9,84 | Method 4 | 100 | 1742,9576 | 1743,977 | [M+2H]²⁺ | 90,70 | 89,90 |
| 78 | 7,63 | Method 4 | 100 | 1350,7516 | 1350,7600 | [M+2H]²⁺ | 88,40 | 89,20 |
| 79 | 9,71 | Method 4 | 89,27 | 1671,9204 | 1671,936 | [M+2H]²⁺ | 90,80 | 92,90 |
| 80 | 7,41 | Method 4 | 100 | 1350,7516 | 1350,767 | [M+2H]²⁺ | 99,10 | 95,00 |
| 81 | 7,15 | Method 4 | 100 | 1643,8388 | 1643,8614 | [M+2H]²⁺ | 95,10 | 95,80 |
| 82 | 9,72 | Method 4 | 100 | 1728,9419 | 1729,9614 | [M+2H]²⁺ | 88,1 | 90,1 |
| 83 | 9,77 | Method 4 | 92,72 | 1657,9048 | 1657,9198 | [M+2H]²⁺ | 96,80 | 95,1 |
| 84 | 9,53 | Method 4 | 100 | 1728,9419 | 1729,9622 | [M+2H]²⁺ | 82,8 | 95,1 |
| 85 | 8,25 | Method 4 | 100 | 1445,7887 | 1445,8116 | [M+2H]²⁺ | | |
| 86 | 2,41 | Method 5 | 100 | 1531,8367 | 1531,8528 | [M+2H]²⁺ | | |
| 87 | 7,87 | Method 4 | 100 | 1445,7887 | 1445,8056 | [M+2H]²⁺ | | |
| 88 | 9,17 | Method 4 | 100 | 1319,7094 | 1319,7244 | [M+2H]²⁺ | | |
| 89 | 6,10 | Method 4 | 100 | 1531,8367 | 1531,86 | [M+3H]³⁺ | | |
| 90 | 7,70 | Method 4 | 100 | 1672,9157 | 1672,9342 | [M+2H]²⁺ | 97,7 | 95,8 |
| 91 | 8,08 | Method 4 | 100 | 1787,9426 | 1788,9644 | [M+2H]²⁺ | 97,7 | 95,8 |
| 92 | 9,30 | Method 4 | 100 | 1659,8477 | 1659,8638 | [M+2H]²⁺ | 98,40 | 97,43 |
| 93 | 7,83 | Method 4 | 100 | 1745,8957 | 1746,90 | [M+2H]²⁺ | 97,56 | 88,43 |
| 94 | 9,45 | Method 1 | 97,73 | 1562,7949 | 1562,8106 | [M+2H]²⁺ | | |
| 95 | 5,43 | Method 1 | 100 | 1347,7407 | 1347,7566 | [M+2H]²⁺ | | |
| 96 | 7,16 | Method 4 | 100 | 1490,8102 | 1490,8254 | [M+2H]²⁺ | 90,50 | 93,90 |
| 97 | 5,60 | Method 4 | 95,90 | 1491,8054 | 1491,8298 | [M+3H]³⁺ | 97,60 | 92,00 |
| 98 | 9,75 | Method 4 | 95,01 | 1659,8477 | 1659,8638 | [M+2H]²⁺ | 96,6 | 96,5 |

**Table 21: Analytical Ion Chromatography Data for HCl Salts of peptides prepared according to the invention**

| **Ex. No** | **% Chloride** | **Equiv. Chloride** | **% TFA** | **Method** |
|---|---|---|---|---|
| 78 | 10.9% | 4.53 | < 1% | Method N3 |
| 79 | 4.9% | 2.36 | < 1% | Method N3 |
| 88 | 2.7% | 1.01 | < 1% | Method N 1 |
| 94 | 3.1% | 1.4 | < 0.5% | Method N2 |

In the following, the examples are exemplified by their chemical structure. The present invention includes pharmaceutically acceptable salts, solvates or solvates of the salts of these examples. Chemical structures are displayed as salt free forms, if not indicated differently. Due to the large ring molecules long carbon chains might appear as round bonds, although the -CH₂-chains are drawn correctly. See e.g. Example 13, where the CH₂ groups of the the Ahx chain appear as almost round drawing.

### Example 13

### Sequence: (Ahx)**-GIC+SRSLPPIC+IPD**

[(6S,9S,12S,15S,18R,21S,34S,36aS,42S,45R,48S,50aS,55aS)-21,42,48-tri[(2S)-butan-2-yl]-12-(3-carbamimidamidopropyl)-9,15-bis(hydroxymethyl)-6-(2-methylpropyl)-5,8,11,14,17,20,23,26,33,36,41,44,47,50,55-pentadecaoxotetrapentacontahydro-1H-18,45-(methanodithiomethano)tripyrrolo[2,1-f:2',1'-r:2",1"-u][1,4,7,10,13, 16,19,22,25,28,31,34,37,40,43]pentadecaazacyclononatetracontin-34-yl]acetic acid

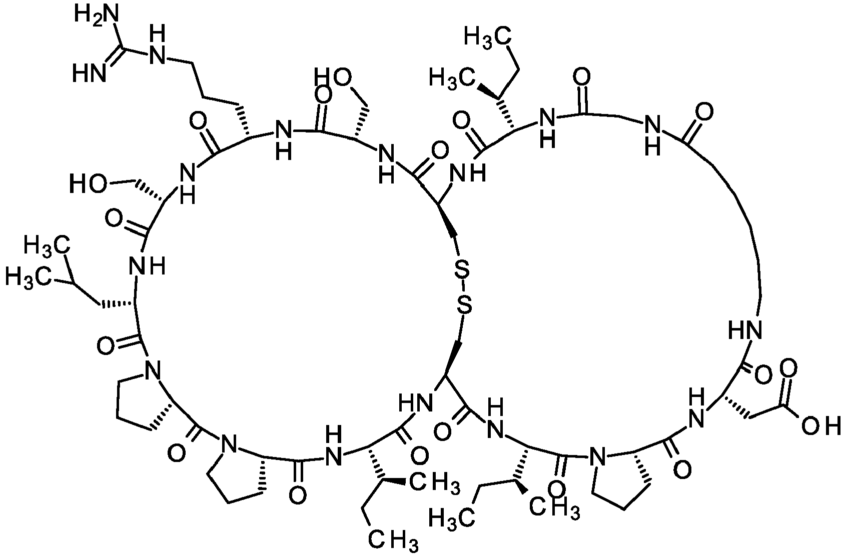

### Example 14

### Sequence: (PEG3(16 atoms))**-GIC+SRSLPPIC+IPD**

[(6S,9S,12S,15S,18R,21S,43S,45aS,51S,54R,57S,59aS,64aS)-21,51,57-tri[(2S)-butan-2-yl]-12-(3-carbamimidamidopropyl)-9,15-bis(hydroxymethyl)-6-(2-methylpropyl)-5,8,11,14,17,20,23,26,42,45,50,53,56,59,64-pentadecaoxooctapentacontahydro-1H,39H-18,54-(methanodithiomethano)tripyrrolo[2,1-r:2',1'-d₁:2",1"-g₁][1,4,7, 10,13,16,19,22,25,28,31,34,37,40,43,46,49,52,55]tetraoxapentadecaazacyclooctapentacontin-43-yl]acetic acid

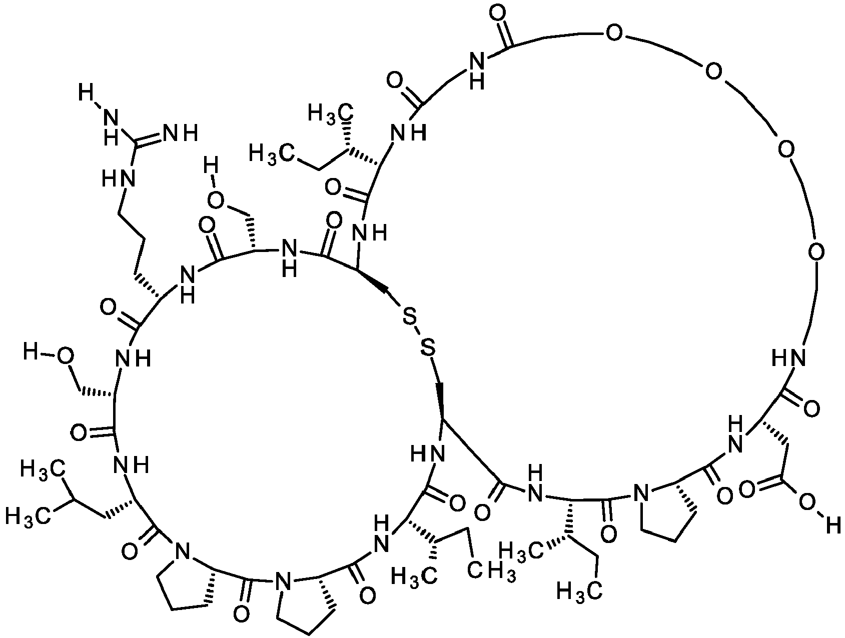

### Example 15

### Sequence: G**GIC+SRSLPPIC+IPD**

[(6S,9S,12S,15S,18R,21S,30S,32aS,38S,41R,44S,46aS,51aS)-21,38,44-tri[(2S)-butan-2-yl]-12-(3-carbamimidamidopropyl)-9,15-bis(hydroxymethyl)-6-(2-methylpropyl)-5,8,11,14,17,20,23,26,29,32,37,40,43,46,51-pentadecaoxopentacontahydro-1H-18,41-(methanodithiomethano)tripyrrolo[1,2-a:1',2'-d:1",2"-p][1,4,7,10,13,16, 19,22,25,28,31,34,37,40,43]pentadecaazacyclopentatetracontin-30-yl]acetic acid

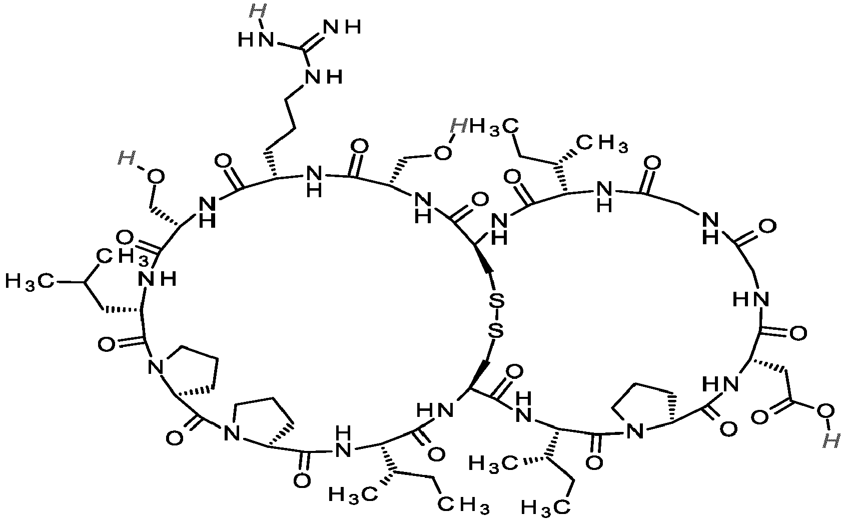

### Example 16

### Sequence: (Ahx)**-GIC+SRSLPPIC+IPd**

[(6S,9S,12S,15S,18R,21S,34R,36aS,42S,45R,48S,50aS,55aS)-21,42,48-tri[(2S)-butan-2-yl]-12-(3-carbamimidamidopropyl)-9,15-bis(hydroxymethyl)-6-(2-methylpropyl)-5,8,11,14,17,20,23,26,33,36,41,44,47,50,55-pentadecaoxotetrapentacontahydro-1H-18,45-(methanodithiomethano)tripyrrolo[2,1-f:2',1'-r:2",1"-u][1,4,7,10,13, 16,19,22,25,28,31,34,37,40,43]pentadecaazacyclononatetracontin-34-yl]acetic acid

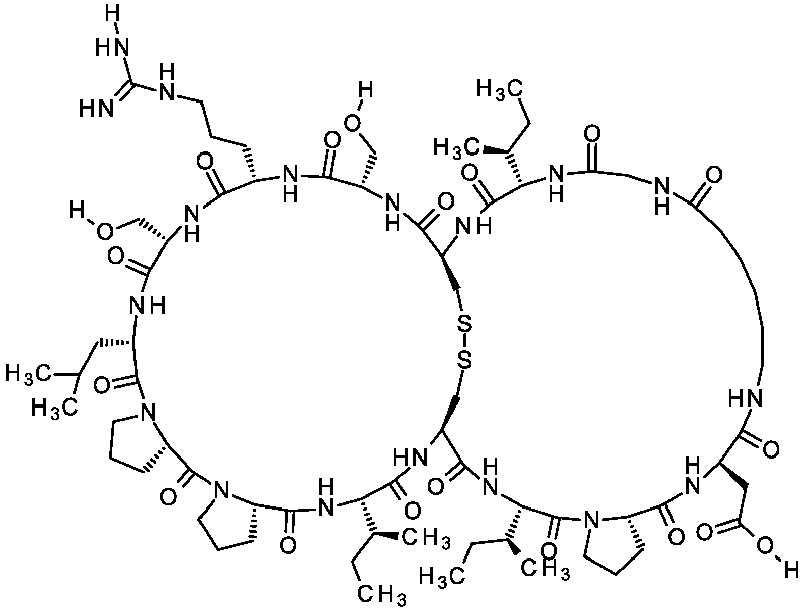

### Example 17

### Sequence: A**GGIC+SRSLPPIC+IPd**

[(6S,9S,12S,15S,18R,21S,30S,33R,35aS,41S,44R,47S,49aS,54aS)-21,41,47-tri[(2S)-butan-2-yl]-12-(3-carbamimidamidopropyl)-9,15-bis(hydroxymethyl)-30-methyl-6-(2-methylpropyl)-5,8,11,14,17,20,23,26,29,32,35, 40,43,46,49,54-hexadecaoxodopentacontahydro-1H,5H-18,44-(methanodithiomethano)tripyrrolo[1,2-a:1',2'-d:1",2"-p][1,4,7,10,13,16,19,22,25,28,31,34,37,40,43,46]hexadecaazacyclooctatetracontin-33-yl]acetic acid

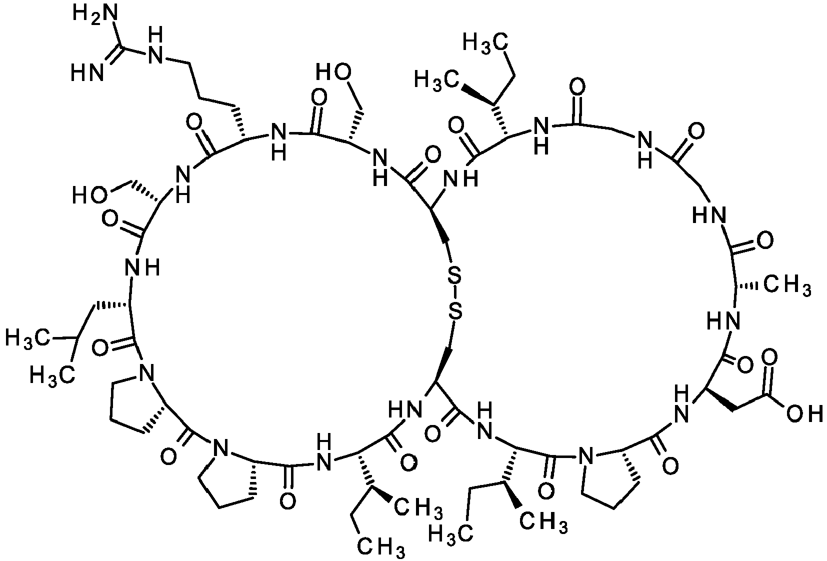

### Example 18

### Sequence: A**GGIC+SRSLPPIC+IPD**

[(6S,9S,12S,15S,18R,21S,30S,33S,35aS,41S,44R,47S,49aS,54aS)-21,41,47-tri[(2S)-butan-2-yl]-12-(3-carbamimidamidopropyl)-9,15-bis(hydroxymethyl)-30-methyl-6-(2-methylpropyl)-5,8,11,14,17,20,23,26,29,32,35, 40,43,46,49,54-hexadecaoxodopentacontahydro-1H,5H-18,44-(methanodithiomethano)tripyrrolo[1,2-a: 1',2'-d:1",2"-p][1,4,7,10,13,16,19,22,25,28,31,34,37,40,43,46]hexadecaazacyclooctatetracontin-33-yl]acetic acid

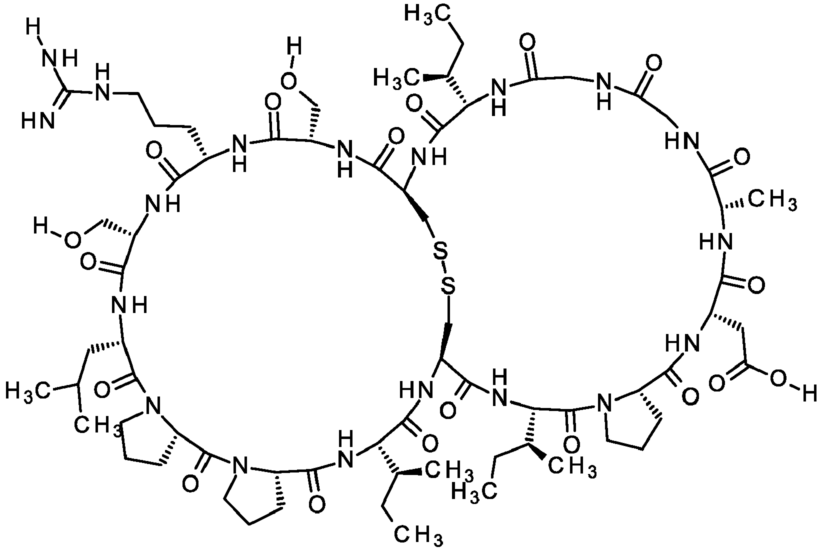

### Example 19

### Sequence: a**GGIC+SRSLPPIC+IPD**

[(6S,9S,12S,15S,18R,21S,30R,33S,35aS,41S,44R,47S,49aS,54aS)-21,41,47-tri[(2S)-butan-2-yl]-12-(3-carbamimidamidopropyl)-9,15-bis(hydroxymethyl)-30-methyl-6-(2-methylpropyl)-5,8,11,14,17,20,23,26,29,32,35, 40,43,46,49,54-hexadecaoxodopentacontahydro-1H,5H-18,44-(methanodithiomethano)tripyrrolo[1,2-a: 1',2'-d:1",2"-p][1,4,7,10,13,16,19,22,25,28,31,34,37,40,43,46]hexadecaazacyclooctatetracontin-33-yl]acetic acid

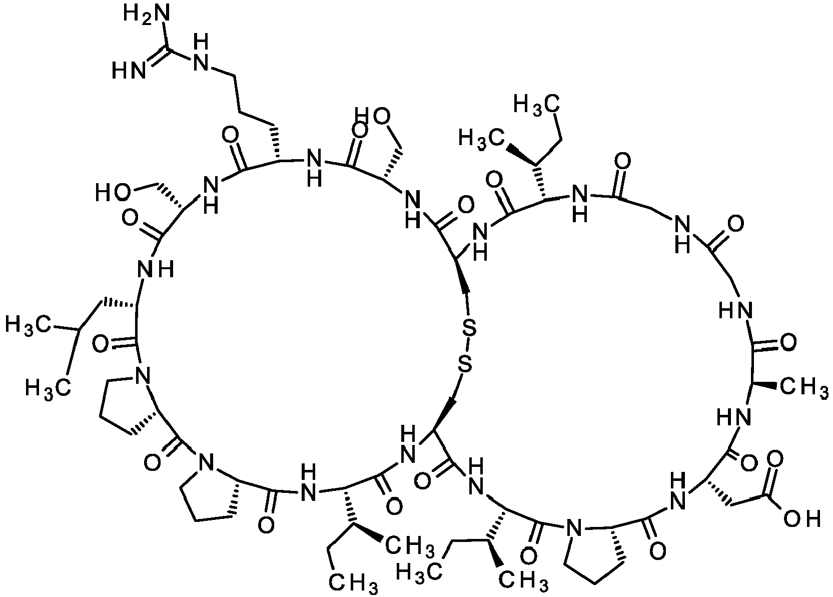

### Example 20

### Sequence: (Dap)++-GGIC+SRSLPPIC+IPD***

[(6S,9S,12S,15S,18R,21S,30S,34S,36aS,42S,45R,48S,50aS,55aS)-30-amino-21,42,48-tri[(2S)-butan-2-yl]-12-(3-carbamimidamidopropyl)-9,15-bis(hydroxymethyl)-6-(2-methylpropyl)-5,8,11,14,17,20,23,26,29,33,36,41, 44,47,50,55-hexadecaoxotetrapentacontahydro-1H-18,45-(methanodithiomethano)tripyrrolo[2,1-f:2',1'-r:2",1"-u][1,4,7,10,13,16,19,22,25,28,31,34,37,40,43,46]hexadecaazacyclononatetracontin-34-yl]acetic acid

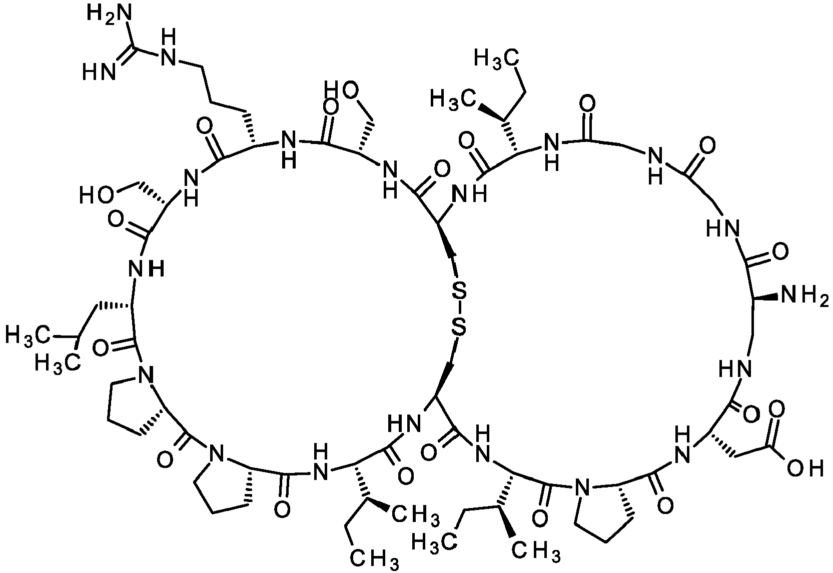

### Example 21

### Sequence: G**SGIC+SRSLPPIC+IPDS**

[(6S,9S,12S,15S,18F,21S,27S,33S,36S,38aS,44S,47F,50S,52aS,57aS)-21,44,50-tri[(2S)-butan-2-yl]-12-(3-carbamimidamidopropyl)-9,15,27,33-tetrakis(hydroxymethyl)-6-(2-methylpropyl)-5,8,11,14,17,20,23,26,29, 32,35,38,43,46,49,52,57-heptadecaoxohexapentacontahydro-1H-18,47-(methanodithiomethano)tripyrrolo[1,2-a:1',2'-d:1",2"-p][1,4,7,10,13,16,19,22,25,28,31,34,37,40,43,46,49]heptadecaazacyclohenpentacontin-36-yl]acetic acid

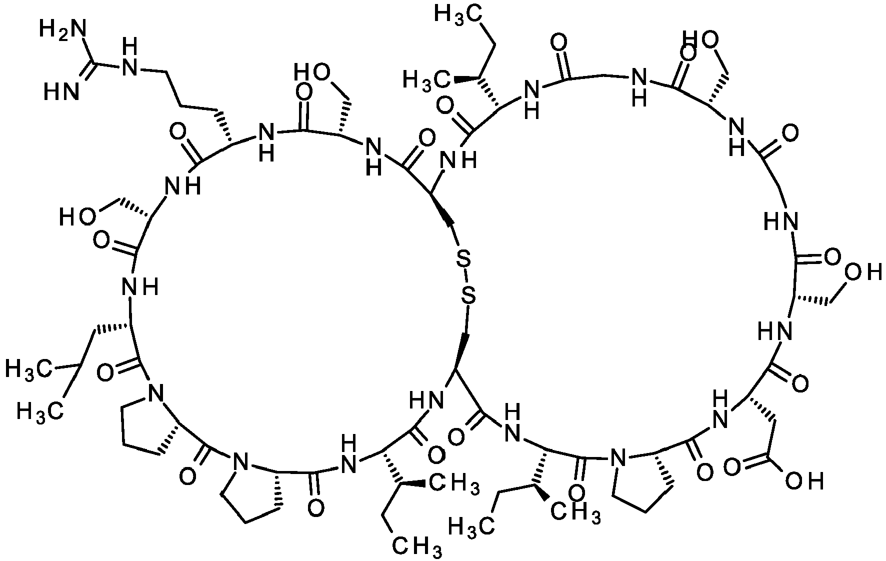

### Example 22

### Sequence: K++GIC+SRSLPPIC+IPD**

[(6S,9S,12S,15S,18R,21S,27S,34S,36aS,42S,45R,48S,50aS,55aS)-27-amino-21,42,48-tri[(2S)-butan-2-yl]-12-(3-carbamimidamidopropyl)-9,15-bis(hydroxymethyl)-6-(2-methylpropyl)-5,8,11,14,17,20,23,26,33,36,41,44, 47,50,55-pentadecaoxotetrapentacontahydro-1H-18,45-(methanodithiomethano)tripyrrolo[2,1-f:2',r-r:2",1"-u][1,4,7,10,13,16,19,22,25,28,31,34,37,40,43]pentadecaazacyclononatetracontin-34-yl]acetic acid

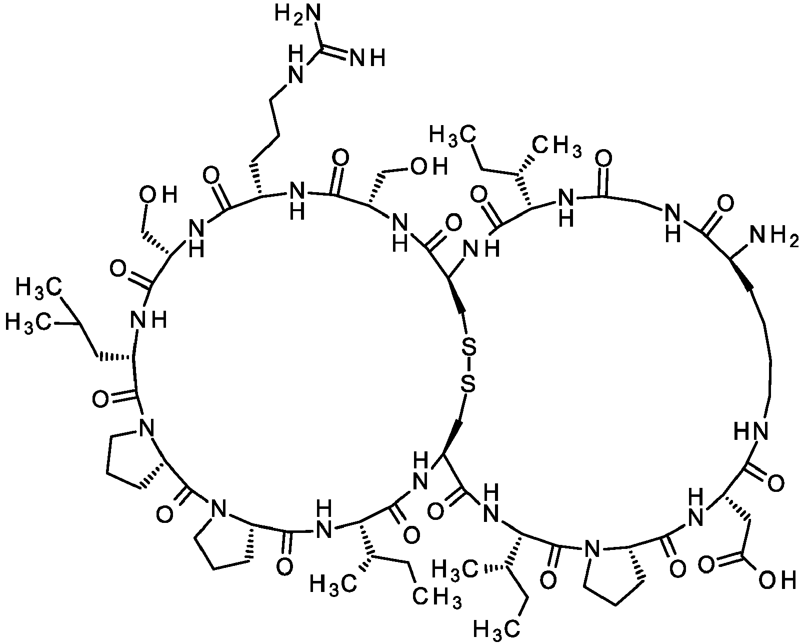

### Example 23

### Sequence: (Dap)**-(Dap)-GIC+SRSLPPIC+IPD**

[(6S,9S,12S,15S,18R,21S,27S,30S,33S,35aS,41S,44R,47S,49aS,54aS)-27,30-bis(aminomethyl)-21,41,47-tri-[(2S)-butan-2-yl] -12-(3 -carbamimidamidopropyl)-9,15-bis(hydroxymethyl)-6-(2-methylpropyl)-5,8,11,14, 17,20,23,26,29,32,35,40,43,46,49,54-hexadecaoxodopentacontahydro-1H,5H-18,44-(methanodithiomethano)-tripyrrolo[1,2-a:1',2'-d:1",2"-p][1,4,7,10,13,16,19,22,25,28,31,34,37,40,43,46]hexadecaazacyclooctatetracontin-33-yl]acetic acid

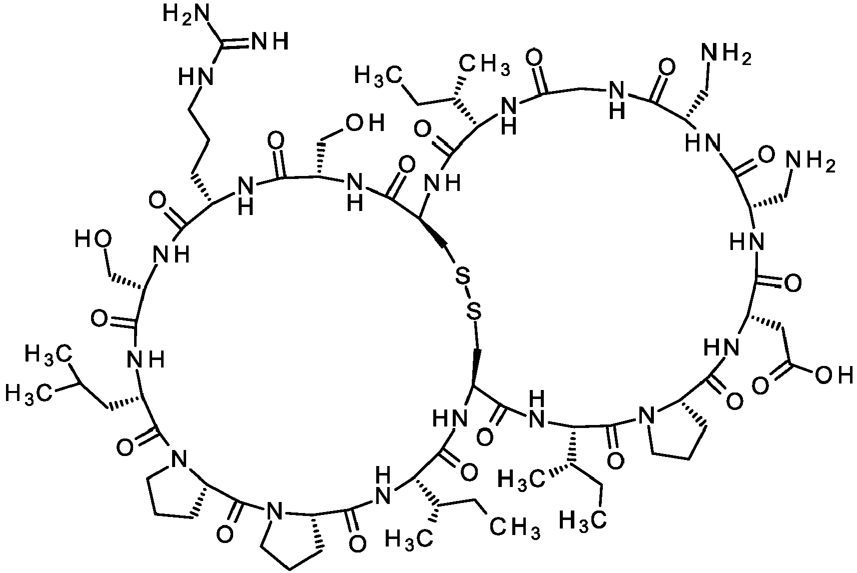

### Example 24

### Sequence: (PEG1(10 atoms))**-GIC+SRSLPPIC+IPD**

[(6S,9S,12S,15S,18R,21S,37S,39aS,45S,48R,51S,53aS,58aS)-21,45,51-tri[(2S)-butan-2-yl]-12-(3-carbamimidamidopropyl)-9,15-bis(hydroxymethyl)-6-(2-methylpropyl)-5,8,11,14,17,20,23,26,36,39,44,47,50,53,58-pentadecaoxotetrapentacontahydro-1H,33H-18,48-(methanodithiomethano)tripyrrolo[2,1-1:2',1'-x:2",1"-a₁][1,4,7, 10,13,16,19,22,25,28,31,34,37,40,43,46,49]dioxapentadecaazacyclodopentacontin-37-yl]acetic acid

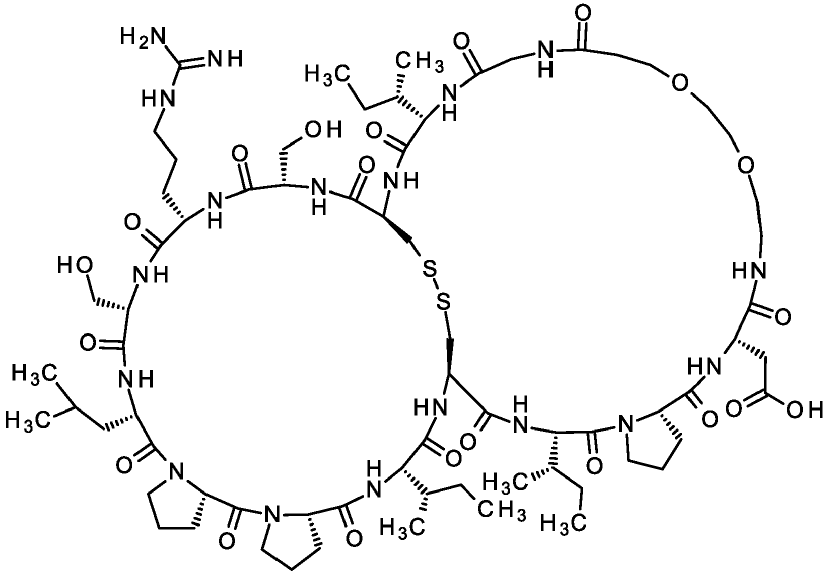

### Example 25

### Sequence: (Ahx)**-AIC+SRSLP-(Oic)-IC+IP**

N-{3-[(3aS,13S,16S,19R,22S,25S,28S,31S,36aS,38aS,42aS,43aS,46S,49R,52S)-16,46,52-tri[(2S)-butan-2-yl]-22,28-bis(hydroxymethyl)-13-methyl-31-(2-methylpropyl)-4,11,14,17,20,23,26,29,32,37,44,47,50,53-tetradecaoxotetrapentacontahydro-1H,34H-19,49-(methanodithiomethano)dipyrrolo[2',1':18,19;2",13,4][1,4,7, 10,13,16,19,22,25,28,31,34,37,40]tetradecaazacyclohexatetracontino[16,15-a]indol-25-yl]propyl}guanidine

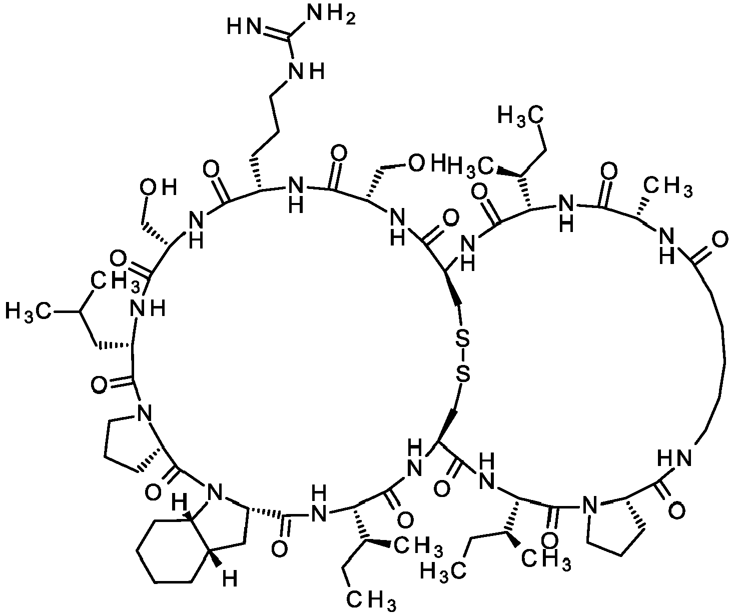

### Example 26

### Sequence: A**GAIC+SRS-((tBu)A)-PPI-(Pen)+-IPD**

[(6S,9S,12S,15S,18F,21S,24S,30S,33S,35aS,41S,44F,47S,49aS,54aS)-21,41,47-tri[(2S)-butan-2-yl]-12-(3-carbamimidamidopropyl)-6-(2,2-dimethylpropyl)-9,15-bis(hydroxymethyl)-24,30,55,55-tetramethyl-5,8,11, 14,17,20,23,26,29,32,35 ,40,43,46,49,54-hexadecaoxodopentacontahydro-1H,5H-18,44-(methanodithiomethano)tripyrrolo[1,2-a:1',2'-d:1",2"-p][1,4,7,10,13,16,19,22,25,28,31,34,37,40,43,46]hexadecaazacyclooctatetracontin-33-yl]acetic acid

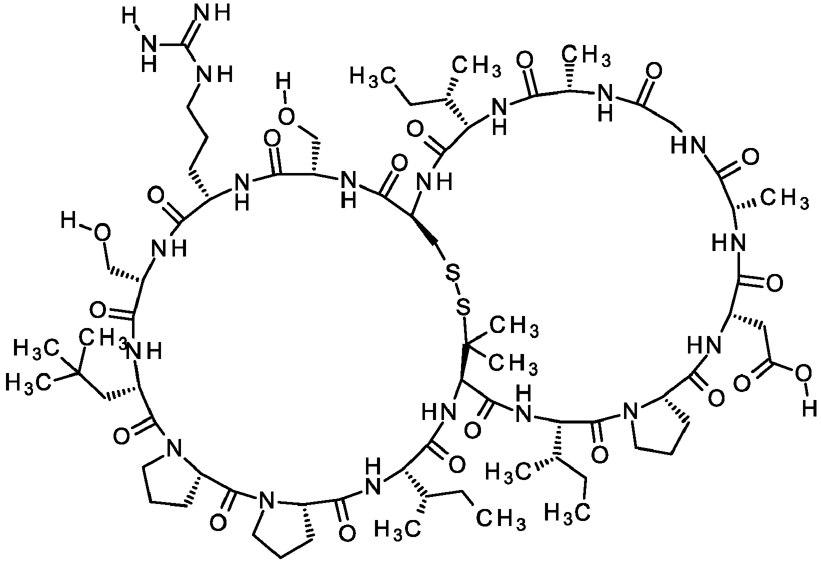

### Example 27

### Sequence: (Ahx)**-AIC+SRS-((tBu)A)-PPI-(Pen)+-IPD**

[(6S,9S,12S,15S,18F,21S,24S,34S,36aS,42S,45F,48S,50aS,55aS)-21,42,48-tri[(2S)-butan-2-yl]-12-(3-carbamimidamidopropyl)-6-(2,2-dimethylpropyl)-9,15-bis(hydroxymethyl)-24,56,56-trimethyl-5,8,11,14,17,20, 23,26,33,36,41,44,4 7,50,55-pentadecaoxotetrapentacontahydro-1H-18,45-(methanodithiomethano)tripyrrolo[2,1-f:2',1'-r:2",1"-u][1,4,7,10,13,16,19,22,25,28,31,34,37,40,43]pentadecaazacyclononatetracontin-34-yl]-acetic acid

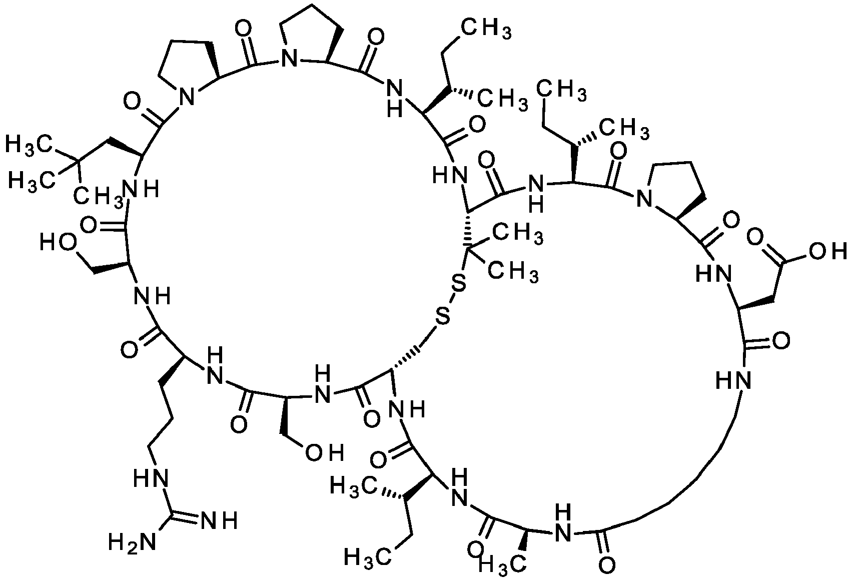

### Example 28

### Sequence: (Ahx)**-GIC+SRS-((tBu)A)-PPI-(Pen)+-IPD**

[(6S,9S,12S,15S,18R,21S,34S,36aS,42S,45R,48S,50aS,55aS)-21,42,48-tri[(2S)-butan-2-yl]-12-(3-carbamimidamidopropyl)-6-(2,2-dimethylpropyl)-9,15-bis(hydroxymethyl)-56,56-dimethyl-5,8,11,14,17,20,23,26,33, 36,41,44,47,50,55-pentadecaoxotetrapentacontahydro-1H-18,45-(methanodithlomethano)tripyrrolo[2,1-f:2',1'-r:2",1"-u][1,4,7,10,13,16,19,22,25,28,31,34,37,40,43]pentadecaazacyclononatetracontin-34-yl]acetic acid

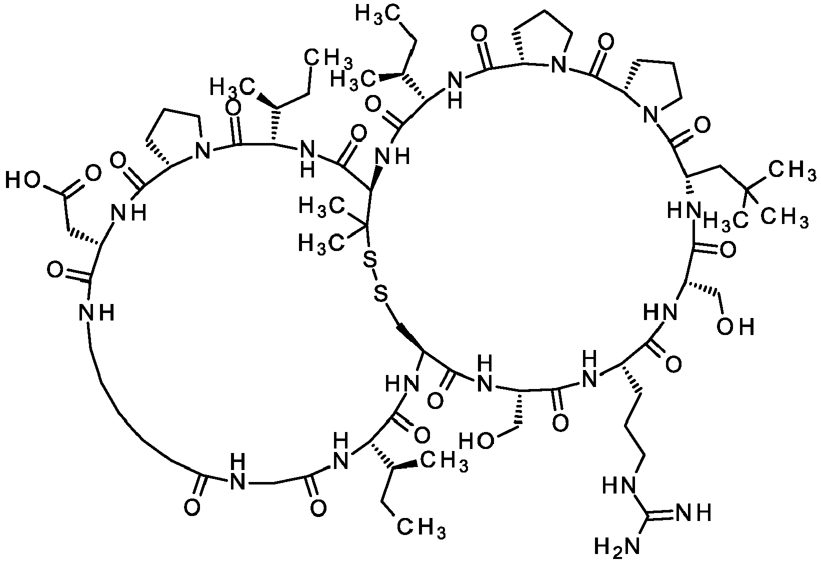

### Example 29

### Sequence: A**GGIC+SRS-((tBu)A)-PPI-(Pen)+-IPD**

[(6S,9S,12S,15S,18R,21S,30S,33S,35aS,41S,44R,47S,49aS,54aS)-21,41,47-tri[(2S)-butan-2-yl]-12-(3-carbamimidamidopropyl)-6-(2,2-dimethylpropyl)-9,15-bis(hydroxymethyl)-30,55,55-trimethyl-5,8,11,14,17,20, 23,26,29,32,35,40,43,46,49,54-hexadecaoxodopentacontahydro-1H,5H-18,44-(methanodithiomethano)tripyrrolo[1,2-a:1',2'-d:1",2"-p][1,4,7,10,13,16,19,22,25,28,31,34,37,40,43,46]hexadecaazacyclooctatetracontin-33-yl]acetic acid

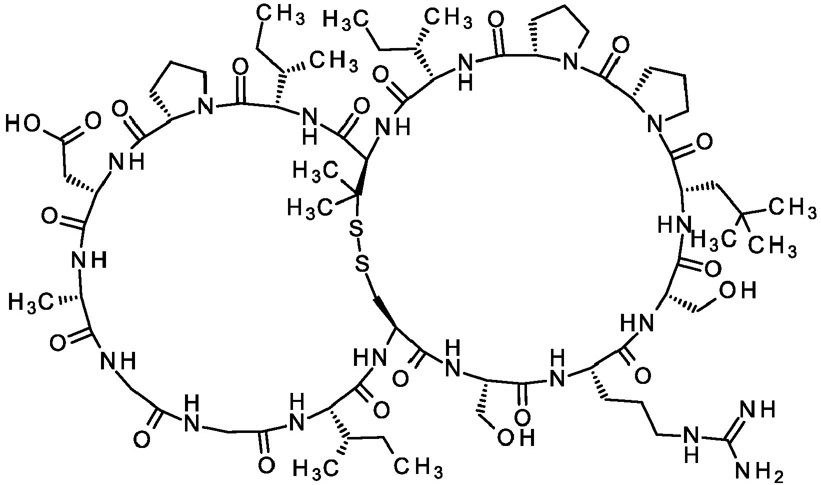

### Example 30

### Sequence: (Ahx)**-GIC+SRS-((tBu)A)-PPI-((N-Me)C)+-IPD**

[(6S,9S,12S,15S,18R,21S,34S,36aS,42S,45R,48S,50aS,55aS)-21,42,48-tri[(2S)-butan-2-yl]-12-(3-carbamimidamidopropyl)-6-(2,2-dimethylpropyl)-9,15-bis(hydroxymethyl)-46-methyl-5,8,11,14,17,20,23,26,33,36, 41,44,47,50,55-pentadecaoxotetrapentacontahydro-1H-18,45-(methanodithiomethano)tripyrrolo[2,1-f:2',1 r:2",1"-u][1,4,7,10,13,16,19,22,25,28,31,34,37,40,43]pentadecaazacyclononatetracontin-34-yl]aceticacid

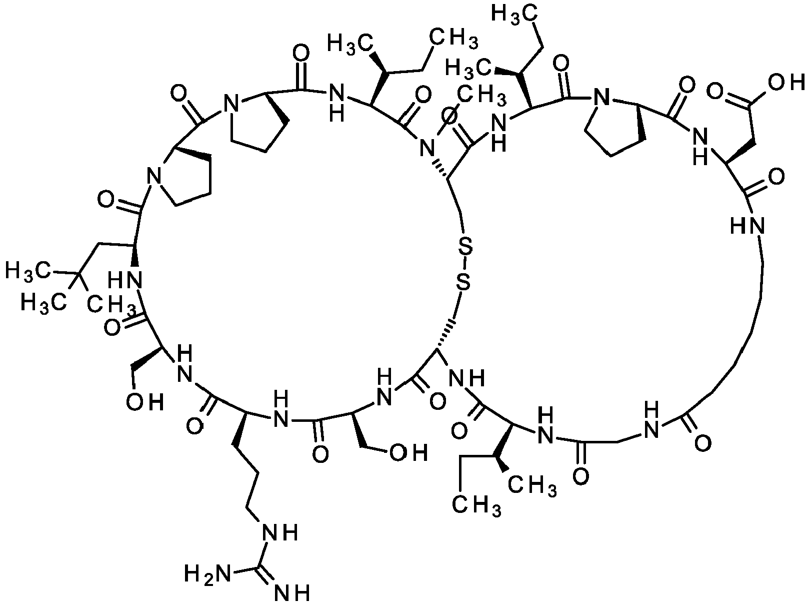

### Example 31

### Sequence: A**GAIC+SRS-((tBu)A)-PPI-((N-Me)C)+-IPD**

[(6S,9S,12S,15S,18F,21S,24S,30S,33S,35aS,41S,44F,47S,49aS,54aS)-21,41,47-tri[(2S)-butan-2-yl]-12-(3-carbamimidamidopropyl)-6-(2,2-dimethylpropyl)-9,15-bis(hydroxymethyl)-24,30,45-trimethyl-5,8,11,14,17, 20,23,26,29,32,35,40,43,46,49,54-hexadecaoxodopentacontahydro-1H,5H-18,44-(methanodithiomethano)tri-pyrrolo[1,2-a:1',2'-d:1",2"-p][1,4,7,10,13,16,19,22,25,28,31,34,37,40,43,46]hexadecaazacyclooctatetracontin-33-yl]acetic acid

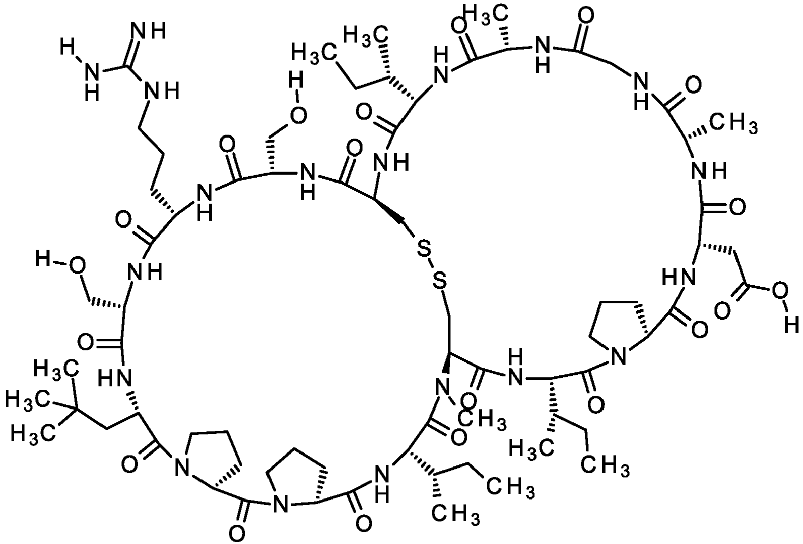

### Example 32

### Sequence: (Ahx)**-AIC+SRS-((tBu)A)-PPI-((N-Me)C)+-IPD**

[(6S,9S,12S,15S,18F,21S,24S,34S,36aS,42S,45F,48S,50aS,55aS)-21,42,48-tri[(2S)-butan-2-yl]-12-(3-carbamimidamidopropyl)-6-(2,2-dimethylpropyl)-9,15-bis(hydroxymethyl)-24,46-dimethyl-5,8,11,14,17,20,23, 26,33,36,41,44,47,50,55-pentadecaoxotetrapentacontahydro-1H-18,45-(methanodithiomethano)tripyrrolo-[2,1-f:2',1'-r:2",1"-u][1,4,7,10,13,16,19,22,25,28,31,34,37,40,43]pentadecaazacyclononatetracontin-34-yl]-acetic acid

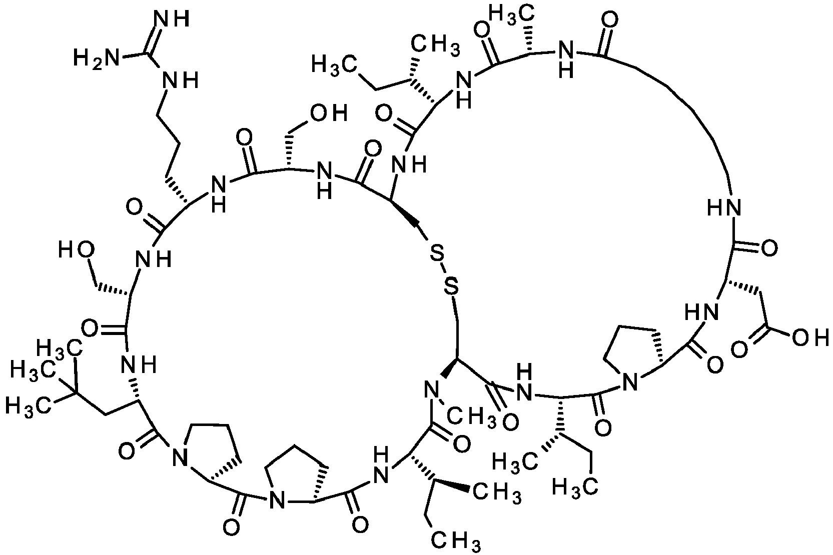

### Example 33

### Sequence: (Ahx)**-(Abu)-IC+SRS-((tBu)A)-PPI-((N-Me)C)+-IPD**

[(6S,9S,12S,15S,18R,21S,24S,34S,36aS,42S,45R,48S,50aS,55aS)-21,42,48-tri[(2S)-butan-2-yl]-12-(3-carbamimidamidopropyl)-6-(2,2-dimethylpropyl)-24-ethyl-9,15-bis(hydroxymethyl)-46-methyl-5,8,11,14,17,20, 23,26,33,36,41,44,47,50,55-pentadecaoxotetrapentacontahydro-1H-18,45-(methanodithiomethano)tripyrrolo-[2,1-f:2',1'-r:2",1"-u][1,4,7,10,13,16,19,22,25,28,31,34,37,40,43]pentadecaazacyclononatetracontin-34-yl]acetic acid

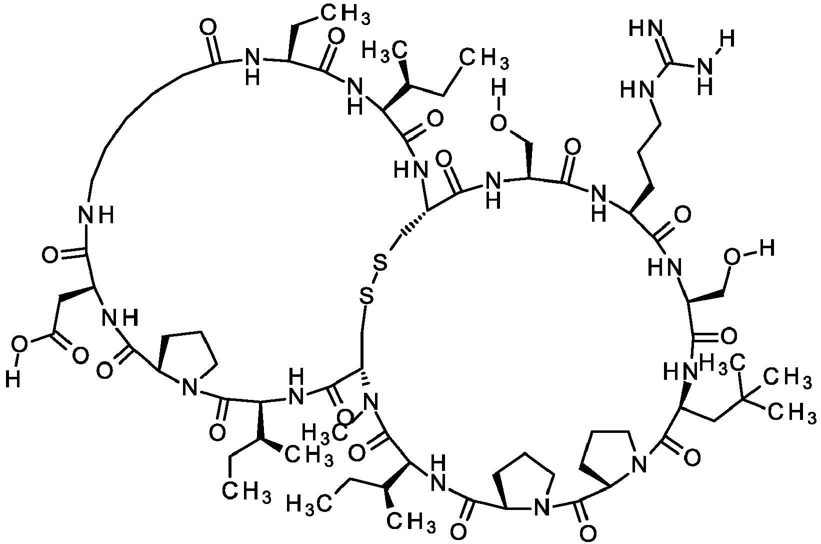

### Example 34

### Sequence: (PEG1(10 atoms))**-AIC+SRS-((tBu)A)-PPI-(Pen)+-IPD**

[(6S,9S,12S,15S,18R,21S,24S,37S,39aS,45S,48R,51S,53aS,58aS)-21,45,51-tri[(2S)-butan-2-yl]-12-(3-carbamimidamidopropyl)-6-(2,2-dimethylpropyl)-9,15-bis(hydroxymethyl)-24,59,59-trimethyl-5,8,11,14,17,20,23, 26,36,39,44,47,50,53,58-pentadecaoxotetrapentacontahydro-1H,33H-18,48-(methanodithiomethano)tripyrrolo[2,1-1:2',1'-x:2",1"-a₁][1,4,7,10,13,16,19,22,25,28,31,34,37,40,43,46,49]dioxapentadecaazacyclodopentacontin-37-yl]acetic acid

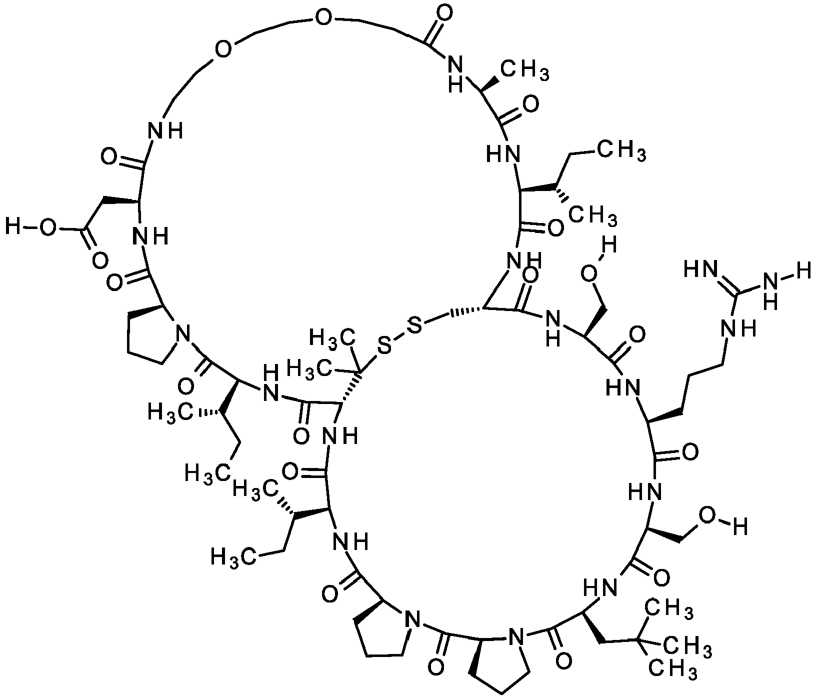

### Example 35

### Sequence: (Gamma-Abu)**-IC+SRS-((tBu)A)-PPI-(Pen)+-IPD**

[(6S,9S,12S,15S,18R,21S,29S,31aS,37S,40R,43S,45aS,50aS)-21,37,43-tri[(2S)-butan-2-yl]-12-(3-carbamimidarnidopropyl)-6-(2,2-dimethylpropyl)-9,15-bis(hydroxymethyl)-51,51-dimethyl-5,8,11,14,17,20,23,28, 31,36,39,42,45,50-tetradecaoxooctatetracontahydro-1H,5H-18,40-(methanodithiomethano)tripyrrolo[2,1-f:2',1'-r:2",1"-u][1,4,7,10,13,16,19,22,25,28,31,34,37,40]tetradecaazacyclotetratetracontin-29-yl]acetic acid

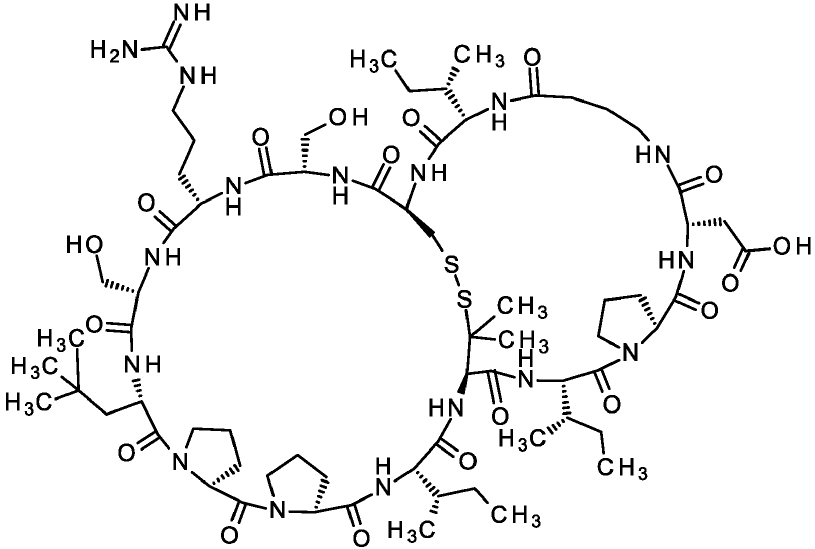

### Example 36

### Sequence: (Gamma-Abu)**-IC+SRS-((tBu)A)-PPI-((N-Me)C)+-IPD**

[(6S,9S,12S,15S,18R,21S,29S,31aS,37S,40R,43S,45aS,50aS)-21,37,43-tri[(2S)-butan-2-yl]-12-(3-carbamimidamidopropyl)-6-(2,2-dimethylpropyl)-9,15-bis(hydroxymethyl)-41-methyl-5,8,11,14,17,20,23,28, 31,36,39,42,45,50-tetradecaoxooctatetracontahydro-1H,5H-18,40-(methanodithiomethano)tripyrrolo[2,1-f:2',1'-r:2",1"-u][1,4,7,10,13,16,19,22,25,28,31,34,37,40]tetradecaazacyclotetratetracontin-29-yl]acetic acid

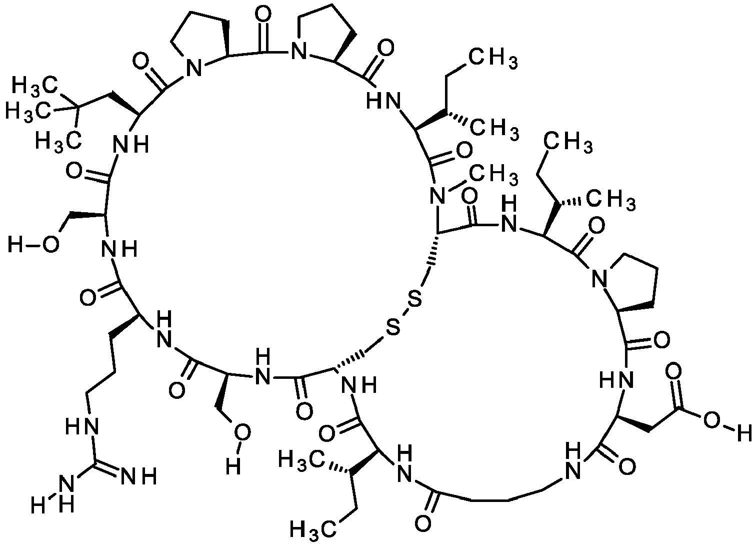

### Example 37

### Sequence: G**AIC+SRSLPPIC+IPD++-NH₂

(6S,9S,12S,15S,18R,21S,24S,31S,33aS,39S,42R,45S,47aS,52aS)-21,39,45-tri[(2S)-butan-2-yl]-12-(3-carbamimidamidopropyl)-9,15-bis(hydroxymethyl)-24-methyl-6-(2-methylpropyl)-5,8,11,14,17,20,23,26,29,33, 38,41,44,47,52-pentadecaoxopentacontahydro-1H,5H-18,42-(methanodithiomethano)tripyrrolo[2,1-c:2',1'-o:2",1"-r][1,4,7,10,13,16,19,22,25,28,31,34,37,40,43]pentadecaazacyclohexatetracontine-31-carboxamide

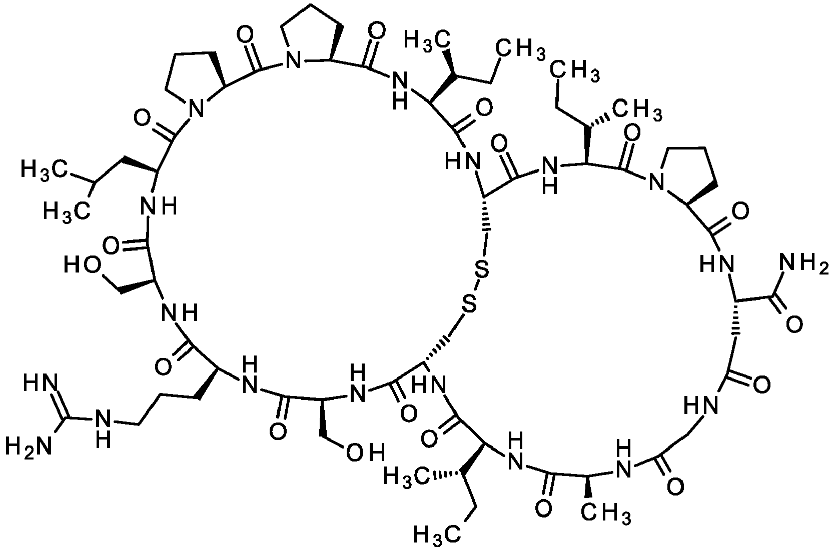

### Example 38

### Sequence: (Ahx)**-(Abu)-IC+SRS-((tBu)A)-PPI-(Pen)+-IPD**

[(6S,9S,12S,15S,18R,21S,24S,34S,36aS,42S,45R,48S,50aS,55aS)-21,42,48-tri[(2S)-butan-2-yl]-12-(3-carbamimidamidopropyl)-6-(2,2-dimethylpropyl)-24-ethyl-9,15-bis(hydroxymethyl)-56,56-dimethyl-5,8,11, 14,17,20,23,26,33,36,41,44,47,50,55-pentadecaoxotetrapentacontahydro-1H-18,45-(methanodithiomethano)tripyrrolo[2,1-f:2',1'-r:2",1"-u][1,4,7,10,13,16,19,22,25,28,31,34,37,40,43]pentadecaazacyclononatetracontin-34-yl]acetic acid

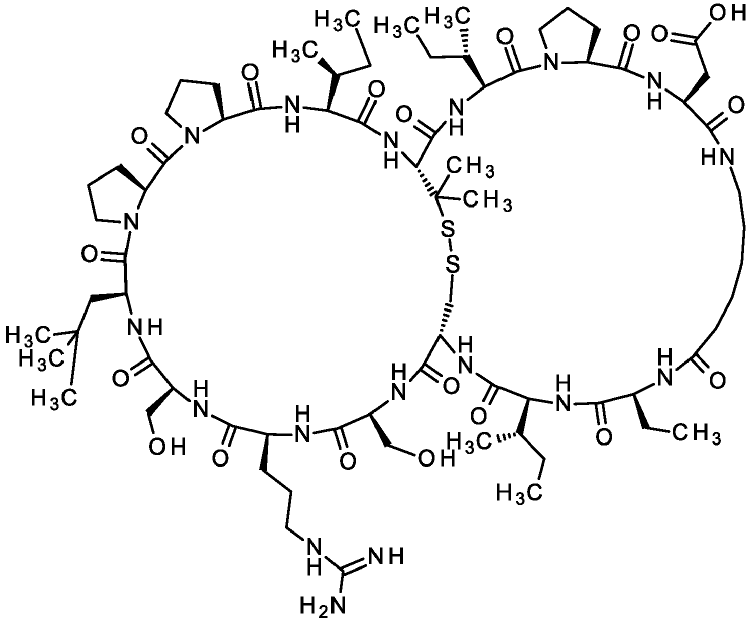

### Example 39

### Sequence: A**GGIC+SRS-((tBu)A)-PPI-(Pen)+-IPd**

[(6S,9S,12S,15S,18R,21S,30S,33R,35aS,41S,44R,47S,49aS,54aS)-21,41,47-tri[(2S)-butan-2-yl]-12-(3-carbamimidamidopropyl)-6-(2,2-dimethylpropyl)-9,15-bis(hydroxymethyl)-30,55,55-trimethyl-5,8,11,14,17,20,23, 26,29,32,35,40,43,46,49,54-hexadecaoxodopentacontahydro-1H,5H-18,44-(methanodithiomethano)tripyrrolo-[1,2-a:1',2'-d:1",2"-p][1,4,7,10,13,16,19,22,25,28,31,34,37,40,43,46]hexadecaazacyclooctatetracontin-33-yl]-acetic acid

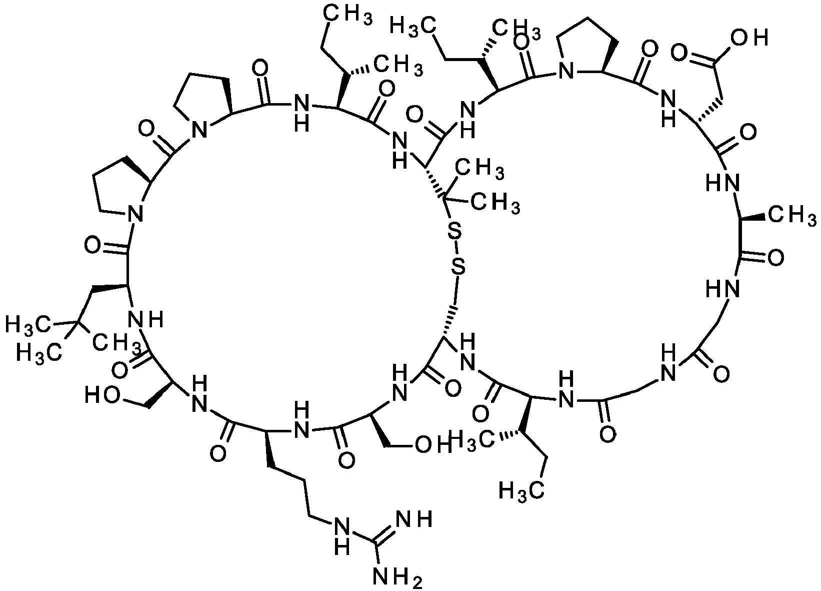

### Example 40

### Sequence: (Ahx)**-GIC+SRS-((tBu)A)-PPI-((N-Me)C)+-IPD++-NH₂

(6S,9S,12S,15S,18R,21S,35S,37aS,43S,46R,49S,51aS,56aS)-21,43,49-tri[(2S)-butan-2-yl]-12-(3-carbamimid-amidopropyl)-6-(2,2-dimethylpropyl)-9,15-bis(hydroxymethyl)-47-methyl-5,8,11,14,17,20,23,26,33,37,42,45, 48,51,56-pentadecaoxotetrapentacontahydro-1H,5H-18,46-(methanodithiomethano)tripyrrolo[1,2-v:1',2'-y:1",2"-k₁][1,4,7,10,13,16,19,22,25,28,31,34,37,40,44]pentadecaazacyclopentacontine-35-carboxamide

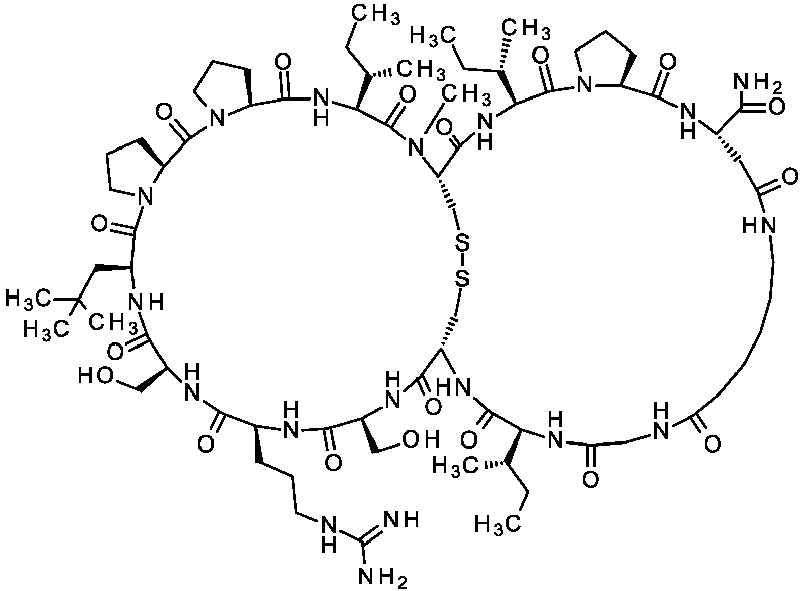

### Example 41

### Sequence: A**GAIC+SRSLP-(Oic)-I-(Pen)+-IPD++-NH₂

(3aS,6S,10S,16S,19S,22R,25S,28S,31S,34S,39aS,41aS,45aS,46aS,49S,52R,55S)-19,49,55tri[(2S)-butan-2-yl]-28-(3 -carbamimidamidopropyl)-25,31-bis(hydroxymethyl)-10,16,58,5 8-tetramethyl-34-(2-methylpropyl)-4,8,11,14,17,20,23,26,29,32,35,40,47,50,53,56-hexadecaoxooctapentacontahydro-3 7H-22,52-(methanodithio-methano)dipyrrolo[2',1':18,19;2",1":3,4][1,4,7,10,13,16,19,22,25,28,31,34,37,40,43,46]hexadecaazacyclonona-tetracontino[16,15 -a]indole-6-carboxamide

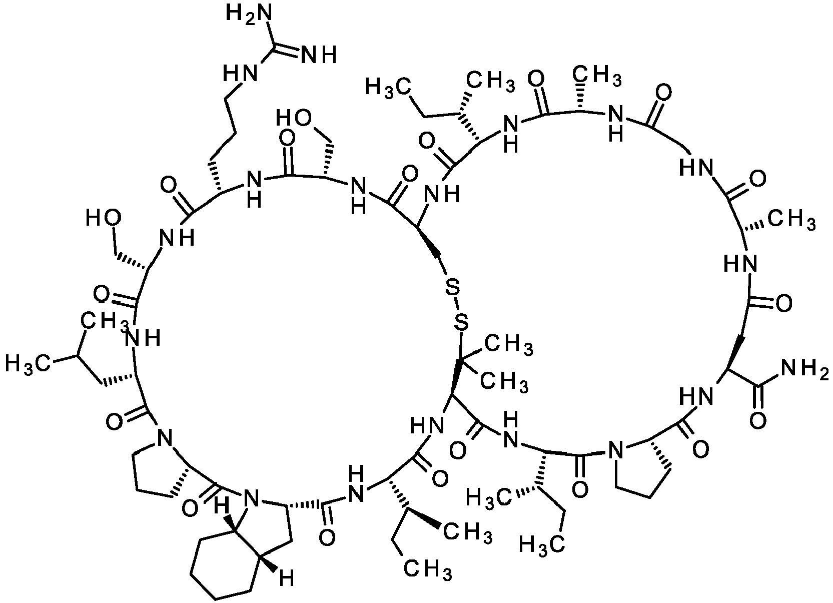

### Example 42

### Sequence: A**GGIC+SRSLP-(Oic)-I-(Pen)+-IPD++-NH₂

(3aS,6S,10S,19S,22R,25S,28S,31S,34S,39aS,41aS,45aS,46aS,49S,52R,55S)-19,49,55-tri[(2S)-butan-2-yl]-28-(3-carbamimidamidopropyl)-25,31-bis(hydroxymethyl)-10,58,58-trimethyl-34-(2-methylpropyl)-4,8,11, 14,17,20,23,26,29,32,35,40,47,50,53,56-hexadecaoxooctapentacontahydro-37H-22,52-(methanodithiometh-ano)dipyrrolo[2',1':18,19;2",1":3,4][1,4,7,10,13,16,19,22,25,28,31,34,37,40,43,46]hexadecaazacyclonona-tetracontino[16,15-a]indole-6-carboxamide

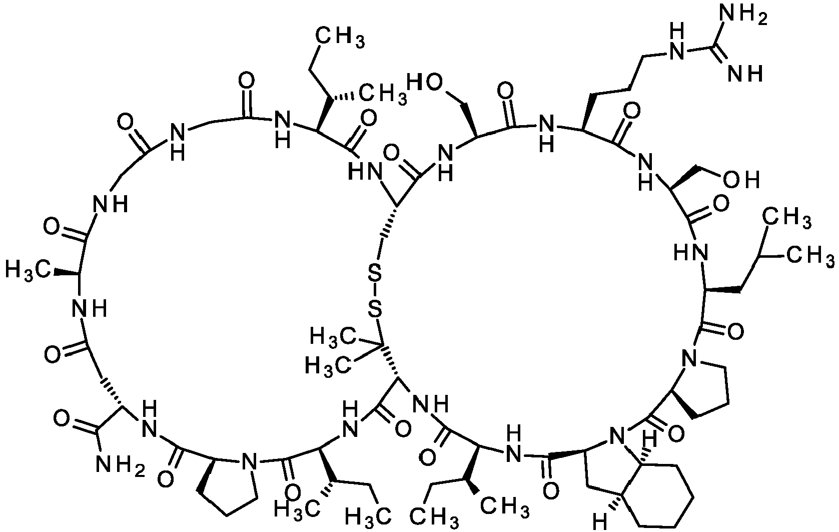

### Example 43

### Sequence: (Ahx)**AIC+SRSLP-(Oic)-I-(Pen)+-IPD++-NH2

(3aS,6S,17S,20S,23R,26S,29S,32S,35S,40aS,42aS,46aS,47aS,50S,53R,56S)-20,50,56-tri[(2S)-butan-2-yl]-29-(3-carbamimidamidopropyl)-26,32-bis(hydroxymethyl)-17,59,59-trimethyl-35-(2-methylpropyl)-4,8,15,18,21, 24,27,30,33,36,41,48,51,54,57-pentadecaoxooctapentacontahydro-1H,38H-23,53-(methanodithiomethano)di-pyrrolo[1',2':22,23;1",2":37,38][1,4,7,10,13,16,19,22,25,28,31,34,37,40,44]pentadecaazacyclopentacontino-[25,26-a]indole-6-carboxamide

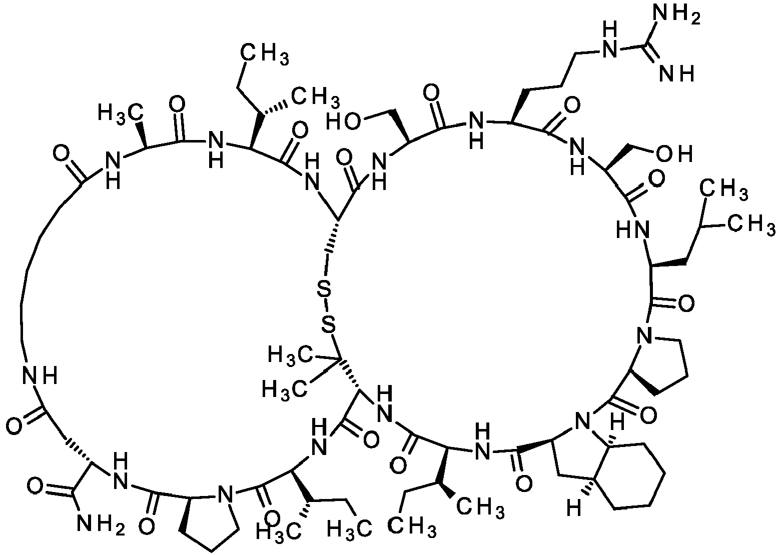

### Example 44

### Sequence: (Orn)++-AIC+SRS-((tBu)A)-PPI-((N-Me)C)+-IPD++-NH₂

(6S,9S,12S,15S,18R,21S,24S,27S,34S,36aS,42S,45R,48S,50aS,55aS)-27-amino-21,42,48-tri[(2S)-butan-2-yl]-12-(3-carbamimidamidopropyl)-6-(2,2-dimethylpropyl)-9,15-bis(hydroxymethyl)-24,46-dimethyl-5,8,11, 14,17,20,23,26,32,36,41,44,47,50,55-pentadecaoxotetrapentacontahydro-1H-18,45-(methanodithiomethano)-tripyrrolo[1,2-v:1',2'-y:1",2"-k₁[1,4,7,10,13,16,19,22,25,28,31,34,37,40,44]pentadecaazacyclononatetracon-tine-34-carboxamide

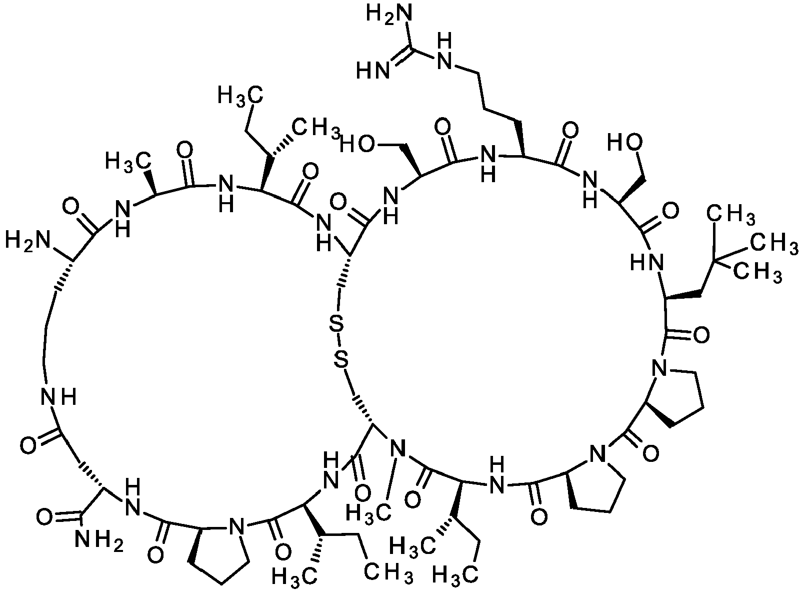

### Example 45

### Sequence: (Ahx)**-AIC+SRSLPPIC+IPD**

[(6S,9S,12S,15S,18R,21S,24S,34S,36aS,42S,45R,48S,50aS,55aS)-21,42,48-tri[(2S)-butan-2-yl]-12-(3-carb-amimidamidopropyl)-9,15-bis(hydroxymethyl)-24-methyl-6-(2-methylpropyl)-5,8,11,14,17,20,23,26,33,36, 41,44,47,50,55-pentadecaoxotetrapentacontahydro-1H-18,45-(methanodithiomethano)tripyrrolo[2,1-f:2',1'-r:2",1"-u][1,4,7,10,13,16,19,22,25,28,31,34,37,40,43]pentadecaazacyclononatetracontin-34-yl]acetic acid

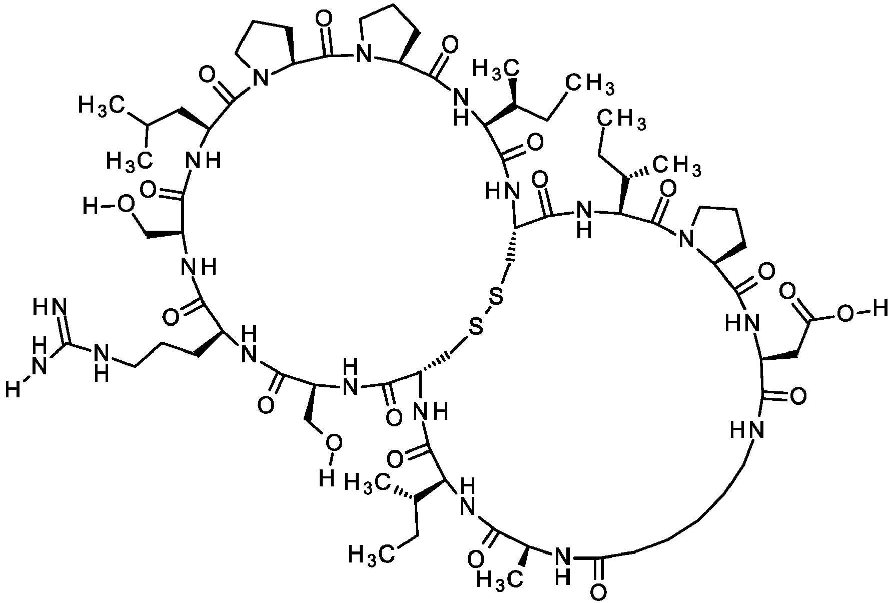

### Example 46

### Sequence: (Ahx)**-AIC+SRS-((tBu)A)-PPIC+IPD**

[(6S,9S,12S,15S,18R,21S,24S,34S,36aS,42S,45R,48S,50aS,55aS)-21,42,48-tri[(2S)-butan-2-yl]-12-(3-carb-amimidamidopropyl)-6-(2,2-dimethylpropyl)-9,15-bis(hydroxymethyl)-24-methyl-5,8,11,14,17,20,23,26,33, 36,41,44,47,50,55-pentadecaoxotetrapentacontahydro-1H-18,45-(methanodithiomethano)tripyrrolo[2,1-f2',1'-r:2",1"-u][1,4,7,10,13,16,19,22,25,28,31,34,37,40,43]pentadecaazacyclononatetracontin-34-yl]acetic acid

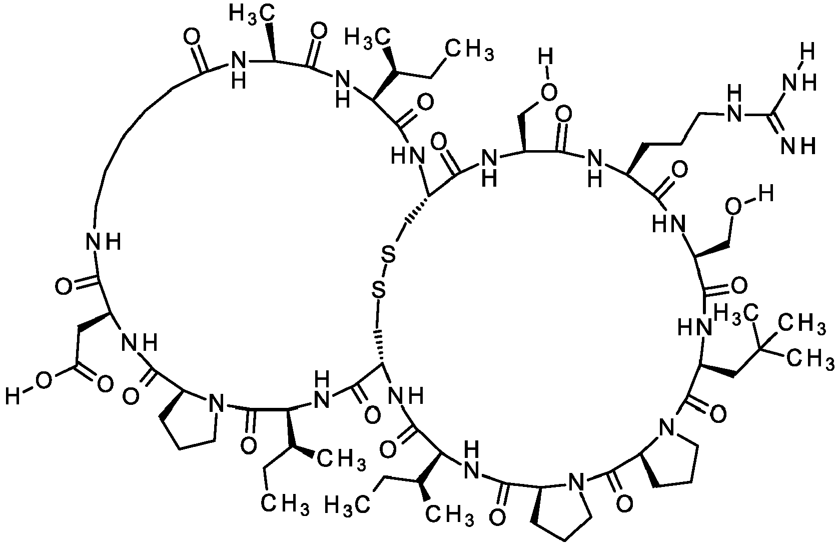

### Example 47

### Sequence: A**GGIC+SRS-((tBu)A)-PPI-((N-Me)C)+-IPd**

[(6S,9S,12S,15S,18R,21S,30S,33R,35aS,41S,44R,47S,49aS,54aS)-21,41,47-tri[(2S)-butan-2-yl]-12-(3-carb-amimidamidopropyl)-6-(2,2-dimethylpropyl)-9,15-bis(hydroxymethyl)-30,45-dimethyl-5,8,11,14,17,20,23,26, 29,32,35,40,43,46,49,54-hexadecaoxodopentacontahydro-1H,5H-18,44-(methanodithiomethano)tripyrrolo-[1,2-a:1',2'-d:1",2"-p][1,4,7,10,13,16,19,22,25,28,31,34,37,40,43,46]hexadecaazacyclooctatetracontin-33-yl]-acetic acid

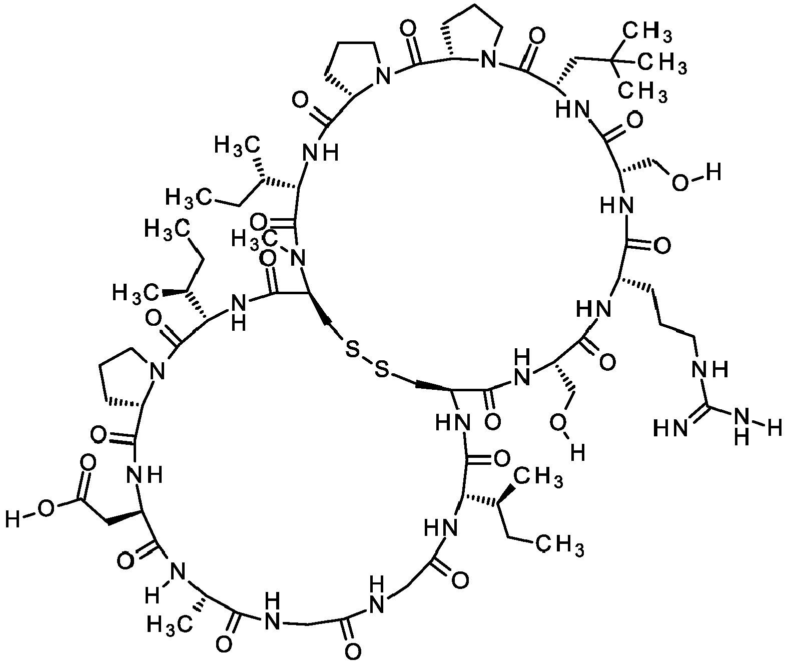

### Example 48

### Sequence: (Ahx)**-GIC+SRS-((tBu)A)-PPI-(Pen)+-IPD++-NH₂

(6S,9S,12S,15S,18R,21S,35S,37aS,43S,46R,49S,51aS,56aS)-21,43,49-tri[(2S)-butan-2-yl]-12-(3-carbamimid-amidopropyl)-6-(2,2-dimethylpropyl)-9,15-bis(hydroxymethyl)-57,57-dimethyl-5,8,11,14,17,20,23,26,33,37, 42,45,48,51,56-pentadecaoxotetrapentacontahydro-1H,5H-18,46-(methanodithiomethano)tripyrrolo[1,2-v:1',2' -y:1",2"-k₁][1,4,7,10,13,16,19,22,25,28,31,34,37,40,44]pentadecaazacyclopentacontine-35-carboxamide

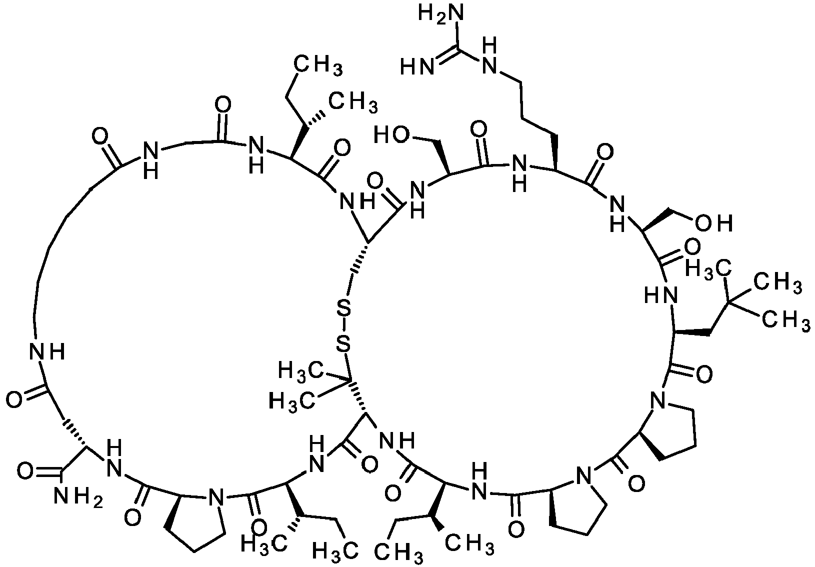

### Example 49

### Sequence: (Ahx)**-GIC+SRSLP-(Oic)-I-(Pen)+-IPD++-NH₂

(3aS,6S,20S,23R,26S,29S,32S,35S,40aS,42aS,46aS,47aS,50S,53R,56S)-20,50,56-tri[(2S)-butan-2-yl]-29-(3-carbamimidamidopropyl)-26,32-bis(hydroxymethyl)-59,59-dimethyl-35-(2-methylpropyl)-4,8,15,18,21,24, 27,30,33,36,41,48,51,54,57-pentadecaoxooctapentacontahydro-1H,38H-23,53-(methanodithiomethano)dipyrrolo[1',2':22,23;1",2":37,38][1,4,7,10,13,16,19,22,25,28,31,34,37,40,44]pentadecaazacyclopentacontino[25,26-a]indole-6-carboxamide

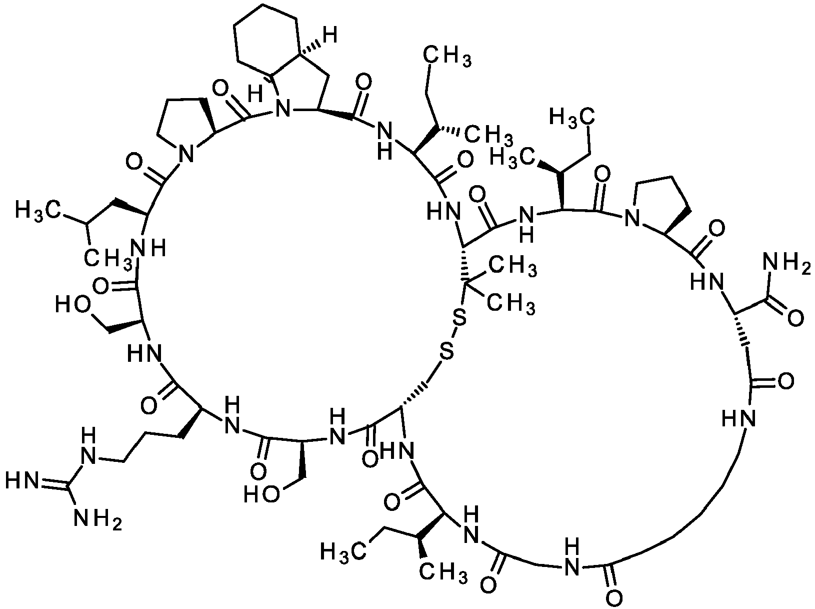

### Example 50

### Sequence: C++AIC+SRS-((tBu)A)-PPI-(Pen)+-IPDC++-NH₂

[(6S,9S,12S,15S,18R,21S,24S,27R,32R,35S,37aS,43S,46R,49S,51aS,56aS)-27-amino-21,43,49-tri[(2S)-butan-2-yl]-12-(3-carbamimidamidopropyl)-32-carbamoyl-6-(2,2-dimethylpropyl)-9,15-bis(hydroxymethyl)-24, 57,57-trimethyl-5,8,11,14,17,20,23,26,34,37,42,45,48,51,56-pentadecaoxodopentacontahydro-1H,31H-18,46-(methanodithiomethano)tripyrrolo[2,1-j:2',1'-v:2",1"-y][1,2,5,8,11,14,17,20,23,26,29,32,35,38,41,44, 47]dithiapentadecaazacyclopentacontin-35-yl] acetic acid

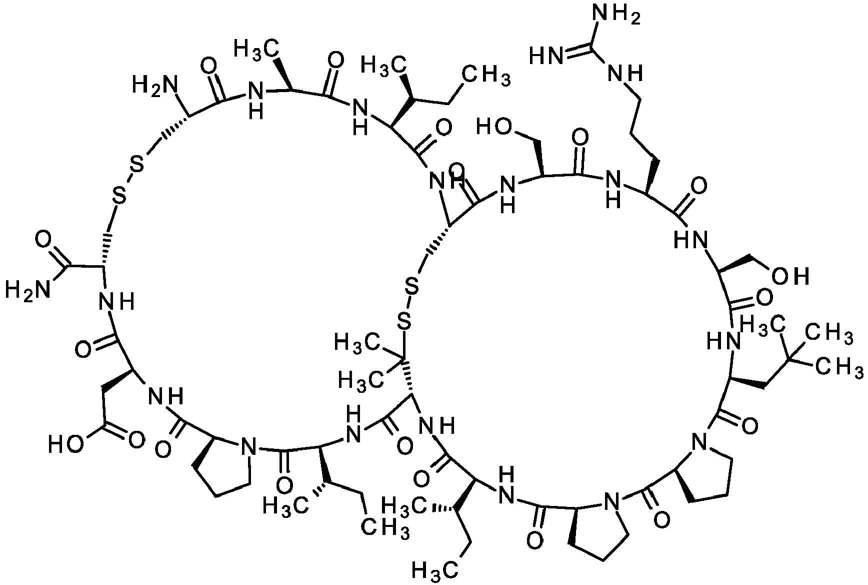

### Example 51

### Sequence: (Ahx)**-IC+SRS-((tBu)A)-PPI-(Pen)+-IP**

N-{13-[(6S,9S,12S,15S,18F,21S,30aS,36S,39F,42S,44aS,49aS)-21,36,42-tri[(2S)-butan-2-yl]-6-(2,2-dimethylpropyl)-9,15-bis(hydroxymethyl)-50,50-dimethyl-5,8,11,14,17,20,23,30,35,38,41,44,49-tridecaoxooctatetracontahydro-1H-18,39-(methanodithiomethano)tripyrrolo[2,1-c:2',1'-o:2",1"-r][1,4,7,10,13,16,19,22,25,28,31, 34,37]tridecaazacyclotritetracontin-12-yl]propyl}guanidine

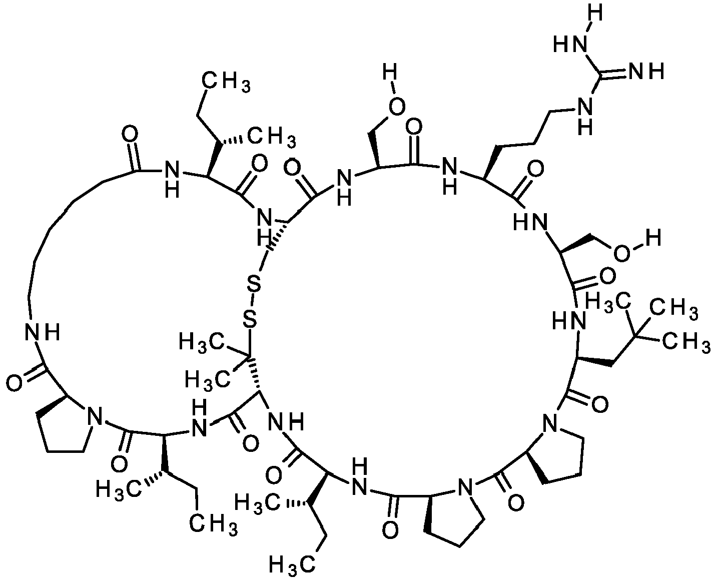

### Example 52

### Sequence: (Ahx)**-IC+SRS-((tBu)A)-PPI-(Pen)+-I**

N-{3-[(5aS,11S,14S,17S,20S,23R,26S,36S,39R,42S,44aS)-26,36,42-tri[(2S)-butan-2-yl]-11-(2,2-dimethylpropyl)-14,20-bis(hydroxymethyl)-45,45-dimethyl-5,10,13,16,19,22,25,28,35,38,41,44-dodecaoxodotetracontahydro-1H,5H-23,39-(methanodithiomethano)dipyrrolo[2,1-1:2',1'-o][1,4,7,10,13,16,19,22,25,28,31,34]dodecaazacyclotetracontin-17-yl]propyl}guanidine

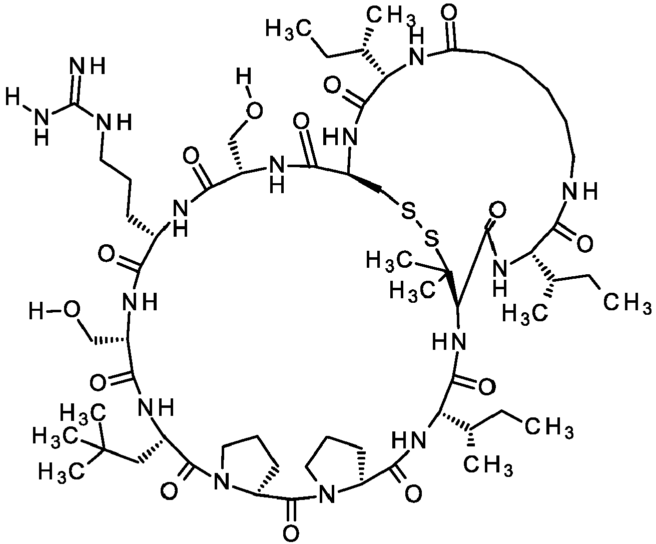

### Example 53

### Sequence: (Ahx)**-AIC+SRS-((tBu)A)-PPI-(Pen)+-IP**

N-{3-[(6S,9S,12S,15S,18R,21S,24S,33aS,39S,42R,45S,47aS,52aS)-21,39,45-tri[(2S)-butan-2-yl]-6-(2,2-dimethylpropyl)-9,15-bis(hydroxymethyl)-24,53,53-trimethyl-5,8,11,14,17,20,23,26,33,38,41,44,47,52-tetradecaoxopentacontahydro-1H,5H-18,42-(methanodithiomethano)tripyrrolo[2,1-c:2',r-o:2",1"-r][1,4,7,10,13,16, 19,22,25,28,31,34,37,40]tetradecaazacyclohexatetracontin-12-yl]propyl}guanidine

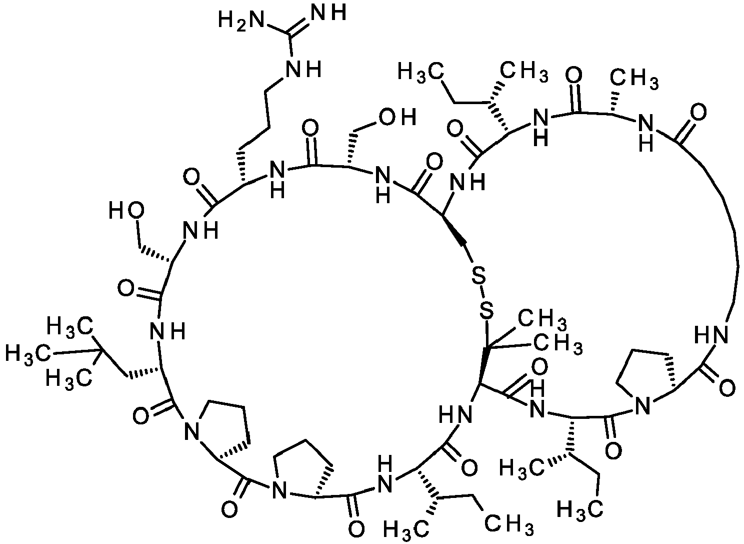

### Example 54

### Sequence: (Ahx)**-((N-Me)G)-IC+SRS-((tBu)A)-PPI-(Pen)+-IP**

N-{3-[(6S,9S,12S,15S,18R,21S,33aS,39S,42R,45S,47aS,52aS)-21,39,45-tri[(2S)-butan-2-yl]-6-(2,2-dimethylpropyl)-9,15-bis(hydroxymethyl)-25,53,53-trimethyl-5,8,11,14,17,20,23,26,33,38,41,44,47,52-tetradecaoxopentacontahydro-1H,5H-18,42-(methanodithiomethano)tripyrrolo[2,1-c:2',1'-o:2",1"-r][1,4,7,10,13,16,19,22, 25,28,31,34,37,40]tetradecaazacyclohexatetracontin-12-yl]propyl}guanidine

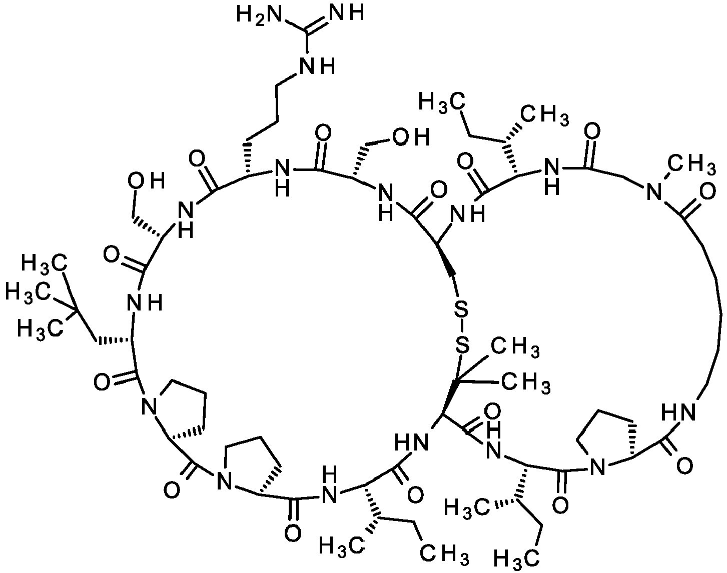

### Example 55

### Sequence: (Dap)**-IC+SRS-((tBu)A)-PPI-(Pen)+-IP**

N-{3-[(6S,9S,12S,15S,18R,21S,24S,26aS,32S,35R,38S,40aS,45aS)-24-(aminomethyl)-21,32,38-tri[(2S)-butan-2-yl]-6-(2,2-dimethylpropyl)-9,15-bis(hydroxymethyl)-46,46-dimethyl-5,8,11,14,17,20,23,26,31,34,37,40, 45-tirdecaoxotetratetracontahydro-1H-18,35-(methanodithiomethano)tripyrrolo[1,2-a:1',2'-d:1",2"-p][1,4,7,10, 13,16,19,22,25,28,31,34,37]tridecaazacyclononatriacontin-12-yl]propyl}guanidine

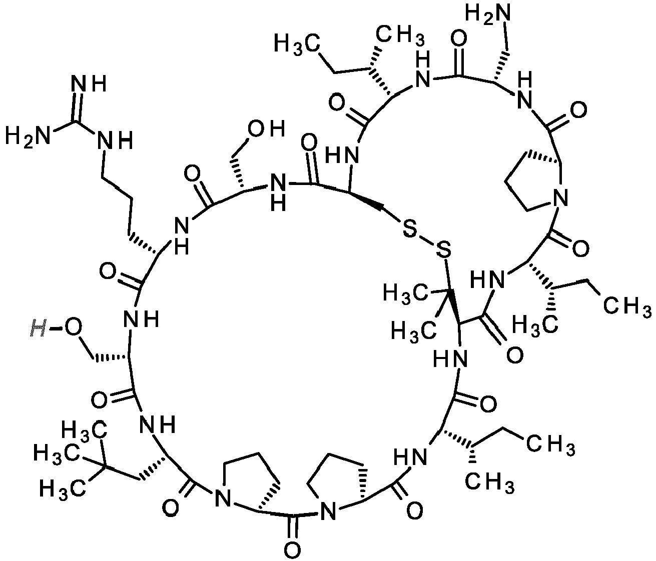

### Example 56

### Sequence: (Dab)**-IC+SRS-((tBu)A)-PPI-(Pen)+-IP**

N-{3-[(6S,9S,12S,15S,18R,21S,24S,26aS,32S,35R,38S,40aS,45aS)-24-(2-aminoethyl)-21,32,38-tri[(2S)-butan-2-yl]-6-(2,2-dimethylpropyl)-9,15-bis(hydroxymethyl)-46,46-dimethyl-5,8,11,14,17,20,23,26,31,34,37, 40,45-tridecaoxotetratetracontahydro-1H-18,35-(methanodithiomethano)tripyrrolo[1,2-a:1',2'-d:1"",2"-p]-[1,4,7,10,13,16,19,22,25,28,31,34,37]tridecaazacyclononatriacontin-12-yl]propyl}guanidine

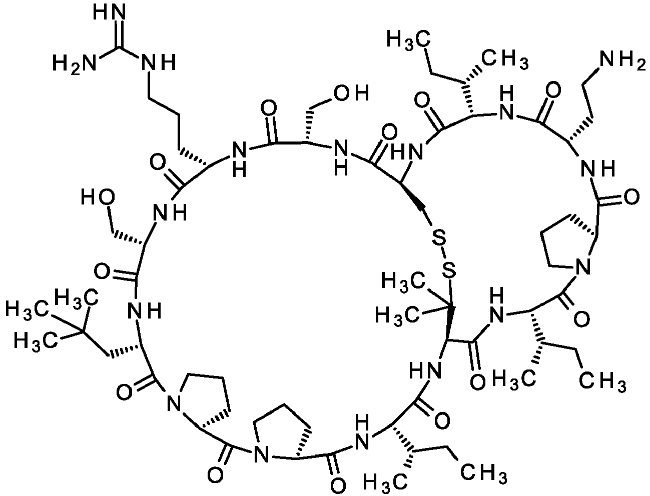

### Example 57

### Sequence:(Dap)**-(Dap)-IC+SRS-((tBu)A)-PPI-(Pen)+-I**

N-{3-[(5aS,11S,14S,17S,20S,23R26S,29S,32S,35S,38R41S,43aS)-29,32-bis(aminomethyl)-26,35,41-tri-[(2S)-butan-2-yl]-11-(2,2-dimethylpropyl)-14,20-bis(hydroxymethyl)-44,44-dimethyl-5,10,13,16,19,22,25, 28,31,34,37,40,43-tridecaoxodotetracontahydro-5H-23,38-(methanodithiomethano)dipyrrolo[1,2-a:1',',2'-d]-[1,4,7,10,13,16,19,22,25,28,31,34,37]tridecaazacyclononatriacontin-17-yl]propyl}guanidine

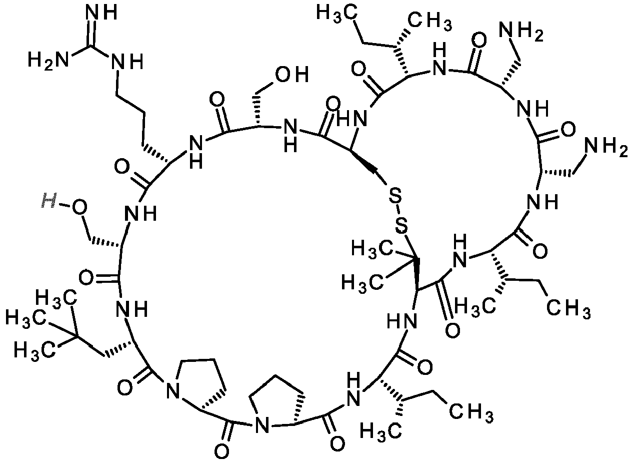

### Example 58

### Sequence: I**C+SRS-((tBu)A)-PPI-(Pen)+-IPP**

N-{3-[(6S,9S,12S,15S,18R,21S,23aS,28aS,34S,37R,40S,42aS,47aS)-21,34,40-tri[(2S)-butan-2-yl]-6-(2,2-dimethylpropyl)-9,15-bis(hydroxymethyl)-48,48-dimethyl-5,8,11,14,17,20,23,28,33,36,39,42,47-tridecaoxotetratetracontahydro-1H,5H,28H-18,37-(methanodithiomethano)tetrapyrrolo[1,2-a:1',2'-d:1",2"-p:1''',2'''-s][1,4,7,10,13,16,19,22,25,28,31,34,37]tridecaazacyclononatriacontin-12-yl]propyl}guanidine

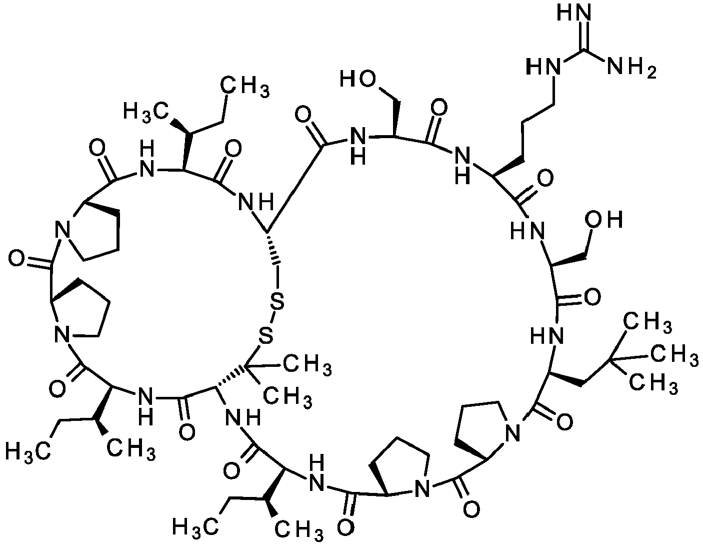

### Example 59

### Sequence: (Ahx)**-(TXA)-IC+SRS-((tBu)A)-PPI-(Pen)+-IP**

N-{3-[(1R4S,7S,13S,19S,22S,25S,28S,31R34S,37S,40S,51S,57S)-4,34,57-tri[(2S)-butan-2-yl]-19-(2,2-dimethylpropyl)-22,28-bis(hydroxymethyl)-60,60-dimethyl-3,6,12,18,21,24,27,30,33,36,43,50,56,59-tetradecaoxo-61,62-dithia-2,5,11,17,20,23,26,29,32,35,42,49,55,58-tetradecaazahexacyclo-[29.28.4.2^{37,40}.0^{7,11}.0^{13,17}.0^{51,55}]pentahexacontan-25-yl]propyl}guanidine

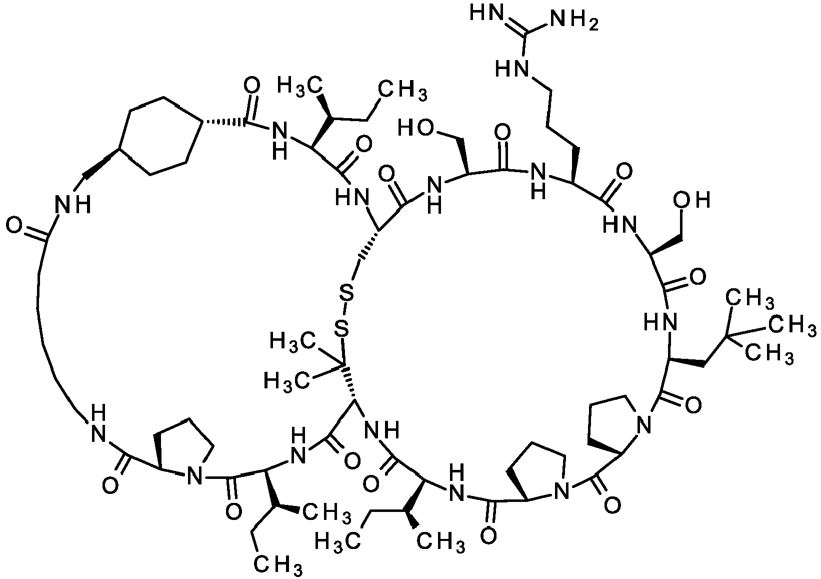

### Example 60

### Sequence: (Adipic acid)**-IC+SRS-((tBu)A)-PPI-(Pen)+-IPD-(Dap)++-NH₂

[(6S,9S,12S,15S,18R,21S,31S,34S,36aS,42S,45R,48S,50aS,55aS)-21,42,48-tri[(2S)-butan-2-yl]-12-(3-carbamimidamidopropyl)-3l-carbamoyl-6-(2,2-dimethylpropyl)-9,15-bis(hydroxymethyl)-56,56-dimethyl-5,8,11, 14,17,20,23,28,33,36,41,44,47,50,55-pentadecaoxotetrapentacontahydro-1H-18,45-(methanodithiomethano)-tripyrrolo[2,1-i:2',1'-u:2",1"-x][1,4,7,10,13,16,19,22,25,28,31,34,37,40,43]pentadecaazacyclononatetracontin-34-yl]acetic acid

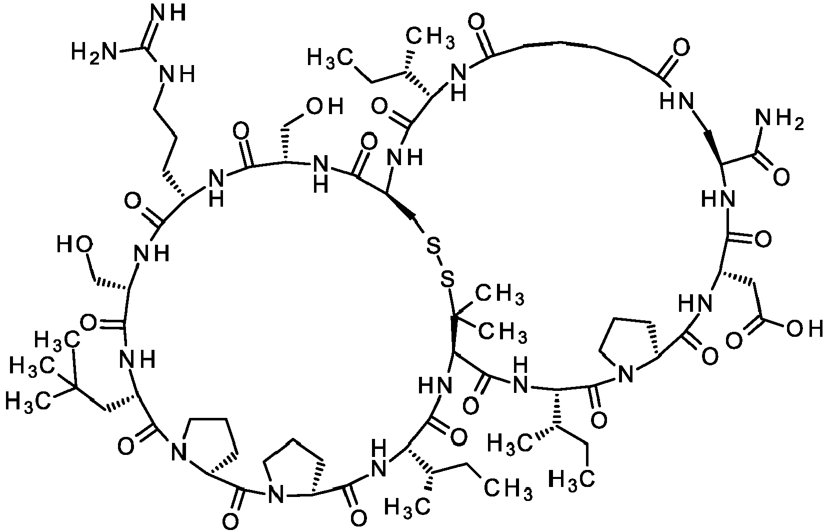

### Example 61

### Sequence: (Orn)++-AIC+SRS-((tBu)A)-PPI-(Pen)+-IPD++-NH₂

(6S,9S,12S,15S,18R,21S,24S,27S,34S,36aS,42S,45R,48S,50aS,55aS)-27-amino-21,42,48-tri[(2S)-butan-2-yl]-12-(3-carbamimidamidopropyl)-6-(2,2-dimethylpropyl)-9,15-bis(hydroxymethyl)-24,56,56-trimethyl-5,8,11,14,17,20,23,26,32,36,41,44,47,50,55-pentadecaoxotetrapentacontahydro-1H-18,45-(methanodithiomethano)tripyrrolo[1,2-v:1',2'-y:1",2"-k₁][1,4,7,10,13,16,19,22,25,28,31,34,37,40,44]pentadecaazacyclononatetracontine-34-carboxamide

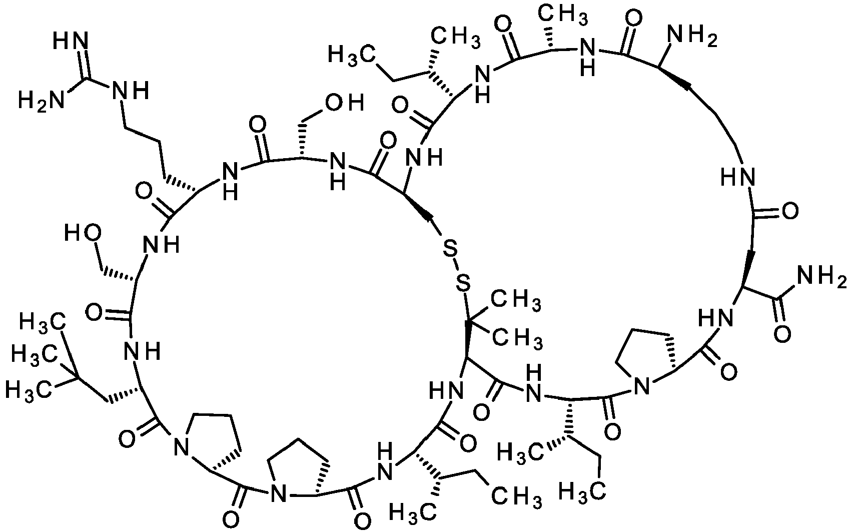

### Example 62

### Sequence: G**-(TXA)-GIC+SRS-((tBu)A)-PPI-(Pen)+-IPD++-NH₂

(1R,4S,10S,21S,24S,30S,33R,36S,39S,42S,45S,51S,57S,60S)-4,30,60 tri[(2S)-butan-2-yl]-39-(3-carbamimidamidopropyl)-45-(2,2-dimethylpropyl)-36,42-bis(hydroxymethyl)-63,63-dimethyl-2,5,11,15,18,25,28,31,34, 37,40,43,46,52,58,61-hexadecaoxo-64,65-dithia-3,6,12,16,19,26,29,32,35,38,41,44,47,53,59,62-hexadecaazahexacyclo[31.29.4.2^{21,21}.0^{6,10}.0^{47,51}.0^{51,57}]octahexacontane-13-carboxamide

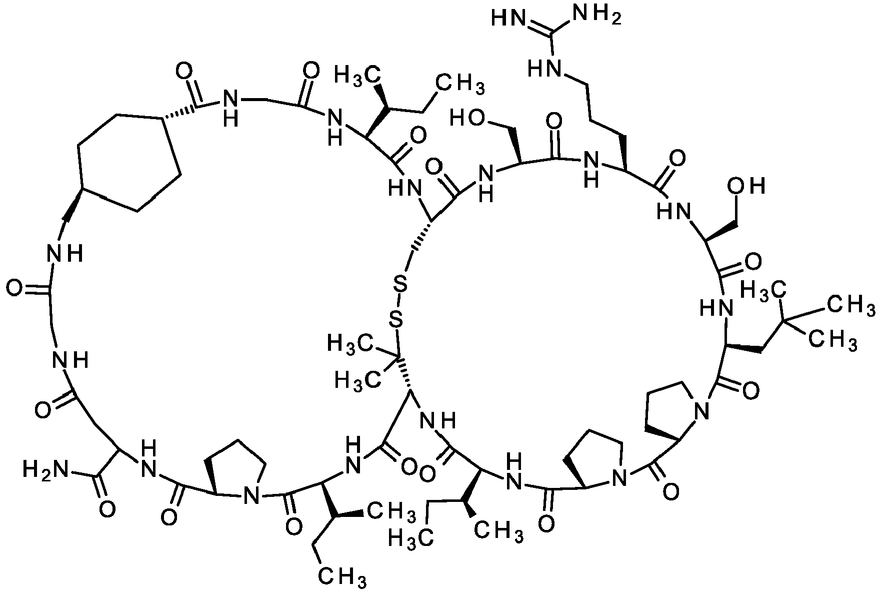

### Example 63

### Sequence: (TTDS)**-AIC+SRS-((tBu)A)-PPI-(Pen)+-IPD**

[(6S,9S,12S,15S,18R,21S,24S,46S,48aS,54S,57R,60S,62aS,67aS)-21,54,60-tri[(2S)-butan-2-yl]-12-(3-carbamimidamidopropyl)-6-(2,2-dimethylpropyl)-9,15-bis(hydroxymethyl)-24,68,68-trimethyl-5,8,11,14,17,20, 23,26,29,45,48,53,56,59,62,67-hexadecaoxodohexacontahydro-1H,5H-18,57-(methanodithiomethano)tripyrrolo[2,1-p:2',1'-b₁:2",1"-e₁][1,4,7,11,14,17,20,23,26,29,32,35,38,41,44,47,50,53,58]trioxahexadecaazacyclohenhexacontin-46-yl]acetic acid

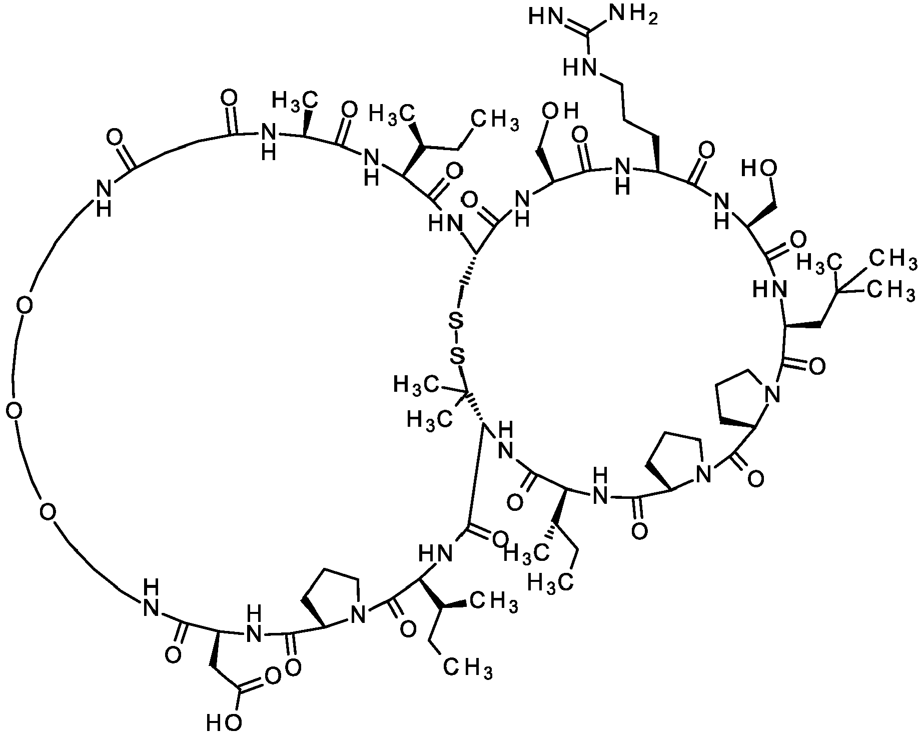

### Example 64

### Sequence: (Ahx)**-((N-Me)G)-IC+SRS-((tBu)A)-PPI-(Pen)+-I**

N-{3-[(5aS,11S,14S,17S,20S,23R,26S,39S,42R,45S,47aS)-26,39,45-tri[(2S)-butan-2-yl]-11-(2,2-dimethylpropyl)-14,20-bis(hydroxymethyl)-30,48,48-trimethyl-5,10,13,16,19,22,25,28,31,38,41,44,47-tridecaoxohexatetracontahydro-5H-23,42-(methanodithiomethano)dipyrrolo[2,1-1:2',1'-o][1,4,7,10,13,16,19,22,25,28,31,34, 37]tridecaazacyclotritetracontin-17-yl]propyl}guanidine

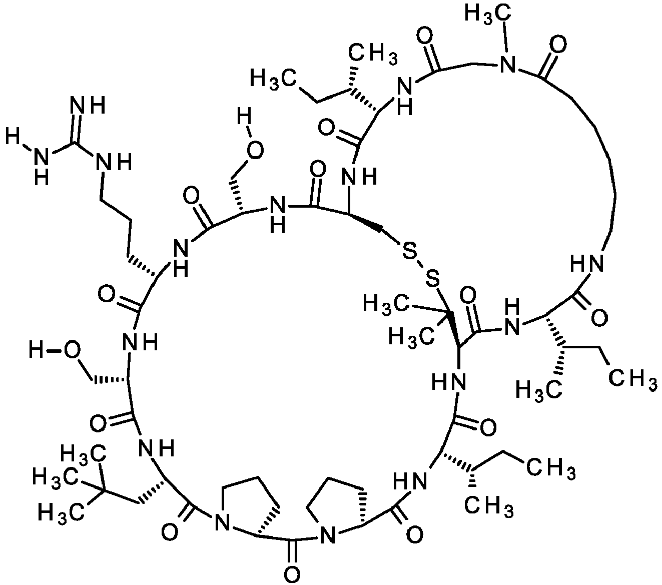

### Example 65

### Sequence: (Orn)**-GIC+SRS-((tBu)A)-PPI-(Pen)+-I**

N-{3-[(SaS,11S,14S,17S,20S,23R,26S,32S,35S,38R,41S,43aS)-32-(3-aminopropyl)-26,35,41tri[(2S)-butan-2-yl]-11-(2,2-dimethylpropyl)-14,20-bis(hydroxymethyl)-44,44-dimethyl-5,10,13,16,19,22,25,28,31,34,37,40, 43-tridecaoxodotetracontahydro-5H-23,38-(methanodithiomethano)dipyrrolo[1,2-a:1',2'-d][1,4,7,10,13,16,19, 22,25,28,31,34,37]tridecaazacyclononatriacontin-17-yl]propyl}guanidine

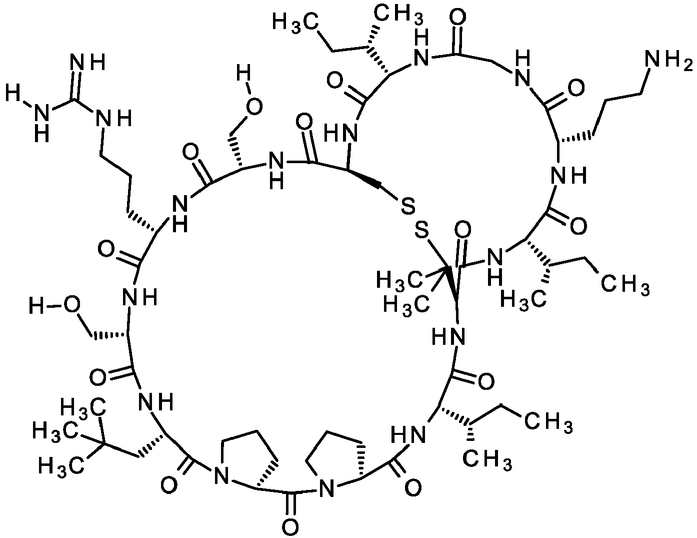

### Example 66

### Sequence: (Orn)++-IC+SRS-((tBu)A)-PPI-(Pen)+-I**

N-{3-[(5aS,11S,14S,17S,20S,23R,26S,29R,35S,38S,41S,43aS)-29-amino-26,35,41-tri[(2S)-butan-2-yl]-11-(2,2-dimethylpropyl)-14,20-bis(hydroxymethyl)-44,44-dimethyl-5,10,13,16,19,22,25,28,34,3 7,40,43-dodecaoxodotetracontahydro-5H-23,38-(methanodithiomethano)dipyrrolo[2,1-1:2',1'-o][1,4,7,10,13,16,19,22,25,28, 31,34]dodecaazacyclononatriacontin-17-yl]propyl}guanidine

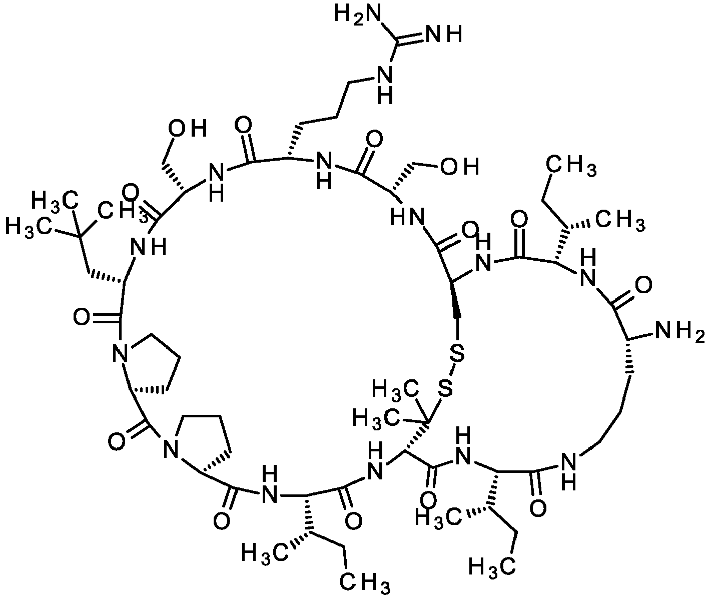

### Example 67

### Sequence: K++-IC+SRS-((tBu)A)-PPI-(Pen)+-I***

N-{3-[(5aS,11S,14S,17S,20S,23R,26S,29S,36S,39R,42S,44aS)-29-amino-26,36,42-tri[(2S)-butan-2-yl]-11-(2,2-dimethylpropyl)-14,20-bis(hydroxymethyl)-45,45-dimethyl-5,10,13,16,19,22,25,28,35,38,41,44-dodecaoxodotetracontahydro-1H,5H-23,39-(methanodithiomethano)dipyrrolo[2,1-1:2',1'-o][1,4,7,10,13,16,19,22,25, 28,31,34]dodecaazacyclotetracontin-17-yl]propyl}guanidine

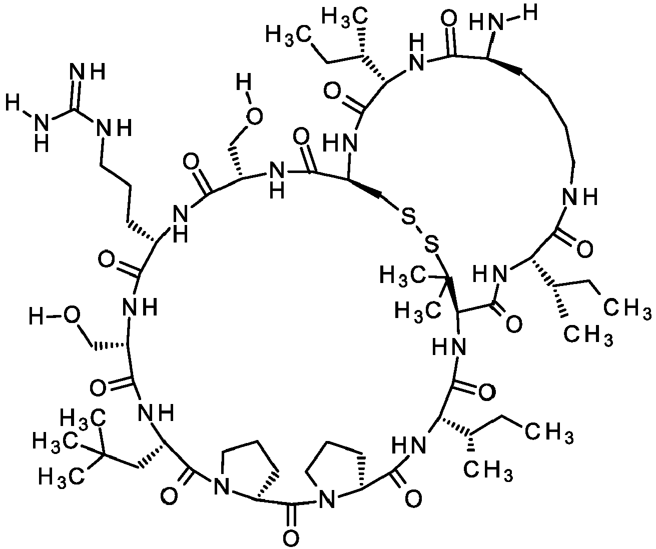

### Example 68

### Sequence: (Ahx)**-(4-(Aminomethyl)benzoic acid)-IC+SRS-((tBu)A)-PPI-(Pen)+-IP**

N-13-[(1R,4S,7S,13S,19S,22S,25S,28S,31R,34S,51S,57S)-4,34,57-tri[(2S)-butan-2-yl]-19-(2,2-dimethylpropyl)-22,28-bis(hydroxymethyl)-60,60-dimethyl-3,6,12,18,21,24,27,30,33,36,43,50,56,59-tetradecaoxo-61,62-dithia-2,5,11,17,20,23,26,29,32,35,42,49,55,58-tetradecaazahexacyclo[29.28.4.2^{37,40}.0^{7,11}.0^{13,17}.0^{51,55}]pentahexaconta-37,39,64-trien-25-yl]propyl}guanidine

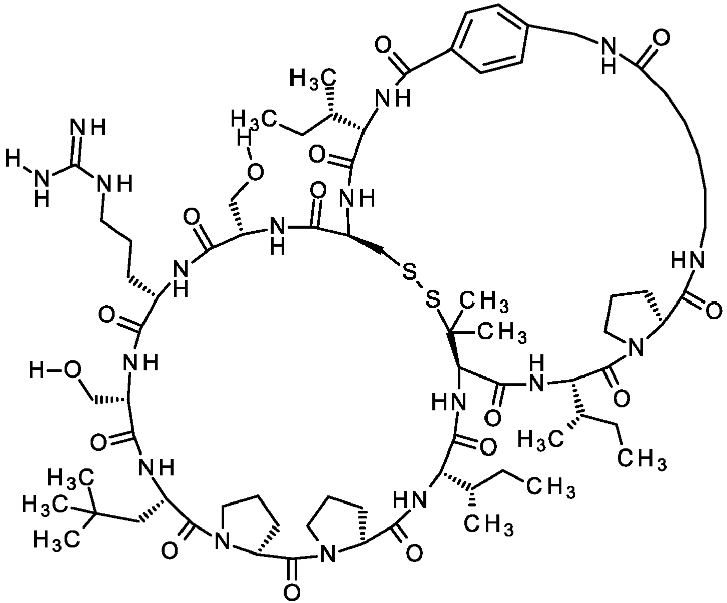

### Example 69

### Sequence: G**-(TXA)-GIC+SRS-((tBu)A)-PPI-((N-Me)C)+-IPD++-NH₂

(1R,4S,10S,13S,21S,24S,30S,33R,36S,39S,42S,45S,51S,57S,60S)-4,30,60tri[(2S)-butan-2-yl]-39-(3-carbamimidamidopropyl)-45-(2,2-dimethylpropyl)-36,42-bis(hydroxymethyl)-62-methyl-2,5,11,15,18,25,28,31, 34,37,40,43,46,52,58,61-hexadecaoxo-64,65-dithia-3,6,12,16,19,26,29,32,35,38,41,44,47,53,59,62-hexadecaazahexacyclo[31.29.4.2^{21,24}.0^{6,10}.0^{47,51}.0^{51,57}]octahexacontane-13-carboxamide

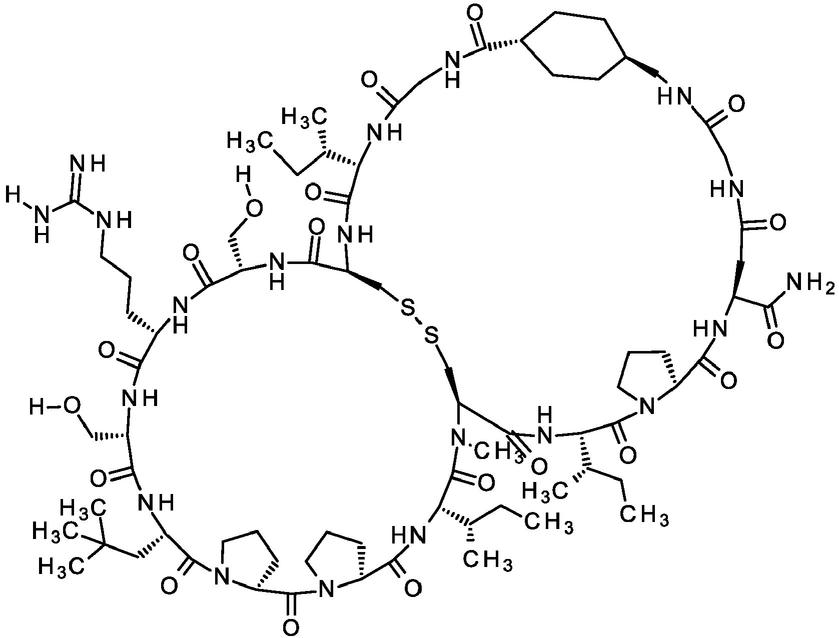

### Example 71

### Sequence: (Orn)++-IC+SRS-((tBu)A)-PPI-(Pen)+-IP**

N-{3-[(6S,9S,12S,15S,18R,21S,24S,29aS,35S,38R,41S,43aS,48aS)-24-amino-21,35,41-tri[(2S)-butan-2-yl]-6-(2,2-dimethylpropyl)-9,15-bis(hydroxymethyl)-49,49-dimethyl-5,8,11,14,17,20,23,29,34,37,40,43,48-tridecaoxohexatetracontahydro-1H,5H-18,38-(methanodithiomethano)tripyrrolo[2,1-c:2',1'-o:2",1"-r][1,4,7,10,13, 16,19,22,25,28,31,34,37]tridecaazacyclodotetracontin-12-yl]propyl}guanidine

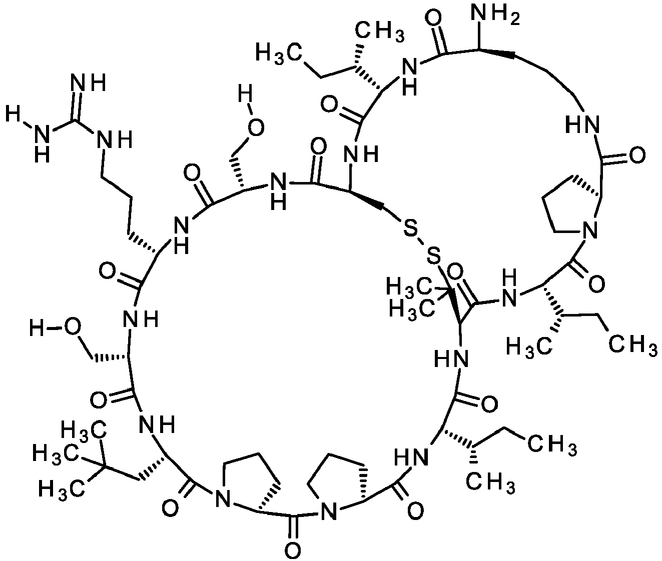

### Example 72

### Sequence: (Ahx)**-AIC+SRS-((tBu)A)-PPI-(Pen)+-I**

N-{3-[(SaS,11S,14S,17S,20S,23R,26S,29S,39S,42R,45S,47aS)-26,39,45-tri[(2S)-butan-2-yl]-11-(2,2-dimethylpropyl)-14,20-bis(hydroxymethyl)-29,48,48-trimethyl-5,10,13,16,19,22,25,28,31,38,41,44,47-tridecaoxohexatetracontahydro-5H-23,42-(methanodithiomethano)dipyrrolo[2,1-1:2',1'-o][1,4,7,10,13,16,19,22, 25,28,31,34,37]tridecaazacyclotritetracontin-17-yl]propyl}guanidine

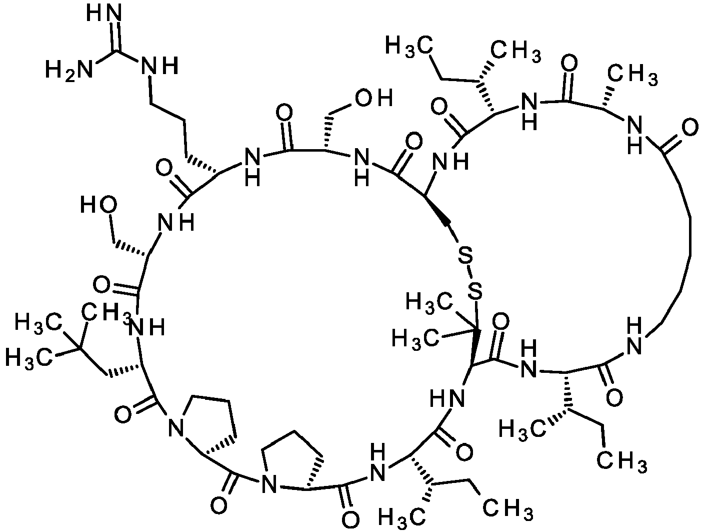

### Example 73

### Sequence: (Dap)**-(Dab)-IC+SRS-((tBu)A)-PPI-(Pen)+-I**

N-{3-[(5aS,11S,14S,17S,20S,23R,26S,29S,32S,35S,38R,41S,43aS)-29-(2-aminoethyl)-32-(aminomethyl)-26, 35,41-tri[(2S)-butan-2-yl]-11-(2,2-dimethylpropyl)-14,20-bis(hydroxymethyl)-44,44-dimethyl-5,10,13,16,19, 22,25,28,31,34,37,40,43-tridecaoxodotetracontahydro-5H-23,38-(methanodithiomethano)dipyrrolo[1,2-a:1',2'-d][1,4,7,10,13,16,19,22,25,28,31,34,37]tridecaazacyclononatriacontin-17-yl]propyl}guanidine

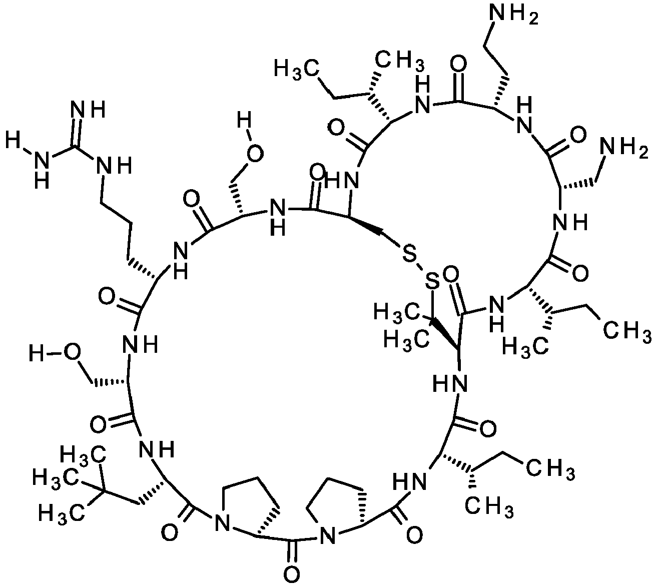

### Example 74

### Sequence: (Ahx)**-AIC+SRSLP-(Oic)-I-(Pen)+-IPE++-NH₂

(3aS,6S,18S,21S,24R,27S,30S,33S,36S,41aS,43aS,47aS,48aS,51S,54R,57S)-21,51,57-tri[(2S)-butan-2-yl]-30-(3-carbamimidamidopropyl)-27,33-bis(hydroxymethyl)-18,60,60-trimethyl-36-(2-methylpropyl)-4,9,16,19,22, 25,28,31,34,37,42,49,52,55,58-pentadecaoxohexacontahydro-39H-24,54-(methanodithiomethano)dipyrrolo-[1',2':22,23;1",2":37,38][1,4,7,10,13,16,19,22,25,28,31,34,37,40,45]pentadecaazacyclohenpentacontino[25,26-a]indole-6-carboxamide

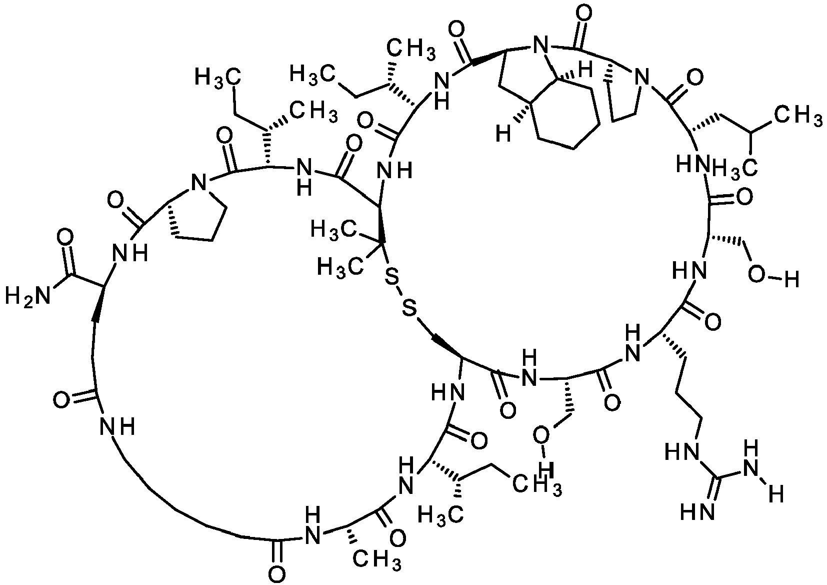

### Example 75

### Sequence: (Ahx)**-IC+SRSLP-(Oic)-I-(Pen)+-IPE++-NH₂

(3aS,6S,18S,21R,24S,27S,30S,33S,38aS,40aS,44aS,45aS,48S,51R,54S)-18,48,54-tri[(2S)-butan-2-yl]-27-(3-carbamimidamidopropyl)-24,30-bis(hydroxymethyl)-57,57-dimethyl-33-(2-methylpropyl)-4,9,16,19,22, 25,28,31,34,39,46,49,52,55-tetradecaoxohexapentacontahydro-1H,36H-21,51-(methanodithiomethano)dipyrrolo[1',2':19,20;1",2":34,35][1,4,7,10,13,16,19,22,25,28,31,34,37,42]tetradecaazacyclooctatetracontino-[22,23-a]indole-6-carboxamide

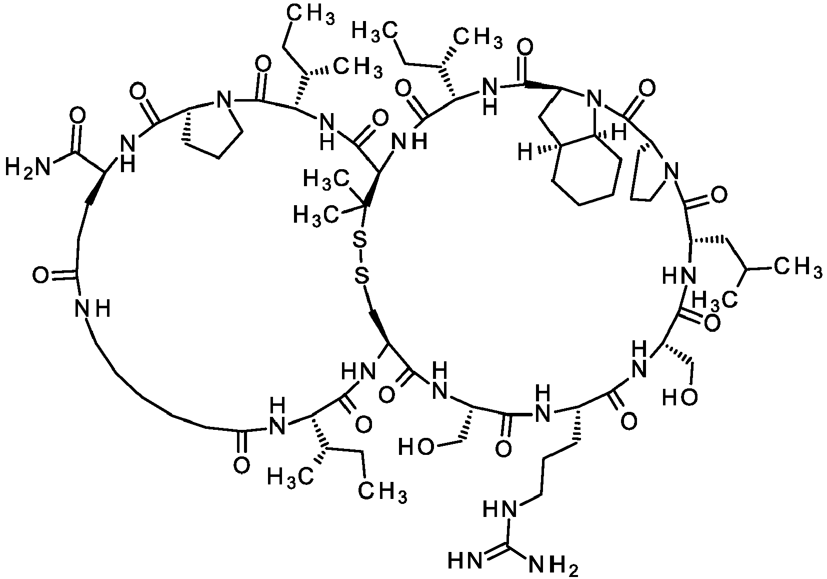

### Example 76

### Sequence: (Ahx)**-((N-Me)G)-GIC+SRSLP-(Oic)-I-(Pen)+-IPE++-NH₂

(3aS,6S,24S,27R,30S,33S,36S,39S,44aS,46aS,50aS,51aS,54S,57R,60S)-24,54,60-tri[(2S)-butan-2-yl]-33-(3-carbamimidamidopropyl)-30,36-bis(hydroxymethyl)-20,63,63-trimethyl-39-(2-methylpropyl)-4,9,16,19,22,25, 28,31,34,37,40,45,52,55,58,61-hexadecaoxodohexacontahydro-1H,42H-27,57-(methanodithiomethano)dipyrrolo[1',2':25,26;1",2":40,41][1,4,7,10,13,16,19,22,25,28,31,34,37,40,43,48]hexadecaazacyclotetrapentacontino[28,29-a]indole-6-carboxamide

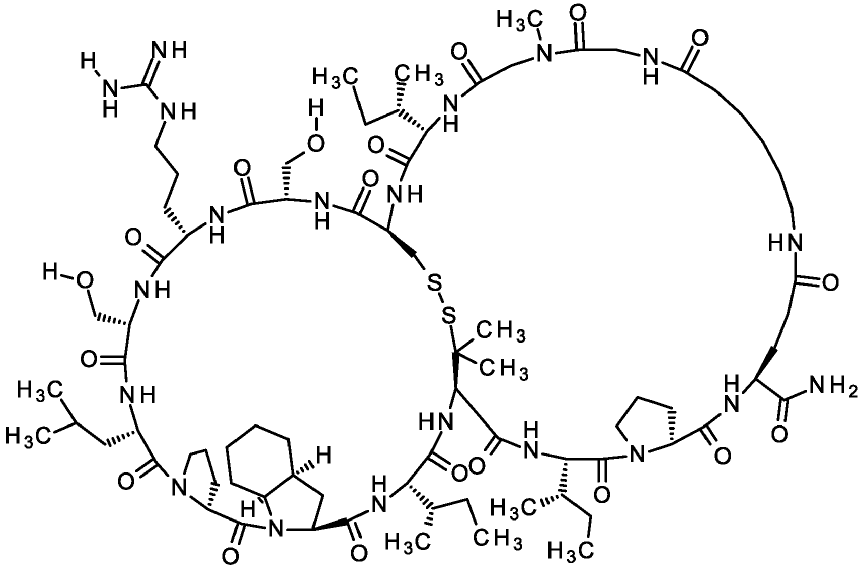

### Example 77

### Sequence: (Ahx)**-((N-Me)G)-AIC+SRSLP-(Oic)-I-(Pen)+-IPE++-NH₂

(3aS,6S,21S,24S,27R,30S,33S,36S,39S,44aS,46aS,50aS,51aS,54S,57R,60S)-24,54,60-tri[(2S)-butan-2-yl]-33-(3-carbamimidamidopropyl)-30,36-bis(hydroxymethyl)-20,21,63,63-tetramethyl-39-(2-methylpropyl)-4,9,16, 19,22,25,28,31,34,37,40,45,52,55,58,61-hexadecaoxodohexacontahydro-1H,42H-27,57-(methanodithiomethano)dipyrrolo[1',2':25,26;1",2":40,41][1,4,7,10,13,16,19,22,25,28,31,34,37,40,43,48]hexadecaazacyclotetrapentacontino[28,29-a]indole-6-carboxamide

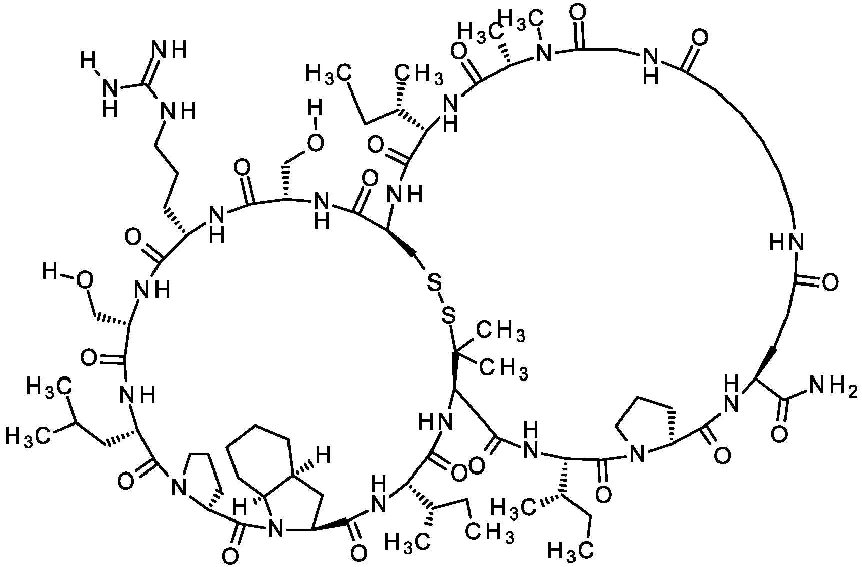

### Example 78

### Sequence: k++-IC+SRS-((tBu)A)-PPI-(Pen)+-I**(HCl Salt)

N-{3-[(5aS,11S,14S,17S,20S,23R,26S,29R,36S,39R,42S,44aS)-29-amino-26,36,42-tri[(2S)-butan-2-yl]-11-(2,2-dimethylpropyl)-14,20-bis(hydroxymethyl)-45,45-dimethyl-5,10,13,16,19,22,25,28,35,38,41,44-dodecaoxodotetracontahydro-1H,5H-23,39-(methanodithiomethano)dipyrrolo[2,1-1:2',1'-o][1,4,7,10,13,16,19,22,25, 28,31,34]dodecaazacyclotetracontin-17-yl]propyl}guanidinehydrogen chloride

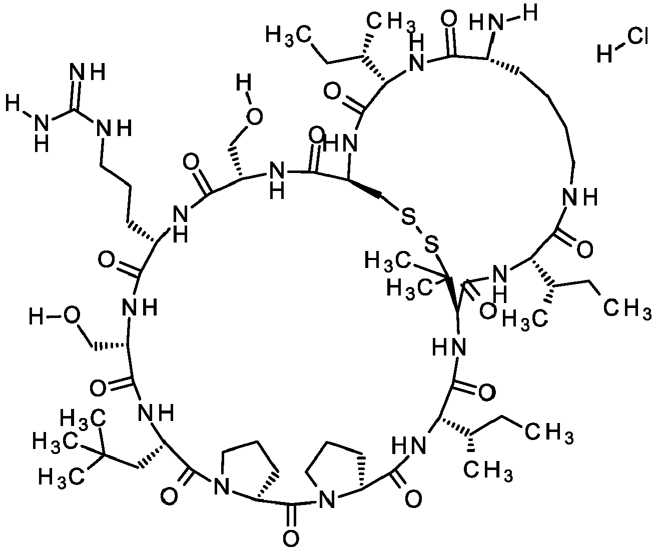

### Example 79

### Sequence: (Ahx)**-aIC+SRSLP-(Oic)-I-(Pen)+-IPE++-NH₂(HCl Salt)

(3aS,6S,18R,21S,24R,27S,30S,33S,36S,41aS,43aS,47aS,48aS,51S,54R,57S)-21,51,57-tri[(2S)-butan-2-yl]-30-(3-carbamimidamidopropyl)-27,33-bis(hydroxymethyl)-18,60,60-trimethyl-36-(2-methylpropyl)-4,9,16, 19,22,25,28,31,34,37,42,49,52,55,58-pentadecaoxohexacontahydro-39H-24,54-(methanodithiomethano)dipyrrolo[1',2':22,23;1",2":37,38][1,4,7,10,13,16,19,22,25,28,31,34,37,40,45]pentadecaazacyclohenpentacontino[25,26-a]indole-6-carboxamide hydrogen chloride

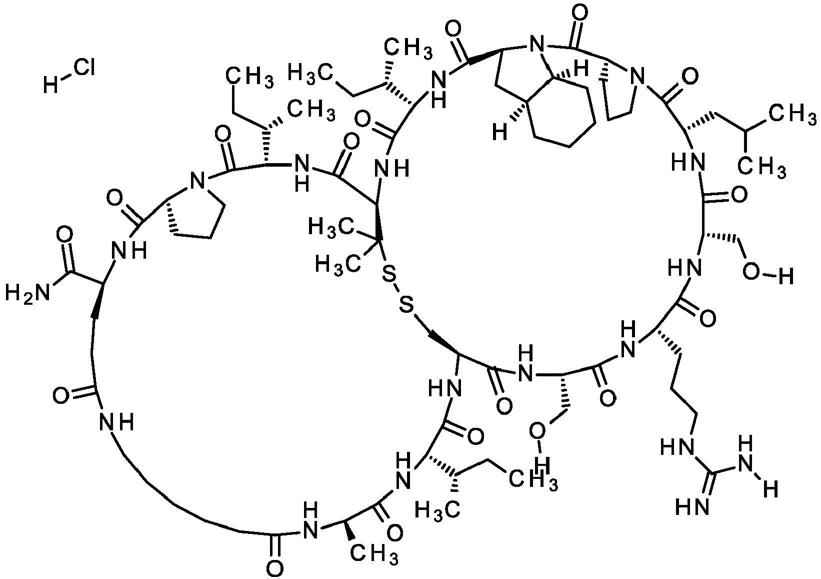

### Example 80

### Sequence: k++-IC+SRS-((tBu)A)-PPI-(Pen)+-I**

N-{3-[(5aS,11S,14S,17S,20S,23R,26S,29R,36S,39R,42S,44aS)-29-amino-26,36,42-tri[(2S)-butan-2-yl]-11-(2,2-dimethylpropyl)-14,20-bis(hydroxymethyl)-45,45-dimethyl-5,10,13,16,19,22,25,28,35,38,41,44-dodecaoxodotetracontahydro-1H,5H-23,39-(methanodithiomethano)dipyrrolo[2,1-1:2',1'-o][1,4,7,10,13,16,19,22,25, 28,31,34]dodecaazacyclotetracontin-17-yl]propyl}guanidine

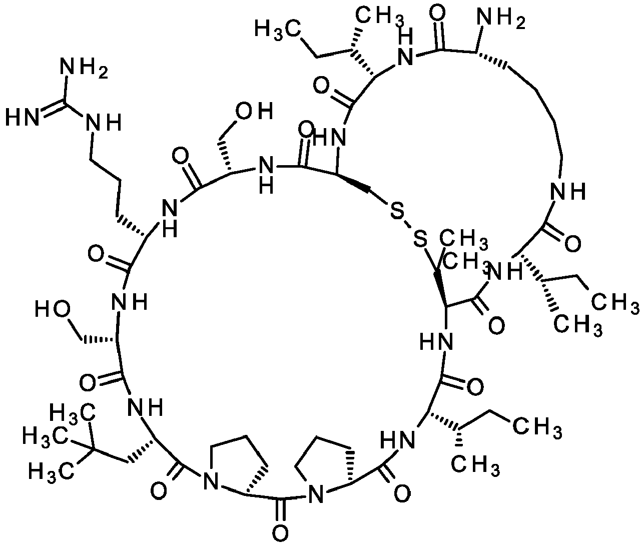

### Example 81

### Sequence: (3-Azido-L-Alanine)++-GAIC+SRS-((tBu)A)-PPIC+IP-(L-Propargylglycine)++-NH₂ (1,2,3-triazole-1,4-diyl)

(1R,4S,10S,13S,20S,26S,29R,32R,35S,38S,41S,44S,50S,56S,59S)-20-amino-4,29,59-tri[(2S)-butan-2-yl]-38-(3-carbamimidamidopropyl)-44-(2,2-dimethylpropyl)-35,41-bis(hydroxymethyl)-26-methyl-2,5,11,21, 24,27,30,33,36,39,42,45,51,57,60-pentadecaoxo-63,64-dithia-3,6,12,16,17,18,22,25,28,31,34,37,40,43,46, 52,58,61-octadecaazahexacyclo[30.29.4.1^{15,18}.0^{6,10}.0^{46,50}.0^{52,56}]hexahexaconta-15(66),16-diene-13-carboxamide

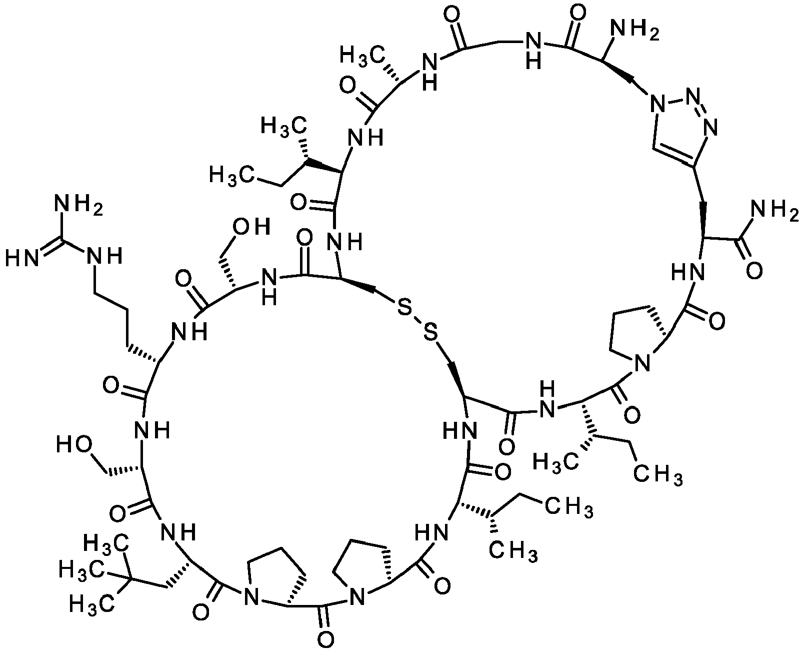

### Example 82

### Sequence: (Ahx)**-GAIC+SRSLP-(Oic)-I-(Pen)+-IPE++-NH₂

(3aS,6S,21S,24S,27R,30S,33S,36S,39S,44aS,46aS,50aS,51aS,54S,57R,60S)-24,54,60-tri[(2S)-butan-2-yl]-33-(3-carbamimidamidopropyl)-30,36-bis(hydroxymethyl)-21,63,63-trimethyl-39-(2-methylpropyl)-4,9,16,19,22, 25,28,31,34,37,40,45,52,55,58,61-hexadecaoxodohexacontahydro-1H,42H-27,57-(methanodithiomethano)dipyrrolo[1',2':25,26;1",2":40,41][1,4,7,10,13,16,19,22,25,28,31,34,37,40,43,48]hexadecaazacyclotetrapentacontino[28,29-a]indole-6-carboxamide

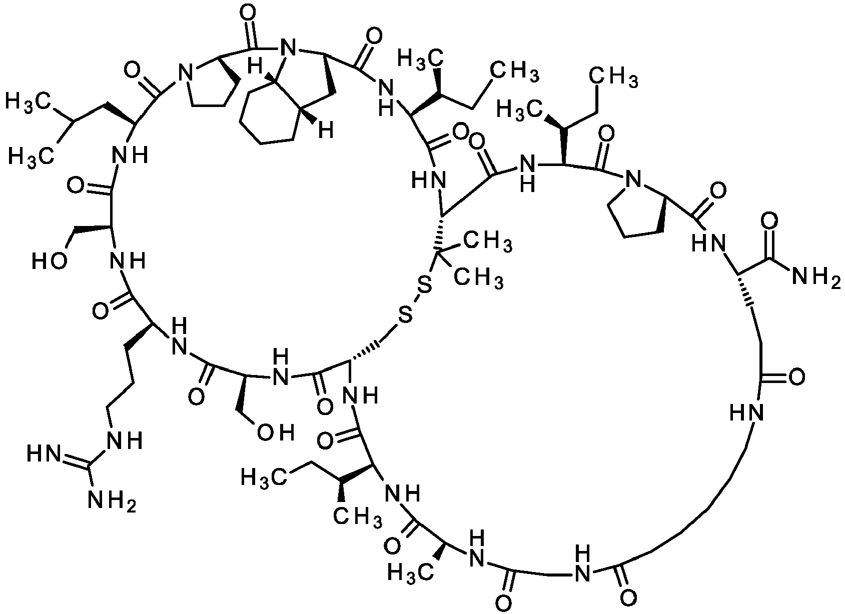

### Example 83

### Sequence: (Ahx)**-GIC+SRSLP-(Oic)-I-(Pen)+-IPE++-NH₂

(3aS,6S,21S,24R,27S,30S,33S,36S,41aS,43aS,47aS,48aS,51S,54R,57S)-21,51,57-tri[(2S)-butan-2-yl]-30-(3-carbamimidamidopropyl)-27,33-bis(hydroxymethyl)-60,60-dimethyl-36-(2-methylpropyl)-4,9,16,19,22, 25,28,31,34,37,42,49,52,55,58-pentadecaoxohexacontahydro-39H-24,54-(methanodithiomethano)dipyrrolo[1',2':22,23;1",2":37,38][1,4,7,10,13,16,19,22,25,28,31,34,37,40,45]pentadecaazacyclohenpentacontino[25,26-a]indole-6-carboxamide

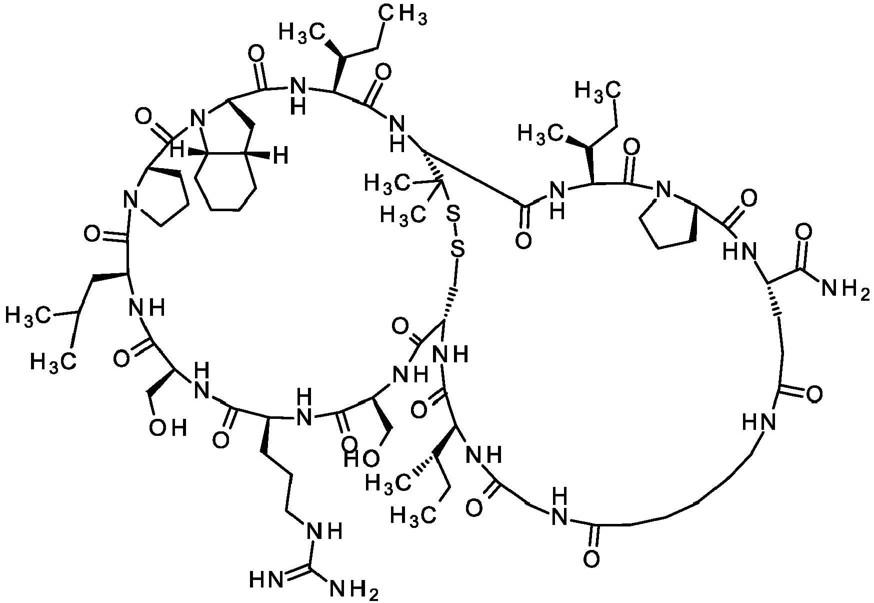

### Example 87

### Sequence: (Ahx)**-GaIC+SRSLP-(Oic)-I-(Pen)+-IPE++-NH₂

(3aS,6S,21R,24S,27R,30S,33S,36S,39S,44aS,46aS,50aS,51aS,54S,57R,60S)-24,54,60-tri[(2S)-butan-2-yl]-33-(3-carbamimidamidopropyl)-30,36-bis(hydroxymethyl)-21,63,63-trimethyl-39-(2-methylpropyl)-4,9,16,19, 22,25,28,31,34,37,40,45,52,55,58,61-hexadecaoxodohexacontahydro-lH,42H-27,57-(methanodithiomethano)dipyrrolo[1',2':25,26;1",2":40,41][1,4,7,10,13,16,19,22,25,28,31,34,37,40,43,48]hexadecaazacyclotetrapentacontino[28,29-a]indole-6-carboxamide

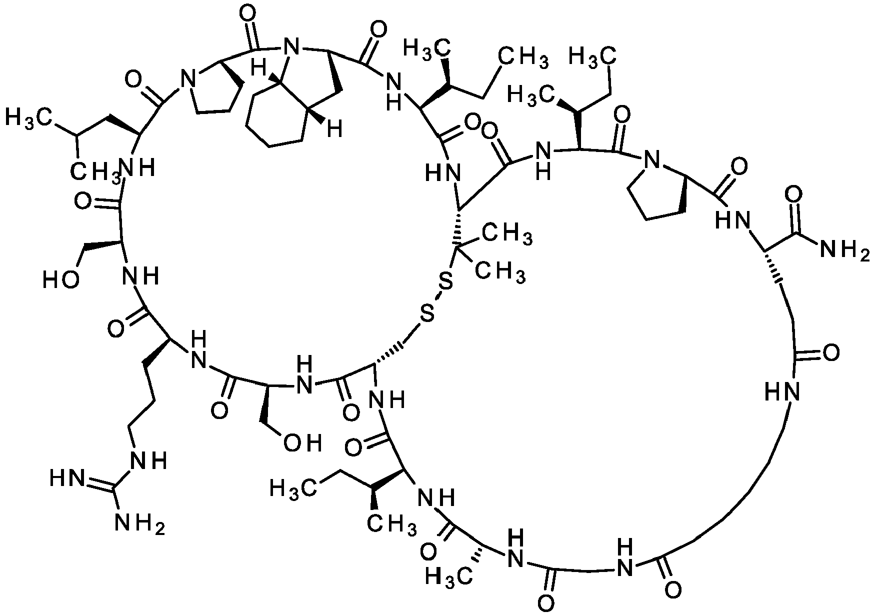

### Example 85

### Sequence: (Dap)++-IC+SRSLP-(Oic)-I-(Pen)+-IP**

N-{3-[(3aS,7S,10S,13R,16S,19S,22S,25S,30aS,32aS,36aS,37aS,40S,43R,46S)-7-amino-10,40,46tri[(2S)-butan-2-yl]-16,22-bis(hydroxymethyl)-49,49-dimethyl-25-(2-methylpropyl)-4,8,11,14,17,20,23,26,31,38,41,44, 47-tridecaoxooctatetracontahydro-1H,28H-13,43-(methanodithiomethano)dipyrrolo[2',1':18,19;2",1":3,4]-[1,4,7,10,13,16,19,22,25,28,31,34,37]tridecaazacyclotetracontino[16,15-a]indol-19-yl]propyl}guanidine

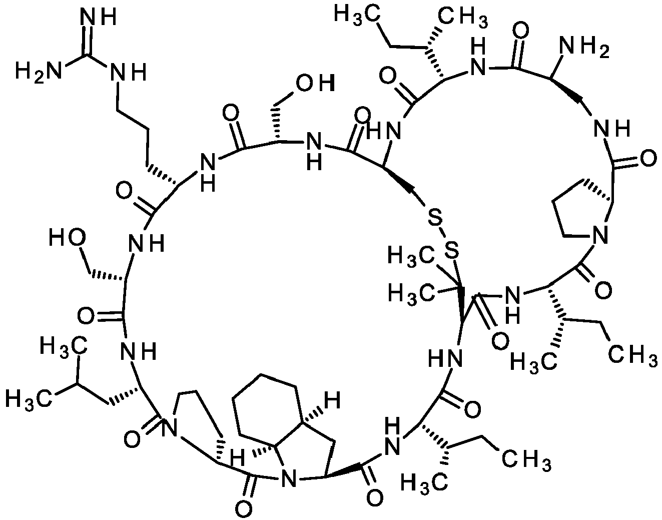

### Example 86

### Sequence: (Dap)++-(Dap)-IC+SRSLP-(Oic)-I-(Pen)+-IP***

N-{3-[(3aS,7S,10S,13S,16R,19S,22S,25S,28S,33aS,35aS,39aS,40aS,43S,46R,49S)-7-amino-10-(aminomethyl)-13,43,49-tri[(2S)-butan-2-yl]-19,25-bis(hydroxymethyl)-52,52-dimethyl-28-(2-methylpropyl)-4,8,11,14, 17,20,23,26,29,34,41,44,47,50-tetradecaoxodopentacontahydro-31H-16,46-(methanodithiomethano)dipyrrolo-[2',1':18,19;2",1":3,4][1,4,7,10,13,16,19,22,25,28,31,34,37,40]tetradecaazacyclotritetracontino[16,15-a]indol-22-yl]propyl}guanidine

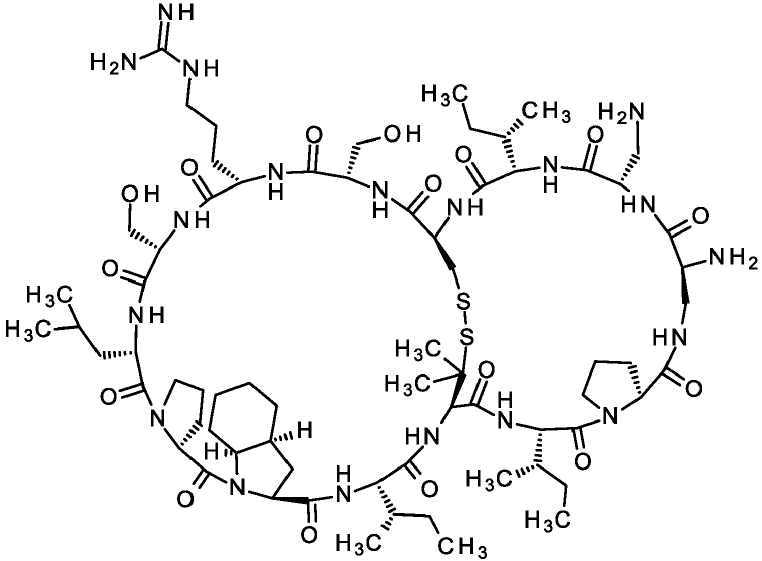

### Example 87

### Sequence: (Dap)**-IC+SRSLP-(Oic)-I-(Pen)+-IP**

N-{3-[(3aS,6S,9S,12R,15S,18S,21S,24S,29aR,31aS,35aS,36aS,39S,42R,45S)-6-(aminomethyl)-9,39,45-tri-[(2S)-butan-2-yl]-15,21-bis(hydroxymethyl)-48,48-dimethyl-24-(2-methylpropyl)-4,7,10,13,16,19,22,25,30, 37,40,43,46-tridecaoxooctatetracontahydro-27H-12,42-(methanodithiomethano)dipyrrolo[1',2': 13,14; 1",2":37,38][1,4,7,10,13,16,19,22,25,28,31,34,37]tridecaazacyclononatriacontino[1,2-a]indol-18-yl]propyl}guanidine

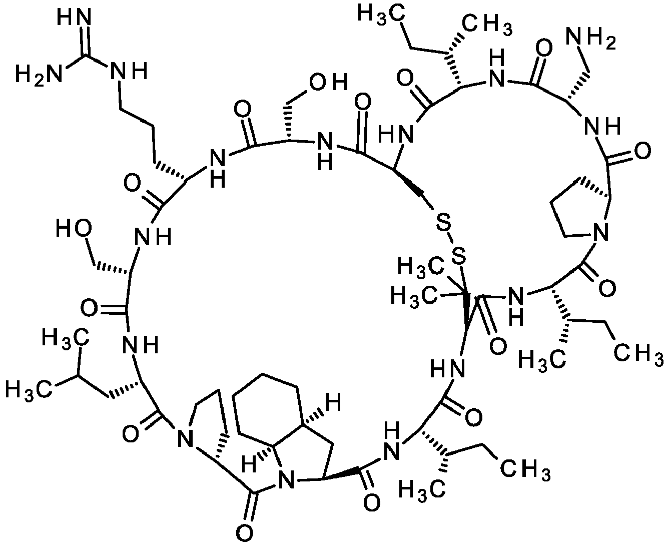

### Example 88

### Sequence: I**C+SRS-((tBu)A)-PPI-(Pen)+-IP**

N-13-[(6S,9S,12S,15S,18R,21S,23aS,29S,32R,35S,37aS,42aS)-21,29,35-tri[(2S)-butan-2-yl]-6-(2,2-dimethylpropyl)-9,15-bis(hydroxymethyl)-43,43-dimethyl-5,8,11,14,17,20,23,28,31,34,37,42-dodecaoxotetracontahydro-1H,5H-18,32-(methanodithiomethano)tripyrrolo[1,2-a:1',2'-d:1",2"-p][1,4,7,10,13,16,19,22,25,28,31,34]-dodecaazacyclohexatriacontin-12-yl]propyl}guanidine hydrogen chloride

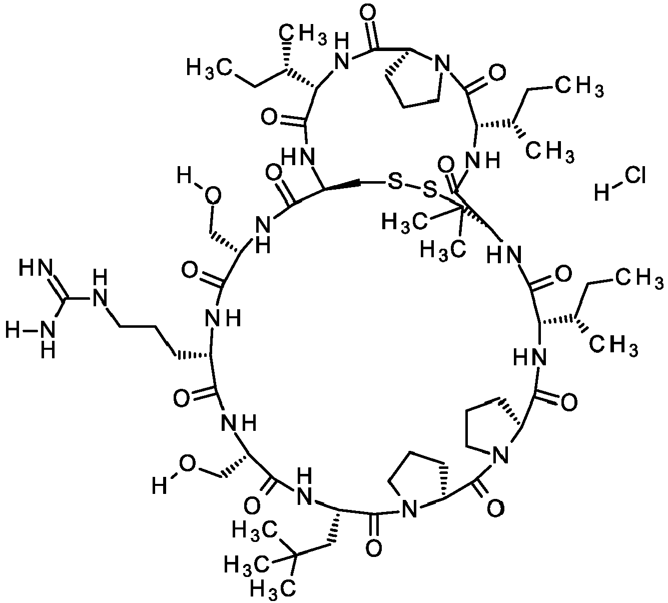

### Example 89

### Sequence: (Dap)**-(Dap)-IC+SRSLP-(Oic)-I-(Pen)+-IP**

N-{3-[(3aS,6S,9S,12S,15R,18S,21S,24S,27S,32aR,34aS,38aS,39aS,42S,45R,48S)-6,9-bis(aminomethyl)-12, 42,48-tri[(2S)-butan-2-yl]-18,24-bis(hydroxymethyl)-51,51-dimethyl-27-(2-methylpropyl)-4,7,10,13,16,19, 22,25,28,33,40,43,46,49-tetradecaoxopentacontahydro-1H,30H-15,45-(methanodithiomethano)dipyrrolo-[1',2':13,14;1",2":40,41][1,4,7,10,13,16,19,22,25,28,31,34,37,40]tetradecaazacyclodotetracontino[1,2-a]indol-21-yl]propyl}guanidine

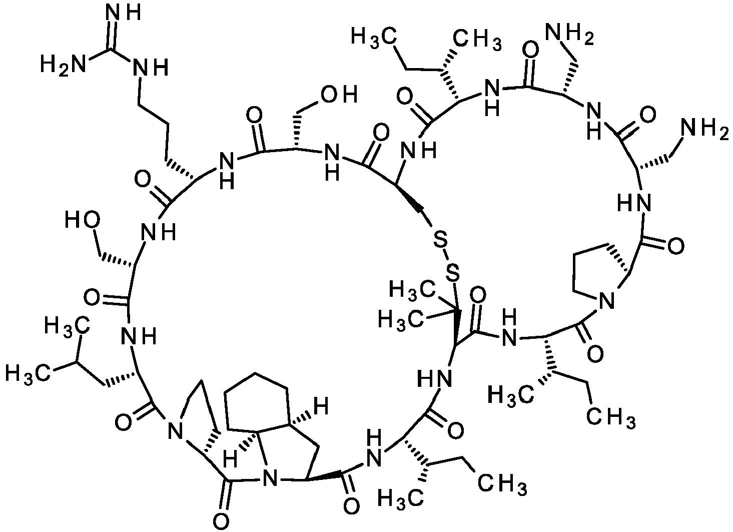

### Example 90

### Sequence: E++GIC+SRSLP-(Oic)-I-(Pen)+-IPK++-NH₂

(3aS,6S,15S,21S,24R,27S,30S,33S,36S,43aS,47aS,48aS,51S,54R,57S)-15-amino-21,51,57-tri[(2S)-butan-2-yl]-30-(3-carbamimidamidopropyl)-27,33-bis(hydroxymethyl)-60,60-dimethyl-36-(2-methylpropyl)-4,12, 16,19,22,25,28,31,34,37,42,49,52,55,58-pentadecaoxohexacontahydro-39H-24,54-(methanodithiomethano)-dipyrrolo[2',1':18,19;2",1":3,4][1,4,7,10,13,16,19,22,25,28,31,34,37,40,46]pentadecaazacyclohenpentacontino[16,15-a]indole-6-carboxamide

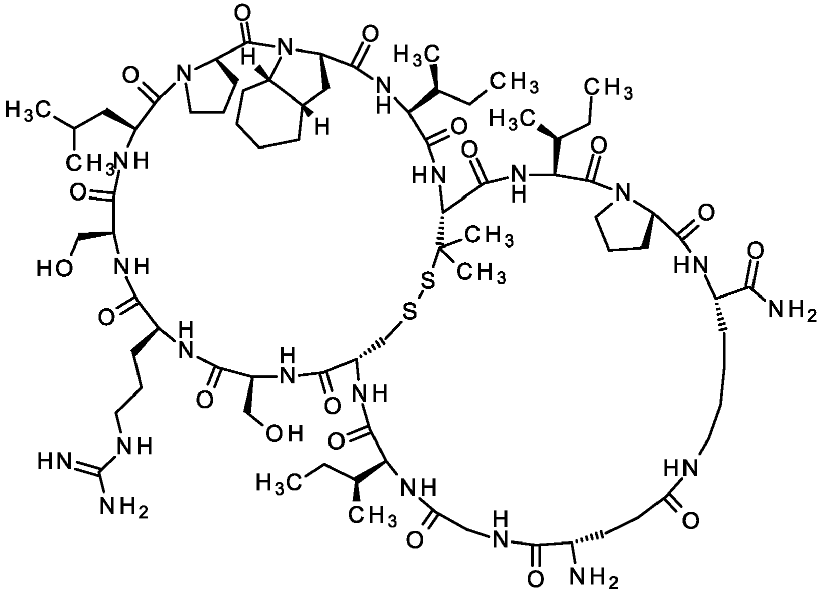

### Example 91

### Sequence: E++GIC+SRSLP-(Oic)-I-(Pen)+-IPDK+-NH₂

[(3aS,6S,9S,18S,24S,27R,30S,33S,36S,39S,44aR,46aS,50aS,51aS,54S,57R,60S)-18-amino-24,54,60-tri[(2S)-butan-2-yl]-33-(3-carbamimidamidopropyl)-9-carbamoyl-30,36-bis(hydroxymethyl)-63,63-dimethyl-39-(2-methylpropyl)-4,7,15,19,22,25,28,31,34,37,40,45,52,55,58,61-hexadecaoxodohexacontahydro-1H,42H-27,57-(methanodithiomethano)dipyrrolo[2',1':21,22;2",1":6,7][1,4,7,10,13,16,19,22,25,28,31,34,37,40,43,49]hexadecaazacyclotetrapentacontino[19,18-a]indol-6-yl]acetic acid

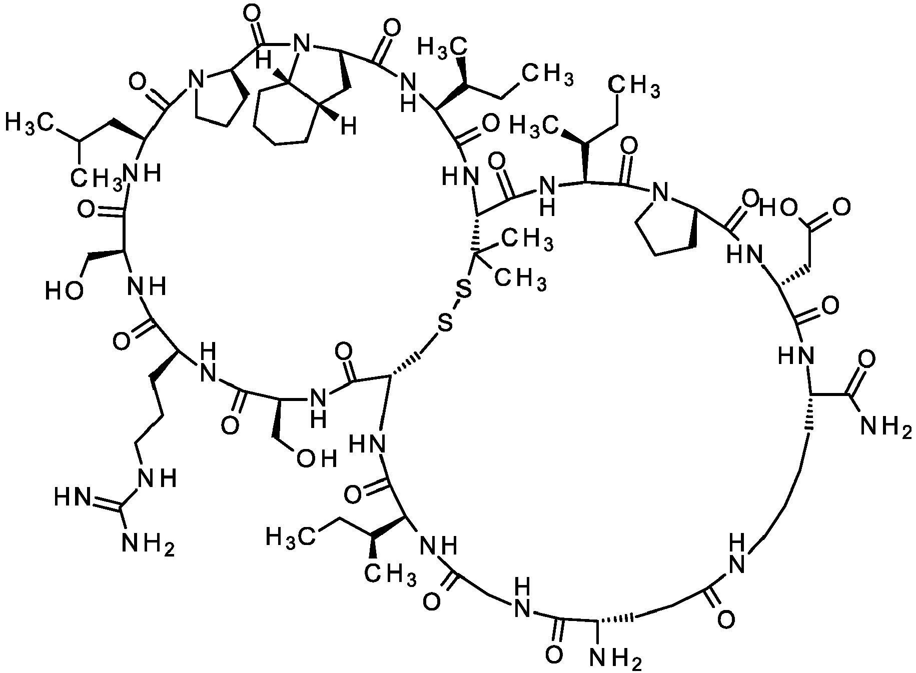

### Example 92

### Sequence: A**GGIC+SRSLP-(Oic)-I-(Pen)+-IPD**

[(3aS,6S,9S,18S,21R,24S,27S,30S,33S,38aS,40aS,44aS,45aS,48S,51R,54S)-18,48,54-tri[(2S)-butan-2-yl]-27-(3-carbamimidamidopropyl)-24,30-bis(hydroxymethyl)-9,57,57-trimethyl-33-(2-methylpropyl)-4,7,10, 13,16,19,22,25,28,31,34,39,46,49,52,55-hexadecaoxohexapentacontahydro-1H,36H-21,51-(methanodithiomethano)dipyrrolo[1',2':13,14;1",2":46,47][1,4,7,10,13,16,19,22,25,28,31,34,37,40,43,46]hexadecaazacyclooctatetracontino[1',2-a]indol-6-yl]acetic acid

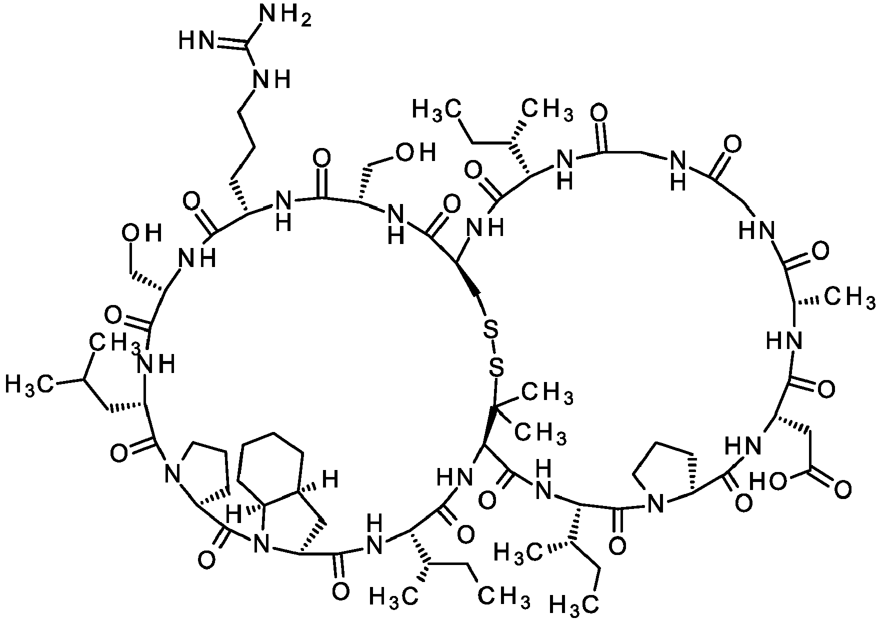

### Example 93

### Sequence: E++GIC+SRSLP-(Oic)-I-(Pen)+-IPD-(Dap)++-NH₂

[(3aS,6S,9S,15S,21S,24R,27S,30S,33S,36S,41aS,43aS,47aS,48aS,51S,54R,57S)-15-amino-21,51,57-tri[(2S)-butan-2-yl]-30-(3-carbamimidamidopropyl)-9-carbamoyl-27,33-bis(hydroxymethyl)-60,60-dimethyl-36-(2-methylpropyl)-4,7,12,16,19,22,25,28,31,34,37,42,49,52,55,58-hexadecaoxohexacontahydro-39H-24,54-(methanodithiomethano)dipyrrolo[2',1':24,25;2",1":9,10][1,4,7,10,13,16,19,22,25,28,31,34,37,40,43,46]hexadecaazacyclohenpentacontino[22,21-a]indol-6-yl]acetic acid

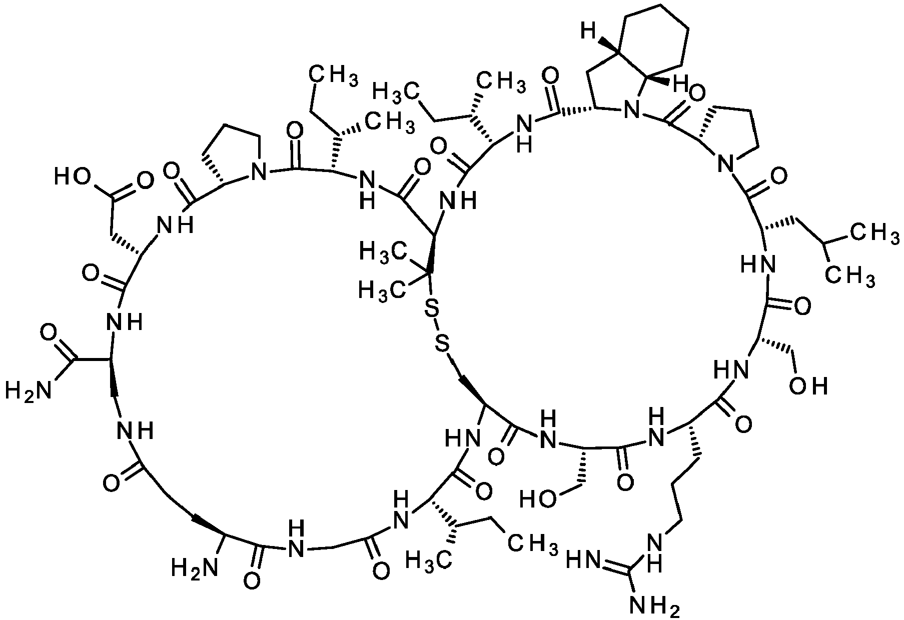

### Example 94

### Sequence: (Ahx)**-GIC+SRSLPPIC+IPD**(HCl Salt)

[(6S,9S,12S,15S,18R,21S,34S,36aS,42S,45R,48S,50aS,55aS)-21,42,48-tri[(2S)-butan-2-yl]-12-(3-carbamimidamidopropyl)-9,15-bis(hydroxymethyl)-6-(2-methylpropyl)-5,8,11,14,17,20,23,26,33,36,41,44,47,50,55-pentadecaoxotetrapentacontahydro-1H-18,45-(methanodithiomethano)tripyrrolo[2,1-f:2',1'-r:2",1"-u][1,4,7,10, 13,16,19,22,25,28,31,34,37,40,43]pentadecaazacyclononatetracontin-34-yl]acetic acid hydrogen chloride

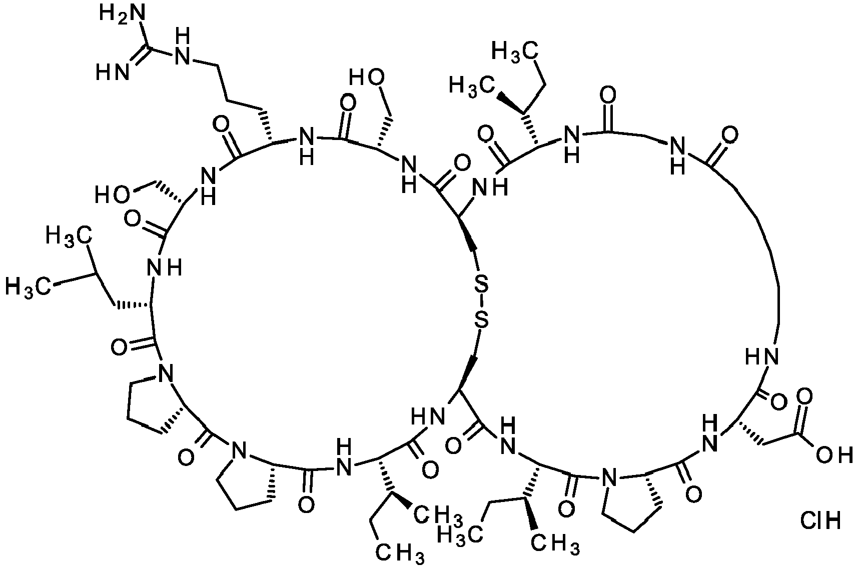

### Example 95

### Sequence: (Ahx)*-IC+SRSLP-(Oic)-IC+I*

N-13-[(6S,9S,12S,15S,18R,21S,31S,34R,37S,39aS,40aS,44aS,46aS)-21,31,37-tri[(2S)-butan-2-yl]-9,15-bis-(hydroxymethyl)-6-(2-methylpropyl)-5,8,11,14,17,20,23,30,33,36,39,46-dodecaoxohexatetracontahydro-1H,5H-18,34-(methanodithiomethano)pyrrolo[2',1':15,16][1,4,7,10,13,16,19,22,25,28,31,34]dodecaazacyclotetracontino[13,12-a]indol-12-yl]propyl}guanidine

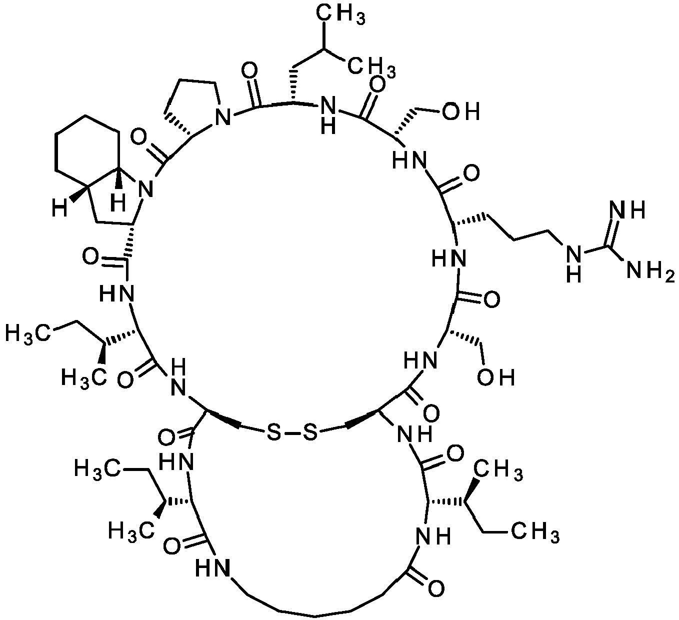

### Example 96

### Sequence: (Orn)**-GIC+SRS-((tBu)A)-PPI-(Pen)+-IP**

N-{3-[(6S,9S,12S,15S,18R,21S,27S,29aS,35S,38R,41S,43aS,48aS)-27-(3-aminopropyl)-21,35,41-tri[(2S)-butan-2-yl]-6-(2,2-dimethylpropyl)-9,15-bis(hydroxymethyl)-49,49-dimethyl-5,8,11,14,17,20,23,26,29,34, 37,40,43,48-tetradecaoxohexatetracontahydro-1H,5H-18,38-(methanodithiomethano)tripyrrolo[1,2-a:1',2'-d:1",2"-p][1,4,7,10,13,16,19,22,25,28,31,34,37,40]tetradecaazacyclodotetracontin-12-yl]propyl}guanidine

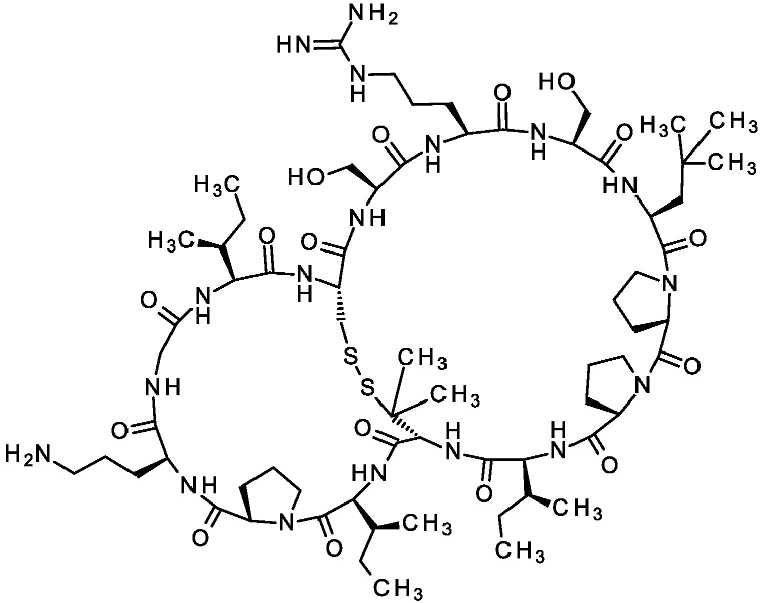

### Example 97

### Sequence: (Dap)**-(Dap)-IC+SRS-((tBu)A)-PPI-(Pen)+-IP**

N-{3-[(6S,9S,12S,15S,18R,21S,24S,27S,29aS,35S,38R,41S,43aS,48aS)-24,27-bis(aminomethyl)-21,35,41-tri[(2S)-butan-2-yl]-6-(2,2-dimethylpropyl)-9,15-bis(hydroxymethyl)-49,49-dimethyl-5,8,11,14,17,20,23,26, 29,34,37,40,43,48-tetradecaoxohexatetracontahydro-1H,5H-18,38-(methanodithiomethano)tripyrrolo[1,2-a:1', 2'-d:1",2"-p][1,4,7,10,13,16,19,22,25,28,31,34,37,40]tetradecaazacyclodotetracontin-12-yl]propyl}guanidine

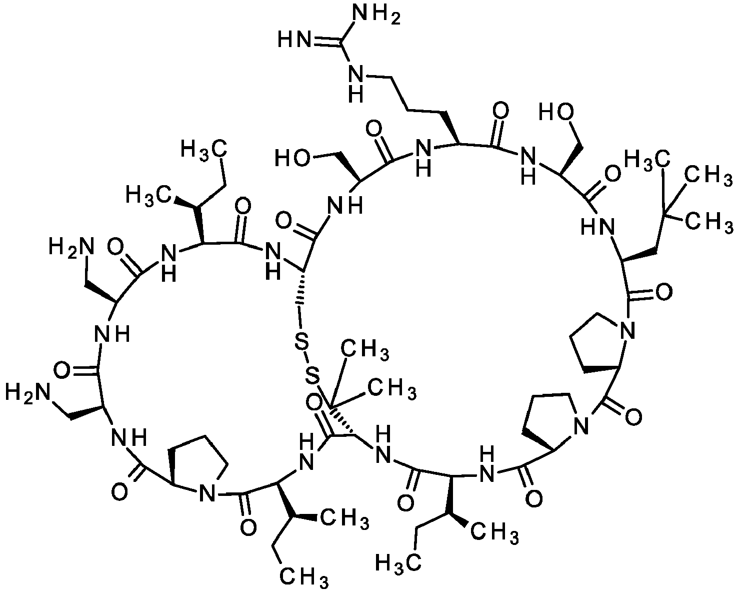

### Example 98

### Sequence: A**GGIC+SRSLP-(Oic)-I-(Pen)+-IPd**

[(3aS,6R,9S,18S,21R,24S,27S,30S,33S,38aS,40aS,44aS,45aS,48S,51R,54S)-18,48,54-tri[(2S)-butan-2-yl]-27-(3-carbamimidamidopropyl)-24,30-bis(hydroxymethyl)-9,57,57-trimethyl-33-(2-methylpropyl)-4,7,10,13,16, 19,22,25,28,31,34,39,46,49,52,55-hexadecaoxohexapentacontahydro-lH,36H-21,51-(methanodithiomethano)-dipyrrolo[1',2':13,14;1",2":46,47][1,4,7,10,13,16,19,22,25,28,31,34,37,40,43,46]hexadecaazacyclooctatetracontino[1,2-a]indol-6-yl]acetic acid

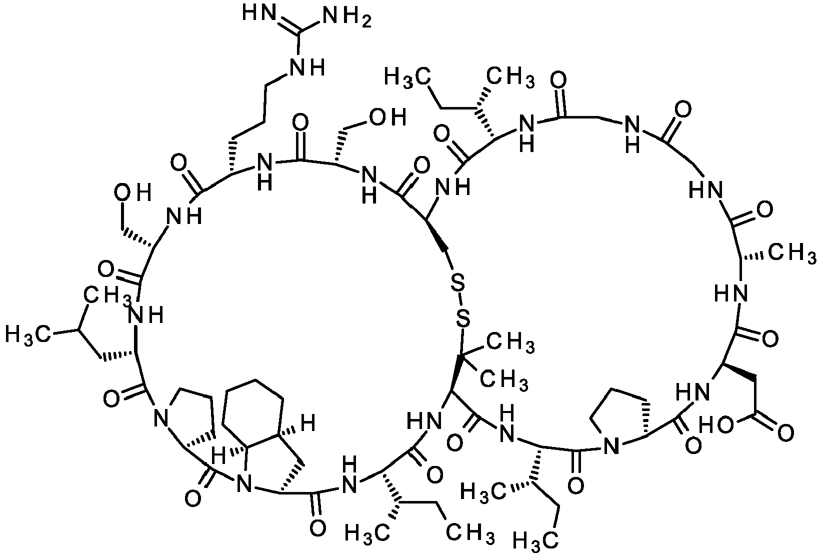

### Biological in vitro Testings

### 1. Serine protease profiling of test compounds

Test compounds were tested in a protease panel consisting of different human serine proteases including kallikrein, plasmin, FXIa, thrombin, factor Xa, tPA, and trypsin.

### Test description

Inhibitory potency and/or selectivity of test compounds were determined. The assays are based on the fluorescent detection of aminomethylcoumarine (AMC), released from the fluorogenic peptidic protease substrates upon protease catalyzed cleavage. The active proteases or zymogenes, typically purified from human plasma or for trypsin from human pancreas, and corresponding substrates are commercially available.

Serine protease assays comprise of the following enzymes and substrates. All enzymes and substrates are diluted in assay buffer (50 mM Tris/HCl pH7.4, 100 mM NaCl, 5 mM CaCl2, 0.1% BSA). The final assay concentrations are given:
- Kallikrein (Kordia; 0.2 nM), H-Pro-Phe-Arg-AMC (Bachem I-1295; 5 µM)
- Plasmin (Kordia; 0.1 µg/mL, 1.2 nM), MeOSuc-Ala-Phe-Lys-AMC (Bachem 1-1275; 50 µM)
- Factor XIa (Kordia; 0.15 nM), Boc-Glu(OBzl)-Ala-Arg-AMC (Bachem I-1575; 5 µM)
- Thrombin (Kordia; 0.02 nM), Boc-Asp(OBzl)-Pro-Arg-AMC (Bachem I-1560; 5 µM)
- Factor Xa (Kordia; 1.3 nM), Boc-Ile-Glu-Gly-Arg-AMC (Bachem I-1100; 5 µM)
- Tissue plasminogen activator (tPA, Loxo; 2 nM), CH3SO2-D-Phe-Gly-Arg-AMC (Pentapharm 091-06; 5 µM)
- Trypsin (Sigma; 0.042 U/mL), substrate Boc-Ile-Glu-Gly-Arg-AMC (Bachem I-1100; 5 µM)

For determination of test compound potency, the enzyme and corresponding substrate dilutions are used to perform protease assays:
To 384 well microtiter plates (white, Greiner), containing 1 µL/well serial dilutions of test or reference compounds, 20 µL assay buffer, 20 µl enzyme dilution, and 20 µl substrate are added. Control reactions do not contain test compound (DMSO only). After incubation for typically 30 min (linear reaction kinetics) at room temperature, fluorescence (ex 360 nm, em 465 nm) is measured in a microtiter plate fluorescence reader (e.g Tecan Safire II). IC₅₀ values are determined by plotting log test compound concentration against the percentage protease activity.

### 2. Biochemical Human MASP-1 and MASP-2 assay

### 2.1 Recombinant expression and protein production of recombinant human MASP1 and MASP2 active proteases.

A truncated cDNA sequence of human MASP1 encoding the fragment corresponding to the amino acids 297-699 with a C-terminal His Tag and N-terminal Ig-kappa secretion signal (SEQ ID No: 2) was subcloned into the mammalian expression vector pcDNA3.1 (Invitrogen).

A truncated cDNA sequence of human MASP1 encoding the fragment corresponding to the amino acids 297-686 with a C-terminal His Tag and N-terminal Ig-kappa secretion signal (SEQ ID No: 3) was subcloned into the mammalian expression vector pcDNA3.1 (Invitrogen).

The MASP1 or MASP2 expression vectors were transfected into the HEK293 (ATCC No. CRL-1573) cell line using Lipofectamine LTX^{®} Reagent (Thermo-Fischer), as described by the manufacturer. The mature form of the recombinant human MASP1 and MASP2 proteases were secreted into the culture medium. The MASP1 and MASP2 proteins were purified from the conditioned media by affinity chromatography on Ni-NTA Superflow resin (Qiagen) as described by the manufacturer.

### 2.2 Biochemical human MASP1 assay

Recombinant human MASP1 enzyme produced in the HEK 293 cells was diluted in the reaction buffer (50 mM HEPES pH 8,0; 100 mM NaCl; 0,01% CHAPS; 0,5 mM Gluthathione) to the concentration of 20 nM and 25 µl was transferred into each single well of 384-well white microtiter plate (Greiner Bio One 781075). 1 µl of the inhibitor compound solution (dissolved in DMSO, at the corresponding concentration) or pure DMSO as a control was added to the same wells. The enzymatic reaction was initiated by addition of 25 µl of 20 µM solution of the FRET substrate ABZ-MYGGARRL-Lys (Dnp)-NH₂; (ABZ - 2-aminobenzoyl; DNP - 2,4-dinitrophenyl; custom synthesis by Jerini Peptide Technologies, Berlin) in the reaction buffer. The microtiter plate was incubated for 60-120 min at the temperature of 32 C. The increase of fluorescence intensity was measured in appropriate fluorescence plate reader (e.g. TECAN Ultra) using excitation wavelength of 320 nm and emission wavelength of 420 nm. IC50 values were calculated from percentage of inhibition of human MASP1 activity as a function of test compound concentration.

### 2.3 Biochemical human MASP2 assay

Recombinant human MASP2 enzyme produced in the HEK 293 cells was diluted in the reaction buffer (50 mM HEPES pH 8,0; 100 mM NaCl; 0,01% CHAPS; 0,5 mM Gluthathione) to the concentration of 20 nM and 25 µl was transferred into each single well of 384-well white microtiter plate (Greiner Bio One 781075). 1 µl of the inhibitor compound solution (dissolved in DMSO, at the corresponding concentration) or pure DMSO as a control was added to the same wells. The enzymatic reaction was initiated by addition of 25 µl of 60 µM solution of the FRET substrate DABCYL-KISPQGYGRR-Glu(EDANS)-NH₂; (Dabcyl - 4 - ((4 - (dimethylamino)phenyl)azo)benzoic acid; Edans - 5-[(2-Aminoethyl) amino]naphthalene-1-sulfonyl; custom synthesis by Jerini Peptide Technologies, Berlin) in the reaction buffer. The microtiter plate was incubated for 60-120 min at the temperature of 32 C. The increase of fluorescence intensity was measured in appropriate fluorescence plate reader (e.g. TECAN Ultra) using excitation wavelength of 340 nm and emission wavelength of 490 nm. IC50 values were calculated from percentage of inhibition of human MASP2 activity as a function of test compound concentration.

### 3. C3 Deposition assay (human, rat, mouse, dog, pig))

The C3 deposition assay was conducted essentially as described (reference). Multiwell plates (Greiner-Nunc 384 Maxi Sorp #464718) were coated over night with Mannan from Saccharomyces cerevisiae (Sigma M7504, 10 µg/mL in 0.05 M carbonate-bicarbonate buffer, pH 9.6) at 4°C. Wells were washed three times with TBS and subsequently incubated for 2 hours with 50 µL of 1% bovine serum albumin (BSA) in Tris-buffered saline (TBS) at 37°C in order to block non-specific binding. After this step and each of the following incubation steps wells were washed three times with C3 wash buffer (TBS; 0.05% Tween 20; 5 mM CaCl₂). Wells were next incubated for 30 min at 37°C with 50 µL of a mixture of test compounds with diluted serum in Veronal buffer (Veronal Puffer (Lonza 12624E). Serum was used at concentrations that did not show detectable C3 deposition to uncoated plates in pre-tests. Appropriate dilutions were found to be in the range of 1:100 - 1:200 for human, rat, mouse and dog serum and 1:20 - 1:100 for mini pig serum, respectively. In typical experiments compounds were tested in a range of concentrations between 1x10⁻⁹ and 5x10⁻⁵ mol/L. After washing C3 deposition was detected by incubation with a polyclonal rabbit anti-human C3 antibody (Dako (Biozol) A0062) for 1 hour followed by washing and incubation with a peroxidase conjugated Anti Rabbit IgG (Sigma A1949) for 30 min at 37°C and subsequent washing and incubation with TMB substrate solution in the dark. When appropriately developed the color reaction was stopped by addition of 25 µL of stop solution (Sigma S5814) and quantified on a photometer by measuring absorption at a wavelength of 450 nm. Antibodies were diluted in C3 wash buffer supplemented with 0.5% BSA.

### 4. Biochemical Rat MASP-1 and MASP-2 assay

### 4.1 Recombinant expression and protein production of recombinant human and rat MASP1 and MASP2 active proteases.

A truncated cDNA sequence of rat MASPlencoding the fragment corresponding to the amino acids 302-704 with a C-terminal His Tag and N-terminal Ig-kappa secretion signal (SEQ ID No: 4) was subcloned into the mammalian expression vector pcDNA3.1 (Invitrogen).

A truncated cDNA sequence of rat MASP2 encoding the fragment corresponding to the amino acids 296-685 with a C-terminal His Tag and N-terminal Ig-kappa secretion signal (SEQ ID No: 5) was subcloned into the mammalian expression vector pcDNA3.1 (Invitrogen).

The MASP1 or MASP2 expression vectors were transfected into the HEK293 (ATCC No. CRL-1573) cell line using Lipofectamine LTX^{®} Reagent (Thermo-Fischer), as described by the manufacturer. The mature form of the recombinant rat MASP1 and MASP2 proteases were secreted into the culture medium. The MASP1 and MASP2 proteins were purified from the conditioned media by affinity chromatography on Ni-NTA Superflow resin (Qiagen) as described by the manufacturer.

### 4.2 Biochemical rat MASP1 assay.

Recombinant rat MASP1 enzyme produced in the HEK 293 cells was diluted in the reaction buffer (50 mM HEPES pH 8,0; 100 mM NaCl; 0,01% CHAPS; 0,5 mM Gluthathione) to the concentration of 4 nM and 25 µl was transferred into each single well of 384-well white microtiter plate (Greiner Bio One 781075). 1 µl of the inhibitor compound solution (dissolved in DMSO, at the corresponding concentration) or pure DMSO as a control was added to the same wells. The enzymatic reaction was initiated by addition of 25 µl of 40 µM solution of the FRET substrate Dabcyl-MYGGARRL-Glu(Edans)-NH2; (Dabcyl - 4 - ((4 - (dimethylamino)phenyl)azo)benzoic acid; Edans - 5-[(2-Aminoethyl) amino]naphthalene-1-sulfonyl; custom synthesis by Jerini Peptide Technologies, Berlin) in the reaction buffer. The microtiter plate was incubated for 60-120 min at the temperature of 32 C. The increase of fluorescence intensity was measured in appropriate fluorescence plate reader (e.g. TECAN Ultra) using excitation wavelength of 340 nm and emission wavelength of 490 nm. IC50 values were calculated from percentage of inhibition of rat MASP2 activity as a function of test compound concentration.

### 4.3 Biochemical rat MASP2 assay.

Recombinant rat MASP2 enzyme produced in the HEK 293 cells was diluted in the reaction buffer (50 mM HEPES pH 8,0; 100 mM NaCl; 0,01% CHAPS; 0,5 mM Gluthathione) to the concentration of 20 nM and 25 µl was transferred into each single well of 384-well white microtiter plate (Greiner Bio One 781075). 1 µl of the inhibitor compound solution (dissolved in DMSO, at the corresponding concentration) or pure DMSO as a control was added to the same wells. The enzymatic reaction was initiated by addition of 25 µl of 30 µM solution of the FRET substrate Abz-IEGRTSED-(Lys)Dnp-NH2; (ABZ - 2-aminobenzoyl; DNP - 2,4-dinitrophenyl; custom synthesis by Jerini Peptide Technologies, Berlin) in the reaction buffer. The microtiter plate was incubated for 60-120 min at the temperature of 32 C. The increase of fluorescence intensity was measured in appropriate fluorescence plate reader (e.g. TECAN Ultra) using excitation wavelength of 320 nm and emission wavelength of 420 nm. IC50 values were calculated from percentage of inhibition of rat MASP2 activity as a function of test compound concentration.

**Table 22: Average IC50 of the reference peptides**

| **Ref. No** | **human MASP1 IC50 [mol/L]** | **human MASP2 \|IC50 [mol/L]** | **Human C3-DEPOSITION SERUM\| IC50 [mol/L]** |
|---|---|---|---|
| 2 | > 3.00 E-06 | > 3.00 E-06 | > 5.00 E-4 |
| 7 | 1.45 E-07 | 8.95 E-07 | 1.12 E-06 |
| 8 | 8.13 E-06 | | 2.60 E-06 |
| 12 | 4.80 E-06 | 5.00 E-06 | 5.00 E-05 |

**Table 23: Average IC50 of the peptides of the invention**

| **Ex. No** | **human MASP1 IC50 [mol/L]** | **human MASP2 \|IC50 [mol/L]** | **Human C3-DEPOSITION SERUM\| IC50 [mol/L]** |
|---|---|---|---|
| 13 | 1.40 E-07 | 1.30 E-07 | 3.51 E-07 |
| 14 | 1.70 E-07 | 2.40 E-07 | 7.35 E-07 |
| 15 | 1.20 E-07 | 3.00 E-07 | 5.71 E-07 |
| 16 | 1.50 E-07 | 3.70 E-07 | 3.84 E-07 |
| 17 | 2.60 E-08 | 8.80 E-08 | 3.04 E-07 |
| 18 | 5.00 E-08 | 1.50 E-08 | 2.07 E-07 |
| 19 | 4.90 E-08 | 2.10 E-07 | 4.41 E-07 |
| 20 | 5.50 E-08 | 1.10 E-07 | 8.50 E-07 |
| 21 | 1.70 E-07 | 1.20 E-07 | 3.26 E-07 |
| 22 | 1.90 E-07 | 9.10 E-08 | 3.08 E-07 |
| 23 | 1.80 E-07 | 2.00 E-08 | 1.21 E-07 |
| 24 | 1.10 E-07 | 2.10 E-07 | 7.59 E-07 |
| 25 | 6.00 E-08 | 6.10 E-07 | 1.91 E-07 |
| 26 | 1.20 E-08 | 1.70 E-07 | 1.63 E-07 |
| 27 | 9.10 E-09 | 3.75 E-08 | 1.27 E-07 |
| 28 | 1.90 E-08 | 2.90 E-08 | 9.19 E-08 |
| 29 | 8.60 E-09 | 6.80 E-08 | 8.46 E-08 |
| 30 | 1.60 E-08 | 6.50 E-08 | 1.11 E-07 |
| 31 | 1.60 E-08 | 1.30 E-07 | 1.10 E-07 |
| 32 | 2.20 E-08 | 6.35 E-08 | 1.21 E-07 |
| 33 | 2.80 E-08 | 6.20 E-08 | 2.06 E-07 |
| 34 | 1.10 E-08 | 9.80 E-08 | 4.26 E-08 |
| 35 | 4.10 E-09 | 1.70 E-08 | 3.30 E-09 |
| 36 | 8.70 E-09 | 1.10 E-07 | 8.68 E-09 |
| 37 | 1.60 E-08 | 1.40 E-07 | 5.94 E-08 |
| 38 | 2.00 E-08 | 4.40 E-08 | |
| 39 | 6.00 E-09 | 7.50 E-08 | 3.36 E-08 |
| 40 | 7.10 E-09 | 9.00 E-08 | 7.26 E-08 |
| 41 | 5.90 E-09 | 1.90 E-07 | 2.24 E-08 |
| 42 | 1.30 E-08 | 2.00 E-07 | 9.47 E-08 |
| 43 | 6.70 E-09 | 1.20 E-07 | 2.82 E-08 |
| 44 | 4.70 E-09 | 2.30 E-07 | |
| 45 | 9.20 E-08 | 7.40 E-08 | 3.31 E-07 |
| 46 | 5.60 E-08 | 8.90 E-08 | 2.28 E-07 |
| 47 | 8.20 E-09 | 6.60 E-08 | 7.29 E-08 |
| 48 | 1.00 E-08 | 7.00 E-08 | 5.34 E-08 |
| 49 | 1.90 E-08 | 1.40 E-07 | 5.62 E-08 |
| 50 | 1.30 E-08 | 2.10 E-07 | 2.28 E-07 |
| 51 | 6.30 E-09 | 1.70 E-08 | 2.82 E-08 |
| 52 | 7.90 E-09 | 9.30 E-09 | 2.37 E-08 |
| 53 | 6.75 E-09 | 8.35 E-08 | 4.45 E-08 |
| 54 | 8.50 E-09 | 3.30 E-08 | 3.96 E-08 |
| 55 | 4.60 E-08 | 9.00 E-07 | 3.82 E-07 |
| 56 | 8.60 E-08 | 1.10 E-06 | 5.66 E-07 |
| 57 | 4.60 E-08 | 3.40 E-07 | 2.36 E-07 |
| 58 | 9.00 E-09 | 6.50 E-07 | 8.17 E-08 |
| 59 | 1.10 E-08 | 5.70 E-09 | 1.77 E-07 |
| 60 | 5.80 E-08 | 5.40 E-09 | 2.05 E-08 |
| 61 | 3.10 E-09 | 3.10 E-07 | 2.34 E-08 |
| 62 | 2.20 E-08 | 5.40 E-07 | 1.49 E-07 |
| 63 | 2.80 E-08 | 1.70 E-07 | 4.09 E-08 |
| 64 | 9.40 E-09 | 1.20 E-08 | 1.78 E-08 |
| 65 | 4.90 E-08 | 5.40 E-07 | 6.70 E-08 |
| 66 | 8.10 E-09 | 2.20 E-08 | 2.58 E-08 |
| 67 | 3.25 E-09 | 1.50 E-07 | 1.91 E-08 |
| 68 | 6.10 E-09 | 2.57 E-09 | 4.64 E-09 |
| 69 | 2.00 E-08 | 1.90 E-07 | 3.64 E-07 |
| 71 | 2.10 E-08 | 2.40 E-08 | 5.38 E-08 |
| 72 | 6.30 E-09 | 4.80 E-08 | 5.34 E-08 |
| 73 | 4.20 E-08 | 4.30 E-07 | 4.82 E-08 |
| 74 | 1.40 E-08 | 1.60 E-07 | 1.43 E-08 |
| 75 | 7.20 E-09 | 1.40 E-08 | 8.14 E-09 |
| 76 | 9.70 E-09 | 1.80 E-07 | 1.93 E-08 |
| 77 | 1.10 E-08 | 2.10 E-07 | 1.99 E-08 |
| 78 | 1.00 E-08 | 7.00 E-09 | 1.20 E-08 |
| 79 | 2.20 E-08 | 4.50 E-08 | 1.03 E-08 |
| 80 | 1.10 E-08 | 6.80 E-09 | 1.39 E-08 |
| 81 | 1.80 E-08 | 2.30 E-07 | 2.28 E-08 |
| 82 | 6.90 E-08 | 7.00 E-07 | 2.04 E-08 |
| 83 | 1.80 E-08 | 9.10 E-08 | 2.65 E-08 |
| 84 | 1.50 E-08 | 8.70 E-08 | 2.56 E-08 |
| 85 | 1.00 E-06 | 1.00 E-06 | 3.13 E-06 |
| 86 | 2.70 E-08 | 1.00 E-06 | 4.63 E-08 |
| 87 | 4.60 E-08 | 7.00 E-07 | 7.86 E-08 |
| 88 | 1.00 E-06 | 1.00 E-06 | |
| 89 | 5.70 E-09 | 9.10 E-08 | 1.85 E-08 |
| 90 | 9.80 E-09 | 1.10 E-07 | 5.86 E-08 |
| 91 | 2.90 E-08 | 7.10 E-08 | 1.47 E-07 |
| 92 | 8.40E-09 | 7.70 E-8 | 1.11 E-07 |
| 93 | 3.50 E-08 | 4.50 E-08 | 2.74 E-07 |
| 94 | 1.20 E-07 | 1.30 E-07 | 1.02 E-06 |
| 95 | 1.00 E-07 | 1.10 E-07 | |
| 96 | 2.30 E-08 | 4.00 E-07 | 1.03 E-07 |
| 97 | 5.00 E-09 | 7.60 E-07 | 5.64 E-08 |
| 98 | 1.90 E-08 | 1.60 E-07 | 3.57 E-07 |

**Table 24: Average IC50 of the reference peptides**

| **Ref. No** | **rat MASP1 IC50 [mol/L]** | **rat MASP2 \|IC50 [mol/L]** | **rat C3-DEPOSITION SERUM\| IC50 [mol/L]** |
|---|---|---|---|
| 2 | 1.00 E-06 | 1.00 E-06 | |
| 7 | 1.00 E-06 | 2.32 E-07 | |
| 8 | 1.00 E-06 | 1.26 E-07 | |
| 12 | 1.00 E-06 | 1.00 E-06 | |

**Table 25: Average IC50 of representative peptides of the invention**

| **Ex. No** | **rat MASP1 IC50 [mol/L]** | **rat MASP2 \|IC50 [mol/L]** | **rat C3-DEPOSITION SERUM\| IC50 [mol/L]** |
|---|---|---|---|
| 28 | 7.90 E-08 | 1.60 E-08 | |
| 35 | 6.30 E-08 | 2.40 E-08 | |
| 39 | 4.40 E-08 | 8.00 E-09 | |
| 52 | 7.90 E-08 | 1.16 E-08 | |
| 53 | 9.60 E-08 | 1.90 E-08 | |
| 54 | 1.80 E-07 | 1.80 E-08 | |
| 58 | 1.10 E-07 | 4.60 E-08 | |
| 59 | 4.86 E-08 | 4.57 E-09 | |
| 60 | 3.30 E-07 | 1.30 E-08 | |
| 64 | 1.57 E-07 | 1.30 E-08 | |
| 68 | 3.08 E-08 | 2.97 E-09 | |
| 72 | 8.50 E-08 | 1.40 E-08 | |
| 75 | 2.70 E-08 | 1.40 E-09 | |
| 89 | 2.90 E-07 | 6.40 E-08 | |
| 92 | 1.70 E-07 | 4.10 E-08 | |
| 93 | 4.10 E-07 | 6.80 E-08 | |
| 98 | 1.70 E-07 | | |

### 4. Kidney ischemia reperfusion injury (IRI) in rats after unilateral nephrectomy

All procedures conformed to national legislation (dt. Tierschutzgesetz) and EU directives for the use of animals for scientific purposes and were approved by the institutional animal care office of Bayer AG and by the competent regional authority (LANUV Recklinghausen). Standard laboratory diet and tap water were available ad libitum. In a typical experiment, the number of animals used was n = 6 to 12. Animals were randomly assigned to experimental groups. Kidney ischemia reperfusion injury (IRI) was performed in male unilaterally nephrectomized Wistar rats of a preferred body weight in the range of 250 to 350 g. For unilateral nephrectomy rats were kept anesthetized under inhalation of 2% isoflurane in air. Analgesia was provided as a subcutaneous injection of 400 µl/kg of a mixture of 25% Ketavet and 8% Rompun in 0.9 NaCl. Unilateral nephrectomy was performed after protruding the right kidney through a small incision in the dorsolateral abdominal wall and ligating of its peduncle. After unilateral nephrectomy abdominal incision was closed by surgical sutures in layers and animals were allowed to recover for 7 to 8 days before IRI. IRI was performed under anesthesia and analgesia as described above. The remnant left kidney was protruded through a small incision of the abdominal wall and blood vessels of the kidney peduncle were clamped with an atraumatic microvascular clamp for 45 minutes in a typical setting. During this time the kidney together with the clamp in situ was repositioned into the abdominal cavity to ensure warm ischemia. After 45 min the clamp was opened and removed and the incision closed by sutures as described above. Test compound or vehicle was administered intravenously via a polyethylene catheter placed before surgery into the jugular vein.

Compounds were dissolved in appropriate vehicle and administered either preventive before IRI or therapeutically after completion of IRI. Typical dose range applied was 0.1 - 30 mg/kg i.v.. Vehicle without compound was administered to animals that served as controls. Sham control animals underwent the whole procedure described above without closure of the clamp for induction of ischemia.

Blood samples were taken under anesthesia at day 1 and 8 after IRI. In a typical setting, animals were sacrificed 8 days after IRI and kidneys were sampled and frozen in liquid nitrogen. In another typical setting the animals were sacrificed 1 day after IRI.

Typical laboratory parameters measured in plasma samples to assess kidney function were creatinine and urea. For determination of creatinine clearance animals were kept in metabolic cages and urine was collected for at least 16 hours. After determination of urine volume flow (Vu) and determination of urinary and plasma creatinine concentrations ([Crea]_{U} and [Crea]_{P1}, respectively) creatinine clearance (Cl_{Crea}) was calculated according to the standard formula: Cl_{Crea}= V_{U} ** [Crea]_{U} / [Crea]_{P1}

RNA Extraction and Quantitative Real-Time Polymerase Chain Reaction: Total RNA was extracted from tissue samples by the Trizol method. Integrity of obtained RNA was checked on a Bioanalyzer (Agilent). For reverse transcription, 1 µg of total RNA was first digested with RNase-free DNase I (Gibco) for 15 min at room temperature and then reversetranscribed using Promiscript (Promega) in a total reaction volume of 40 µl according to the standard protocol of the kit supplier. After inactivation of the enzyme by heating for 15 min to 65 °C, the obtained cDNA was diluted to a final volume of 150 µl with bidest. water and 4 µl were chosen per PCR reaction. Real-Time PCR including normalization of raw data to cytosolic beta-actin as a housekeeping gene was carried out as described (Ellinghaus et al., 2005). The resulting expression is given in arbitrary units. Sequences of used oligonucleotide primers and probes are given in table 1.

### 5. Kidney ischemia reperfusion injury (IRI) in pigs after aortic balloon occlusion

All procedures conformed to national legislation (dt. Tierschutzgesetz) and EU directives for the use of animals for scientific purposes and were approved by the institutional animal care office of Bayer AG and by the competent regional authority (LANUV Recklinghausen). Female Göttingen mini pigs (Ellegaard, Denmark) of a body weight ranging preferably from 12 to 16 kg were used for the experiments. Animals were randomly assigned to experimental groups.

Minimal invasive methods were applied with modifications as described (Simon et al., Effects of intravenous sulfide during porcine aortic occlusion-induced kidney ischemia/reperfusion injury. Shock. 2011; 35:156-163*;* Matejkova et al., Carbamylated erythropoietin-FC fusion protein and recombinant human erythropoietin during porcine kidney ischemia/reperfusion injury. Intensive Care Med. 2011;39:497-510)*.* In brief: Pigs were kept anesthetized by a continuous i.v.-infusion of Ketavet^{®}, Dormicum^{®} and Pancuronium^{®} after premedication with an intramuscular injection of Ketavet^{®} / Stresnil^{®}. After intratracheal intubation animals were artificially ventilated using a pediatric respirator (Avance CS², GE Healthcare) with an oxygen air mixture at a tidal volume of 6 to 8 mL/kg at a constant positive end-expiratory pressure (PEEP) of 3 - 4 cm H₂O and a frequency of 13 to 20 min⁻¹. Ventilation was adjusted to keep arterial PaCO2 at about 40 mmHg at baseline. A catheter was placed into the right jugular vein for drug and fluid administration. Ringer-lactate solution was infused intravenously at a constant rate of 10 mL/kg/h. Animals received 50 i.E./kg Heparin i.v.. Routinely the following cardiovascular and respiratory parameters were measured after placement of necessary probes and catheters fitted to appropriate pressure transducers and recording equipment: central venous pressure (via left jugular vein), arterial blood pressure and heart rate (BP and HR; via left carotid artery) and cardiac output (CO) and systemic vascular resistance (SVR) by use of the PiCCO^{®} system (Pulsion, Germany) connected to a Pulsion 4F Thermodilutioncatheter (PV2014L08N) placed into the right carotid artery. Catheters for measurement of CVP, BP and HR were fitted to a Ponemah recording system via Combitrans transducers (Braun, REF 5203660). A Fogarty occlusion catheter (8F/14F, Edwards Lifesiences, REF 6208014F) was inserted into the abdominal aorta via the left femoral artery so that the tip with the inflatable balloon was placed upstream of the kidney arteries. A catheter was introduced into the urinary bladder via a small abdominal incision and urine continuously collected. Arterial blood samples were collected at regular intervals in which creatinine, urea, liver enzymes, blood cells and compound concentrations were determined. Arterial pO2, pCO2 and pH were determined on a Stat Profile^{®} PRIME^{®} (Nova Biomedical) blood gas analyzer at regular intervals in arterial blood samples. Kidney perfusion was assessed at regular intervals by Doppler ultrasound determination of the resistive index using a LOGIQ e Veterinary ultrasound apparatus (General Electrics) fitted with a 2,0 to 5,0 MHz broad-spectrum convex transducer (C1-5-RS, REF 5384874). Renal resistive index (RRI) is a suitable parameter to assess severity of acute kidney injury in patients (Darmon et al., Diagnostic accuracy of Doppler renal resistive index for reversibility of acute kidney injury in critically ill patients. Intensive Care Med. 2011;37(1):68-76)

When cardiovascular parameters showed a stable baseline (which was normally the case 60 min after surgery) recordings were started and samples for baseline parameters were collected. HR and MABP were continuously measured and for recording averaged over 2 min intervals. At the end of experimentation pigs were sacrificed by exsanguination.

Kidney injury was induced by inflating the balloon of the Fogarty balloon catheter with saline which immediately interrupted blood flow to the kidneys and the abdominal organs and led to a sharp increase of aortic blood pressure upstream of the balloon. Stop of blood flow was further confirmed by Doppler ultrasound examination of the kidney blood vessels. In typical experiments the aorta is kept occluded for 90 to 120 min until reperfusion by deflating the balloon. After reperfusion the Ringer-lactate infusion rate is doubled to 20 mL/kg/h in order to stabilize blood pressure and to enable diuresis. All parameters were monitored for up to 6 hours after reperfusion.

Compounds were dissolved in appropriate vehicle and administered either preventive before IRI or therapeutically after completion of IRI. Typical dose range applied was 0.1 - 10 mg/kg i.v. In a typical experiment up to three doses were tested in up to 6 animals per dose group. Vehicle without compound was administered to animals that served as controls. Sham control animals underwent the whole procedure described above without induction of ischemia.

As a measure for kidney function after reperfusion preferably but not exclusively changes in diuresis, serum creatinine, serum potassium, serum bicarbonate and in resistive index determined by Doppler ultrasound examination were used.

**Table 26: Sequence Listing**

| **SEQ ID** | **No** | **Sequence** |
|---|---|---|
| SEQ ID | 1 | G**RC+TKSIPPIC+FPD** |
| SEQ ID | 2 | METDTLLLWVLLLWVPGSTGDAGNECPELQPPVHGKIEPSQAKYFFKDQVLVSC DTGYKVLKDNVEMDTFQIECLKDGTWSNKIPTCKIVDCRAPGELEHGLITFSTRN NLTTYKSEIKYSCQEPYYKMLNNNTGIYTCSAQGVWMNKVLGRSLPTCLPVCGL PKFSRKLMARIFNGRPAQKGTTPWIAMLSHLNGQPFCGGSLLGSSWIVTAAHCLH QSLDPEDPTLRDSDLLSPSDFKIILGKHWRLRSDENEQHLGVKHTTLHPQYDPNTF ENDVALVELLESPVLNAFVMPICLPEGPQQEGAMVIVSGWGKQFLQRFPETLMEI EIPIVDHSTCQKAYAPLKKKVTRDMICAGEKEGGKDACAGDSGGPMVTLNRERG QWYLVGTVSWGDDCGKKDRYGVYSYIHHNKDWIQRVTGVRNHHHHHH |
| SEQ ID | 3 | METDTLLLWVLLLWVPGSTGDAQPCPYPMAPPNGHVSPVQAKYILKDSFSIFCET GYELLQGHLPLKSFTAVCQKDGSWDRPMPACSIVDCGPPDDLPSGRVEYITGPGV TTYKAVIQYSCEETFYTMKVNDGKYVCEADGFWTSSKGEKSLPVCEPVCGLSAR TTGGRIYGGQKAKPGDFPWQVLILGGTTAAGALLYDNWVLTAAHAVYEQKHDA SALDIRMGTLKRLSPHYTQAWSEAVFIHEGYTHDAGFDNDIALIKLNNKVVINSNI TPICLPRKEAESFMRTDDIGTASGWGLTQRGFLARNLMYVDIPIVDHQKCTAAYE KPPYPRGSVTANMLCAGLESGGKDSCRGDSGGALVFLDSETERWFVGGIVSWGS MNCGEAGQYGVYTKVINYIPWIENIISDFHHHHHH |
| SEQ ID | 4 | METDTLLLWVLLLWVPGSTGDAGNECPKLQPPVYGKIEPSQAVYSFKDQVLISC DTGYKVLKDNEVMDTFQIECLKDGAWSNKIPTCKIVDCGVPAVLKHGLVTFSTR NNLTTYKSEIRYSCQQPYYKMLHNTTGVYTCSAHGTWTNEVLKRSLPTCLPVCG LPKFSRKHISRIFNGRPAQKGTTPWIAMLSQLNGQPFCGGSLLGSNWVLTAAHCL HHPLDPEEPILHNSHLLSPSDFKIIMGKHWRRRSDEDEQHLHVKHIMLHPLYNPST FENDLGLVELSESPRLNDFVMPVCLPEHPSTEGTMVIVSGWGKQFLQRLPENLME IEIPIVNYHTCQEAYTPLGKKVTQDMICAGEKEGGKDACAGDSGGPMVTKDAER DQWYLVGVVSWGEDCGKKDRYGVYSYIYPNKDWIQRVTGVRNHHHHHH |
| SEQ ID | 5 | METDTLLLWVLLLWVPGSTGDTAQPCPDPTAPPNGHISPVQATYVLKDSFSVFCK TGFELLQGSVPLKSFTAVCQKDGSWDRPIPECSIIDCGPPDDLPNGHVDYITGPEV TTYKAVIQYSCEETFYTMSSNGKYVCEADGFWTSSKGEKSLPVCKPVCGLSTHTS GGRIIGGQPAKPGDFPWQVLLLGETTAAGALIHDDWVLTAAHAVYGKTEAMSSL DIRMGILKRLSLIYTQAWPEAVFIHEGYTHGAGFDNDIALIKLKNKVTINRNIMPIC LPRKEAASLMKTDFVGTVAGWGLTQKGFLARNLMFVDIPIVDHQKCATAYTKQ PYPGAKVTVNMLCAGLDRGGKDSCRGDSGGALVFLDNETQRWFVGGIVSWGSI NCGGSEQYGVYTKVTNYIPWIENIINNFHHHHHH |
| SEQ ID | 6 | Abz-MYGGARRL-Lys (Dnp)-NH₂ |
| SEQ ID | 7 | DABCYL-KISPQGYGRR-Glu(EDANS)-NH₂ |
| SEQ ID | 8 | Dabcyl-MYGGARRL-Glu(Edans)-NH₂ |
| SEQ ID | 9 | Abz-IEGRTSED-(Lys)Dnp-NH₂ |
| SEQ ID | 10 | G**IC+SRSLPPIC+IPD** |
| SEQ ID | 11 | G**YC+SRSYPPVC+IPD** |
| SEQ ID | 12 | P**FC+IPPISKTC+RGD** |

### References

- Dunkelberger and Song, Complement and its role in innate and adaptive immune responses. Cell Res. 2010;20(1):34-50
- Garred et al., A journey through the lectin pathway of complement-MBL and beyond. Immunol Rev. 2016;274(1):74-97
- Héja et al, Revised mechanism of complement lectin-pathway activation revealing the role of serine protease MASP-1 as the exclusive activator of MASP-2. Proc Natl Acad Sci U S A. 2012;109(26):10498-503
- Sieve et al., Regulation and function of endothelial glycocalyx layer in vascular diseases. Vascul Pharmacol. 2018;100:26-33
- Moller-Kristensen et al., Mannan-binding lectin recognizes structures on ischaemic reperfused mouse kidneys and is implicated in tissue injury. Scand J Immunol. 2005;61(5):426-34
- Schwaeble et al., Targeting of mannan-binding lectin-associated serine protease-2 confers protection from myocardial and gastrointestinal ischemia/reperfusion injury. Proc Natl Acad Sci U S A. 2011;108(18):7523-8
- Farrar et al., Collectin-11 detects stress-induced L-fucose pattern to trigger renal epithelial injury. J Clin Invest. 2016;126(5):1911-1925
- Kocsis et al., Selective inhibition of the lectin pathway of complement with phage display selected peptides against mannose-binding lectin-associated serine protease (MASP)-1 and -2: significant contribution of MASP-1 to lectin pathway activation. J Immunol. 2010;185(7):4169-78
- Héja et al., Monospecific inhibitors show that both mannan-binding lectin-associated serine protease-1 (MASP-1) and are essential for lectin pathway activation and reveal structural plasticity of MASP-2. J Biol Chem. 2012;287(24):20290-300
- Encyclopaedia of Pharmaceutical Technology", 3rd edition, James Swarbrick (Ed.),pp. 3177-3187
- Hong-Kui Cui, Ye Guo, Yao He, Feng-Liang Wang, Hao-Nan Chang, Yu-Jia Wang, Fang-Ming Wu, Chang-Lin Tian, Lei Liu Angew. Chem. Int. Ed. 2013, 52, 9558-9562
- Ye Guo, De-Meng Sun, Feng-Liang Wang, Yao He, Lei Liu, Chang-Lin Tian Angew. Chem. Int. Ed. 2015, 54, 14276-14281
- Yang Xu, Tao Wang, Chao-Jian Guan, Yi-Ming Li, Lei Liu, Jing Shi, Donald Bierer Tetrahedron Letters 2017, 58, 1677-1680
- Tao Wang, Yi-Fu Kong, Yang Xu, Jian Fan, Hua-Jian Xu, Donald Bierer, Jun Wang, Jing Shi, Yi-Ming Li Tetrahedron Letters 2017, 58, 3970-3973
- Tao Wang, Jian Fan, Xiao-Xu Chen, Rui Zhao, Yang Xu, Donald Bierer, Lei Liu, Yi-Ming Li, Jing Shi, Ge-Min Fang Org. Lett. 2018, 20, 6074-6078
- Jan-Patrick Fischer, Ria Schönauer, Sylvia Els-Heindl, Donald Bierer, Johannes Koebberling, Bernd Riedl, Annette G. Beck-Sickinger J Pep Sci. 2019; e3147
- Dong-Liang Huang, Jing-Si Bai, Meng Wu, Xia Wang, Bernd Riedl, Elisabeth Pook, Carsten Alt, Marion Erny, Yi-Ming Li, Donald Bierer, Jing Shi, Ge-Min Fang Chem. Commun., 2019, 55, 2821-2824
- Shuai-Shuai Sun, Junyou Chen, Rui Zhao, Donald Bierer, Jun Wang, Ge-Min Fang, Yi-Ming Li Tetrahedron Letters 2019, 60, 1197-1201
- C. M. B. K. Kourra and N. Cramer Chem. Sci., 2016, 7, 7007-7012
- Qian Qu, Shuai Gao, Fangming Wu, Meng-Ge Zhang, Ying Li, Long-Hua Zhang, Donald Bierer, Chang-Lin Tian, Ji-Shen Zheng, Lei Liu Angew. Chem. Int. Ed. 2020, 59, 6037-6045
- Rui Zhao, Pan Shi, Junyou Chen, Shuaishuai Sun, Jingnan Chen, Jibin Cui, Fangming Wu, Gemin Fang, Changlin Tian, Jing Shi, Donald Bierer, Lei Liu, Yi-Ming Li Chem. Sci., 2020, 11, 7927-7932; 10.1039/d0sc02374d
- Junyou Chen, Shuaishuai Sun, Rui Zhao, Chen-Peng Xi, Wenjie Qiu, Ning Wang, Ya Wang, Donald Bierer, Jing Shi, Yi-Ming Li ChemistrySelect 2020, 5, 1359-1363; 10.1002/slct.201904042
- Yun-Kun Qi, Qian Qu, Donald Bierer, Lei Liu Chem Asian J. 2020, 15, 2793-2802; 10.1002/asia.202000609.
- Caliceti P.,Veronese F.M., Adv. Drug Deliv. Rev.2003, 55, 1261-1277
- T. Peleg-Shulman et al., J. Med.Chem., 2004, 47, 4897-4904
- Simon et al., Effects of intravenous sulfide during porcine aortic occlusion-induced kidney ischemia/reperfusion injury. Shock. 2011;35:156-163;
- Matejkova et al., Carbamylated erythropoietin-FC fusion protein and recombinant human erythropoietin during porcine kidney ischemia/reperfusion injury. Intensive Care Med. 2011;39:497-510
- Darmon et al., Diagnostic accuracy of Doppler renal resistive index for reversibility of acute kidney injury in critically ill patients. Intensive Care Med. 2011;37(1):68-76
- Nomenclature of α-Amino Acids (Recommendations, 1974), Biochemistry, 14(2), (1975*)*
- http://www.researchdisclosure.com/searching-disclosures, Research Disclosure Database Number 605005, 2014, 01 Aug 2014
- Helv. Chim. Acta 2003, 86, 4061-4072
- Ellinghaus et al., 2005

## Claims

1. A bicyclic compound, which may be isolated and/or purified, comprising, essentially consisting of, or consisting of the formula (I): 
X1—X2—X3—Ile4—Cys5—Ser6—Arg7—Ser8—X9—Pro10—X11—Ile12—X13—Ile14—X15—X16—X17
(I)
 or a derivative, analogue, pharmaceutically acceptable salt, solvate or solvate of the salt, wherein
X¹ represents a natural amino acid, which can be in D- or L-stereoconfiguration, selected from the group consisting of alanine, glycine, lysine, cysteine and glutamic acid, or a moiety selected from the group consisting of 6-aminohexanoic acid (Ahx), L-2,3-Diaminopropionic acid (Dap), L-2,4-Diaminobutyric acid (Dab), 3-azido-L-Alanine, L-2-aminobutyric acid (Abu), gamma-aminobutyric acid (gamma-Abu), 2-aminoisobutyric acid (Aib), L-Ornithine (Orn), 1,13-diamino-4,7,10-trioxatridecan-succinamic acid (TTDS), 9-Amino-4,7-dioxanonanoic acid [PEG1(10 atoms)], 12-Amino-4,7,10-trioxadodecanoic acid [PEG2(13 atoms)], 15-Amino-4,7,10,13-tetraoxapentadecanoic acid [PEG3(16 atoms)] and adipic acid, or X¹ may be absent,
X² represents a natural amino acid, which can be in D- or L-stereoconfiguration, selected from the group consisting of glycine and serine, or a moiety selected from the group consisting of N-methyl-glycine, L-2,3-Diaminopropionic acid (Dap), L-2,4-Diaminobutyric acid (Dab), L-2-Aminobutyric acid (Abu), gamma-aminobutyric acid (gamma-Abu), tranexamic acid (TXA), 3-(aminomethyl)benzoic acid and 4-(aminomethyl)benzoic acid, or X² may be absent,
X³ represents a natural amino acid, which can be in D- or L-stereoconfiguration, selected from the group consisting of glycine and alanine, or X³ may be absent,
Ile⁴ represents L-Isoleucine,
Cys⁵ represents L-Cysteine,
Ser⁶ represents L-Serine,
Arg⁷ represents L-Arginine,
Ser⁸ represents L-Serine,
X⁹ represents L-Lysine or L-tert-Butylalanine [(tBu)A)],
Pro¹⁰ represents L-Proline,
X¹¹ represents L-Proline or 2,3,3a,4,5,6,7,7a-octahydroindole-2-carboxylic acid (Oic),
Ile¹² represents L-Isoleucine,
X¹³ represents L-Cysteine, L-N-Methylcysteine [(N-Me)C] or L-Penicillamine (Pen),
Ile¹⁴ represents L-Isoleucine,
X¹⁵ represents L-Proline or 2-aminoisobutyric acid (Aib), or X¹⁵ may be absent,
X¹⁶ represents a natural amino acid, which can be in D- or L-stereoconfiguration, selected from the group consisting of aspartic acid and glutamic acid, or X¹⁶ may be absent,
X¹⁷ represents a natural amino acid, which can be in D- or L-stereoconfiguration, selected from the group consisting of serine, cysteine, proline and lysine, or a moiety selected from the group consisting of L-2,3-Diaminopropionic acid (Dap), L-2,4-Diaminobutyric acid (Dab) and L-Propargylglycine, or X¹⁷ may be absent,
wherein Cys⁵ and X¹³ are linked by a disulfide bond between the sulfur atoms of the two groups forming a first ring,
wherein a second ring is formed between X¹ (in case X¹ is not absent), X² (in case X¹ is absent and X² is not absent), X³ (in case X¹ and X² are absent and X³ is not absent) or Ile⁴ (in case X¹, X² and X³ are all absent) at the N-terminus and Ile¹⁴ (in case X¹⁵, X¹⁶ and X¹⁷ are all absent), X¹⁵ (in case X¹⁶ and X¹⁷ are absent and X¹⁵ is not absent), X¹⁶ (in case X¹⁷ is absent and X¹⁶ is not absent) or X¹⁷ (in case X¹⁷ is not absent) at the C-terminus,
and wherein such second ring may be formed either via an α-peptide bond in the backbone or via one or two of the amino acid side chains, where in the case the second ring is formed not using the C-terminal carboxylic acid then the C-terminal carboxy group may be transformed in to an amide group wherein in the case that X¹ represents 3-azido-L-Alanine and X¹⁷ represents L-Propargylglycine the ring formation results in an 1,2,3-triazole ring, which is attached in 1-position to the alanine and in 4-position to the glycine.

2. A bicyclic compound, which may be isolated and/or purified, comprising, essentially consisting of, or consisting of the formula (I) or a derivative, analogue, pharmaceutically acceptable salt, solvate or solvate of the salt thereof, according to Claim 1, wherein
X¹ represents a natural amino acid selected from the group consisting of D-alanine, L-Alanine, Glycine, D-lysine, L-Lysine, L-Cysteine and L-Glutamic acid, or a moiety selected from the group consisting of 6-aminohexanoic acid (Ahx), L-2,3-Diaminopropionic acid (Dap), L-2,4-Diaminobutyric acid (Dab), gamma-aminobutyric acid (gamma-Abu), L-Ornithine (Orn), 1,13-diamino-4,7,10-trioxatridecan-succinamic acid (TTDS), 9-Amino-4,7-dioxanonanoic acid [PEG1(10 atoms)], 15-Amino-4,7,10,13-tetraoxapentadecanoic acid [PEG3(16 atoms)] and adipic acid, or X¹ may be absent,
X² represents a natural amino acid selected from the group consisting of Glycine and L-Serine, or a moiety selected from the group consisting of N-methyl-glycine, L-2,3-Diaminopropionic acid (Dap), L-2,4-Diaminobutyric acid (Dab), L-2-Aminobutyric acid (Abu), tranexamic acid (TXA), and 4-(aminomethyl)benzoic acid, or X² may be absent,
X³ represents a natural amino acidselected from the group consisting of Glycine, L-Alanine and D-alanine, or X³ may be absent,
Ile⁴ represents L-Isoleucine,
Cys⁵ represents L-Cysteine,
Ser⁶ represents L-Serine,
Arg⁷ represents L-Arginine,
Ser⁸ represents L-Serine,
X⁹ represents L-Lysine or L-tert-Butylalanine [(tBu)A)],
Pro¹⁰ represents L-Proline,
X¹¹ represents L-proline or 2,3,3a,4,5,6,7,7a-octahydroindole-2-carboxylic acid (Oic),
Ile¹² represents L-Isoleucine,
X¹³ represents L-Cysteine, L-N-Methylcysteine [(N-Me)C] or L-Penicillamine (Pen),
Ile¹⁴ represents L-Isoleucine,
X¹⁵ represents L-Proline, or X¹⁵ may be absent,
X¹⁶ represents a natural amino acid selected from the group consisting of L-Aspartic acide, D-aspartic acid and L-Glutamic acid, or X¹⁶ may be absent,
X¹⁷ represents a natural amino acid selected from the group consisting of L-Serine, L-Cysteine, L-Proline and L-Lysine, or a moiety selected from the group consisting of L-2,3-Diaminopropionic acid (Dap), or X¹⁷ may be absent,
wherein Cys⁵ and X¹³ are linked by a disulfide bond between the sulfur atoms of the two groups forming a first ring,
wherein a second ring is formed between X¹ (in case X¹ is not absent), X² (in case X¹ is absent and X² is not absent), X³ (in case X¹ and X² are absent and X³ is not absent) or Ile⁴ (in case X¹, X² and X³ are all absent) at the N-terminus and Ile¹⁴ (in case X¹⁵, X¹⁶ and X¹⁷ are all absent), X¹⁵ (in case X¹⁶ and X¹⁷ are absent and X¹⁵ is not absent), X¹⁶ (in case X¹⁷ is absent and X¹⁶ is not absent) or X¹⁷ (in case X¹⁷ is not absent) at the C-terminus,
and wherein such second ring may be formed either via an α-peptide bond in the backbone or via one or two of the amino acid side chains, where in the case the second ring is formed not using the C-terminal carboxylic acid then the C-terminal carboxy group may be transformed in to an amide group.

3. A bicyclic compound, which may be isolated and/or purified, comprising, essentially consisting of, or consisting of the formula (I) or a derivative, analogue, pharmaceutically acceptable salt, solvate or solvate of the salt thereof, according to Claim 1 or 2, wherein
X¹ represents a natural amino acid selected from the group consisting of L-Alanine, Glycine, L-Lysine and L-Glutamic acid, or a moiety selected from the group consisting of 6-aminohexanoic acid (Ahx), L-2,3-Diaminopropionic acid (Dap), L-2,4-Diaminobutyric acid (Dab), gamma-aminobutyric acid (gamma-Abu), L-Ornithine (Orn), 1,13-diamino-4,7,10-trioxatridecan-succinamic acid (TTDS), 9-Amino-4,7-dioxanonanoic acid [PEG1(10 atoms)], 15-Amino-4,7,10,13-tetraoxapentadecanoic acid [PEG3(16 atoms)] and adipic acid,
X² represents a natural amino acid selected from the group consisting of Glycine and L-Serine, or a moiety selected from the group consisting of N-methyl-glycine, L-2,3-Diaminopropionic acid (Dap), L-2-Aminobutyric acid (Abu), tranexamic acid (TXA), and 4-(aminomethyl)benzoic acid, or X² may be absent,
X³ represents a natural amino acidselected from the group consisting of Glycine and L-Alanine, or X³ may be absent,
Ile⁴ represents L-Isoleucine,
Cys⁵ represents L-Cysteine,
Ser⁶ represents L-Serine,
Arg⁷ represents L-Arginine,
Ser⁸ represents L-Serine,
X⁹ represents L-Lysine or L-tert-Butylalanine [(tBu)A)],
Pro¹⁰ represents L-Proline,
X¹¹ represents L-proline or 2,3,3a,4,5,6,7,7a-octahydroindole-2-carboxylic acid (Oic),
Ile¹² represents L-Isoleucine,
X¹³ represents L-N-Methylcysteine [(N-Me)C] or L-Penicillamine (Pen),
Ile¹⁴ represents L-Isoleucine,
X¹⁵ represents L-Proline, or X¹⁵ may be absent,
X¹⁶ represents a natural amino acid selected from the group consisting of L-Aspartic acide and L-Glutamic acid, or X¹⁶ may be absent,
X¹⁷ represents a natural amino acid selected from the group consisting of L-Proline and L-Lysine, or a moiety selected from the group consisting of L-2,3-Diaminopropionic acid (Dap), or X¹⁷ may be absent,
wherein Cys⁵ and X¹³ are linked by a disulfide bond between the sulfur atoms of the two groups forming a first ring,
wherein a second ring is formed between X¹ at the N-terminus and Ile¹⁴ (in case X¹⁵, X¹⁶ and X¹⁷ are all absent), X¹⁵ (in case X¹⁶ and X¹⁷ are absent and X¹⁵ is not absent), X¹⁶ (in case X¹⁷ is absent and X¹⁶ is not absent) or X¹⁷ (in case X¹⁷ is not absent) at the C-terminus,
and wherein such second ring may be formed either via an α-peptide bond in the backbone or via one or two of the amino acid side chains, where in the case the second ring is formed not using the C-terminal carboxylic acid then the C-terminal carboxy group may be transformed in to an amide group.

4. A bicyclic compound, which may be isolated and/or purified, comprising, essentially consisting of, or consisting of the formula (I) or a derivative, analogue, pharmaceutically acceptable salt, solvate or solvate of the salt thereof, according to any of Claims 1, 2 or 3, wherein
X¹ represents a natural amino acid selected from the group consisting of L-Alanine and Glycine, L-Lysine, or a moiety selected from the group consisting of 6-aminohexanoic acid (Ahx), L-2,3-Diaminopropionic acid (Dap), gamma-aminobutyric acid (gamma-Abu), L-Ornithine (Orn),
X² represents the natural amino acid Glycine, or a moiety selected from the group consisting L-2,3-Diaminopropionic acid (Dap), L-2-Aminobutyric acid (Abu), tranexamic acid (TXA), and 4-(aminomethyl)benzoic acid, or X² may be absent,
X³ represents a natural amino acidselected from the group consisting of Glycine and L-Alanine, or X³ may be absent,
Ile⁴ represents L-Isoleucine,
Cys⁵ represents L-Cysteine,
Ser⁶ represents L-Serine,
Arg⁷ represents L-Arginine,
Ser⁸ represents L-Serine,
X⁹ represents L-tert-Butylalanine [(tBu)A)],
Pr0¹⁰ represents L-Proline,
X¹¹ represents 2,3,3a,4,5,6,7,7a-octahydroindole-2-carboxylic acid (Oic),
Ile¹² represents L-Isoleucine,
X¹³ represents L-Penicillamine (Pen),
Ile¹⁴ represents L-Isoleucine,
X¹⁵ represents L-Proline, or X¹⁵ may be absent,
X¹⁶ represents a natural amino acid selected from the group consisting of L-Aspartic acide and L-Glutamic acid, or X¹⁶ may be absent,
X¹⁷ is absent,
wherein Cys⁵ and X¹³ are linked by a disulfide bond between the sulfur atoms of the two groups forming a first ring,
wherein a second ring is formed between X¹ at the N-terminus and Ile¹⁴ (in case X¹⁵ and X¹⁶ are absent), X¹⁵ (in case X¹⁶ is absent and X¹⁵ is not absent) or X¹⁶ (X¹⁶ is not absent) at the C-terminus,
and wherein such second ring may be formed either via an α-peptide bond in the backbone or via one or two of the amino acid side chains, where in the case the second ring is formed not using the C-terminal carboxylic acid then the C-terminal carboxy group may be transformed in to an amide group.

5. A bicyclic compound, which may be isolated and/or purified, comprising, essentially consisting of, or consisting of the formula (I) or a derivative, analogue, pharmaceutically acceptable salt, solvate or solvate of the salt thereof, according to any of Claims 1, 2, 3 or 4, wherein
X¹ is present.

6. A bicyclic compound according to any of Claims 1 to 5 or a pharmaceutically acceptable salt, solvate or solvate of the salt thereof, which acts as a MASP-1 and/or MASP-2 inhibitor and/or which inhibits C3 deposition.

7. A process for preparing a bicyclic compound according to any of Claims 1 to 6 or a pharmaceutically acceptable salt, solvate or solvate of the salt thereof, using solid phase peptide synthesis.

8. A bicyclic compound according to any of Claims 1 to 6 or a pharmaceutically acceptable salt, solvate or solvate of the salt thereof, for the use in the prophylaxis and treatment of diseases.

9. A bicyclic compound according to any of Claims 1 to 6 or a pharmaceutically acceptable salt, solvate or solvate of the salt thereof, for the use in the prophylaxis and treatment of cardiovascular and cardiopulmonary disorders, shock, inflammatory disorders, cardiovascular, pulmonary, cerebral and renal sequels of sepsis, ischemia and/or reperfusion-related damage, acute kidney injury, transplant protection and delayed graft function, diseases of the blood and blood-forming organs and the immune system, sequels of diabetes mellitus, inflammatory diseases of the nervous system, diseases of the eye, diseases of the skin, diseases of the respiratory, digestive or genitourinary system and sequels of burns and injuries.

10. A pharmaceutical composition comprising at least one bicyclic compound according to any of Claims 1 to 6 or a pharmaceutically acceptable salt, solvate or solvate of the salt thereof, in combination with one or more inert, nontoxic, pharmaceutically suitable excipients.

11. A pharmaceutical composition comprising at least one bicyclic compound according to any of Claims 1 to 6 or a pharmaceutically acceptable salt, solvate or solvate of the salt thereof, in combination with one or more further active ingredients selected from the group consisting of inhibitors of phosphordiesterases, stimulators or activators of guanylate cyclase, IP receptor agonists, mineralocorticoid-receptor antagonist, diuretic, PPAR-gamma agonist, PPAR-delta agonist, corticosteroids, active ingredients which reduce damage to organs under oxidative stress, compounds which inhibit induction of cell death and apoptosis pathway, compounds which inhibit inflammatory response and T cell proliferation, antithrombotic agents, platelet aggregation inhibitor, thrombin inhibitor, GPIIb/IIIa antagonist, factor Xa inhibitor, heparin or a low molecular weight (LMW) heparin derivative and inhibitors of coagulation factor XI.

12. A pharmaceutical composition according to Claims 10 or 11 for the prophylaxis and/or treatment of cardiovascular and cardiopulmonary disorders, shock, inflammatory disorders, cardiovascular, pulmonary, cerebral and renal sequels of sepsis, ischemia and/or reperfusion-related damage, acute kidney injury, transplant protection and delayed graft function, diseases of the blood and blood-forming organs and the immune system, sequels of diabetes mellitus, inflammatory diseases of the nervous system, diseases of the eye, diseases of the skin, diseases of the respiratory, digestive or genitourinary system and sequels of burns and injuries.

13. Method for treatment and/or prevention of cardiovascular and cardiopulmonary disorders, shock, inflammatory disorders, cardiovascular, pulmonary, cerebral and renal sequels of sepsis, ischemia and/or reperfusion-related damage, acute kidney injury, transplant protection and delayed graft function, diseases of the blood and blood-forming organs and the immune system, sequels of diabetes mellitus, inflammatory diseases of the nervous system, diseases of the eye, diseases of the skin, diseases of the respiratory, digestive or genitourinary system and sequels of burns and injuries in humans and animals by administration of an effective amount of at least one bicyclic compound as defined in any of Claims 1 to 6, or of a pharmaceutical composition according to Claims 10 or 11.
